# EUROPEAN PATENT SPECIFICATION

(11) **EP 3 938 369 B1**
(45) Date of publication and mention of the grant of the patent: **16.09.2026**
(21) Application number: 20769748.3
(22) Date of filing: 10.03.2020
(51) Int. Cl.: A61K 31/519, A61K 31/5025, A61P 25/00, A61P 29/00, A61P 37/00, C07D 498/16, C07D 498/22

(54) **TYK2 INHIBITORS AND USES THEREOF**
TYK2-INHIBITOREN UND VERWENDUNGEN DAVON
INHIBITEURS DE TYK2 ET LEURS UTILISATIONS

(30) Priority: 11.03.2019 US 201962816698 P; 17.04.2019 US 201962835376 P; 23.07.2019 US 201962877741 P; 05.11.2019 US 201962931119 P
(43) Date of publication of application: 19.01.2022
(73) Proprietor: Alumis Inc., South San Francisco, CA 94080 (US)
(72) Inventor: JIN, Bohan, San Diego, California 92121 (US); DONG, Qing, San Diego, California 92121 (US); HUNG, Gene, San Diego, California 92121 (US)
(74) Representative: Harris, Jennifer Lucy
(86) International application number: PCT/US2020/021850
(87) International publication number: WO 2020/185755

(56) References cited:
- WO-A1-2013/001310
- WO-A1-2017/004342
- WO-A1-2018/170381
- WO-A1-2019/023417
- WO-A1-2020/198379
- WO-A2-2018/081417

## Description

### FIELD OF THE INVENTION

Described herein are compounds, methods of making such compounds, pharmaceutical compositions and medicaments comprising such compounds, and such compounds for use in methods of inhibiting nonreceptor tyrosine-protein kinase 2 ("TYK2"), also known as Tyrosine kinase 2.

### BACKGROUND OF THE INVENTION

TYK2 is a non-receptor tyrosine kinase member of the Janus kinase (JAKs) family of protein kinases. The mammalian JAK family consists of four members, TYK2, JAK1, JAK2, and JAK3. JAK proteins, including TYK2, are integral to cytokine signaling. TYK2 associates with the cytoplasmic domain of type I and type II cytokine receptors, as well as interferon types I and III receptors, and is activated by those receptors upon cytokine binding. Cytokines implicated in TYK2 activation include interferons (e.g. IFN-α, IFN-β, IFN-κ, IFN-δ, IFN-ε, IFN-τ, IFN-ω, and IFN-ζ (also known as limitin), and interleukins (e.g. IL-4, IL-6, IL-10, IL-11, IL-12, IL-13, L-22, IL-23, IL-27, IL-31, oncostatin M, ciliary neurotrophic factor, cardiotrophin 1, cardiotrophin-like cytokine, and LIF). The activated TYK2 then goes on to phosphorylate further signaling proteins such as members of the STAT family, including STAT1, STAT2, STAT4, and STAT6.

TYK2 activation by IL-23, has been linked to inflammatory bowel disease (IBD), Crohn's disease, and ulcerative colitis. A genome-wide association study of 2,622 individuals with psoriasis identified associations between disease susceptibility and TYK2. Knockout or tyrphostin inhibition of TYK2 significantly reduces both IL-23 and IL-22-induced dermatitis.

TYK2 also plays a role in respiratory diseases such as asthma, chronic obstructive pulmonary disease (COPD), lung cancer, and cystic fibrosis. Goblet cell hyperplasia (GCH) and mucous hypersecretion is mediated by IL-13-induced activation of TYK2, which in turn activates STAT6.

Decreased TYK2 activity leads to protection of joints from collagen antibody-induced arthritis, a model of human rheumatoid arthritis. Mechanistically, decreased Tyk2 activity reduced the production of Th1/Th17-related cytokines and matrix metalloproteases, and other key markers of inflammation.

TYK2 knockout mice showed complete resistance in experimental autoimmune encephalomyelitis (EAE, an animal model of multiple sclerosis (MS)), with no infiltration of CD4 T cells in the spinal cord, as compared to controls, suggesting that TYK2 is essential to pathogenic CD4-mediated disease development in MS. This corroborates earlier studies linking increased TYK2 expression with MS susceptibility. Loss of function mutation in TYK2, leads to decreased demyelination and increased remyelination of neurons, further suggesting a role for TYK2 inhibitors in the treatment of MS and other CNS demyelination disorders.

TYK2 is the sole signaling messenger common to both IL-12 and IL-23. TYK2 knockout reduced methylated BSA injection-induced footpad thickness, imiquimod-induced psoriasis-like skin inflammation, and dextran sulfate sodium or 2,4,6-trinitrobenzene sulfonic acid-induced colitis in mice.

Joint linkage and association studies of various type I IFN signaling genes with systemic lupus erythematosus (SLE, an autoimmune disorder), showed a strong, and significant correlation between loss of function mutations to TYK2 and decreased prevalence of SLE in families with affected members. Genome-wide association studies of individuals with SLE versus an unaffected cohort showed highly significant correlation between the TYK2 locus and SLE.

TYK2 has been shown to play an important role in maintaining tumor surveillance and TYK2 knockout mice showed compromised cytotoxic T cell response, and accelerated tumor development. However, these effects were linked to the efficient suppression of natural killer (NK) and cytotoxic T lymphocytes, suggesting that TYK2 inhibitors would be highly suitable for the treatment of autoimmune disorders or transplant rejection. Although other JAK family members such as JAK3 have similar roles in the immune system, TYK2 has been suggested as a superior target because of its involvement in fewer and more closely related signaling pathways, leading to fewer off-target effects.

Studies in T-cell acute lymphoblastic leukemia (T-ALL) indicate that T-ALL is highly dependent on IL-10 via TYK2 via STAT1-mediated signal transduction to maintain cancer cell survival through upregulation of anti-apoptotic protein BCL2. Knockdown of TYK2, but not other JAK family members, reduced cell growth. Specific activating mutations to TYK2 that promote cancer cell survival include those to the FERM domain (G36D, S47N, and R425H), the JH2 domain (V73 11), and the kinase domain (E957D and R1027H). However, it was also identified that the kinase function of TYK2 is required for increased cancer cell survival, as TYK2 enzymes featuring kinase-dead mutations (M978Y or M978F) in addition to an activating mutation (E957D) resulted in failure to transform.

Thus, selective inhibition of TYK2 has been suggested as a suitable target for patients with IL-10 and/or BCL2-addicted tumors, such as 70% of adult T-cell leukemia cases. TYK2 mediated STAT3 signaling has also been shown to mediate neuronal cell death caused by amyloid-β (Aβ) peptide. Decreased TYK2 phosphorylation of STAT3 following Aβ administration lead to decreased neuronal cell death, and increased phosphorylation of STAT3 has been observed in postmortem brains of Alzheimer's patients.

Inhibition of JAK-STAT signaling pathways is also implicated in hair growth, and the reversal of the hair loss associated with alopecia areata.

Accordingly, compounds that inhibit the activity of TYK2 are beneficial, especially those with selectivity over JAK2. Such compounds should deliver a pharmacological response that favorably treats one or more of the conditions described herein without the side-effects associated with the inhibition of JAK2.

Accordingly there is a need to provide novel inhibitors having more effective or advantageous pharmaceutically relevant properties, like selectivity over other JAK kinases (especially JAK2).

WO 2017/004342 A1 relates to certain chiral diaryl macrocyclic derivatives, pharmaceutical compositions containing them, and methods of using them to treat cancer, pain, neurological diseases, autoimmune diseases, and inflammation.

WO 2020/198379 A1 relates to TYK2 pseudokinase ligands and methods of utilizing TYK2 pseudokinase ligands in the treatment of diseases, disorders or conditions. Also described are pharmaceutical compositions containing such compounds.

### BRIEF SUMMARY OF THE INVENTION

Provided by the presently claimed invention is a compound of Formula (II), or a pharmaceutically acceptable salt, solvate, or stereoisomer thereof: 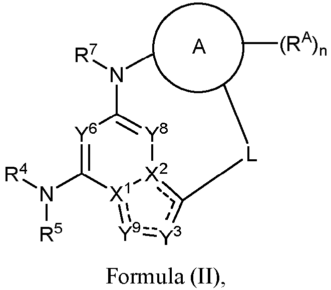 wherein:
L is 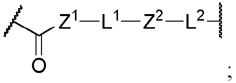 wherein Z¹ and Z² are independently -O-, -S-, or -NR^{Z}; each R^{Z} is independently hydrogen or C₁-C₆alkyl; and L¹ and L² are independently C₁-C₆ alkylene optionally substituted with one or more R^{L};
each R^{L} is independently deuterium, halogen, -CN, -OR^{b}, -SR^{b}, -S(=O)R^{a}, -S(=O)₂R^{a}, -NO₂, -NR^{c}R^{d}, - NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, - OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{L} on the same carbon are taken together to form an oxo, a cycloalkyl, or heterocycloalkyl; or two R^{L} on different carbons are taken together to form a cycloalkyl or heterocycloalkyl;
Ring A is cycloalkyl, heterocycloalkyl, aryl, or heteroaryl;
each R^{A} is independently deuterium, halogen, -CN, -OR^{b}, -SR^{b}, -S(=O)R^{a}, -S(=O)₂R^{a}, -NO₂, -NR^{c}R^{d}, - NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, - OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more R^{A1}; or two R^{A} on the same carbon are taken together to form an oxo;
each R^{A1}; is independently deuterium, halogen, -CN, -OR^{b}, -SR^{b}, -S(=O)R^{a}, -S(=O)₂R^{a}, -NO₂, -NR^{c}R^{d}, - NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, - OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{A1}; on the same carbon are taken together to form an oxo
n is 0-4;
-̅ -̅ -̅ is a single bond or a double bond;
X¹ and X² are -N- or -C=; provided that one of X¹ or X² is -N- and the other is -C=;
Y⁸ is CR⁸ or N;
Y⁶ is CR⁶ or N;
Y³ is CR³ or N;
Y⁹ is CR⁹ or N;
R³, R⁶, R⁸, and R⁹ are independently hydrogen, deuterium, halogen, -CN, -OR^{b}, -SR^{b}, -S(=O)R^{a}, - S(=O)₂R^{a}, -NO₂, -NR^{c}R^{d}, -NHS(=O)₂R^{a}, -S(=O)₂NR^{c}R^{d}, -C(=O)R^{a}, -OC(=O)R^{a}, -C(=O)OR^{b}, - OC(=O)OR^{b}, -C(=O)NR^{c}R^{d}, -OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, or C₂-C₆alkynyl;
R⁴ is hydrogen, C₁-C₆alkyl, C₁-C₆heteroalkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl are optionally substituted with one or more R^{4a};
each R^{4a} is independently deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; or two R^{4a} on the same carbon are taken together to form an oxo;
R⁵ is hydrogen, C₁-C₆alkyl, C₁-C₆haloalkyl, or C₁-C₆deuteroalkyl;
R⁷ is hydrogen, C₁-C₆alkyl, C₁-C₆haloalkyl, or C₁-C₆deuteroalkyl;
each R^{a} is independently C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OMe, - NH₂, -C(=O)Me, -C(=O)OH, -C(=O)OMe, C₁-C₆alkyl, or C₁-C₆haloalkyl;
each R^{b} is independently hydrogen, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OMe, - NH₂, -C(=O)Me, -C(=O)OH, -C(=O)OMe, C₁-C₆alkyl, or C₁-C₆haloalkyl; and
each R^{c} and R^{d} is independently hydrogen, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OMe, -NH₂, -C(=O)Me, -C(=O)OH, -C(=O)OMe, C₁-C₆alkyl, or C₁-C₆haloalkyl;
or R^{c} and R^{d} are taken together with the nitrogen atom to which they are attached to form a heterocycloalkyl optionally substituted with one or more oxo, deuterium, halogen, -CN, -OH, -OMe, -NH₂, -C(=O)Me, -C(=O)OH, -C(=O)OMe, C₁-C₆alkyl, or C₁-C₆haloalkyl.

Also provided by the presently claimed invention is a compound, or a pharmaceutically acceptable salt, stereoisomer, or solvate thereof wherein the compound is: 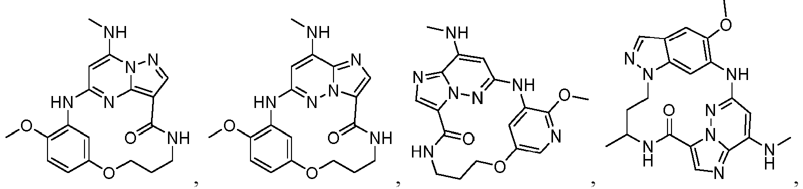 or

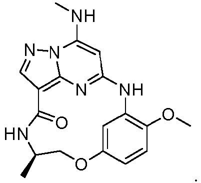

Also disclosed herein, but not presently claimed unless identified above, is a compound of Formula (II), or a pharmaceutically acceptable salt, stereoisomer, or solvate thereof, wherein:
L is a 4-10 atom linker; optionally substituted with one or more R^{L}; and
all other substituents are as described in paragraph [0016] above.

Also disclosed herein is a compound of Formula (IIa), or a pharmaceutically acceptable salt, stereoisomer, or solvate thereof:

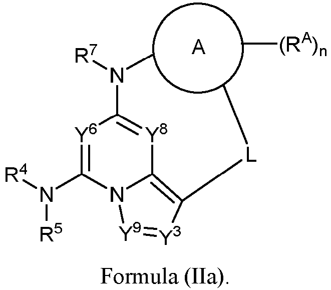

Also disclosed herein is a compound of Formula (IIb), or a pharmaceutically acceptable salt, stereoisomer, or solvate thereof:

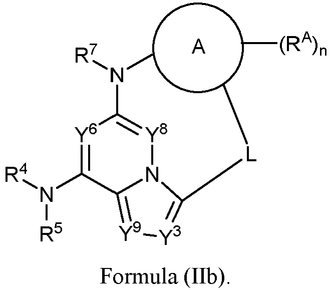

Also disclosed herein is a pharmaceutical composition comprising a therapeutically effective amount of the compound disclosed herein, or a pharmaceutically acceptable salt, stereoisomer, or solvate thereof, and a pharmaceutically acceptable excipient.

Also provided by the presently claimed invention is a compound, or a pharmaceutically acceptable salt, stereoisomer, or solvate thereof, for use in a method of (a) inhibiting a TYK2 enzyme or (b) treating a TYK2-mediated disorder, in accordance with claim 15.

Also disclosed herein is a method of inhibiting a TYK2 enzyme in a patient or biological sample comprising contacting said patient or biological sample with a compound disclosed herein, or a pharmaceutically acceptable salt, stereoisomer, or solvate thereof.

Also disclosed herein is a method of treating a TYK2-mediated disorder comprising administering to a patient in need thereof a compound disclosed herein, or a pharmaceutically acceptable salt, stereoisomer, or solvate thereof. In some embodiments, the TYK2-mediated disorder is an autoimmune disorder, an inflammatory disorder, a proliferative disorder, an endocrine disorder, a neurological disorder, or a disorder associated with transplantation. In some embodiments, the disorder is associated with type I interferon, IL-10, IL-12, or IL-23 signaling.

### DETAILED DESCRIPTION OF THE INVENTION

The extent of the protection is determined by the claims, as interpreted by the remaining specification. The technical information set out below may, in some respects, go beyond the scope of the present claims. Such technical information is provided to place the claim scope in a broader technical context and to illustrate possible related technical developments.

The references to methods of treatment in the subsequent paragraphs of this description are to be interpreted as references to the compounds, pharmaceutical compositions and medicaments of the present invention for use in a method for treatment of the human or animal body by therapy or for diagnosis.

### Definitions

As used herein and in the appended claims, the singular forms "a," "an," and "the" include plural referents unless the context clearly dictates otherwise. Thus, for example, reference to "an agent" includes a plurality of such agents, and reference to "the cell" includes reference to one or more cells (or to a plurality of cells) and equivalents thereof known to those skilled in the art, and so forth. When ranges are used herein for physical properties, such as molecular weight, or chemical properties, such as chemical formulae, all combinations and subcombinations of ranges and specific embodiments therein are intended to be included. The term "about" when referring to a number or a numerical range means that the number or numerical range referred to is an approximation within experimental variability (or within statistical experimental error), and thus the number or numerical range, in some instances, will vary between 1% and 15% of the stated number or numerical range. The term "comprising" (and related terms such as "comprise" or "comprises" or "having" or "including") is not intended to exclude that in other certain embodiments, for example, an embodiment of any composition of matter, composition, method, or process, or the like, described herein, "consist of" or "consist essentially of" the described features.

As used in the specification and appended claims, unless specified to the contrary, the following terms have the meaning indicated below.

"Aliphatic chain" refers to a linear chemical moiety that is composed of only carbons and hydrogens. In some embodiments, the aliphatic chain is saturated. In some embodiments, the aliphatic chain is unsaturated. In some embodiments, the unsaturated aliphatic chain contains one unsaturation. In some embodiments, the unsaturated aliphatic chain contains more than one unsaturation. In some embodiments, the unsaturated aliphatic chain contains two unsaturations. In some embodiments, the unsaturated aliphatic chain contains one double bond. In some embodiments, the unsaturated aliphatic chain contains two double bonds.

"Oxo" refers to =O.

"Alkyl" refers to an optionally substituted straight-chain, or optionally substituted branched-chain saturated hydrocarbon monoradical having from one to about ten carbon atoms, or from one to six carbon atoms. Examples include, but are not limited to, methyl, ethyl, n-propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, n-butyl, isobutyl, sec-butyl, t-butyl, n-pentyl, isopentyl, neopentyl, tert-amyl and hexyl, and longer alkyl groups, such as heptyl, octyl, and the like. Whenever it appears herein, a numerical range such as "C₁-C₆ alkyl" means that the alkyl group consists of 1 carbon atom, 2 carbon atoms, 3 carbon atoms, 4 carbon atoms, 5 carbon atoms or 6 carbon atoms, although the present definition also covers the occurrence of the term "alkyl" where no numerical range is designated. In some embodiments, the alkyl is a C₁-C₁₀ alkyl, a C₁-C₉ alkyl, a C₁-C₈ alkyl, a C₁-C₇ alkyl, a C₁-C₆ alkyl, a C₁-C₅ alkyl, a C₁-C₄ alkyl, a C₁-C₃ alkyl, a C₁-C₂ alkyl, or a C₁ alkyl. Unless stated otherwise specifically in the specification, an alkyl group is optionally substituted, for example, with oxo, halogen, amino, nitrile, nitro, hydroxyl, haloalkyl, alkoxy, aryl, cycloalkyl, heterocycloalkyl, heteroaryl, and the like. In some embodiments, the alkyl is optionally substituted with oxo, halogen, -CN, -CF₃, -OH, -OMe, -NH₂, or -NO₂. In some embodiments, the alkyl is optionally substituted with oxo, halogen, -CN, -CF₃, -OH, or -OMe. In some embodiments, the alkyl is optionally substituted with halogen.

"Alkenyl" refers to an optionally substituted straight-chain, or optionally substituted branched-chain hydrocarbon monoradical having one or more carbon-carbon double-bonds and having from two to about ten carbon atoms, more preferably two to about six carbon atoms. The group may be in either the *cis* or *trans* conformation about the double bond(s), and should be understood to include both isomers. Examples include, but are not limited to, ethenyl (-CH=CH₂), 1-propenyl (-CH₂CH=CH₂), isopropenyl [-C(CH₃)=CH₂], butenyl, 1,3-butadienyl and the like. Whenever it appears herein, a numerical range such as "C₂-C₆ alkenyl" means that the alkenyl group may consist of 2 carbon atoms, 3 carbon atoms, 4 carbon atoms, 5 carbon atoms or 6 carbon atoms, although the present definition also covers the occurrence of the term "alkenyl" where no numerical range is designated. In some embodiments, the alkenyl is a C₂-C₁₀ alkenyl, a C₂-C₉ alkenyl, a C₂-C₈ alkenyl, a C₂-C₇ alkenyl, a C₂-C₆ alkenyl, a C₂-C₅ alkenyl, a C₂-C₄ alkenyl, a C₂-C₃ alkenyl, or a C₂ alkenyl. Unless stated otherwise specifically in the specification, an alkenyl group is optionally substituted, for example, with oxo, halogen, amino, nitrile, nitro, hydroxyl, haloalkyl, alkoxy, aryl, cycloalkyl, heterocycloalkyl, heteroaryl, and the like. In some embodiments, an alkenyl is optionally substituted with oxo, halogen, -CN, -CF₃, -OH, -OMe, -NH₂, or -NO₂. In some embodiments, an alkenyl is optionally substituted with oxo, halogen, -CN, -CF₃, -OH, or -OMe. In some embodiments, the alkenyl is optionally substituted with halogen.

"Alkynyl" refers to an optionally substituted straight-chain or optionally substituted branched-chain hydrocarbon monoradical having one or more carbon-carbon triple-bonds and having from two to about ten carbon atoms, more preferably from two to about six carbon atoms. Examples include, but are not limited to, ethynyl, 2-propynyl, 2-butynyl, 1,3-butadiynyl and the like. Whenever it appears herein, a numerical range such as "C₂-C₆ alkynyl" means that the alkynyl group may consist of 2 carbon atoms, 3 carbon atoms, 4 carbon atoms, 5 carbon atoms or 6 carbon atoms, although the present definition also covers the occurrence of the term "alkynyl" where no numerical range is designated. In some embodiments, the alkynyl is a C₂-C₁₀ alkynyl, a C₂-C₉ alkynyl, a C₂-C₈ alkynyl, a C₂-C₇ alkynyl, a C₂-C₆ alkynyl, a C₂-C₅ alkynyl, a C₂-C₄ alkynyl, a C₂-C₃ alkynyl, or a C₂ alkynyl. Unless stated otherwise specifically in the specification, an alkynyl group is optionally substituted, for example, with oxo, halogen, amino, nitrile, nitro, hydroxyl, haloalkyl, alkoxy, aryl, cycloalkyl, heterocycloalkyl, heteroaryl, and the like. In some embodiments, an alkynyl is optionally substituted with oxo, halogen, -CN, - CF₃, -OH, -OMe, -NH₂, or -NO₂. In some embodiments, an alkynyl is optionally substituted with oxo, halogen, -CN, -CF₃, -OH, or -OMe. In some embodiments, the alkynyl is optionally substituted with halogen.

"Alkylene" refers to a straight or branched divalent hydrocarbon chain. Unless stated otherwise specifically in the specification, an alkylene group may be optionally substituted, for example, with oxo, halogen, amino, nitrile, nitro, hydroxyl, haloalkyl, alkoxy, aryl, cycloalkyl, heterocycloalkyl, heteroaryl, and the like. In some embodiments, an alkylene is optionally substituted with oxo, halogen, -CN, - CF₃, -OH, -OMe, -NH₂, or -NO₂. In some embodiments, an alkylene is optionally substituted with oxo, halogen, -CN, -CF₃, -OH, or -OMe. In some embodiments, the alkylene is optionally substituted with halogen.

"Alkoxy" refers to a radical of the formula -ORₐ where Rₐ is an alkyl radical as defined. Unless stated otherwise specifically in the specification, an alkoxy group may be optionally substituted, for example, with oxo, halogen, amino, nitrile, nitro, hydroxyl, haloalkyl, alkoxy, aryl, cycloalkyl, heterocycloalkyl, heteroaryl, and the like. In some embodiments, an alkoxy is optionally substituted with oxo, halogen, -CN, -CF₃, -OH, -OMe, -NH₂, or -NO₂. In some embodiments, an alkoxy is optionally substituted with oxo, halogen, -CN, -CF₃, -OH, or -OMe. In some embodiments, the alkoxy is optionally substituted with halogen.

"Aminoalkyl" refers to an alkyl radical, as defined above, that is substituted by one or more amines. In some embodiments, the alkyl is substituted with one amine. In some embodiments, the alkyl is substituted with one, two, or three amines. Aminoalkyl include, for example, aminomethyl, aminoethyl, aminopropyl, aminobutyl, or aminopentyl. In some embodiments, the aminoalkyl is aminomethyl.

"Aryl" refers to a radical derived from a hydrocarbon ring system comprising hydrogen, 6 to 30 carbon atoms and at least one aromatic ring. The aryl radical may be a monocyclic, bicyclic, tricyclic or tetracyclic ring system, which may include fused (when fused with a cycloalkyl or heterocycloalkyl ring, the aryl is bonded through an aromatic ring atom) or bridged ring systems. In some embodiments, the aryl is a 6- to 10-membered aryl. In some embodiments, the aryl is a 6-membered aryl. Aryl radicals include, but are not limited to, aryl radicals derived from the hydrocarbon ring systems of anthrylene, naphthylene, phenanthrylene, anthracene, azulene, benzene, chrysene, fluoranthene, fluorene, as-indacene, s-indacene, indane, indene, naphthalene, phenalene, phenanthrene, pleiadene, pyrene, and triphenylene. In some embodiments, the aryl is phenyl. Unless stated otherwise specifically in the specification, an aryl may be optionally substituted, for example, with halogen, amino, nitrile, nitro, hydroxyl, alkyl, alkenyl, alkynyl, haloalkyl, alkoxy, aryl, cycloalkyl, heterocycloalkyl, heteroaryl, and the like. In some embodiments, an aryl is optionally substituted with halogen, methyl, ethyl, -CN, - CF₃, -OH, -OMe, -NH₂, or -NO₂. In some embodiments, an aryl is optionally substituted with halogen, methyl, ethyl, -CN, -CF₃, -OH, or -OMe. In some embodiments, the aryl is optionally substituted with halogen.

"Cycloalkyl" refers to a partially or fully saturated, monocyclic or polycyclic carbocyclic ring, which may include fused (when fused with an aryl or a heteroaryl ring, the cycloalkyl is bonded through a non-aromatic ring atom) or bridged ring systems. Representative cycloalkyls include, but are not limited to, cycloalkyls having from three to fifteen carbon atoms (C₃-C₁₅ cycloalkyl), from three to ten carbon atoms (C₃-C₁₀ cycloalkyl), from three to eight carbon atoms (C₃-C₈ cycloalkyl), from three to six carbon atoms (C₃-C₆ cycloalkyl), from three to five carbon atoms (C₃-C₅ cycloalkyl), or three to four carbon atoms (C₃-C₄ cycloalkyl). In some embodiments, the cycloalkyl is a 3- to 6-membered cycloalkyl. In some embodiments, the cycloalkyl is a 5- to 6-membered cycloalkyl. Monocyclic cycloalkyls include, for example, cyclopropyl, cyclobutyl, cyclopentyl, cyclohexyl, cycloheptyl, and cyclooctyl. Polycyclic cycloalkyls or carbocycles include, for example, adamantyl, norbornyl, decalinyl, bicyclo[3.3.0]octane, bicyclo[4.3.0]nonane, cis-decalin, trans-decalin, bicyclo[2.1.1]hexane, bicyclo[2.2.1]heptane, bicyclo[2.2.2]octane, bicyclo[3.2.2]nonane, and bicyclo[3.3.2]decane, and 7,7-dimethyl-bicyclo[2.2.1]heptanyl. Partially saturated cycloalkyls include, for example cyclopentenyl, cyclohexenyl, cycloheptenyl, and cyclooctenyl. Unless stated otherwise specifically in the specification, a cycloalkyl is optionally substituted, for example, with oxo, halogen, amino, nitrile, nitro, hydroxyl, alkyl, alkenyl, alkynyl, haloalkyl, alkoxy, aryl, cycloalkyl, heterocycloalkyl, heteroaryl, and the like. In some embodiments, a cycloalkyl is optionally substituted with oxo, halogen, methyl, ethyl, -CN, - CF₃, -OH, -OMe, -NH₂, or -NO₂. In some embodiments, a cycloalkyl is optionally substituted with oxo, halogen, methyl, ethyl, -CN, -CF₃, -OH, or -OMe. In some embodiments, the cycloalkyl is optionally substituted with halogen.

"Deuteroalkyl" refers to an alkyl radical, as defined above, that is substituted by one or more deuterium atoms. In some embodiments, the alkyl is substituted with one deuterium atom. In some embodiments, the alkyl is substituted with one, two, or three deuterium atoms. In some embodiments, the alkyl is substituted with one, two, three, four, five, or six deuterium atoms. Deuteroalkyl includes, for example, CD₃, CH₂D, CHD₂, CH₂CD₃, CD₂CD₃, CHDCD₃, CH₂CH₂D, or CH₂CHD₂. In some embodiments, the deuteroalkyl is CD₃.

"Haloalkyl" refers to an alkyl radical, as defined above, that is substituted by one or more halogen atoms. In some embodiments, the alkyl is substituted with one, two, or three halogen atoms. In some embodiments, the alkyl is substituted with one, two, three, four, five, or six halogen halogens. Haloalkyl includes, for example, trifluoromethyl, difluoromethyl, fluoromethyl, trichloromethyl, 2,2,2-trifluoroethyl, 1,2-difluoroethyl, 3-bromo-2-fluoropropyl, 1,2-dibromoethyl, and the like. In some embodiments, the haloalkyl is trifluoromethyl.

"Halo" or "halogen" refers to bromo, chloro, fluoro or iodo. In some embodiments, halogen is fluoro or chloro. In some embodiments, halogen is fluoro.

"Heteroalkyl" refers to an alkyl group in which one or more skeletal atoms of the alkyl are selected from an atom other than carbon, e.g., oxygen, nitrogen (e.g., -NH-, -N(alkyl)-), sulfur, or combinations thereof. A heteroalkyl is attached to the rest of the molecule at a carbon atom of the heteroalkyl. In one aspect, a heteroalkyl is a C₁-C₆ heteroalkyl wherein the heteroalkyl is comprised of 1 to 6 carbon atoms and one or more atoms other than carbon, e.g., oxygen, nitrogen (e.g. -NH-, -N(alkyl)-), sulfur, or combinations thereof wherein the heteroalkyl is attached to the rest of the molecule at a carbon atom of the heteroalkyl. Examples of such heteroalkyl are, for example, -CH₂OCH₃, - CH₂CH₂OCH₃, -CH₂CH₂OCH₂CH₂OCH₃, or -CH(CH₃)OCH₃. Unless stated otherwise specifically in the specification, a heteroalkyl is optionally substituted for example, with oxo, halogen, amino, nitrile, nitro, hydroxyl, alkyl, alkenyl, alkynyl, haloalkyl, alkoxy, aryl, cycloalkyl, heterocycloalkyl, heteroaryl, and the like. In some embodiments, a heteroalkyl is optionally substituted with oxo, halogen, methyl, ethyl, - CN, -CF₃, -OH, -OMe, -NH₂, or -NO₂. In some embodiments, a heteroalkyl is optionally substituted with oxo, halogen, methyl, ethyl, -CN, -CF₃, -OH, or -OMe. In some embodiments, the heteroalkyl is optionally substituted with halogen.

"Hydroxyalkyl" refers to an alkyl radical, as defined above, that is substituted by one or more hydroxyls. In some embodiments, the alkyl is substituted with one hydroxyl. In some embodiments, the alkyl is substituted with one, two, or three hydroxyls. Hydroxyalkyl include, for example, hydroxymethyl, hydroxyethyl, hydroxypropyl, hydroxybutyl, or hydroxypentyl. In some embodiments, the hydroxyalkyl is hydroxymethyl.

"Heterocycloalkyl" refers to a 3- to 24-membered partially or fully saturated ring radical comprising 2 to 23 carbon atoms and from one to 8 heteroatoms selected from the group consisting of nitrogen, oxygen, phosphorous and sulfur. In some embodiments, the heterocycloalkyl comprises 1 or 2 heteroatoms selected from nitrogen and oxygen. Unless stated otherwise specifically in the specification, the heterocycloalkyl radical may be a monocyclic, bicyclic, tricyclic or tetracyclic ring system, which may include fused (when fused with an aryl or a heteroaryl ring, the heterocycloalkyl is bonded through a non-aromatic ring atom) or bridged ring systems; and the nitrogen, carbon or sulfur atoms in the heterocycloalkyl radical may be optionally oxidized; the nitrogen atom may be optionally quaternized. Representative heterocycloalkyls include, but are not limited to, heterocycloalkyls having from two to fifteen carbon atoms (C₂-C₁₅ heterocycloalkyl), from two to ten carbon atoms (C₂-C₁₀ heterocycloalkyl), from two to eight carbon atoms (C₂-C₈ heterocycloalkyl), from two to six carbon atoms (C₂-C₆ heterocycloalkyl), from two to five carbon atoms (C₂-C₅ heterocycloalkyl), or two to four carbon atoms (C₂-C₄ heterocycloalkyl). In some embodiments, the heterocycloalkyl is a 3- to 6-membered heterocycloalkyl. In some embodiments, the cycloalkyl is a 5- to 6-membered heterocycloalkyl. Examples of such heterocycloalkyl radicals include, but are not limited to, aziridinyl, azetidinyl, dioxolanyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, 1,1-dioxo-thiomorpholinyl, 1,3-dihydroisobenzofuran-1-yl, 3-oxo-1,3-dihydroisobenzofuran-1-yl, methyl-2-oxo-1,3-dioxol-4-yl, and 2-oxo-1,3-dioxol-4-yl. The term heterocycloalkyl also includes all ring forms of the carbohydrates, including but not limited to, the monosaccharides, the disaccharides and the oligosaccharides. It is understood that when referring to the number of carbon atoms in a heterocycloalkyl, the number of carbon atoms in the heterocycloalkyl is not the same as the total number of atoms (including the heteroatoms) that make up the heterocycloalkyl (i.e. skeletal atoms of the heterocycloalkyl ring). Unless stated otherwise specifically in the specification, a heterocycloalkyl is optionally substituted, for example, with oxo, halogen, amino, nitrile, nitro, hydroxyl, alkyl, alkenyl, alkynyl, haloalkyl, alkoxy, aryl, cycloalkyl, heterocycloalkyl, heteroaryl, and the like. In some embodiments, a heterocycloalkyl is optionally substituted with oxo, halogen, methyl, ethyl, -CN, - CF₃, -OH, -OMe, -NH₂, or -NO₂. In some embodiments, a heterocycloalkyl is optionally substituted with oxo, halogen, methyl, ethyl, -CN, -CF₃, -OH, or -OMe. In some embodiments, the heterocycloalkyl is optionally substituted with halogen.

"Heteroalkyl" refers to an alkyl group in which one or more skeletal atoms of the alkyl are selected from an atom other than carbon, e.g., oxygen, nitrogen (e.g. -NH-, -N(alkyl)-), sulfur, or combinations thereof. A heteroalkyl is attached to the rest of the molecule at a carbon atom of the heteroalkyl. In one aspect, a heteroalkyl is a C₁-C₆ heteroalkyl. Unless stated otherwise specifically in the specification, a heteroalkyl is optionally substituted, for example, with oxo, halogen, amino, nitrile, nitro, hydroxyl, alkyl, alkenyl, alkynyl, haloalkyl, alkoxy, aryl, cycloalkyl, heterocycloalkyl, heteroaryl, and the like. In some embodiments, a heteroalkyl is optionally substituted with oxo, halogen, methyl, ethyl, - CN, -CF₃, -OH, -OMe, -NH₂, or -NO₂. In some embodiments, a heteroalkyl is optionally substituted with oxo, halogen, methyl, ethyl, -CN, -CF₃, -OH, or -OMe. In some embodiments, the heteroalkyl is optionally substituted with halogen.

"Heteroaryl" refers to a 5- to 14-membered ring system radical comprising hydrogen atoms, one to thirteen carbon atoms, one to six heteroatoms selected from the group consisting of nitrogen, oxygen, phosphorous and sulfur, and at least one aromatic ring. The heteroaryl radical may be a monocyclic, bicyclic, tricyclic or tetracyclic ring system, which may include fused (when fused with a cycloalkyl or heterocycloalkyl ring, the heteroaryl is bonded through an aromatic ring atom) or bridged ring systems; and the nitrogen, carbon or sulfur atoms in the heteroaryl radical may be optionally oxidized; the nitrogen atom may be optionally quaternized. In some embodiments, the heteroaryl is a 5-to 10-membered heteroaryl. In some embodiments, the heteroaryl is a 5- to 6-membered heteroaryl. Examples include, but are not limited to, azepinyl, acridinyl, benzimidazolyl, benzothiazolyl, benzindolyl, benzodioxolyl, benzofuranyl, benzooxazolyl, benzothiazolyl, benzothiadiazolyl, benzo[b][1,4]dioxepinyl, 1,4-benzodioxanyl, benzonaphthofuranyl, benzoxazolyl, benzodioxolyl, benzodioxinyl, benzopyranyl, benzopyranonyl, benzofuranyl, benzofuranonyl, benzothienyl (benzothiophenyl), benzotriazolyl, benzo[4,6]imidazo[1,2-a]pyridinyl, carbazolyl, cinnolinyl, dibenzofuranyl, dibenzothiophenyl, furanyl, furanonyl, isothiazolyl, imidazolyl, indazolyl, indolyl, indazolyl, isoindolyl, indolinyl, isoindolinyl, isoquinolyl, indolizinyl, isoxazolyl, naphthyridinyl, oxadiazolyl, 2-oxoazepinyl, oxazolyl, oxiranyl, 1-oxidopyridinyl, 1-oxidopyrimidinyl, 1-oxidopyrazinyl, 1-oxidopyridazinyl, 1-phenyl-1H-pyrrolyl, phenazinyl, phenothiazinyl, phenoxazinyl, phthalazinyl, pteridinyl, purinyl, pyrrolyl, pyrazolyl, pyridinyl, pyrazinyl, pyrimidinyl, pyridazinyl, quinazolinyl, quinoxalinyl, quinolinyl, quinuclidinyl, isoquinolinyl, tetrahydroquinolinyl, thiazolyl, thiadiazolyl, triazolyl, tetrazolyl, triazinyl, and thiophenyl (i.e., thienyl). Unless stated otherwise specifically in the specification, a heteroaryl is optionally substituted, for example, with halogen, amino, nitrile, nitro, hydroxyl, alkyl, alkenyl, alkynyl, haloalkyl, alkoxy, aryl, cycloalkyl, heterocycloalkyl, heteroaryl, and the like. In some embodiments, a heteroaryl is optionally substituted with halogen, methyl, ethyl, -CN, - CF₃, -OH, -OMe, -NH₂, or -NO₂. In some embodiments, a heteroaryl is optionally substituted with halogen, methyl, ethyl, -CN, -CF₃, -OH, or -OMe. In some embodiments, the heteroaryl is optionally substituted with halogen.

The terms "treat," "prevent," "ameliorate," and "inhibit," as well as words stemming therefrom, as used herein, do not necessarily imply 100% or complete treatment, prevention, amelioration, or inhibition. Rather, there are varying degrees of treatment, prevention, amelioration, and inhibition of which one of ordinary skill in the art recognizes as having a potential benefit or therapeutic effect. In this respect, the disclosed methods can provide any amount of any level of treatment, prevention, amelioration, or inhibition of the disorder in a mammal. For example, a disorder, including symptoms or conditions thereof, may be reduced by, for example, about 100%, about 90%, about 80%, about 70%, about 60%, about 50%, about 40%, about 30%, about 20%, or about 10%. Furthermore, the treatment, prevention, amelioration, or inhibition provided by the methods disclosed herein can include treatment, prevention, amelioration, or inhibition of one or more conditions or symptoms of the disorder, e.g., cancer or an inflammatory disease. Also, for purposes herein, "treatment," "prevention," "amelioration," or "inhibition" encompass delaying the onset of the disorder, or a symptom or condition thereof.

The terms "effective amount" or "therapeutically effective amount," as used herein, refer to a sufficient amount of a compound disclosed herein being administered which will relieve to some extent one or more of the symptoms of the disease or condition being treated, e.g., cancer or an inflammatory disease. In some embodiments, the result is a reduction and/or alleviation of the signs, symptoms, or causes of a disease, or any other desired alteration of a biological system. For example, an "effective amount" for therapeutic uses is the amount of the composition comprising a compound disclosed herein required to provide a clinically significant decrease in disease symptoms. In some embodiments, an appropriate "effective" amount in any individual case is determined using techniques, such as a dose escalation study.

As used herein, the term "TYK2-mediated" disorders, diseases, and/or conditions as used herein means any disease or other deleterious condition in which TYK2 or a mutant thereof is known to play a role. Accordingly, another embodiment relates to treating or lessening the severity of one or more diseases in which TYK2, or a mutant thereof, is known to play a role. Such TYK2-mediated disorders include but are not limited to autoimmune disorders, inflammatory disorders, proliferative disorders, endocrine disorders, neurological disorders and disorders associated with transplantation.

### Compounds

Described herein are compounds that are useful in treating a TYK2-mediated disorder. In some embodiments, the TYK2-mediated disorder is an autoimmune disorder, an inflammatory disorder, a proliferative disorder, an endocrine disorder, a neurological disorder, or a disorder associated with transplantation.

Disclosed herein is a compound of Formula (I), or a pharmaceutically acceptable salt, solvate, or stereoisomer thereof: 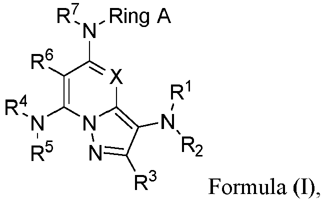 wherein:
Ring A is optionally substituted cycloalkyl, optionally substituted heterocycloalkyl, optionally substituted aryl or optionally substituted heteroaryl;
X is CR⁸ or N;
R¹ is -S(=O)R¹⁰, -S(=O)₂R¹⁰, -S(=O)₂NR¹²R¹³, -C(=O)R¹⁰, -C(=O)OR¹¹, -C(=O)NR¹²R¹³, optionally substituted C₁-C₆alkyl, optionally substituted C₁-C₆heteroalkyl, optionally substituted C₁-C₆haloalkyl, optionally substituted C₁-C₆deuteroalkyl, optionally substituted C₁-C₆hydroxyalkyl, optionally substituted C₁-C₆aminoalkyl, optionally substituted C₂-C₆alkenyl, optionally substituted C₂-C₆alkynyl, optionally substituted cycloalkyl, optionally substituted heterocycloalkyl, optionally substituted aryl, or optionally substituted heteroaryl;
R² is hydrogen, optionally substituted C₁-C₆alkyl, optionally substituted C₁-C₆haloalkyl, or optionally substituted C₁-C₆deuteroalkyl;
R³, R⁶, and R⁸ are independently hydrogen, deuterium, halogen, -CN, -OR^{b}, -SR^{b}, -S(=O)R^{a}, -S(=O)₂R^{a}, - NO₂, -NR^{c}R^{d}, -NHS(=O)₂R^{a}, -S(=O)₂NR^{c}R^{d}, -C(=O)R^{a}, -OC(=O)R^{a}, -C(=O)OR^{b}, -OC(=O)OR^{b}, - C(=O)NR^{c}R^{d}, -OC(=O)NR^{c}R^{d}, -NR^{b}C(=O)NR^{c}R^{d}, -NR^{b}C(=O)R^{a}, -NR^{b}C(=O)OR^{b}, optionally substituted C₁-C₆alkyl, optionally substituted C₁-C₆haloalkyl, optionally substituted C₁-C₆deuteroalkyl, optionally substituted C₁-C₆hydroxyalkyl, optionally substituted C₁-C₆aminoalkyl, optionally substituted C₂-C₆alkenyl, or optionally substituted C₂-C₆alkynyl;
R⁴ is hydrogen, optionally substituted C₁-C₆alkyl, optionally substituted C₁-C₆heteroalkyl, optionally substituted C₁-C₆haloalkyl, optionally substituted C₁-C₆deuteroalkyl, optionally substituted C₁-C₆hydroxyalkyl, optionally substituted C₁-C₆aminoalkyl, optionally substituted C₂-C₆alkenyl, optionally substituted C₂-C₆alkynyl, optionally substituted cycloalkyl, optionally substituted heterocycloalkyl, optionally substituted aryl, or optionally substituted heteroaryl;
R⁵ is hydrogen, optionally substituted C₁-C₆alkyl, optionally substituted C₁-C₆haloalkyl, or optionally substituted C₁-C₆deuteroalkyl;
R⁷ is hydrogen, optionally substituted C₁-C₆alkyl, optionally substituted C₁-C₆haloalkyl, or optionally substituted C₁-C₆deuteroalkyl;
each R¹⁰ is independently optionally substituted C₁-C₆alkyl, optionally substituted C₁-C₆heteroalkyl, optionally substituted C₁-C₆haloalkyl, optionally substituted C₁-C₆deuteroalkyl, optionally substituted C₁-C₆hydroxyalkyl, optionally substituted C₁-C₆aminoalkyl, optionally substituted C₂-C₆alkenyl, optionally substituted C₂-C₆alkynyl, optionally substituted cycloalkyl, optionally substituted heterocycloalkyl, optionally substituted aryl, or optionally substituted heteroaryl;
each R¹¹ is independently hydrogen, optionally substituted C₁-C₆alkyl, optionally substituted C₁-C₆haloalkyl, optionally substituted C₁-C₆deuteroalkyl, optionally substituted C₁-C₆hydroxyalkyl, optionally substituted C₁-C₆aminoalkyl, optionally substituted C₂-C₆alkenyl, optionally substituted C₂-C₆alkynyl, optionally substituted cycloalkyl, optionally substituted heterocycloalkyl, optionally substituted aryl, or optionally substituted heteroaryl;
each R¹² and R¹³ is independently hydrogen, optionally substituted C₁-C₆alkyl, optionally substituted C₁-C₆haloalkyl, optionally substituted C₁-C₆deuteroalkyl, optionally substituted C₁-C₆hydroxyalkyl, optionally substituted C₁-C₆aminoalkyl, optionally substituted C₂-C₆alkenyl, optionally substituted C₂-C₆alkynyl, optionally substituted cycloalkyl, optionally substituted heterocycloalkyl, optionally substituted aryl, or optionally substituted heteroaryl;
or R¹² and R¹³ are taken together with the nitrogen atom to which they are attached to form an optionally substituted heterocycloalkyl;
each R^{a} is independently C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OMe, - NH₂, -C(=O)Me, -C(=O)OH, -C(=O)OMe, C₁-C₆alkyl, or C₁-C₆haloalkyl;
each R^{b} is independently hydrogen, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OMe, - NH₂, -C(=O)Me, -C(=O)OH, -C(=O)OMe, C₁-C₆alkyl, or C₁-C₆haloalkyl; and
each R^{c} and R^{d} is independently hydrogen, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OMe, -NH₂, -C(=O)Me, -C(=O)OH, -C(=O)OMe, C₁-C₆alkyl, or C₁-C₆haloalkyl;
or R^{c} and R^{d} are taken together with the nitrogen atom to which they are attached to form a heterocycloalkyl optionally substituted with one or more oxo, deuterium, halogen, -CN, -OH, -OMe, -NH₂, -C(=O)Me, -C(=O)OH, -C(=O)OMe, C₁-C₆alkyl, or C₁-C₆haloalkyl.

Disclosed herein is a compound of Formula (I), or a pharmaceutically acceptable salt, solvate, or stereoisomer thereof: 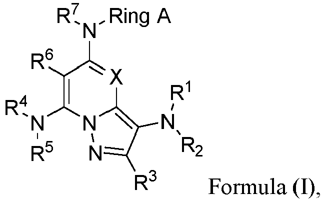 wherein:
Ring A is cycloalkyl, heterocycloalkyl, aryl, or heteroaryl; each optionally substituted with one or more R^{A};
each R^{A} is independently deuterium, halogen, -CN, -OR¹⁵, -SR¹⁵, -S(=O)R¹⁴, -S(=O)₂R¹⁴, -NO₂, - NR¹⁶R¹⁷, -NHS(=O)₂R¹⁴, -S(=O)₂NR¹⁶R¹⁷, -C(=O)R¹⁴, -OC(=O)R¹⁴, -C(=O)OR¹⁵, -OC(=O)OR¹⁵, - C(=O)NR¹⁶R¹⁷, -OC(=O)NR¹⁶R¹⁷, -NR¹⁵C(=O)NR¹⁶R¹⁷, -NR¹⁵C(=O)R¹⁴, -NR¹⁵C(=O)OR¹⁵, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more R^{A1}; or two R^{A} on the same carbon are taken together to form an oxo;
each R^{A1}; is independently deuterium, halogen, -CN, -OR¹⁵, -SR¹⁵, -S(=O)R¹⁴, -S(=O)₂R¹⁴, -NO₂, - NR¹⁶R¹⁷, -NHS(=O)₂R¹⁴, -S(=O)₂NR¹⁶R¹⁷, -C(=O)R¹⁴, -OC(=O)R¹⁴, -C(=O)OR¹⁵, -OC(=O)OR¹⁵, - C(=O)NR¹⁶R¹⁷, -OC(=O)NR¹⁶R¹⁷, -NR¹⁵C(=O)NR¹⁶R¹⁷, -NR¹⁵C(=O)R¹⁴, -NR¹⁵C(=O)OR¹⁵, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{A1}; on the same carbon are taken together to form an oxo;
each R¹⁴ is independently C₁-C₆alkyl, C₁-C₆heteroalkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more R^{14a};
each R^{14a} is independently deuterium, halogen, -CN, -OR^{b}, -SR^{b}, -S(=O)R^{a}, -S(=O)₂R^{a}, -NO₂, -NR^{c}R^{d}, - NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, - OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{14a} on the same carbon are taken together to form an oxo;
each R¹⁵ is independently hydrogen, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more R^{15a};
each R^{15a} is independently deuterium, halogen, -CN, -OR^{b}, -SR^{b}, -S(=O)R^{a}, -S(=O)₂R^{a}, -NO₂, -NR^{c}R^{d}, - NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, - OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{15a} on the same carbon are taken together to form an oxo;
each R¹⁶ and R¹⁷ is independently hydrogen, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more R^{16a};
or R¹⁶ and R¹⁷ are taken together with the nitrogen atom to which they are attached to form a heterocycloalkyl optionally substituted with one or more R^{16b};
each R^{16a} is independently deuterium, halogen, -CN, -OR^{b}, -SR^{b}, -S(=O)R^{a}, -S(=O)₂R^{a}, -NO₂, -NR^{c}R^{d}, - NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, - OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl, or two R^{16a} on the same carbon are taken together to form an oxo;
each R^{16b} is independently deuterium, halogen, -CN, -OR^{b}, -SR^{b}, -S(=O)R^{a}, -S(=O)₂R^{a}, -NO₂, -NR^{c}R^{d}, - NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, - OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{16b} on the same carbon are taken together to form an oxo;
X is CR⁸ or N;
R¹ is -S(=O)R¹⁰, -S(=O)₂R¹⁰, -S(=O)₂NR¹²R¹³, -C(=O)R¹⁰, -C(=O)OR¹¹, -C(=O)NR¹²R¹³, C₁-C₆alkyl, C₁-C₆heteroalkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more R^{1a};
each R^{1a} is independently deuterium, halogen, -CN, -OR^{b}, -SR^{b}, -S(=O)R^{a}, -S(=O)₂R^{a}, -NO₂, -NR^{c}R^{d}, - NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, - OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more R^{1b}; or two R^{1a} on the same carbon are taken together to form an oxo;
each R^{1b} is independently deuterium, halogen, -CN, -OR^{b}, -SR^{b}, -S(=O)R^{a}, -S(=O)₂R^{a}, -NO₂, -NR^{c}R^{d}, - NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, - OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{1b} on the same carbon are taken together to form an oxo;
R² is hydrogen, C₁-C₆alkyl, C₁-C₆haloalkyl, or C₁-C₆deuteroalkyl;
R³, R⁶, and R⁸ are independently hydrogen, deuterium, halogen, -CN, -OR^{b}, -SR^{b}, -S(=O)R^{a}, -S(=O)₂R^{a}, - NO₂, -NR^{c}R^{d}, -NHS(=O)₂R^{a}, -S(=O)₂NR^{c}R^{d}, -C(=O)R^{a}, -OC(=O)R^{a}, -C(=O)OR^{b}, -OC(=O)OR^{b}, - C(=O)NR^{c}R^{d}, -OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, or C₂-C₆alkynyl;
R⁴ is hydrogen, C₁-C₆alkyl, C₁-C₆heteroalkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl are optionally substituted with one or more R^{4a};
each R^{4a} is independently deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; or two R^{4a} on the same carbon are taken together to form an oxo;
R⁵ is hydrogen, C₁-C₆alkyl, C₁-C₆haloalkyl, or C₁-C₆deuteroalkyl;
R⁷ is hydrogen, C₁-C₆alkyl, C₁-C₆haloalkyl, or C₁-C₆deuteroalkyl;
each R¹⁰ is independently C₁-C₆alkyl, C₁-C₆heteroalkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more R^{10a};
each R^{10a} is independently deuterium, halogen, -CN, -OR^{b}, -SR^{b}, -S(=O)R^{a}, -S(=O)₂R^{a}, -NO₂, -NR^{c}R^{d}, - NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, - OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{10a} on the same carbon are taken together to form an oxo;
each R¹¹ is independently hydrogen, C₁-C₆alkyl, C₁-C₆heteroalkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more R^{11a};
each R^{11a} is independently hydrogen, deuterium, halogen, -CN, -OR^{b}, -SR^{b}, -S(=O)R^{a}, -S(=O)₂R^{a}, -NO₂, - NR^{c}R^{d}, -NHS(=O)₂R^{a}, -S(=O)₂NR^{c}R^{d}, -C(=O)R^{a}, -OC(=O)R^{a}, -C(=O)OR^{b}, -OC(=O)OR^{b}, - C(=O)NR^{c}R^{d}, -OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{11a} on the same carbon are taken together to form an oxo
each R¹² and R¹³ is independently hydrogen, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more R^{12a};
each R^{12a} is independently deuterium, halogen, -CN, -OR^{b}, -SR^{b}, -S(=O)R^{a}, -S(=O)₂R^{a}, -NO₂, -NR^{c}R^{d}, - NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, - OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{12a} on the same carbon are taken together to form an oxo;
or R¹² and R¹³ are taken together with the nitrogen atom to which they are attached to form a heterocycloalkyl optionally substituted with one or more R^{12b}.
each R^{12b} is independently deuterium, halogen, -CN, -OR^{b}, -SR^{b}, -S(=O)R^{a}, -S(=O)₂R^{a}, -NO₂, -NR^{c}R^{d}, - NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, - OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{12b} on the same carbon are taken together to form an oxo;
each R^{a} is independently C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OMe, - NH₂, -C(=O)Me, -C(=O)OH, -C(=O)OMe, C₁-C₆alkyl, or C₁-C₆haloalkyl;
each R^{b} is independently hydrogen, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OMe, - NH₂, -C(=O)Me, -C(=O)OH, -C(=O)OMe, C₁-C₆alkyl, or C₁-C₆haloalkyl; and
each R^{c} and R^{d} is independently hydrogen, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OMe, -NH₂, -C(=O)Me, -C(=O)OH, -C(=O)OMe, C₁-C₆alkyl, or C₁-C₆haloalkyl;
or R^{c} and R^{d} are taken together with the nitrogen atom to which they are attached to form a heterocycloalkyl optionally substituted with one or more oxo, deuterium, halogen, -CN, -OH, -OMe, -NH₂, -C(=O)Me, -C(=O)OH, -C(=O)OMe, C₁-C₆alkyl, or C₁-C₆haloalkyl.

In some embodiments of a compound of Formula (I), Ring A is cycloalkyl, heterocycloalkyl, aryl, or heteroaryl; each optionally substituted with one or more R^{A}. In some embodiments of a compound of Formula (I), Ring A is heterocycloalkyl, aryl, or heteroaryl; each optionally substituted with one or more R^{A}. In some embodiments of a compound of Formula (I), Ring A is heterocycloalkyl optionally substituted with one or more R^{A}. In some embodiments of a compound of Formula (I), Ring A is aryl or heteroaryl; each optionally substituted with one or more R^{A}. In some embodiments of a compound of Formula (I), Ring A is aryl optionally substituted with one or more R^{A}. In some embodiments of a compound of Formula (I), Ring A is phenyl, pyridyl, pyrimidyl, pyrazinyl, or pyridazinyl, each optionally substituted with one or more R^{A}.

In some embodiments of a compound of Formula (I), each R^{A} is independently deuterium, halogen, -CN, -OR¹⁵, -SR¹⁵, -S(=O)R¹⁴, -S(=O)₂R¹⁴, -NO₂, -NR¹⁶R¹⁷, -NHS(=O)₂R¹⁴, -S(=O)₂NR¹⁶R¹⁷, - C(=O)R¹⁴, -OC(=O)R¹⁴, -C(=O)OR¹⁵, -OC(=O)OR¹⁵, -C(=O)NR¹⁶R¹⁷, -OC(=O)NR¹⁶R¹⁷, - NR¹⁵C(=O)NR¹⁶R¹⁷, -NR¹⁵C(=O)R¹⁴, -NR¹⁵C(=O)OR¹⁵, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more R^{A1}; or two R^{A} on the same carbon are taken together to form an oxo.

In some embodiments of a compound of Formula (I), each R^{A} is independently deuterium, halogen, -CN, -OR¹⁵, -NR¹⁶R¹⁷, -C(=O)R¹⁴, -C(=O)OR¹⁵, -C(=O)NR¹⁶R¹⁷, C₁-C₆alkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; wherein each alkyl, cycloalkyl, heterocycloalkyl, aryl, and heteroaryl is independently optionally substituted with one or more R^{A1}; or two R^{A} on the same carbon are taken together to form an oxo.

In some embodiments of a compound of Formula (I), each R^{A} is independently deuterium, halogen, -CN, -OR¹⁵, -NR¹⁶R¹⁷, C₁-C₆alkyl, C₁-C₆haloalkyl, C₁-C₆deuteroalkyl, cycloalkyl, heterocycloalkyl, aryl, or heteroaryl; wherein each alkyl, cycloalkyl, heterocycloalkyl, aryl, and heteroaryl is independently optionally substituted with one or more R^{A1}; or two R^{A} on the same carbon are taken together to form an oxo.

In some embodiments of a compound of Formula (I), each R^{A1}; is independently deuterium, halogen, -CN, -OR¹⁵, -SR¹⁵, -S(=O)R¹⁴, -S(=O)₂R¹⁴, -NO₂, -NR¹⁶R¹⁷, -NHS(=O)₂R¹⁴, -S(=O)₂NR¹⁶R¹⁷, - C(=O)R¹⁴, -OC(=O)R¹⁴, -C(=O)OR¹⁵, -OC(=O)OR¹⁵, -C(=O)NR¹⁶R¹⁷, -OC(=O)NR¹⁶R¹⁷, - NR¹⁵C(=O)NR¹⁶R¹⁷, -NR¹⁵C(=O)R¹⁴, -NR¹⁵C(=O)OR¹⁵, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{A1}; on the same carbon are taken together to form an oxo.

In some embodiments of a compound of Formula (I), each R^{A1}; is independently deuterium, halogen, -CN, -OR¹⁵, -NR¹⁶R¹⁷, -C(=O)R¹⁴, -C(=O)OR¹⁵, -C(=O)NR¹⁶R¹⁷, C₁-C₆alkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; or two R^{A1} on the same carbon are taken together to form an oxo.

In some embodiments of a compound of Formula (I), each R^{A1} is independently deuterium, halogen, -CN, -OR¹⁵, -NR¹⁶R¹⁷, C₁-C₆alkyl, C₁-C₆haloalkyl, C₁-C₆deuteroalkyl, cycloalkyl, or heterocycloalkyl; or two R^{A1} on the same carbon are taken together to form an oxo.

In some embodiments of a compound of Formula (I), each R¹⁴ is independently C₁-C₆alkyl, C₁-C₆heteroalkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more R^{14a}.

In some embodiments of a compound of Formula (I), each R¹⁴ is independently C₁-C₆alkyl, C₁-C₆heteroalkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; wherein each alkyl, cycloalkyl, heterocycloalkyl, aryl, and heteroaryl is independently optionally substituted with one or more R^{14a}.

In some embodiments of a compound of Formula (I), each R¹⁴ is independently C₁-C₆alkyl, C₁-C₆heteroalkyl, C₁-C₆haloalkyl, C₁-C₆deuteroalkyl, cycloalkyl, or heterocycloalkyl; wherein each alkyl, cycloalkyl, and heterocycloalkyl is independently optionally substituted with one or more R^{14a}.

In some embodiments of a compound of Formula (I), each R^{14a} is independently deuterium, halogen, -CN, -OR^{b}, -SR^{b}, -S(=O)R^{a}, -S(=O)₂R^{a}, -NO₂, -NR^{c}R^{d}, -NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, -OC(=O)NR^{c}R^{d}, -NR^{b}C(=O)NR^{c}R^{d}, - NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{14a} on the same carbon are taken together to form an oxo.

In some embodiments of a compound of Formula (I), each R^{14a} is independently deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; or two R^{14a} on the same carbon are taken together to form an oxo.

In some embodiments of a compound of Formula (I), each R¹⁵ is independently hydrogen, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more R^{15a}.

In some embodiments of a compound of Formula (I), each R¹⁵ is independently hydrogen, C₁-C₆alkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; wherein each alkyl, cycloalkyl, heterocycloalkyl, aryl, and heteroaryl is independently optionally substituted with one or more R^{15a}.

**In** some embodiments of a compound of Formula (I), each R¹⁵ is independently hydrogen, C₁-C₆alkyl, C₁-C₆haloalkyl, C₁-C₆deuteroalkyl, cycloalkyl, or heterocycloalkyl; wherein each alkyl, cycloalkyl, or heterocycloalkyl is independently optionally substituted with one or more R^{15a}.

**In** some embodiments of a compound of Formula (I), each R^{15a} is independently deuterium, halogen, -CN, -OR^{b}, -SR^{b}, -S(=O)R^{a}, -S(=O)₂R^{a}, -NO₂, -NR^{c}R^{d}, -NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, -OC(=O)NR^{c}R^{d}, -NR^{b}C(=O)NR^{c}R^{d}, - NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{15a} on the same carbon are taken together to form an oxo.

**In** some embodiments of a compound of Formula (I), each R^{15a} is independently deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; or two R^{15a} on the same carbon are taken together to form an oxo.

**In** some embodiments of a compound of Formula (I), each R^{15a} is independently deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, cycloalkyl, or heterocycloalkyl; or two R^{15a} on the same carbon are taken together to form an oxo.

In some embodiments of a compound of Formula (I), each R¹⁶ and R¹⁷ is independently hydrogen, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more R^{16a}.

**In** some embodiments of a compound of Formula (I), each R¹⁶ and R¹⁷ is independently hydrogen, C₁-C₆alkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; wherein each alkyl, cycloalkyl, heterocycloalkyl, aryl, and heteroaryl is independently optionally substituted with one or more R^{16a}.

**In** some embodiments of a compound of Formula (I), each R¹⁶ and R¹⁷ is independently hydrogen, C₁-C₆alkyl, C₁-C₆haloalkyl, C₁-C₆deuteroalkyl, cycloalkyl, heterocycloalkyl, aryl, or heteroaryl; wherein each alkyl, cycloalkyl, or heterocycloalkyl is independently optionally substituted with one or more R^{16a}.

In some embodiments of a compound of Formula (I), each R^{16a} is independently deuterium, halogen, -CN, -OR^{b}, -SR^{b}, -S(=O)R^{a}, -S(=O)₂R^{a}, -NO₂, -NR^{c}R^{d}, -NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, -OC(=O)NR^{c}R^{d}, -NR^{b}C(=O)NR^{c}R^{d}, - NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl, or two R^{16a} on the same carbon are taken together to form an oxo.

In some embodiments of a compound of Formula (I), each R^{16a} is independently deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl, or two R^{16a} on the same carbon are taken together to form an oxo.

In some embodiments of a compound of Formula (I), each R^{16a} is independently deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, cycloalkyl, or heterocycloalkyl, or two R^{16a} on the same carbon are taken together to form an oxo.

In some embodiments of a compound of Formula (I), R¹⁶ and R¹⁷ are taken together with the nitrogen atom to which they are attached to form a heterocycloalkyl optionally substituted with one or more R^{16b}.

In some embodiments of a compound of Formula (I), each R^{16b} is independently deuterium, halogen, -CN, -OR^{b}, -SR^{b}, -S(=O)R^{a}, -S(=O)₂R^{a}, -NO₂, -NR^{c}R^{d}, -NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, -OC(=O)NR^{c}R^{d}, -NR^{b}C(=O)NR^{c}R^{d}, - NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{16b} on the same carbon are taken together to form an oxo.

In some embodiments of a compound of Formula (I), each R^{16b} is independently deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; or two R^{16b} on the same carbon are taken together to form an oxo.

In some embodiments of a compound of Formula (I), each R^{16b} is independently deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, cycloalkyl, or heterocycloalkyl; or two R^{16b} on the same carbon are taken together to form an oxo.

In some embodiments of a compound of Formula (I), X is N. In some embodiments of a compound of Formula (I), X is CR⁸.

In some embodiments of a compound of Formula (I), R¹ is -S(=O)R¹⁰, -S(=O)₂R¹⁰, - S(=O)₂NR¹²R¹³, -C(=O)R¹⁰, -C(=O)OR¹¹, -C(=O)NR¹²R¹³, C₁-C₆alkyl, C₁-C₆heteroalkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more R^{1a}.

In some embodiments of a compound of Formula (I), R¹ is -C(=O)R¹⁰, C₁-C₆alkyl, C₁-C₆heteroalkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; wherein each alkyl, cycloalkyl, heterocycloalkyl, aryl, and heteroaryl is independently optionally substituted with one or more R^{1a}. In some embodiments of a compound of Formula (I), R¹ is -C(=O)R¹⁰. In some embodiments of a compound of Formula (I), R¹ is C₁-C₆alkyl, C₁-C₆heteroalkyl, cycloalkyl, heterocycloalkyl, aryl, or heteroaryl; wherein each alkyl, cycloalkyl, heterocycloalkyl, aryl, and heteroaryl is independently optionally substituted with one or more R^{1a}. In some embodiments of a compound of Formula (I), R¹ is heteroaryl optionally substituted with one or more R^{1a}. In some embodiments of a compound of Formula (I), R¹ is -C(=O)R¹⁰ or heteroaryl optionally substituted with one or more R^{1a}.

In some embodiments of a compound of Formula (I), each R^{1a} is independently deuterium, halogen, -CN, -OR^{b}, -SR^{b}, -S(=O)R^{a}, -S(=O)₂R^{a}, -NO₂, -NR^{c}R^{d}, -NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, -OC(=O)NR^{c}R^{d}, -NR^{b}C(=O)NR^{c}R^{d}, - NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more R^{1b}; or two R^{1a} on the same carbon are taken together to form an oxo.

In some embodiments of a compound of Formula (I), each R^{1a} is independently deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; wherein each alkyl, cycloalkyl, heterocycloalkyl, aryl, and heteroaryl is independently optionally substituted with one or more R^{1b}; or two R^{1a} on the same carbon are taken together to form an oxo.

In some embodiments of a compound of Formula (I), each R^{1a} is independently deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, cycloalkyl, or heterocycloalkyl; wherein each alkyl, cycloalkyl, and heterocycloalkyl is independently optionally substituted with one or more R^{1b}; or two R^{1a} on the same carbon are taken together to form an oxo.

In some embodiments of a compound of Formula (I), each R^{1b} is independently deuterium, halogen, -CN, -OR^{b}, -SR^{b}, -S(=O)R^{a}, -S(=O)₂R^{a}, -NO₂, -NR^{c}R^{d}, -NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, -OC(=O)NR^{c}R^{d}, -NR^{b}C(=O)NR^{c}R^{d}, - NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{1b} on the same carbon are taken together to form an oxo.

In some embodiments of a compound of Formula (I), each R^{1b} is independently deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; or two R^{1b} on the same carbon are taken together to form an oxo.

In some embodiments of a compound of Formula (I), each R^{1b} is independently deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, cycloalkyl, or heterocycloalkyl; or two R^{1b} on the same carbon are taken together to form an oxo.

In some embodiments of a compound of Formula (I), each R¹⁰ is independently C₁-C₆alkyl, C₁-C₆heteroalkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more R^{10a}.

In some embodiments of a compound of Formula (I), each R¹⁰ is independently C₁-C₆alkyl, C₁-C₆heteroalkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; wherein each alkyl, cycloalkyl, heterocycloalkyl, aryl, and heteroaryl is independently optionally substituted with one or more R^{10a}.

In some embodiments of a compound of Formula (I), each R¹⁰ is independently C₁-C₆alkyl, C₁-C₆heteroalkyl, C₁-C₆haloalkyl, C₁-C₆deuteroalkyl, C₁-C₆hydroxyalkyl, C₁-C₆aminoalkyl, cycloalkyl, or heterocycloalkyl; wherein each alkyl, cycloalkyl, or heterocycloalkyl is independently optionally substituted with one or more R^{10a}.

In some embodiments of a compound of Formula (I), each R^{10a} is independently deuterium, halogen, -CN, -OR^{b}, -SR^{b}, -S(=O)R^{a}, -S(=O)₂R^{a}, -NO₂, -NR^{c}R^{d}, -NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, -OC(=O)NR^{c}R^{d}, -NR^{b}C(=O)NR^{c}R^{d}, - NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{10a} on the same carbon are taken together to form an oxo.

In some embodiments of a compound of Formula (I), each R^{10a} is independently deuterium, halogen, -CN, -OR^{b}, -NO₂, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; or two R^{10a} on the same carbon are taken together to form an oxo.

In some embodiments of a compound of Formula (I), each R^{10a} is independently deuterium, halogen, -CN, -OR^{b}, -NO₂, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, cycloalkyl, or heterocycloalkyl; or two R^{10a} on the same carbon are taken together to form an oxo.

In some embodiments of a compound of Formula (I), each R¹¹ is independently hydrogen, C₁-C₆alkyl, C₁-C₆heteroalkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more R^{11a}.

In some embodiments of a compound of Formula (I), each R¹¹ is independently hydrogen, C₁-C₆alkyl, C₁-C₆heteroalkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; wherein each alkyl, cycloalkyl, heterocycloalkyl, aryl, and heteroaryl is independently optionally substituted with one or more R^{11a}.

**In** some embodiments of a compound of Formula (I), each R¹¹ is independently hydrogen, C₁-C₆alkyl, C₁-C₆heteroalkyl, C₁-C₆haloalkyl, C₁-C₆deuteroalkyl, cycloalkyl, or heterocycloalkyl; wherein each alkyl, cycloalkyl, and heterocycloalkyl is independently optionally substituted with one or more R^{11a}.

**In** some embodiments of a compound of Formula (I), each R^{11a} is independently hydrogen, deuterium, halogen, -CN, -OR^{b}, -SR^{b}, -S(=O)R^{a}, -S(=O)₂R^{a}, -NO₂, -NR^{c}R^{d}, -NHS(=O)₂R^{a}, - S(=O)₂NR^{c}R^{d}, -C(=O)R^{a}, -OC(=O)R^{a}, -C(=O)OR^{b}, -OC(=O)OR^{b}, -C(=O)NR^{c}R^{d}, -OC(=O)NR^{c}R^{d}, - NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{11a} on the same carbon are taken together to form an oxo.

**In** some embodiments of a compound of Formula (I), each R^{11a} is independently hydrogen, deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; or two R^{11a} on the same carbon are taken together to form an oxo.

In some embodiments of a compound of Formula (I), each R^{11a} is independently hydrogen, deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, cycloalkyl, or heterocycloalkyl; or two R^{11a} on the same carbon are taken together to form an oxo.

**In** some embodiments of a compound of Formula (I), each R¹² and R¹³ is independently hydrogen, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more R^{12a}.

**In** some embodiments of a compound of Formula (I), each R¹² and R¹³ is independently hydrogen, C₁-C₆alkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; wherein each alkyl, cycloalkyl, heterocycloalkyl, aryl, and heteroaryl is independently optionally substituted with one or more R^{12a}.

**In** some embodiments of a compound of Formula (I), each R¹² and R¹³ is independently hydrogen, C₁-C₆alkyl, C₁-C₆haloalkyl, C₁-C₆deuteroalkyl, cycloalkyl, or heterocycloalkyl; wherein each alkyl, cycloalkyl, and heterocycloalkyl is independently optionally substituted with one or more R^{12a}.

**In** some embodiments of a compound of Formula (I), each R^{12a} is independently deuterium, halogen, -CN, -OR^{b}, -SR^{b}, -S(=O)R^{a}, -S(=O)₂R^{a}, -NO₂, -NR^{c}R^{d}, -NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, -OC(=O)NR^{c}R^{d}, -NR^{b}C(=O)NR^{c}R^{d}, - NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{12a} on the same carbon are taken together to form an oxo.

In some embodiments of a compound of Formula (I), each R^{12a} is independently deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; or two R^{12a} on the same carbon are taken together to form an oxo.

In some embodiments of a compound of Formula (I), each R^{12a} is independently deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, cycloalkyl, or heterocycloalkyl; or two R^{12a} on the same carbon are taken together to form an oxo.

In some embodiments of a compound of Formula (I), R¹² and R¹³ are taken together with the nitrogen atom to which they are attached to form a heterocycloalkyl optionally substituted with one or more R^{12b}.

In some embodiments of a compound of Formula (I), each R^{12b} is independently deuterium, halogen, -CN, -OR^{b}, -SR^{b}, -S(=O)R^{a}, -S(=O)₂R^{a}, -NO₂, -NR^{c}R^{d}, -NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, -OC(=O)NR^{c}R^{d}, -NR^{b}C(=O)NR^{c}R^{d}, - NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{12b} on the same carbon are taken together to form an oxo.

In some embodiments of a compound of Formula (I), each R^{12b} is independently deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; or two R^{12b} on the same carbon are taken together to form an oxo.

In some embodiments of a compound of Formula (I), each R^{12b} is independently deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, cycloalkyl, or heterocycloalkyl; or two R^{12b} on the same carbon are taken together to form an oxo.

In some embodiments of a compound of Formula (I), R² is hydrogen, C₁-C₆alkyl, C₁-C₆haloalkyl, or C₁-C₆deuteroalkyl. In some embodiments of a compound of Formula (I), R² is hydrogen or C₁-C₆alkyl. In some embodiments of a compound of Formula (I), R² is hydrogen.

In some embodiments of a compound of Formula (I), R³, R⁶, and R⁸ are independently hydrogen, deuterium, halogen, -CN, -OR^{b}, -SR^{b}, -S(=O)R^{a}, -S(=O)₂R^{a}, -NO₂, -NR^{c}R^{d}, -NHS(=O)₂R^{a}, - S(=O)₂NR°R^{d}, -C(=O)R^{a}, -OC(=O)R^{a}, -C(=O)OR^{b}, -OC(=O)OR^{b}, -C(=O)NR^{c}R^{d}, -OC(=O)NR^{c}R^{d}, - NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, or C₂-C₆alkynyl. In some embodiments of a compound of Formula (I), R³, R⁶, and R⁸ are independently hydrogen, deuterium, halogen, -CN, -OR^{b}, - NR^{c}R^{d}, -C(=O)NR^{c}R^{d}, C₁-C₆alkyl, C₁-C₆haloalkyl, or C₁-C₆deuteroalkyl. In some embodiments of a compound of Formula (I), R³, R⁶, and R⁸ are independently hydrogen, deuterium, halogen, or C₁-C₆alkyl. In some embodiments of a compound of Formula (I), R³, R⁶, and R⁸ are hydrogen.

In some embodiments of a compound of Formula (I), R⁴ is hydrogen, C₁-C₆alkyl, C₁-C₆heteroalkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl are optionally substituted with one or more R^{4a}.

In some embodiments of a compound of Formula (I), R⁴ is hydrogen, C₁-C₆alkyl, C₁-C₆heteroalkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; wherein each alkyl, cycloalkyl, heterocycloalkyl, aryl, and heteroaryl are optionally substituted with one or more R^{4a}.

In some embodiments of a compound of Formula (I), R⁴ is hydrogen, C₁-C₆alkyl, C₁-C₆heteroalkyl, C₁-C₆haloalkyl, C₁-C₆deuteroalkyl, cycloalkyl, or heterocycloalkyl; wherein each alkyl, cycloalkyl, or heterocycloalkyl are optionally substituted with one or more R^{4a}.

In some embodiments of a compound of Formula (I), R⁴ is hydrogen, C₁-C₆alkyl, C₁-C₆haloalkyl, or C₁-C₆deuteroalkyl.

In some embodiments of a compound of Formula (I), R⁴ is hydrogen, C₁-C₆alkyl, or C₁-C₆deuteroalkyl.

In some embodiments of a compound of Formula (I), R⁴ is hydrogen or C₁-C₆alkyl optionally substituted with one or more R^{4a}. In some embodiments of a compound of Formula (I), R⁴ is C₁-C₆alkyl optionally substituted with one or more R^{4a}.

In some embodiments of a compound of Formula (I), R⁴ is hydrogen or C₁-C₆alkyl. In some embodiments of a compound of Formula (I), R⁴ is C₁-C₆alkyl.

In some embodiments of a compound of Formula (I), each R^{4a} is independently deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; or two R^{4a} on the same carbon are taken together to form an oxo.

In some embodiments of a compound of Formula (I), each R^{4a} is independently deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, cycloalkyl, or heterocycloalkyl; or two R^{4a} on the same carbon are taken together to form an oxo.

In some embodiments of a compound of Formula (I), each R^{4a} is independently deuterium, halogen, -CN, -OR^{b}, -NR^{c}R^{d}, C₁-C₆alkyl, C₁-C₆haloalkyl, C₁-C₆deuteroalkyl, cycloalkyl, or heterocycloalkyl; or two R^{4a} on the same carbon are taken together to form an oxo.

In some embodiments of a compound of Formula (I), R⁵ is hydrogen, C₁-C₆alkyl, C₁-C₆haloalkyl, or C₁-C₆deuteroalkyl. In some embodiments of a compound of Formula (I), R⁵ is hydrogen or C₁-C₆alkyl. In some embodiments of a compound of Formula (I), R⁵ is hydrogen.

In some embodiments of a compound of Formula (I), R⁷ is hydrogen, C₁-C₆alkyl, C₁-C₆haloalkyl, or C₁-C₆deuteroalkyl. In some embodiments of a compound of Formula (I), R⁷ is hydrogen or C₁-C₆alkyl. In some embodiments of a compound of Formula (I), R⁷ is hydrogen.

In some embodiments of a compound described above, each R^{a} is independently C₁-C₆alkyl, C₁-C₆haloalkyl, C₁-C₆deuteroalkyl, or cycloalkyl; wherein each alkyl and cycloalkyl is independently optionally substituted with one or more oxo, deuterium, halogen, -CN, -OH, -OMe, -NH₂, -C(=O)Me, - C(=O)OH, -C(=O)OMe, C₁-C₆alkyl, or C₁-C₆haloalkyl. In some embodiments of a compound described above, each R^{a} is independently C₁-C₆alkyl, C₁-C₆haloalkyl, or C₁-C₆deuteroalkyl. In some embodiments of a compound described above, each R^{a} is independently C₁-C₆alkyl.

In some embodiments of a compound described above, each R^{b} is independently hydrogen, C₁-C₆alkyl, C₁-C₆haloalkyl, C₁-C₆deuteroalkyl, or cycloalkyl; wherein each alkyl and cycloalkyl is independently optionally substituted with one or more oxo, deuterium, halogen, -CN, -OH, -OMe, -NH₂, -C(=O)Me, -C(=O)OH, -C(=O)OMe, C₁-C₆alkyl, or C₁-C₆haloalkyl. In some embodiments of a compound described above, each R^{b} is independently hydrogen, C₁-C₆alkyl, C₁-C₆haloalkyl, or C₁-C₆deuteroalkyl. In some embodiments of a compound described above, each R^{b} is independently hydrogen or C₁-C₆alkyl. In some embodiments of a compound described above, each R^{b} is hydrogen. In some embodiments of a compound described above, each R^{b} is independently C₁-C₆alkyl.

In some embodiments of a compound described above, each R^{c} and R^{d} is independently hydrogen, C₁-C₆alkyl, C₁-C₆haloalkyl, C₁-C₆deuteroalkyl, or cycloalkyl; wherein each alkyl and cycloalkyl is independently optionally substituted with one or more oxo, deuterium, halogen, -CN, -OH, - OMe, -NH₂, -C(=O)Me, -C(=O)OH, -C(=O)OMe, C₁-C₆alkyl, or C₁-C₆haloalkyl. In some embodiments of a compound described above, each R^{c} and R^{d} is independently hydrogen, C₁-C₆alkyl, C₁-C₆haloalkyl, or C₁-C₆deuteroalkyl. In some embodiments of a compound described above, each R^{c} and R^{d} is independently hydrogen or C₁-C₆alkyl. In some embodiments of a compound described above, each R^{c} and R^{d} is hydrogen. In some embodiments of a compound described above, each R^{c} and R^{d} is independently C₁-C₆alkyl.

In some embodiments of a compound of Formula (I), each Ring A, R^{A}, R¹⁴, R¹⁵, R¹⁶, R¹⁷, R¹, R⁴, R¹⁰, R¹¹, R¹², R¹³, R^{a}, R^{b}, R^{c}, and R^{d} is independently optionally substituted with one, two, three, or four substituents as defined herein. In some embodiments of a compound of Formula (I), each Ring A, R^{A}, R¹⁴, R¹⁵, R¹⁶, R¹⁷, R¹, R⁴, R¹⁰, R¹¹, R¹², R¹³, R^{a}, R^{b}, R^{c}, and R^{d} is independently optionally substituted with one, two, or three substituents as defined herein. In some embodiments of a compound of Formula (I), each Ring A, R^{A}, R¹⁴, R¹⁵, R¹⁶, R¹⁷, R¹, R⁴, R¹⁰, R¹¹, R¹², R¹³, R^{a}, R^{b}, R^{c}, and R^{d} is independently optionally substituted with one or two substituents as defined herein. In some embodiments of a compound of Formula (I), each Ring A, R^{A}, R¹⁴, R¹⁵, R¹⁶, R¹⁷, R¹, R⁴, R¹⁰, R¹¹, R¹², R¹³, R^{a}, R^{b}, R^{c}, and R^{d} is independently optionally substituted with one substituent as defined herein.

Also disclosed herein is a compound of Formula (II), or a pharmaceutically acceptable salt, solvate, or stereoisomer thereof: 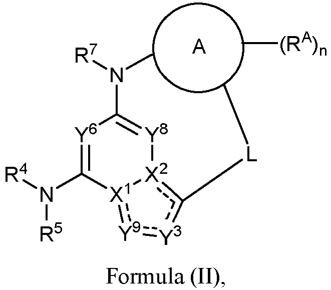 wherein:
L is a 4-10 atom optionally substituted linker;
Ring A is optionally substituted cycloalkyl, optionally substituted heterocycloalkyl, optionally substituted aryl or optionally substituted heteroaryl;
-̅-̅-̅ is a single bond or a double bond;
X¹ and X² are -N- or -C=; provided that one of X¹ or X² is -N- and the other is -C=;
Y⁸ is CR⁸ or N;
Y⁶ is CR⁶ or N;
Y³ is CR³ or N;
Y⁹ is CR⁹ or N;
R³, R⁶, R⁸, and R⁹ are independently hydrogen, deuterium, halogen, -CN, -OR^{b}, -SR^{b}, -S(=O)R^{a}, - S(=O)₂R^{a}, -NO₂, -NR^{c}R^{d}, -NHS(=O)₂R^{a}, -S(=O)₂NR^{c}R^{d}, -C(=O)R^{a}, -OC(=O)R^{a}, -C(=O)OR^{b}, - OC(=O)OR^{b}, -C(=O)NR^{c}R^{d}, -OC(=O)NR^{c}R^{d}, -NR^{b}C(=O)NR^{c}R^{d}, -NR^{b}C(=O)R^{a}, -NR^{b}C(=O)OR^{b}, optionally substituted C₁-C₆alkyl, optionally substituted C₁-C₆haloalkyl, optionally substituted C₁-C₆deuteroalkyl, optionally substituted C₁-C₆hydroxyalkyl, optionally substituted C₁-C₆aminoalkyl, optionally substituted C₂-C₆alkenyl, or optionally substituted C₂-C₆alkynyl;
R⁴ is hydrogen, optionally substituted C₁-C₆alkyl, optionally substituted C₁-C₆heteroalkyl, optionally substituted C₁-C₆haloalkyl, optionally substituted C₁-C₆deuteroalkyl, optionally substituted C₁-C₆hydroxyalkyl, optionally substituted C₁-C₆aminoalkyl, optionally substituted C₂-C₆alkenyl, optionally substituted C₂-C₆alkynyl, optionally substituted cycloalkyl, optionally substituted heterocycloalkyl, optionally substituted aryl, or optionally substituted heteroaryl;
R⁵ is hydrogen, optionally substituted C₁-C₆alkyl, optionally substituted C₁-C₆haloalkyl, or optionally substituted C₁-C₆deuteroalkyl;
R⁷ is hydrogen, optionally substituted C₁-C₆alkyl, optionally substituted C₁-C₆haloalkyl, or optionally substituted C₁-C₆deuteroalkyl;
each R^{a} is independently C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OMe, - NH₂, -C(=O)Me, -C(=O)OH, -C(=O)OMe, C₁-C₆alkyl, or C₁-C₆haloalkyl;
each R^{b} is independently hydrogen, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OMe, - NH₂, -C(=O)Me, -C(=O)OH, -C(=O)OMe, C₁-C₆alkyl, or C₁-C₆haloalkyl; and
each R^{c} and R^{d} is independently hydrogen, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OMe, -NH₂, -C(=O)Me, -C(=O)OH, -C(=O)OMe, C₁-C₆alkyl, or C₁-C₆haloalkyl;
or R^{c} and R^{d} are taken together with the nitrogen atom to which they are attached to form a heterocycloalkyl optionally substituted with one or more oxo, deuterium, halogen, -CN, -OH, -OMe, -NH₂, -C(=O)Me, -C(=O)OH, -C(=O)OMe, C₁-C₆alkyl, or C₁-C₆haloalkyl.

Also disclosed herein is a compound of Formula (II), or a pharmaceutically acceptable salt, solvate, or stereoisomer thereof: 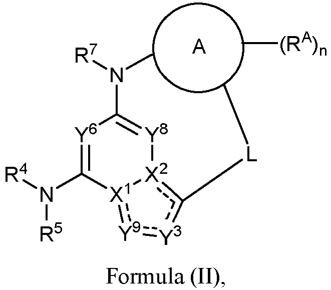 wherein:
L is a 4-10 atom linker; optionally substituted with one or more R^{L};
each R^{L} is independently deuterium, halogen, -CN, -OR^{b}, -SR^{b}, -S(=O)R^{a}, -S(=O)₂R^{a}, -NO₂, -NR^{c}R^{d}, - NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, - OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{L} on the same carbon are taken together to form an oxo, a cycloalkyl, or heterocycloalkyl; or two R^{L} on different carbons are taken together to form a cycloalkyl or heterocycloalkyl;
Ring A is cycloalkyl, heterocycloalkyl, aryl, or heteroaryl;
each R^{A} is independently deuterium, halogen, -CN, -OR^{b}, -SR^{b}, -S(=O)R^{a}, -S(=O)₂R^{a}, -NO₂, -NR^{c}R^{d}, - NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, - OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more R^{A1}; or two R^{A} on the same carbon are taken together to form an oxo;
each R^{A1} is independently deuterium, halogen, -CN, -OR^{b}, -SR^{b}, -S(=O)R^{a}, -S(=O)₂R^{a}, -NO₂, -NR^{c}R^{d}, - NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, - OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{A1} on the same carbon are taken together to form an oxo;
n is 0-4;
-̅ -̅ -̅ is a single bond or a double bond;
X¹ and X² are -N- or -C=; provided that one of X¹ or X² is -N- and the other is -C=;
Y⁸ is CR⁸ or N;
Y⁶ is CR⁶ or N;
Y³ is CR³ or N;
Y⁹ is CR⁹ or N;
R³, R⁶, R⁸, and R⁹ are independently hydrogen, deuterium, halogen, -CN, -OR^{b}, -SR^{b}, -S(=O)R^{a}, - S(=O)₂R^{a}, -NO₂, -NR^{c}R^{d}, -NHS(=O)₂R^{a}, -S(=O)₂NR^{c}R^{d}, -C(=O)R^{a}, -OC(=O)R^{a}, -C(=O)OR^{b}, - OC(=O)OR^{b}, -C(=O)NR^{c}R^{d}, -OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, or C₂-C₆alkynyl;
R⁴ is hydrogen, C₁-C₆alkyl, C₁-C₆heteroalkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl are optionally substituted with one or more R^{4a};
each R^{4a} is independently deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; or two R^{4a} on the same carbon are taken together to form an oxo;
R⁵ is hydrogen, C₁-C₆alkyl, C₁-C₆haloalkyl, or C₁-C₆deuteroalkyl;
R⁷ is hydrogen, C₁-C₆alkyl, C₁-C₆haloalkyl, or C₁-C₆deuteroalkyl;
each R^{a} is independently C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OMe, - NH₂, -C(=O)Me, -C(=O)OH, -C(=O)OMe, C₁-C₆alkyl, or C₁-C₆haloalkyl;
each R^{b} is independently hydrogen, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OMe, - NH₂, -C(=O)Me, -C(=O)OH, -C(=O)OMe, C₁-C₆alkyl, or C₁-C₆haloalkyl; and
each R^{c} and R^{d} is independently hydrogen, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OMe, -NH₂, -C(=O)Me, -C(=O)OH, -C(=O)OMe, C₁-C₆alkyl, or C₁-C₆haloalkyl;
or R^{c} and R^{d} are taken together with the nitrogen atom to which they are attached to form a heterocycloalkyl optionally substituted with one or more oxo, deuterium, halogen, -CN, -OH, -OMe, -NH₂, -C(=O)Me, -C(=O)OH, -C(=O)OMe, C₁-C₆alkyl, or C₁-C₆haloalkyl.

Also disclosed herein is a compound of Formula (II), or a pharmaceutically acceptable salt, solvate, or stereoisomer thereof: 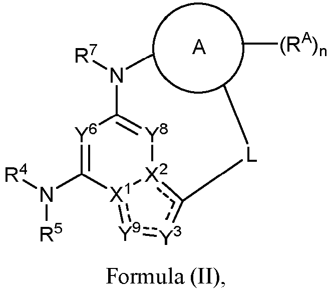 wherein:
L is a 4-10 atom linker; optionally substituted with one or more R^{L};
each R^{L} is independently deuterium, halogen, -CN, -OR^{b}, -SR^{b}, -S(=O)R^{a}, -S(=O)₂R^{a}, -NO₂, -NR^{c}R^{d}, - NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, - OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{L} on the same carbon are taken together to form an oxo, a cycloalkyl, or heterocycloalkyl; or two R^{L} on adjacent carbons are taken together to form a cycloalkyl or heterocycloalkyl;
Ring A is cycloalkyl, heterocycloalkyl, aryl, or heteroaryl;
each R^{A} is independently deuterium, halogen, -CN, -OR^{b}, -SR^{b}, -S(=O)R^{a}, -S(=O)₂R^{a}, -NO₂, -NR^{c}R^{d}, - NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, - OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more R^{A1}; or two R^{A} on the same carbon are taken together to form an oxo;
each R^{A1} is independently deuterium, halogen, -CN, -OR^{b}, -SR^{b}, -S(=O)R^{a}, -S(=O)₂R^{a}, -NO₂, -NR^{c}R^{d}, - NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, - OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{A1} on the same carbon are taken together to form an oxo;
n is 0-4;
-̅ -̅ -̅ is a single bond or a double bond;
X¹ and X² are -N- or -C=; provided that one of X¹ or X² is -N- and the other is -C=;
Y⁸ is CR⁸ or N;
Y⁶ is CR⁶ or N;
Y³ is CR³ or N;
Y⁹ is CR⁹ or N;
R³, R⁶, R⁸, and R⁹ are independently hydrogen, deuterium, halogen, -CN, -OR^{b}, -SR^{b}, -S(=O)R^{a}, - S(=O)₂R^{a}, -NO₂, -NR^{c}R^{d}, -NHS(=O)₂R^{a}, -S(=O)₂NR^{c}R^{d}, -C(=O)R^{a}, -OC(=O)R^{a}, -C(=O)OR^{b}, - OC(=O)OR^{b}, -C(=O)NR^{c}R^{d}, -OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, or C₂-C₆alkynyl;
R⁴ is hydrogen, C₁-C₆alkyl, C₁-C₆heteroalkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl are optionally substituted with one or more R^{4a};
each R^{4a} is independently deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; or two R^{4a} on the same carbon are taken together to form an oxo;
R⁵ is hydrogen, C₁-C₆alkyl, C₁-C₆haloalkyl, or C₁-C₆deuteroalkyl;
R⁷ is hydrogen, C₁-C₆alkyl, C₁-C₆haloalkyl, or C₁-C₆deuteroalkyl;
each R^{a} is independently C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OMe, - NH₂, -C(=O)Me, -C(=O)OH, -C(=O)OMe, C₁-C₆alkyl, or C₁-C₆haloalkyl;
each R^{b} is independently hydrogen, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OMe, - NH₂, -C(=O)Me, -C(=O)OH, -C(=O)OMe, C₁-C₆alkyl, or C₁-C₆haloalkyl; and
each R^{c} and R^{d} is independently hydrogen, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OMe, -NH₂, -C(=O)Me, -C(=O)OH, -C(=O)OMe, C₁-C₆alkyl, or C₁-C₆haloalkyl;
or R^{c} and R^{d} are taken together with the nitrogen atom to which they are attached to form a heterocycloalkyl optionally substituted with one or more oxo, deuterium, halogen, -CN, -OH, -OMe, -NH₂, -C(=O)Me, -C(=O)OH, -C(=O)OMe, C₁-C₆alkyl, or C₁-C₆haloalkyl.

In some embodiments of a compound of Formula (II), or a pharmaceutically acceptable salt, stereoisomer, or solvate thereof, the compound is of Formula (IIa):

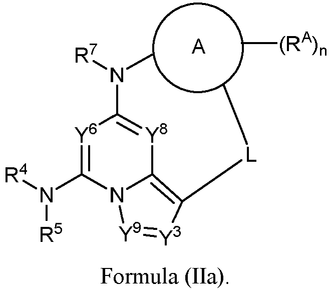

In some embodiments of a compound of Formula (II), or a pharmaceutically acceptable salt, stereoisomer, or solvate thereof, the compound is of Formula (IIb):

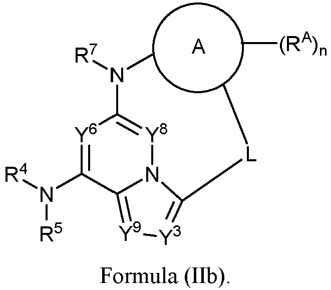

In some embodiments of a compound of Formula (II), (IIa), or (IIb), Y⁹ is N. In some embodiments of a compound of Formula (II), Y⁹ is CR⁹.

In some embodiments of a compound of Formula (II), (IIa), or (IIb), Y⁸ is N. In some embodiments of a compound of Formula (II), Y⁸ is CR⁸.

In some embodiments of a compound of Formula (II), (IIa), or (IIb), or a pharmaceutically acceptable salt, stereoisomer, or solvate thereof, the compound is of Formula (IIc):

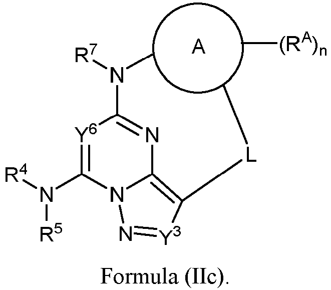

In some embodiments of a compound of Formula (II), (IIa), or (IIb), or a pharmaceutically acceptable salt, stereoisomer, or solvate thereof, the compound is of Formula (IId):

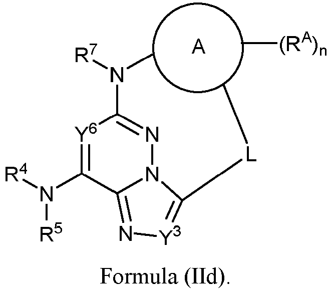

In some embodiments of a compound of Formula (II) or (IIa)-(IId), Y⁶ is CR⁶. In some embodiments of a compound of Formula (II), Y⁶ is N.

In some embodiments of a compound of Formula (II) or (IIa)-(IId), Y³ is CR³. In some embodiments of a compound of Formula (II), Y³ is N.

In some embodiments of a compound of Formula (II) or (IIa)-(IId), Ring A is heterocycloalkyl, aryl, or heteroaryl. In some embodiments of a compound of Formula (II) or (IIa)-(IId), Ring A is heterocycloalkyl. In some embodiments of a compound of Formula (II) or (IIa)-(IId), Ring A is aryl or heteroaryl. In some embodiments of a compound of Formula (II) or (IIa)-(IId), Ring A is heteroaryl. In some embodiments of a compound of Formula (II) or (IIa)-(IId), Ring A is aryl. In some embodiments of a compound of Formula (II) or (IIa)-(IId), Ring A is phenyl, pyridyl, pyrimidyl, pyrazinyl, or pyridazinyl. In some embodiments of a compound of Formula (II) or (IIa)-(IId), Ring A is a bicyclic heteroaryl. In some embodiments of a compound of Formula (II) or (IIa)-(IId), Ring A is indole, indazole, benzimidazole, benzotriazole, benzofuran, benzothiazole, benzoisothiazole, benzoxazole, benzoisoxazole, or benzothiophene.

In some embodiments of a compound of Formula (II) or (IIa)-(IId), n is 0-3. In some embodiments of a compound of Formula (II) or (IIa)-(IId), n is 0-2. In some embodiments of a compound of Formula (II) or (IIa)-(IId), n is 0 or 1. In some embodiments of a compound of Formula (II) or (IIa)-(IId), n is 0. In some embodiments of a compound of Formula (II) or (IIa)-(IId), n is 1. In some embodiments of a compound of Formula (II) or (IIa)-(IId), n is 2. In some embodiments of a compound of Formula (II) or (IIa)-(IId), n is 3. In some embodiments of a compound of Formula (II) or (IIa)-(IId), n is 4.

In some embodiments of a compound of Formula (II) or (IIa)-(IId), each R^{A} is independently deuterium, halogen, -CN, -OR^{b}, -SR^{b}, -S(=O)R^{a}, -S(=O)₂R^{a}, -NO₂, -NR^{c}R^{d}, -NHS(=O)₂R^{a}, - S(=O)₂NR^{c}R^{d}, -C(=O)R^{a}, -OC(=O)R^{a}, -C(=O)OR^{b}, -OC(=O)OR^{b}, -C(=O)NR^{c}R^{d}, -OC(=O)NR^{c}R^{d}, - NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more R^{A1}; or two R^{A} on the same carbon are taken together to form an oxo.

In some embodiments of a compound of Formula (II) or (IIa)-(IId), each R^{A} is independently deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; wherein each alkyl, cycloalkyl, heterocycloalkyl, aryl, and heteroaryl is independently optionally substituted with one or more R^{A1}; or two R^{A} on the same carbon are taken together to form an oxo.

In some embodiments of a compound of Formula (II) or (IIa)-(IId), each R^{A} is independently deuterium, halogen, -CN, -OR^{b}, -NR^{c}R^{d}, C₁-C₆alkyl, C₁-C₆haloalkyl, C₁-C₆deuteroalkyl, cycloalkyl, heterocycloalkyl, aryl, or heteroaryl; wherein each alkyl, cycloalkyl, heterocycloalkyl, aryl, and heteroaryl is independently optionally substituted with one or more R^{A1}; or two R^{A} on the same carbon are taken together to form an oxo.

In some embodiments of a compound of Formula (II) or (IIa)-(IId), each R^{A} is independently deuterium, halogen, -CN, -OR^{b}, -NR^{c}R^{d}, -C(=O)R^{a}, C₁-C₆alkyl, C₁-C₆haloalkyl, or C₁-C₆deuteroalkyl; or two R^{A} on the same carbon are taken together to form an oxo.

In some embodiments of a compound of Formula (II) or (IIa)-(IId), each R^{A} is independently deuterium, halogen, -CN, -OR^{b}, C₁-C₆alkyl, C₁-C₆haloalkyl, or C₁-C₆deuteroalkyl; or two R^{A} on the same carbon are taken together to form an oxo.

In some embodiments of a compound of Formula (II) or (IIa)-(IId), each R^{A} is independently deuterium, halogen, -OR^{b}, or C₁-C₆alkyl; or two R^{A} on the same carbon are taken together to form an oxo.

In some embodiments of a compound of Formula (II) or (IIa)-(IId), each R^{A} is independently deuterium, halogen, -CN, -OR^{b}, -NR^{c}R^{d}, -C(=O)R^{a}, C₁-C₆alkyl, C₁-C₆haloalkyl, or C₁-C₆deuteroalkyl.

In some embodiments of a compound of Formula (II) or (IIa)-(IId), each R^{A} is independently deuterium, halogen, -CN, -OR^{b}, C₁-C₆alkyl, C₁-C₆haloalkyl, or C₁-C₆deuteroalkyl.

In some embodiments of a compound of Formula (II) or (IIa)-(IId), each R^{A} is independently deuterium, halogen, -OR^{b}, or C₁-C₆alkyl.

In some embodiments of a compound of Formula (II) or (IIa)-(IId), each R^{A1} is independently deuterium, halogen, -CN, -OR^{b}, -SR^{b}, -S(=O)R^{a}, -S(=O)₂R^{a}, -NO₂, -NR^{c}R^{d}, -NHS(=O)₂R^{a}, - S(=O)₂NR^{c}R^{d}, -C(=O)R^{a}, -OC(=O)R^{a}, -C(=O)OR^{b}, -OC(=O)OR^{b}, -C(=O)NR^{c}R^{d}, -OC(=O)NR^{c}R^{d}, - NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{A1} on the same carbon are taken together to form an oxo.

In some embodiments of a compound of Formula (II) or (IIa)-(IId), each R^{A1} is independently deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; or two R^{A1} on the same carbon are taken together to form an oxo.

In some embodiments of a compound of Formula (II) or (IIa)-(IId), each R^{A1} is independently deuterium, halogen, -CN, -OR^{b}, -NR^{c}R^{d}, C₁-C₆alkyl, C₁-C₆haloalkyl, C₁-C₆deuteroalkyl, cycloalkyl, or heterocycloalkyl; or two R^{A1} on the same carbon are taken together to form an oxo.

In some embodiments of a compound of Formula (II) or (IIa)-(IId), R³, R⁶, R⁸, and R⁹ are independently hydrogen, deuterium, halogen, -CN, -OR^{b}, -SR^{b}, -S(=O)R^{a}, -S(=O)₂R^{a}, -NO₂, -NR^{c}R^{d}, - NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, - OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, or C₂-C₆alkynyl. In some embodiments of a compound of Formula (II) or (IIa)-(IId), R³, R⁶, R⁸, and R⁹ are independently hydrogen, deuterium, halogen, -CN, -OR^{b}, -NR^{c}R^{d}, -C(=O)NR^{c}R^{d}, C₁-C₆alkyl, C₁-C₆haloalkyl, or C₁-C₆deuteroalkyl. In some embodiments of a compound of Formula (II) or (IIa)-(IId), R³, R⁶, R⁸, and R⁹ are independently hydrogen, deuterium, halogen, or C₁-C₆alkyl. In some embodiments of a compound of Formula (II) or (IIa)-(IId), R³, R⁶, R⁸, and R⁹ are hydrogen.

In some embodiments of a compound of Formula (II) or (IIa)-(IId), R⁴ is hydrogen, C₁-C₆alkyl, C₁-C₆heteroalkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl are optionally substituted with one or more R^{4a}.

In some embodiments of a compound of Formula (II) or (IIa)-(IId), R⁴ is hydrogen, C₁-C₆alkyl, C₁-C₆heteroalkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; wherein each alkyl, cycloalkyl, heterocycloalkyl, aryl, and heteroaryl are optionally substituted with one or more R^{4a}.

In some embodiments of a compound of Formula (II) or (IIa)-(IId), R⁴ is hydrogen, C₁-C₆alkyl, C₁-C₆heteroalkyl, C₁-C₆haloalkyl, C₁-C₆deuteroalkyl, cycloalkyl, or heterocycloalkyl; wherein each alkyl, cycloalkyl, or heterocycloalkyl are optionally substituted with one or more R^{4a}.

In some embodiments of a compound of Formula (II) or (IIa)-(IId), R⁴ is hydrogen, C₁-C₆alkyl, C₁-C₆haloalkyl, or C₁-C₆deuteroalkyl.

In some embodiments of a compound of Formula (II) or (IIa)-(IId), R⁴ is hydrogen, C₁-C₆alkyl, or C₁-C₆deuteroalkyl.

In some embodiments of a compound of Formula (II) or (IIa)-(IId), R⁴ is hydrogen or C₁-C₆alkyl optionally substituted with one or more R^{4a}. In some embodiments of a compound of Formula (II) or (IIa)-(IId), R⁴ is C₁-C₆alkyl optionally substituted with one or more R^{4a}.

In some embodiments of a compound of Formula (II) or (IIa)-(IId), R⁴ is hydrogen or C₁-C₆alkyl. In some embodiments of a compound of Formula (II) or (IIa)-(IId), R⁴ is C₁-C₆alkyl.

In some embodiments of a compound of Formula (II) or (IIa)-(IId), each R^{4a} is independently deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; or two R^{4a} on the same carbon are taken together to form an oxo.

In some embodiments of a compound of Formula (II) or (IIa)-(IId), each R^{4a} is independently deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, cycloalkyl, or heterocycloalkyl; or two R^{4a} on the same carbon are taken together to form an oxo.

In some embodiments of a compound of Formula (II) or (IIa)-(IId), each R^{4a} is independently deuterium, halogen, -CN, -OR^{b}, -NR^{c}R^{d}, C₁-C₆alkyl, C₁-C₆haloalkyl, C₁-C₆deuteroalkyl, cycloalkyl, or heterocycloalkyl; or two R^{4a} on the same carbon are taken together to form an oxo.

In some embodiments of a compound of Formula (II) or (IIa)-(IId), R⁵ is hydrogen, C₁-C₆alkyl, C₁-C₆haloalkyl, or C₁-C₆deuteroalkyl. In some embodiments of a compound of Formula (II) or (IIa)-(IId), R⁵ is hydrogen or C₁-C₆alkyl. In some embodiments of a compound of Formula (II) or (IIa)-(IId), R⁵ is hydrogen.

In some embodiments of a compound of Formula (II) or (IIa)-(IId), R⁷ is hydrogen, C₁-C₆alkyl, C₁-C₆haloalkyl, or C₁-C₆deuteroalkyl. In some embodiments of a compound of Formula (II) or (IIa)-(IId), R⁷ is hydrogen or C₁-C₆alkyl. In some embodiments of a compound of Formula (II) or (IIa)-(IId), R⁷ is hydrogen.

In some embodiments of a compound of Formula (II) or (IIa)-(IId), L is an a C₂₋₁₀ alkylene chain optionally substituted with one or more R^{L}, wherein up to four carbon atoms of L are optionally and independently replaced by -NR^{L}-, -S-, -O-, -OC(=O)-, -C(=O)O-, -C(=O)-, -C(=O)NR^{L}-, - NR^{L}C(=O)-, -S(=O)₂NR^{L}-, -NR^{L}S(=O)₂-, -NR^{L}C(=O)NR^{L}-, -S(O)-, or -S(O)₂-.

In some embodiments of a compound of Formula (II) or (IIa)-(IId), L is an a C₂₋₁₀ alkylene chain optionally substituted with one or more R^{L}, wherein up to four carbon atoms of L are optionally and independently replaced by -NR^{L}-, -S-, -O-, -C(=O)-, -S(O)-, or -S(O)₂-.

In some embodiments of a compound of Formula (II) or (IIa)-(IId), L is an a C₂₋₁₀ alkylene chain optionally substituted with one or more R^{L}, wherein up to four carbon atoms of L are optionally and independently replaced by -NR^{L}-, -O-, or -C(=O)-.

In some embodiments of a compound of Formula (II) or (IIa)-(IId), L is a 4-10 atom linker; optionally substituted with one or more R^{L}.

In some embodiments of a compound of Formula (II) or (IIa)-(IId), L is a 4-8 atom linker; optionally substituted with one or more R^{L}.

In some embodiments of a compound of Formula (II) or (IIa)-(IId), L is a 4-6 atom linker; optionally substituted with one or more R^{L}.

In some embodiments of a compound of Formula (II) or (IIa)-(IId), L is a 4-10 atom linker comprising between 4 and 10 carbons and between 0 and 4 heteroatoms selected from oxygen and nitrogen; the linker being optionally substituted with one or more R^{L}.

In some embodiments of a compound of Formula (II) or (IIa)-(IId), L is a 4-10 atom linker comprising between 3 and 9 carbons and between 1 and 2 heteroatoms selected from oxygen and nitrogen; the linker being optionally substituted with one or more R^{L}.

In some embodiments of a compound of Formula (II) or (IIa)-(IId), L is a 4-8 atom linker comprising between 4 and 8 carbons and between 0 and 4 heteroatoms selected from oxygen and nitrogen; the linker being optionally substituted with one or more R^{L}.

In some embodiments of a compound of Formula (II) or (IIa)-(IId), L is a 4-8 atom linker comprising between 3 and 7 carbons and between 1 and 2 heteroatoms selected from oxygen and nitrogen; the linker being optionally substituted with one or more R^{L}.

In some embodiments of a compound of Formula (II) or (IIa)-(IId), L is a 4-6 atom linker comprising between 4 and 6 carbons and between 0 and 4 heteroatoms selected from oxygen and nitrogen; the linker being optionally substituted with one or more R^{L}.

In some embodiments of a compound of Formula (II) or (IIa)-(IId), L is a 4-6 atom linker comprising between 3 and 5 carbons and between 1 and 2 heteroatoms selected from oxygen and nitrogen; the linker being optionally substituted with one or more R^{L}.

In some embodiments of a compound of Formula (II) or (IIa)-(IId), each R^{L} is independently deuterium, halogen, C₁-C₆alkyl, C₁-C₆haloalkyl, or C₁-C₆deuteroalkyl; or two R^{L} on the same carbon are taken together to form an oxo or a cycloalkyl; or two R^{L} on different carbons are taken together to form a cycloalkyl.

In some embodiments of a compound of Formula (II) or (IIa)-(IId), each R^{L} is independently deuterium, halogen, -CN, -OR^{b}, -NR^{c}R^{d}, C₁-C₆alkyl, C₁-C₆haloalkyl, or C₁-C₆deuteroalkyl; or two R^{L} on the same carbon are taken together to form an oxo.

In some embodiments of a compound of Formula (II) or (IIa)-(IId), each R^{L} is independently deuterium, halogen, C₁-C₆alkyl, C₁-C₆haloalkyl, or C₁-C₆deuteroalkyl; or two R^{L} on the same carbon are taken together to form an oxo.

In some embodiments of a compound of Formula (II) or (IIa)-(IId), each R^{L} is independently deuterium, halogen, or C₁-C₆alkyl; or two R^{L} on the same carbon are taken together to form an oxo.

In some embodiments of a compound of Formula (II) or (IIa)-(IId), each R^{L} is independently deuterium or halogen; or two R^{L} on the same carbon are taken together to form an oxo.

In some embodiments of a compound of Formula (II) or (IIa)-(IId), L is 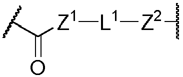 or 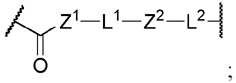 wherein Z¹ and Z² are independently -O-, -S-, or -NR^{Z}; each R^{Z} is independently hydrogen or C₁-C₆alkyl; and L¹ and L² are independently C₁-C₆ alkylene optionally substituted with one or more R^{L}. In the presently claimed invention, L is 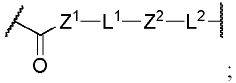 wherein Z¹ and Z² are independently -O-, -S-, or -NR^{Z}; each R^{Z} is independently hydrogen or C₁-C₆alkyl; and L¹ and L² are independently C₁-C₆ alkylene optionally substituted with one or more R^{L}.

In some embodiments, 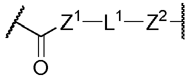 is equivalent to 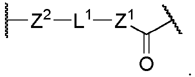 In some embodiments, 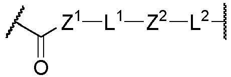 is equivalent to

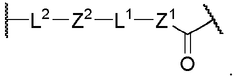

In some embodiments of a compound of Formula (II) or (IIa)-(IId), Z¹ and Z² are independently -O- or -NR^{Z}; each R^{Z} is independently hydrogen or C₁-C₆alkyl. In some embodiments of a compound of Formula (II) or (IIa)-(IId), Z¹ and Z² are independently -O- or -NR^{Z}; each R^{Z} is hydrogen.

In some embodiments of a compound of Formula (II) or (IIa)-(IId), L¹ and L² are independently C₁-C₃ alkylene optionally substituted with one or more R^{L}.

In some embodiments of a compound of Formula (II) or (IIa)-(IId), L is

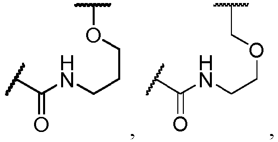

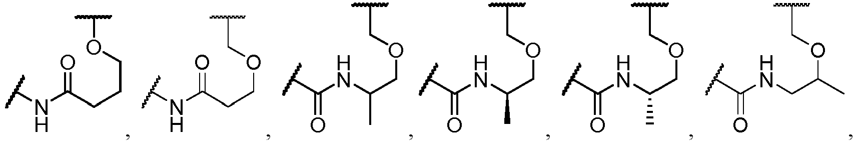

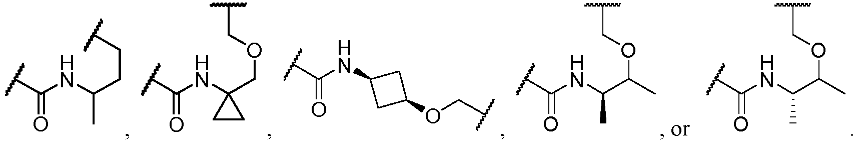

In some embodiments of a compound of Formula (II) or (IIa)-(IId), L is

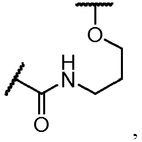

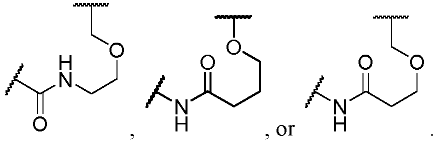

In some embodiments of a compound of Formula (II) or (IIa)-(IId), L is 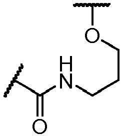 or

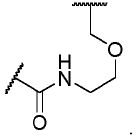

In some embodiments of a compound described above, each R^{a} is independently C₁-C₆alkyl, C₁-C₆haloalkyl, C₁-C₆deuteroalkyl, or cycloalkyl; wherein each alkyl and cycloalkyl is independently optionally substituted with one or more oxo, deuterium, halogen, -CN, -OH, -OMe, -NH₂, -C(=O)Me, - C(=O)OH, -C(=O)OMe, C₁-C₆alkyl, or C₁-C₆haloalkyl. In some embodiments of a compound described above, each R^{a} is independently C₁-C₆alkyl, C₁-C₆haloalkyl, or C₁-C₆deuteroalkyl. In some embodiments of a compound described above, each R^{a} is independently C₁-C₆alkyl.

In some embodiments of a compound described above, each R^{b} is independently hydrogen, C₁-C₆alkyl, C₁-C₆haloalkyl, C₁-C₆deuteroalkyl, or cycloalkyl; wherein each alkyl and cycloalkyl is independently optionally substituted with one or more oxo, deuterium, halogen, -CN, -OH, -OMe, -NH₂, -C(=O)Me, -C(=O)OH, -C(=O)OMe, C₁-C₆alkyl, or C₁-C₆haloalkyl. In some embodiments of a compound described above, each R^{b} is independently hydrogen, C₁-C₆alkyl, C₁-C₆haloalkyl, or C₁-C₆deuteroalkyl. In some embodiments of a compound described above, each R^{b} is independently hydrogen or C₁-C₆alkyl. In some embodiments of a compound described above, each R^{b} is hydrogen. In some embodiments of a compound described above, each R^{b} is independently C₁-C₆alkyl.

In some embodiments of a compound described above, each R^{c} and R^{d} is independently hydrogen, C₁-C₆alkyl, C₁-C₆haloalkyl, C₁-C₆deuteroalkyl, or cycloalkyl; wherein each alkyl and cycloalkyl is independently optionally substituted with one or more oxo, deuterium, halogen, -CN, -OH, - OMe, -NH₂, -C(=O)Me, -C(=O)OH, -C(=O)OMe, C₁-C₆alkyl, or C₁-C₆haloalkyl. In some embodiments of a compound described above, each R^{c} and R^{d} is independently hydrogen, C₁-C₆alkyl, C₁-C₆haloalkyl, or C₁-C₆deuteroalkyl. In some embodiments of a compound described above, each R^{c} and R^{d} is independently hydrogen or C₁-C₆alkyl. In some embodiments of a compound described above, each R^{c} and R^{d} is hydrogen. In some embodiments of a compound described above, each R^{c} and R^{d} is independently C₁-C₆alkyl.

In some embodiments of a compound of Formula (II) or (IIa)-(IId), each L, R^{L}, R^{A}, R⁴, R^{a}, R^{b}, R^{c}, and R^{d} is independently substituted with one, two, three, or four substituents as defined herein. In some embodiments of a compound of Formula (II) or (IIa)-(IId), each L, R^{L}, R^{A}, R⁴, R^{a}, R^{b}, R^{c}, and R^{d} is independently optionally substituted with one, two, or three substituents as defined herein. In some embodiments of a compound of Formula (II) or (IIa)-(IId), each L, R^{L}, R^{A}, R⁴, R^{a}, R^{b}, R^{c}, and R^{d} is independently optionally substituted with one or two substituents as defined herein. In some embodiments of a compound of Formula (II) or (IIa)-(IId), each L, R^{L}, R^{A}, R⁴, R^{a}, R^{b}, R^{c}, and R^{d} is independently optionally substituted with one substituent as defined herein.

In some embodiments of a compound of Formula (II) or (IIa)-(IId), the compound is:

In some embodiments of a compound of Formula (II) or (IIa)-(IId), the compound is:

| **Ex.** | **Structure** | **Ex.** | **Structure** |
|---|---|---|---|
| **1** | 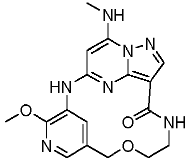 | **32** | 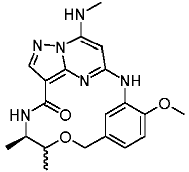 |
| **2** | 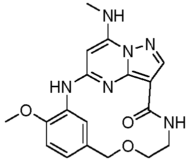 | **33** | 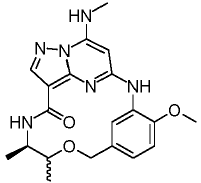 |
| **3** | 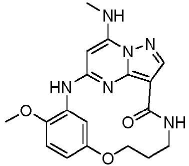 | **34** | 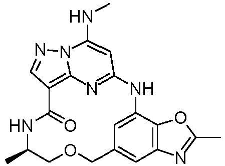 |
| **4** | 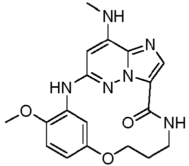 | **35** | 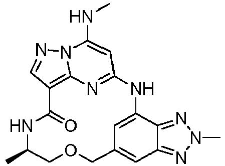 |
| **5** | 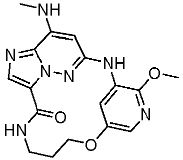 | **36** | 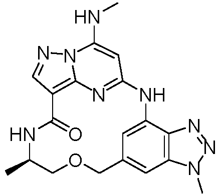 |
| **6** | 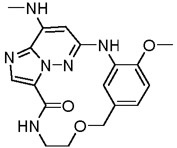 | **37** | 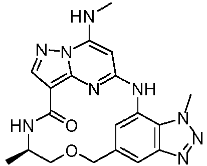 |
| **7** | 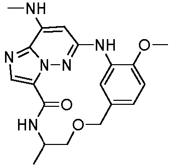 | **38** | 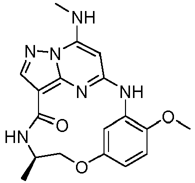 |
| **8** | 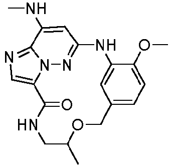 | **40** | 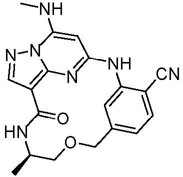 |
| **9** | 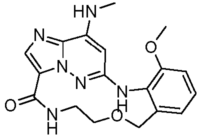 | **41** | 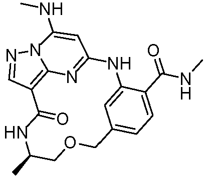 |
| **10** | 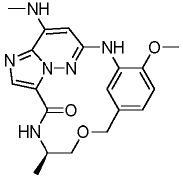 | **42** | 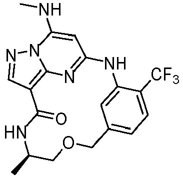 |
| **11** | 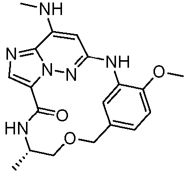 | **43** | 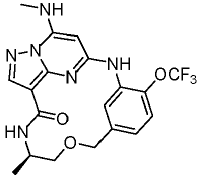 |
| **12** | 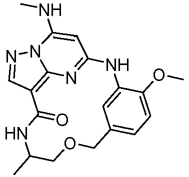 | **44** | 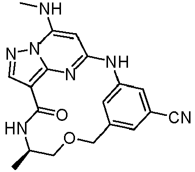 |
| **13** | 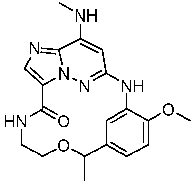 | **45** | 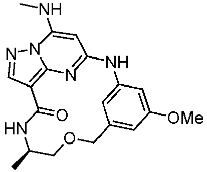 |
| **14** | 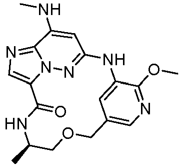 | **46** | 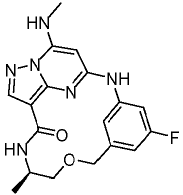 |
| **15** | 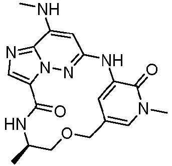 | **47** | 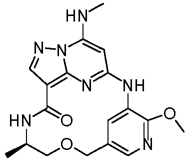 |
| **16** | 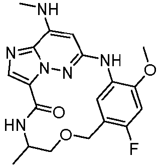 | **48** | 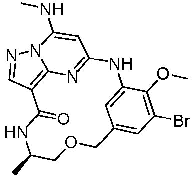 |
| **17** | 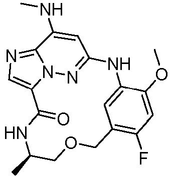 | **49** | 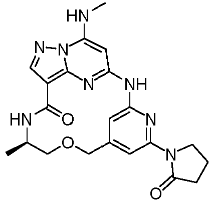 |
| **18** | 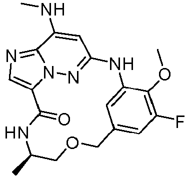 | **50** | 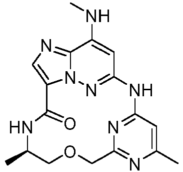 |
| **19** | 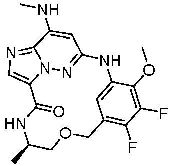 | **51** | 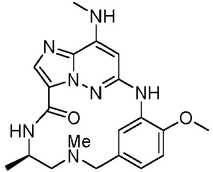 |
| **20** | 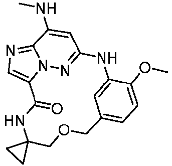 | **52** | 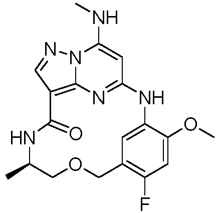 |
| **21** | 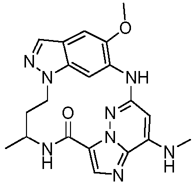 | **53** | 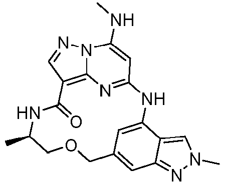 |
| **22** | 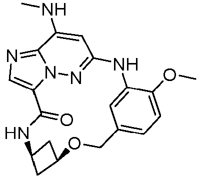 | **54** | 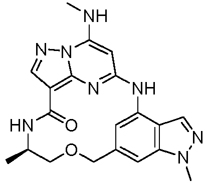 |
| **24** | 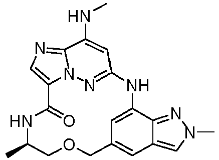 | **55** | 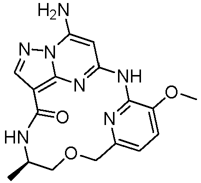 |
| **25** | 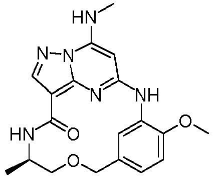 | **56** | 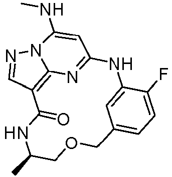 |
| **26** | 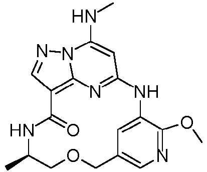 | **57** | 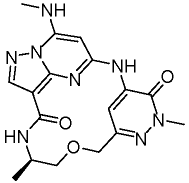 |
| **27** | 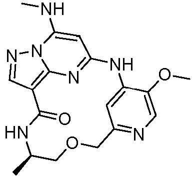 | **58** | 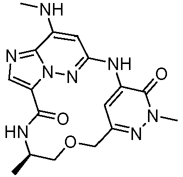 |
| **28** | 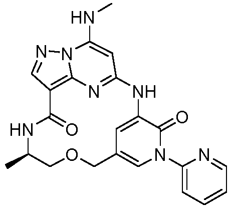 | **59** | 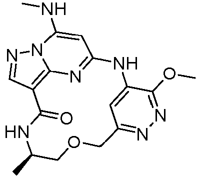 |
| **29** | 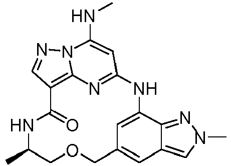 | **60** | 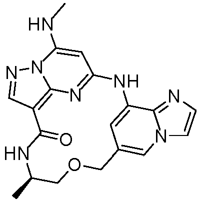 |
| **30** | 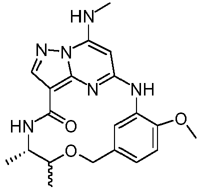 | **62** | 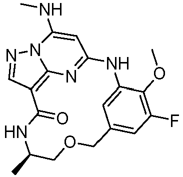 |
| **31** | 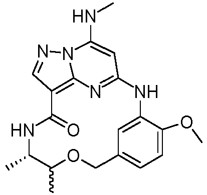 | | |

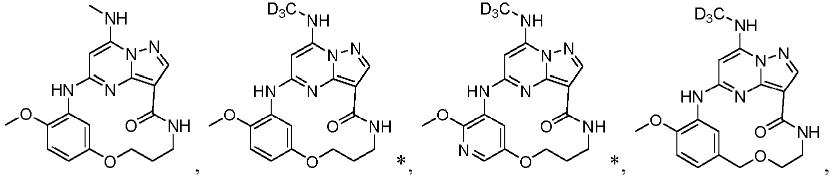

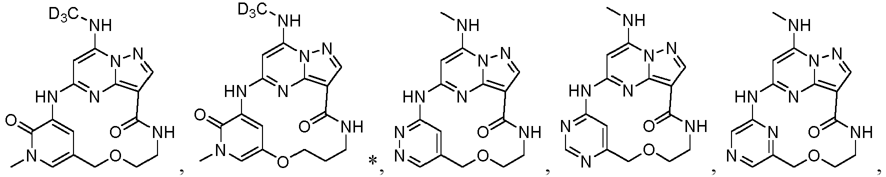

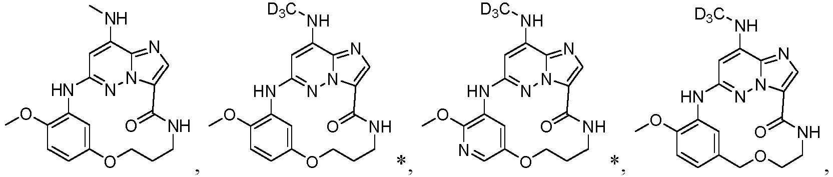

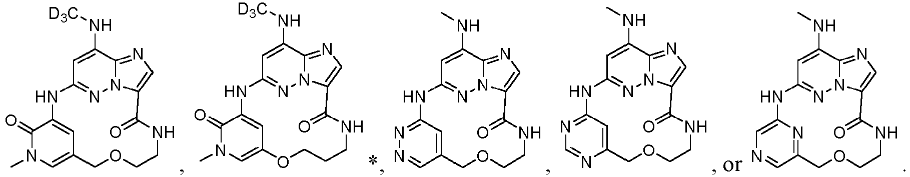

Compounds marked with a * are described herein, but not presently claimed.

In some embodiments of a compound of Formula (II) or (IIa)-(Iid), the compound is: 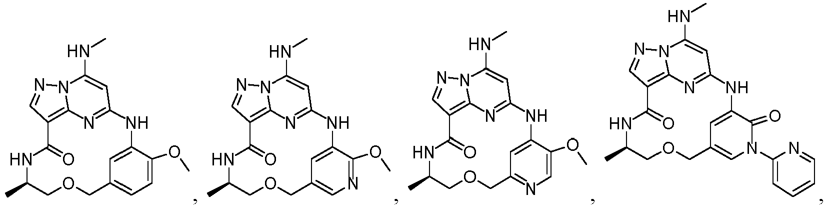 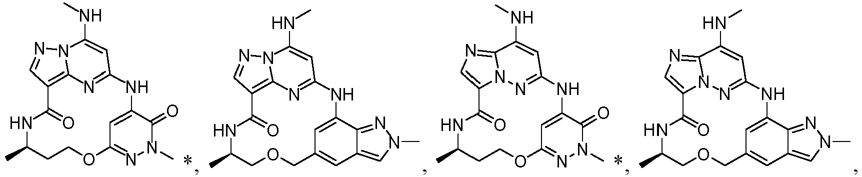 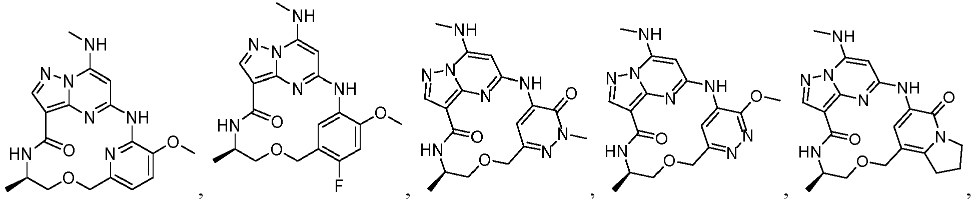 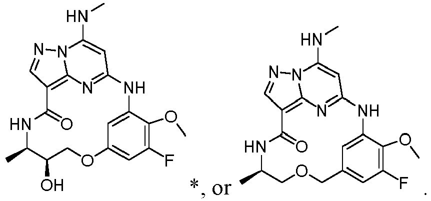 Compounds marked with a * are described herein, but not presently claimed.

### Further Forms of Compounds Disclosed Herein

### Isomers/Stereoisomers

In some embodiments, the compounds described herein exist as geometric isomers. In some embodiments, the compounds described herein possess one or more double bonds. The compounds presented herein include all cis, trans, syn, anti, entgegen (E), and zusammen (Z) isomers as well as the corresponding mixtures thereof. In some situations, the compounds described herein possess one or more chiral centers and each center exists in the R configuration or S configuration. The compounds described herein include all diastereomeric, enantiomeric, and epimeric forms as well as the corresponding mixtures thereof. In additional embodiments of the compounds and methods provided herein, mixtures of enantiomers and/or diastereoisomers, resulting from a single preparative step, combination, or interconversion are useful for the applications described herein. In some embodiments, the compounds described herein are prepared as their individual stereoisomers by reacting a racemic mixture of the compound with an optically active resolving agent to form a pair of diastereoisomeric compounds, separating the diastereomers, and recovering the optically pure enantiomers. In some embodiments, dissociable complexes are preferred. In some embodiments, the diastereomers have distinct physical properties (e.g., melting points, boiling points, solubilities, reactivity, etc.) and are separated by taking advantage of these dissimilarities. In some embodiments, the diastereomers are separated by chiral chromatography, or preferably, by separation/resolution techniques based upon differences in solubility. In some embodiments, the optically pure enantiomer is then recovered, along with the resolving agent.

### Labeled compounds

In some embodiments, the compounds described herein exist in their isotopically-labeled forms. In some embodiments, the methods disclosed herein include methods of treating diseases by administering such isotopically-labeled compounds. In some embodiments, the methods disclosed herein include methods of treating diseases by administering such isotopically-labeled compounds as pharmaceutical compositions. Thus, in some embodiments, the compounds disclosed herein include isotopically-labeled compounds, which are identical to those recited herein, but for the fact that one or more atoms are replaced by an atom having an atomic mass or mass number different from the atomic mass or mass number usually found in nature. Examples of isotopes that can be incorporated into compounds described herein, or a solvate, or stereoisomer thereof, include isotopes of hydrogen, carbon, nitrogen, oxygen, phosphorous, sulfur, fluorine, and chloride, such as ²H, ³H, ¹³C, ¹⁴C, ¹⁵ N, ¹⁸O, ¹⁷O, ³¹P, ³²P, ³⁵S, ¹⁸F, and ³⁶Cl, respectively. Compounds described herein, and the pharmaceutically acceptable salts, solvates, or stereoisomers thereof which contain the aforementioned isotopes and/or other isotopes of other atoms are within the scope of this disclosure. Certain isotopically-labeled compounds, for example those into which radioactive isotopes such as ³H and ¹⁴C are incorporated, are useful in drug and/or substrate tissue distribution assays. Tritiated, i.e., ³H and carbon-14, i.e., ¹⁴C, isotopes are particularly preferred for their ease of preparation and detectability. Further, substitution with heavy isotopes such as deuterium, *i.e.,* ²H, produces certain therapeutic advantages resulting from greater metabolic stability, for example increased in vivo half-life or reduced dosage requirements. In some embodiments, the isotopically labeled compound or a pharmaceutically acceptable salt, solvate, or stereoisomer thereof is prepared by any suitable method.

In some embodiments, the compounds described herein are labeled by other means, including, but not limited to, the use of chromophores or fluorescent moieties, bioluminescent labels, or chemiluminescent labels.

### Pharmaceutically acceptable salts

In some embodiments, the compounds described herein exist as their pharmaceutically acceptable salts. In some embodiments, the methods disclosed herein include methods of treating diseases by administering such pharmaceutically acceptable salts. In some embodiments, the methods disclosed herein include methods of treating diseases by administering such pharmaceutically acceptable salts as pharmaceutical compositions.

In some embodiments, the compounds described herein possess acidic or basic groups and therefor react with any of a number of inorganic or organic bases, and inorganic and organic acids, to form a pharmaceutically acceptable salt. In some embodiments, these salts are prepared *in situ* during the final isolation and purification of the compounds disclosed herein, or by separately reacting a purified compound in its free form with a suitable acid or base, and isolating the salt thus formed.

Examples of pharmaceutically acceptable salts include those salts prepared by reaction of the compounds described herein with a mineral, organic acid, or inorganic base, such salts including acetate, acrylate, adipate, alginate, aspartate, benzoate, benzenesulfonate, bisulfate, bisulfite, bromide, butyrate, butyn-1,4-dioate, camphorate, camphorsulfonate, caproate, caprylate, chlorobenzoate, chloride, citrate, cyclopentanepropionate, decanoate, digluconate, dihydrogenphosphate, dinitrobenzoate, dodecylsulfate, ethanesulfonate, formate, fumarate, glucoheptanoate, glycerophosphate, glycolate, hemisulfate, heptanoate, hexanoate, hexyne-1,6-dioate, hydroxybenzoate, γ-hydroxybutyrate, hydrochloride, hydrobromide, hydroiodide, 2-hydroxyethanesulfonate, iodide, isobutyrate, lactate, maleate, malonate, methanesulfonate, mandelate metaphosphate, methanesulfonate, methoxybenzoate, methylbenzoate, monohydrogenphosphate, 1-napthalenesulfonate, 2-napthalenesulfonate, nicotinate, nitrate, palmoate, pectinate, persulfate, 3-phenylpropionate, phosphate, picrate, pivalate, propionate, pyrosulfate, pyrophosphate, propiolate, phthalate, phenylacetate, phenylbutyrate, propanesulfonate, salicylate, succinate, sulfate, sulfite, succinate, suberate, sebacate, sulfonate, tartrate, thiocyanate, tosylateundeconate, and xylenesulfonate.

Further, the compounds described herein can be prepared as pharmaceutically acceptable salts formed by reacting the free base form of the compound with a pharmaceutically acceptable inorganic or organic acid, including, but not limited to, inorganic acids such as hydrochloric acid, hydrobromic acid, sulfuric acid, nitric acid, phosphoric acid metaphosphoric acid, and the like; and organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, p-toluenesulfonic acid, tartaric acid, trifluoroacetic acid, citric acid, benzoic acid, 3-(4-hydroxybenzoyl)benzoic acid, cinnamic acid, mandelic acid, arylsulfonic acid, methanesulfonic acid, ethanesulfonic acid, 1,2-ethanedisulfonic acid, 2-hydroxyethanesulfonic acid, benzenesulfonic acid, 2-naphthalenesulfonic acid, 4-methylbicyclo-[2.2.2]oct-2-ene-1-carboxylic acid, glucoheptonic acid, 4,4'-methylenebis-(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, and muconic acid.

In some embodiments, those compounds described herein which comprise a free acid group react with a suitable base, such as the hydroxide, carbonate, bicarbonate, or sulfate of a pharmaceutically acceptable metal cation, with ammonia, or with a pharmaceutically acceptable organic primary, secondary, tertiary, or quaternary amine. Representative salts include the alkali or alkaline earth salts, like lithium, sodium, potassium, calcium, and magnesium, and aluminum salts and the like. Illustrative examples of bases include sodium hydroxide, potassium hydroxide, choline hydroxide, sodium carbonate, N⁺(C₁₋₄ alkyl)₄, and the like.

Representative organic amines useful for the formation of base addition salts include ethylamine, diethylamine, ethylenediamine, ethanolamine, diethanolamine, piperazine, and the like. It should be understood that the compounds described herein also include the quaternization of any basic nitrogen-containing groups they contain. In some embodiments, water or oil-soluble or dispersible products are obtained by such quaternization.

### Solvates

In some embodiments, the compounds described herein exist as solvates. The disclosure provides for methods of treating diseases by administering such solvates. The disclosure further provides for methods of treating diseases by administering such solvates as pharmaceutical compositions.

Solvates contain either stoichiometric or non-stoichiometric amounts of a solvent, and, in some embodiments, are formed during the process of crystallization with pharmaceutically acceptable solvents such as water, ethanol, and the like. Hydrates are formed when the solvent is water, or alcoholates are formed when the solvent is alcohol. Solvates of the compounds described herein can be conveniently prepared or formed during the processes described herein. In addition, the compounds provided herein can exist in unsolvated as well as solvated forms. In general, the solvated forms are considered equivalent to the unsolvated forms for the purposes of the compounds and methods provided herein.

### Tautomers

In some situations, compounds exist as tautomers. The compounds described herein include all possible tautomers within the formulas described herein. Tautomers are compounds that are interconvertible by migration of a hydrogen atom, accompanied by a switch of a single bond and adjacent double bond. In bonding arrangements where tautomerization is possible, a chemical equilibrium of the tautomers will exist. All tautomeric forms of the compounds disclosed herein are contemplated. The exact ratio of the tautomers depends on several factors, including temperature, solvent, and pH.

### Preparation of the Compounds

The compounds used in the reactions described herein are made according to organic synthesis techniques known to those skilled in this art, starting from commercially available chemicals and/or from compounds described in the chemical literature. "Commercially available chemicals" are obtained from standard commercial sources including Acros Organics (Pittsburgh, PA), Aldrich Chemical (Milwaukee, WI, including Sigma Chemical and Fluka), Apin Chemicals Ltd. (Milton Park, UK), Avocado Research (Lancashire, U.K.), BDH, Inc. (Toronto, Canada), Bionet (Cornwall, U.K.), Chem Service Inc. (West Chester, PA), Crescent Chemical Co. (Hauppauge, NY), Eastman Organic Chemicals, Eastman Kodak Company (Rochester, NY), Fisher Scientific Co. (Pittsburgh, PA), Fisons Chemicals (Leicestershire, UK), Frontier Scientific (Logan, UT), ICN Biomedicals, Inc. (Costa Mesa, CA), Key Organics (Cornwall, U.K.), Lancaster Synthesis (Windham, NH), Maybridge Chemical Co. Ltd. (Cornwall, U.K.), Parish Chemical Co. (Orem, UT), Pfaltz & Bauer, Inc. (Waterbury, CN), Polyorganix (Houston, TX), Pierce Chemical Co. (Rockford, IL), Riedel de Haen AG (Hanover, Germany), Spectrum Quality Product, Inc. (New Brunswick, NJ), TCI America (Portland, OR), Trans World Chemicals, Inc. (Rockville, MD), and Wako Chemicals USA, Inc. (Richmond, VA).

Suitable reference books and treatises that detail the synthesis of reactants useful in the preparation of compounds described herein, or provide references to articles that describe the preparation, include for example, "Synthetic Organic Chemistry", John Wiley & Sons, Inc., New York; S. R. Sandler et al., "Organic Functional Group Preparations," 2nd Ed., Academic Press, New York, 1983; H. O. House, "Modern Synthetic Reactions", 2nd Ed., W. A. Benjamin, Inc. Menlo Park, Calif. 1972; T. L. Gilchrist, "Heterocyclic Chemistry", 2nd Ed., John Wiley & Sons, New York, 1992; J. March, "Advanced Organic Chemistry: Reactions, Mechanisms and Structure", 4th Ed., Wiley-Interscience, New York, 1992. Additional suitable reference books and treatises that detail the synthesis of reactants useful in the preparation of compounds described herein, or provide references to articles that describe the preparation, include for example, Fuhrhop, J. and Penzlin G. "Organic Synthesis: Concepts, Methods, Starting Materials", Second, Revised and Enlarged Edition (1994) John Wiley & Sons ISBN: 3-527-29074-5; Hoffman, R.V. "Organic Chemistry, An Intermediate Text" (1996) Oxford University Press, ISBN 0-19-509618-5; Larock, R. C. "Comprehensive Organic Transformations: A Guide to Functional Group Preparations" 2nd Edition (1999) Wiley-VCH, ISBN: 0-471-19031-4; March, J. "Advanced Organic Chemistry: Reactions, Mechanisms, and Structure" 4th Edition (1992) John Wiley & Sons, ISBN: 0-471-60180-2; Otera, J. (editor) "Modern Carbonyl Chemistry" (2000) Wiley-VCH, ISBN: 3-527-29871-1; Patai, S. "Patai's 1992 Guide to the Chemistry of Functional Groups" (1992) Interscience ISBN: 0-471-93022-9; Solomons, T. W. G. "Organic Chemistry" 7th Edition (2000) John Wiley & Sons, ISBN: 0-471-19095-0; Stowell, J.C., "Intermediate Organic Chemistry" 2nd Edition (1993) Wiley-Interscience, ISBN: 0-471-57456-2; "Industrial Organic Chemicals: Starting Materials and Intermediates: An Ullmann's Encyclopedia" (1999) John Wiley & Sons, ISBN: 3-527-29645-X, in 8 volumes; "Organic Reactions" (1942-2000) John Wiley & Sons, in over 55 volumes; and "Chemistry of Functional Groups" John Wiley & Sons, in 73 volumes.

Specific and analogous reactants are optionally identified through the indices of known chemicals prepared by the Chemical Abstract Service of the American Chemical Society, which are available in most public and university libraries, as well as through on-line. Chemicals that are known but not commercially available in catalogs are optionally prepared by custom chemical synthesis houses, where many of the standard chemical supply houses (e.g., those listed above) provide custom synthesis services. A reference for the preparation and selection of pharmaceutical salts of the compounds described herein is P. H. Stahl & C. G. Wermuth "Handbook of Pharmaceutical Salts", Verlag Helvetica Chimica Acta, Zurich, 2002.

### Pharmaceutical Compositions

In certain embodiments, the compound described herein is administered as a pure chemical. In some embodiments, the compound described herein is combined with a pharmaceutically suitable or acceptable carrier (also referred to herein as a pharmaceutically suitable (or acceptable) excipient, physiologically suitable (or acceptable) excipient, or physiologically suitable (or acceptable) carrier) selected on the basis of a chosen route of administration and standard pharmaceutical practice as described, for example, in Remington: The Science and Practice of Pharmacy (Gennaro, 21st Ed. Mack Pub. Co., Easton, PA (2005)).

Accordingly, provided herein is a pharmaceutical composition comprising a compound described herein, or a pharmaceutically acceptable salt, solvate, or stereoisomer thereof, and a pharmaceutically acceptable excipient.

In certain embodiments, the compound provided herein is substantially pure, in that it contains less than about 5%, or less than about 1%, or less than about 0.1%, of other organic small molecules, such as unreacted intermediates or synthesis by-products that are created, for example, in one or more of the steps of a synthesis method.

Pharmaceutical compositions are administered in a manner appropriate to the disease to be treated (or prevented). An appropriate dose and a suitable duration and frequency of administration will be determined by such factors as the condition of the patient, the type and severity of the patient's disease, the particular form of the active ingredient, and the method of administration. In general, an appropriate dose and treatment regimen provides the composition(s) in an amount sufficient to provide therapeutic and/or prophylactic benefit (e.g., an improved clinical outcome, such as more frequent complete or partial remissions, or longer disease-free and/or overall survival, or a lessening of symptom severity. Optimal doses are generally determined using experimental models and/or clinical trials. The optimal dose depends upon the body mass, weight, or blood volume of the patient.

In some embodiments, the pharmaceutical composition is formulated for oral, topical (including buccal and sublingual), rectal, vaginal, transdermal, parenteral, intrapulmonary, intradermal, intrathecal and epidural and intranasal administration. Parenteral administration includes intramuscular, intravenous, intraarterial, intraperitoneal, or subcutaneous administration. In some embodiments, the pharmaceutical composition is formulated for intravenous injection, oral administration, inhalation, nasal administration, topical administration, or ophthalmic administration. In some embodiments, the pharmaceutical composition is formulated for oral administration. In some embodiments, the pharmaceutical composition is formulated for intravenous injection. In some embodiments, the pharmaceutical composition is formulated as a tablet, a pill, a capsule, a liquid, an inhalant, a nasal spray solution, a suppository, a suspension, a gel, a colloid, a dispersion, a suspension, a solution, an emulsion, an ointment, a lotion, an eye drop, or an ear drop. In some embodiments, the pharmaceutical composition is formulated as a tablet.

Suitable doses and dosage regimens are determined by conventional range-finding techniques known to those of ordinary skill in the art. Generally, treatment is initiated with smaller dosages that are less than the optimum dose of the compound disclosed herein. Thereafter, the dosage is increased by small increments until the optimum effect under the circumstances is reached. In some embodiments, the present method involve the administration of about 0.1 µg to about 50 mg of at least one compound described herein per kg body weight of the subject. For a 70 kg patient, dosages of from about 10 µg to about 200 mg of the compound disclosed herein would be more commonly used, depending on a subject's physiological response.

By way of example only, the dose of the compound described herein for methods of treating a disease as described herein is about 0.001 to about 1 mg/kg body weight of the subject per day, for example, about 0.001 mg, about 0.002 mg, about 0.005 mg, about 0.010 mg, 0.015 mg, about 0.020 mg, about 0.025 mg, about 0.050 mg, about 0.075 mg, about 0.1 mg, about 0.15 mg, about 0.2 mg, about 0.25 mg, about 0.5 mg, about 0.75 mg, or about 1 mg/kg body weight per day. In some embodiments, the dose of compound described herein for the described methods is about 1 to about 1000 mg/kg body weight of the subject being treated per day, for example, about 1 mg, about 2 mg, about 5 mg, about 10 mg, about 15 mg, about 20 mg, about 25 mg, about 50 mg, about 75 mg, about 100 mg, about 150 mg, about 200 mg, about 250 mg, about 500 mg, about 750 mg, or about 1000 mg per day.

### Methods of Treatment

The compounds disclosed herein, or pharmaceutically acceptable salts, solvates, or stereoisomers thereof, are useful for the inhibition of kinase activity of one or more enzymes. In some embodiments the kinase inhibited by the compounds and methods is TYK2.

Provided herein are compounds that are inhibitors of TYK2 and are therefore useful for treating one or more disorders associated with activity of TYK2 or mutants thereof.

Provided herein are methods for treating a disease or disorder, wherein the disease or disorder is an autoimmune disorders, inflammatory disorders, proliferative disorders, endocrine disorders, neurological disorders, or disorders associated with transplantation, said method comprising administering to a patient in need thereof, a pharmaceutical composition comprising an effective amount of a compound described herein, or a pharmaceutically acceptable salt, solvate, or stereoisomer thereof.

In some embodiments, the disease or disorder is an autoimmune disorder. In some embodiments the disease or disorder is selected from type 1 diabetes, systemic lupus erythematosus, multiple sclerosis, psoriasis, Behçet's disease, POEMS syndrome, Crohn's disease, ulcerative colitis, and inflammatory bowel disease.

In some embodiments, the disease or disorder is an inflammatory disorder. In some embodiments, the inflammatory disorder is rheumatoid arthritis, asthma, chronic obstructive pulmonary disease, psoriasis, hepatomegaly, Crohn's disease, ulcerative colitis, inflammatory bowel disease.

In some embodiments, the disease or disorder is a proliferative disorder. In some embodiments, the proliferative disorder is a hematological cancer. In some embodiments the proliferative disorder is a leukemia. In some embodiments, the leukemia is a T-cell leukemia. In some embodiments the T-cell leukemia is T-cell acute lymphoblastic leukemia (T-ALL). In some embodiments the proliferative disorder is polycythemia vera, myelofibrosis, essential or thrombocytosis.

In some embodiments, the disease or disorder is an endocrine disorder. In some embodiments, the endocrine disorder is polycystic ovary syndrome, Crouzon's syndrome, or type 1 diabetes.

In some embodiments, the disease or disorder is a neurological disorder. In some embodiments, the neurological disorder is Alzheimer's disease.

In some embodiments the proliferative disorder is associated with one or more activating mutations in TYK2. In some embodiments, the activating mutation in TYK2 is a mutation to the FERM domain, the JH2 domain, or the kinase domain. In some embodiments the activating mutation in TYK2 is selected from G36D, S47N, R425H, V731I, E957D, and R1027H.

In some embodiments, the disease or disorder is associated with transplantation. In some embodiments the disease or disorder associated with transplantation is transplant rejection, or graft versus host disease.

In some embodiments the disease or disorder is associated with type I interferon, IL-10, IL-12, or IL-23 signaling. In some embodiments the disease or disorder is associated with type I interferon signaling. In some embodiments the disease or disorder is associated with IL-10 signaling. In some embodiments the disorder is associated with IL-12 signaling. In some embodiments the disease or disorder is associated with IL-23 signaling.

Provided herein are methods for treating an inflammatory or allergic condition of the skin, for example psoriasis, contact dermatitis, atopic dermatitis, alopecia areata, erythema multiforma, dermatitis herpetiformis, scleroderma, vitiligo, hypersensitivity angiitis, urticaria, bullous pemphigoid, lupus erythematosus, systemic lupus erythematosus, pemphigus vulgaris, pemphigus foliaceus, paraneoplastic pemphigus, epidermolysis bullosa acquisita, acne vulgaris, and other inflammatory or allergic conditions of the skin.

Provided herein are methods for treating other diseases or conditions, such as diseases or conditions having an inflammatory component, for example, treatment of diseases and conditions of the eye such as ocular allergy, conjunctivitis, keratoconjunctivitis sicca, and vernal conjunctivitis, diseases affecting the nose including allergic rhinitis, and inflammatory disease in which autoimmune reactions are implicated or having an autoimmune component or etiology, including autoimmune hematological disorders (e.g. hemolytic anemia, aplastic anemia, pure red cell anemia and idiopathic thrombocytopenia), systemic lupus erythematosus, rheumatoid arthritis, polychondritis, scleroderma, Wegener granulamatosis, dermatomyositis, chronic active hepatitis, myasthenia gravis, Steven-Johnson syndrome, idiopathic sprue, autoimmune inflammatory bowel disease (e.g. ulcerative colitis and Crohn's disease), irritable bowel syndrome, celiac disease, periodontitis, hyaline membrane disease, kidney disease, glomerular disease, alcoholic liver disease, multiple sclerosis, endocrine opthalmopathy, Grave's disease, sarcoidosis, alveolitis, chronic hypersensitivity pneumonitis, multiple sclerosis, primary biliary cirrhosis, uveitis (anterior and posterior), Sjogren's syndrome, keratoconjunctivitis sicca and vernal keratoconjunctivitis, interstitial lung fibrosis, psoriatic arthritis, systemic juvenile idiopathic arthritis, cryopyrin-associated periodic syndrome, nephritis, vasculitis, diverticulitis, interstitial cystitis, glomerulonephritis (with and without nephrotic syndrome, e.g. including idiopathic nephrotic syndrome or minal change nephropathy), chronic granulomatous disease, endometriosis, leptospiriosis renal disease, glaucoma, retinal disease, ageing, headache, pain, complex regional pain syndrome, cardiac hypertrophy, musclewasting, catabolic disorders, obesity, fetal growth retardation, hyperchlolesterolemia, heart disease, chronic heart failure, mesothelioma, anhidrotic ecodermal dysplasia, Behcet's disease, incontinentia pigmenti, Paget's disease, pancreatitis, hereditary periodic fever syndrome, asthma (allergic and non-allergic, mild, moderate, severe, bronchitic, and exercise-induced), acute lung injury, acute respiratory distress syndrome, eosinophilia, hypersensitivities, anaphylaxis, nasal sinusitis, ocular allergy, silica induced diseases, COPD (reduction of damage, airways inflammation, bronchial hyperreactivity, remodeling or disease progression), pulmonary disease, cystic fibrosis, acid-induced lung injury, pulmonary hypertension, polyneuropathy, cataracts, muscle inflammation in conjunction with systemic sclerosis, inclusion body myositis, myasthenia gravis, thyroiditis, Addison's disease, lichen planus, Type 1 diabetes, or Type 2 diabetes, appendicitis, atopic dermatitis, asthma, allergy, blepharitis, bronchiolitis, bronchitis, bursitis, cervicitis, cholangitis, cholecystitis, chronic graft rejection, colitis, conjunctivitis, Crohn's disease, cystitis, dacryoadenitis, dermatitis, dermatomyositis, encephalitis, endocarditis, endometritis, enteritis, enterocolitis, epicondylitis, epididymitis, fasciitis, fibrositis, gastritis, gastroenteritis, Henoch-Schonlein purpura, hepatitis, hidradenitis suppurativa, immunoglobulin A nephropathy, interstitial lung disease, laryngitis, mastitis, meningitis, myelitis myocarditis, myositis, nephritis, oophoritis, orchitis, osteitis, otitis, pancreatitis, parotitis, pericarditis, peritonitis, pharyngitis, pleuritis, phlebitis, pneumonitis, pneumonia, polymyositis, proctitis, prostatitis, pyelonephritis, rhinitis, salpingitis, sinusitis, stomatitis, synovitis, tendonitis, tonsillitis, ulcerative colitis, uveitis, vaginitis, vasculitis, or vulvitis.

In some embodiments the inflammatory disease is acute and chronic gout, chronic gouty arthritis, psoriasis, psoriatic arthritis, rheumatoid arthritis, Juvenile rheumatoid arthritis, Systemic juvenile idiopathic arthritis (SJIA), Cryopyrin Associated Periodic Syndrome (CAPS), or osteoarthritis.

In some embodiments the inflammatory disease is a Th1 or Th17 mediated disease. In some embodiments the Th17 mediated disease is selected from Systemic lupus erythematosus, Multiple sclerosis, and inflammatory bowel disease (including Crohn's disease or ulcerative colitis).

In some embodiments the inflammatory disease is Sjogren's syndrome, allergic disorders, osteoarthritis, conditions of the eye such as ocular allergy, conjunctivitis, keratoconjunctivitis sicca, vernal conjunctivitis, or diseases affecting the nose such as allergic rhinitis.

### Combination Therapy

In certain instances, the compound described herein, or a pharmaceutically acceptable salt, solvate, or stereoisomer thereof, is administered in combination with a second therapeutic agent.

In some embodiments, the benefit experienced by a patient is increased by administering one of the compounds described herein with a second therapeutic agent (which also includes a therapeutic regimen) that also has therapeutic benefit.

In one specific embodiment, a compound described herein, or a pharmaceutically acceptable salt, solvate, or stereoisomer thereof, is co-administered with a second therapeutic agent, wherein the compound described herein, or a pharmaceutically acceptable salt, solvate, or stereoisomer thereof, and the second therapeutic agent modulate different aspects of the disease, disorder or condition being treated, thereby providing a greater overall benefit than administration of either therapeutic agent alone.

In any case, regardless of the disease, disorder or condition being treated, the overall benefit experienced by the patient is simply additive of the two therapeutic agents or the patient experiences a synergistic benefit.

In certain embodiments, different therapeutically-effective dosages of the compounds disclosed herein will be utilized in formulating a pharmaceutical composition and/or in treatment regimens when the compounds disclosed herein are administered in combination with a second therapeutic agent. Therapeutically-effective dosages of drugs and other agents for use in combination treatment regimens are optionally determined by means similar to those set forth hereinabove for the actives themselves. Furthermore, the methods of prevention/treatment described herein encompasses the use of metronomic dosing, i.e., providing more frequent, lower doses in order to minimize toxic side effects. In some embodiments, a combination treatment regimen encompasses treatment regimens in which administration of a compound described herein, or a pharmaceutically acceptable salt, solvate, or stereoisomer thereof, is initiated prior to, during, or after treatment with a second agent described herein, and continues until any time during treatment with the second agent or after termination of treatment with the second agent. It also includes treatments in which a compound described herein, or a pharmaceutically acceptable salt, solvate, or stereoisomer thereof, and the second agent being used in combination are administered simultaneously or at different times and/or at decreasing or increasing intervals during the treatment period. Combination treatment further includes periodic treatments that start and stop at various times to assist with the clinical management of the patient.

It is understood that the dosage regimen to treat, prevent, or ameliorate the condition(s) for which relief is sought, is modified in accordance with a variety of factors (e.g. the disease, disorder or condition from which the subject suffers; the age, weight, sex, diet, and medical condition of the subject). Thus, in some instances, the dosage regimen actually employed varies and, in some embodiments, deviates from the dosage regimens set forth herein.

For combination therapies described herein, dosages of the co-administered compounds vary depending on the type of co-drug employed, on the specific drug employed, on the disease or condition being treated, and so forth. In additional embodiments, when co-administered with a second therapeutic agent, the compound provided herein is administered either simultaneously with the second therapeutic agent, or sequentially.

In combination therapies, the multiple therapeutic agents (one of which is one of the compounds described herein) are administered in any order or even simultaneously. If administration is simultaneous, the multiple therapeutic agents are, by way of example only, provided in a single, unified form, or in multiple forms (e.g., as a single pill or as two separate pills).

The compounds described herein, or a pharmaceutically acceptable salt, solvate, or stereoisomer thereof, as well as combination therapies, are administered before, during, or after the occurrence of a disease or condition, and the timing of administering the composition containing a compound varies. Thus, in one embodiment, the compounds described herein are used as a prophylactic and are administered continuously to subjects with a propensity to develop conditions or diseases in order to prevent the occurrence of the disease or condition. In another embodiment, the compounds and compositions are administered to a subject during or as soon as possible after the onset of the symptoms. In specific embodiments, a compound described herein is administered as soon as is practicable after the onset of a disease or condition is detected or suspected, and for a length of time necessary for the treatment of the disease. In some embodiments, the length required for treatment varies, and the treatment length is adjusted to suit the specific needs of each subject. For example, in specific embodiments, a compound described herein or a formulation containing the compound is administered for at least 2 weeks, about 1 month to about 5 years.

In some embodiments, the compound of described herein, or a pharmaceutically acceptable salt, solvate, or stereoisomer thereof, is administered in combination with an adjuvant. In one embodiment, the therapeutic effectiveness of one of the compounds described herein is enhanced by administration of an adjuvant (i.e., by itself the adjuvant has minimal therapeutic benefit, but in combination with another therapeutic agent, the overall therapeutic benefit to the patient is enhanced).

### EXAMPLES

### Example 1: General Procedure for synthesis of compound Example 1

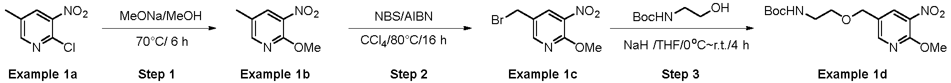

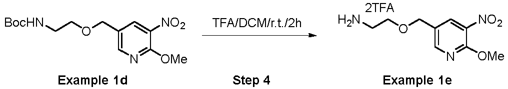

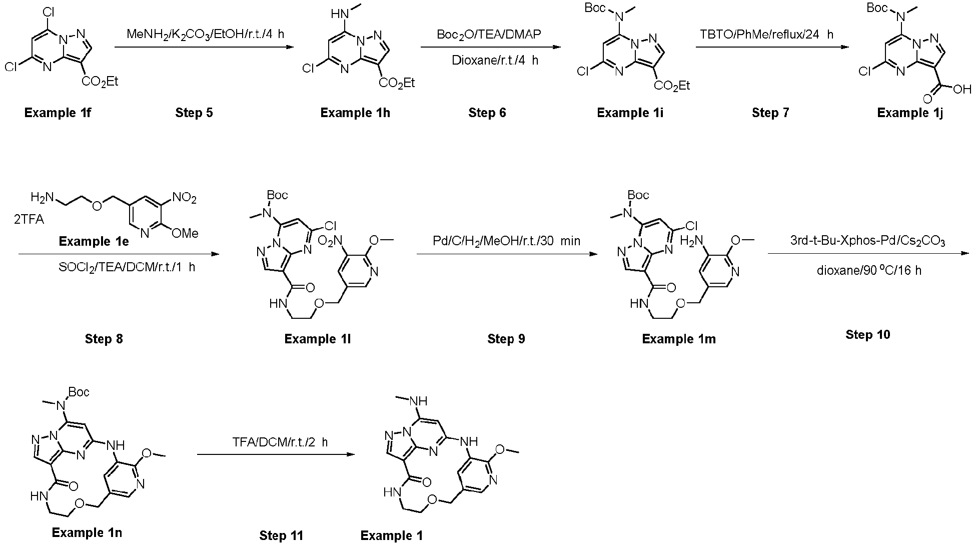

### Step 1: Example 1b

To a solution of Example 1a (11.0 g, 63.74 mmol) in MeOH (200 mL) was added NaOMe (6.88 g, 127.48 mmol). The reaction mixture was stirred at 70°C for 6 h. The mixture was quenched with H₂O (1 L), and a pink precipitate was formed. The solid was collected by filtration and dried in vacuum to afford Example 1b (9.0 g, 84.0% yield) as a pink solid. LCMS [M+1]⁺= 169.1.

### Step 2: Example 1c

To a mixture of Example 1b (5.0 g, 29.76 mmol) in CCl₄ (100 mL) were added NBS (6.36 g, 35.71 mmol) and AIBN (0.98 g, 5.95 mmol). The reaction mixture was stirred at 80°C for 16 h under N₂ protection. After cooled to room temperature, the solvent was removed, and the residue was purified by silica gel flash column chromatography afford the product Example 1c (2.8 g, 38.2% yield) as a yellow solid.

LCMS [M+1]⁺ = 247.1.

### Step 3: Example 1d

To a solution of *tert-butyl* (2-hydroxyethyl)carbamate (1.29 g, 8.02 mmol) in THF (30 mL) was added NaH (320mg, 8.02 mmol) in portions at 0 °C. The mixture was stirred for 30 min at the same temperature, then Example 1c (1.8 g, 7.29 mmol) in THF was added dropwise. The reaction mixture was stirred at r.t. for 4 h. The solvent was removed, and the residue was purified by silica gel flash column chromatography to afford the product Example 1d (600 mg, 25.2% yield) as yellow oil. LCMS [M+1-100]⁺ = 328.2.

### Step 4: Example 1e

To a solution of Example 1d (600 mg, 3.3 mmol) in DCM (5 mL) was added TFA(1 mL). The reaction mixture was stirred at r.t. for 2 h. The solution was concentrated in vacuum to give the crude product Example 1e (710 mg, 113.8%, crude) as yellow oil, which was used to next step directly without purification. LCMS [M+1]⁺ = 228.2.

### Step 5: Example 1h

To a solution of Example 1f (2.0 g, 7.69 mmol) in EtOH (40 mL) were added CH₃NH₂ (7.7 mL, 2M in MeOH, 15.38 mmol) and K₂CO₃(2.12 g, 15.38mmol). The reaction mixture was stirred at r.t. for 4 h. The mixture was poured into H₂O (200 mL), and a white precipitate was formed. The solid was collected by filtration and dried in *vacuo* to afford the product Example 1h (1.85 g, 94.4% yield) as a white solid. LCMS [M+1]⁺ = 255.2.

### Step 6: Example 1i

To a solution of Example 1h (1.85 g, 7.26 mmol) in dioxane (40 mL) were added Boc₂O (1.9 g, 8.72 mmol), TEA (1.09 g, 10.89 mmol) and DMAP (44 mg, 0.36 mmol). The reaction mixture was stirred at r.t. for 4 h. The solvent was removed, and the residue was purified by silica gel flash column chromatography to afford the product Example 1i (2.3 g, 89.3% yield) as an off-white solid. LCMS [M+1]⁺ = 355.2.

### Step 7: Example 1j

To a mixture of Example 1i (800 mg, 2.25 mmol) in toluene (10 mL) was added TBTO (2.96 g, 4.51 mmol). The reaction mixture was stirred at 120°C for 24 h under N₂ protection. After cooled to room temperature, the solvent was removed, and the residue was purified by silica gel flash column chromatography to afford the product Example 1j (620 mg, 84.3% yield) as a yellow solid. LCMS [M+1]⁺ = 327.2.

### Step 8: Example 11

To a solution of Example 1j (500 mg, 1.53 mmol) in DCM (10 mL) was added SOCl₂ (728 mg, 6.12 mmol), and the reaction mixture was stirred at r.t. for 1 h. The solvent was removed, and the residue was diluted with DCM, which was added to a solution of Example 1e (565 mg, 1.53 mmol) and TEA (773 mg, 7.65 mmol) in DCM (10 mL) dropwise at 0°C. The resulting mixture was stirred at r.t. for 1 h, and then concentrated. The residue was purified by silica gel flash column chromatography to afford the product Example 11 (180 mg, 22.0% yield) as an off-white solid. LCMS [M+1]⁺ = 536.3.

### Step 9: Example 1m

To a mixture of Example 11 (160 mg, 0.299 mmol) in MeOH (30 mL) was added Pd/C (16 mg) under N₂ protection. The suspension was degassed under vacuum and purged with H₂, which was stirred at r.t. for 30 min under H₂ balloon. The solid was filtered out, and the filtrate was concentrated. The residue was purified by silica gel flash column chromatography to afford the product Example 1m (82 mg, 54.2% yield) as a yellow solid. LCMS[M+1]⁺ = 506.2.

### Step 10: Example 1n

To a solution of Example 1m (80 mg, 0.16 mmol) in 1,4-dioxane (5 mL) were added Cs₂CO₃ (103 mg, 0.32 mmol) and 3rd-*t*-Bu-Xphos-Pd (14 mg, 0.016 mmol). The reaction mixture was stirred at 90°C for 16 h under N₂. After cooled to room temperature, the solvent was removed, and the residue was purified by silica gel flash column chromatography to afford the product Example 1n (50 mg, 67.4% yield) as an off-white solid. LCMS [M+1]⁺ = 470.2.

### Step 11: Example 1

To a solution of Example 1n (50 mg, 0.11 mmol) in DCM (2 mL) was added TFA (0.5 mL). The reaction mixture was stirred at r.t. for 2 h and then concentrated in vacuum. The residue was purified by prep-HPLC to give the desired product Example 51 (17.0 mg, 43.2% yield) as a white solid. LCMS[M+1] ⁺ = 370.2. ¹H NMR (300 MHz, DMSO-*d*₆) δ 9.14 (s, 1H), 8.57 (d, 1H), 8.14 (s, 1H), 8.06 (brs, 1H), 7.93 (d, 1H), 7.75 (d, 1H), 5.95 (s, 1H), 4.53 (s, 2H), 3.97 (s, 3H), 3.59 (t, 2H), 3.36 - 3.32 (m, 2H), 2.92 (d, 3H).

### Example 2: General Procedure for synthesis of compound Example 2

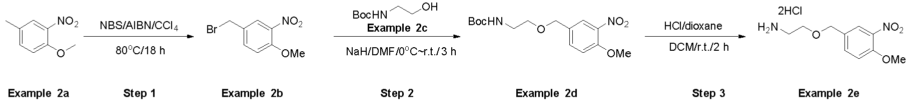

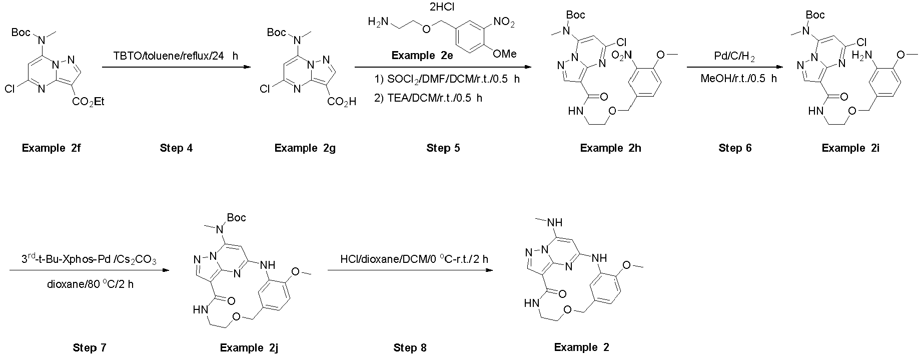

### Step 1: Example 2b

To a solution of Example 2a (10.0 g, 59.8 mmol, 1.0 eq) in CCl₄ (200 mL) were added NBS (10.8 g, 60.4 mmol, 1.01 eq) and AIBN (1.96 g,12.0 mmol, 0.20 eq). The reaction mixture was stirred at 80°C for 18 h under N₂. After cooled to room temperature, the solvent was removed, and the residue was purified by silica gel flash column chromatography to afford the product Example 2b (7.2 g, 49% yield) as a yellow solid.

LCMS [M+1]⁺ = 245.2.

### Step 2: Example 2d

To a solution of Example 2b (1.0 g, 4.06 mmol, 1.0 eq) and Example 2c (720 mg, 4.47 mmol, 1.1 eq) in DMF (20 mL) was added NaH (244 mg, 60% in mineral oil, 6.1 mmol, 1.5 eq) in portions at 0°C. The reaction mixture was stirred for 4 h at r.t., and then poured into a saturated aqueous of NH₄Cl (40 mL), which was extracted with EtOAc (50 mL*3). The combined organic layer was washed with brine, dried over Na₂SO₄, and concentrated. The crude product was purified by silica gel flash column chromatography to afford the product Example 2d (1.2 g, 90% yield) as yellow oil. LCMS [M+1]⁺ = 327.2.

### Step 3: Example 2e

To a solution of Example 2d (800 mg, 2.5 mmol, 1.0 eq) in DCM (8 mL) was added HCl/dioxane (1 mL, 4M in dioxane). The reaction solution was stirred for 2 h at r.t. After completion, the reaction mixture was concentrated to afford the product Example 2e (660 mg, 83% yield) as a yellow solid. LCMS [M+1] ⁺ = 227.2

### Step 4: Example 2g

To a solution of Example 2f (1.0 g, 2.8 mmol, 1.0 eq, from Example **1i**) in toluene (15 mL) was added TBTO (3.3 g, 5.6 mmol, 2.0 eq). The reaction mixture was stirred at reflux for 24 h under N₂. After concentrated, the residue was purified by silica gel flash column chromatography to afford the product Example 2g (800 mg, 82% yield) as a yellow solid. LCMS [M+1]⁺ = 327.2.

### Step 5: Example 2h

To a solution of Example 2g (452 mg, 1.7 mmol, 0.8 eq) in DCM (10 mL) were added SOCl₂ (1.04 g, 8.8 mmol, 4.0 eq) and DMF (0.2 mL), the reaction mixture was stirred for 0.5 h at r.t. After the reaction completed, it was concentrated in *vacuo* to give crude product, which was diluted with DCM and then added to a solution of Example 2e (700 mg, 2.2 mmol, 1.0 eq) and TEA (1.1 g, 11.0 mmol, 5.0 eq) in DCM (10 mL) dropwise at 0°C. The resulting mixture was stirred for 0.5 h at r.t. The solvent was removed, and the residue was purified by silica gel flash column chromatography to afford the product Example 2h (300 mg, 3% yield) as a yellow solid. LCMS [M+1]⁺ = 535.3.

### Step 6: Example 2i

To a solution of Example 2h (170 mg, 0.30 mmol, 1.0 eq) in MeOH (30 mL) was added Pd/C (17 mg). The suspension was degassed under vacuum and purged with H₂, which was stirred for 0.5 h at r.t. under H₂ balloon. The solid was filtered out, and the filtrate was concentrated. The residue was purified by silica gel flash column chromatography to afford the product Example 2i (107 mg, 71% yield) as yellow oil.

LCMS [M+1]⁺ =505.2.

### Step 7: Example 2j

Toa solution of Example 2i (100 mg, 0.20 mmol, 1.0 eq) in dioxane (5 mL) were added Cs₂CO₃ (130.4 mg, 0.40 mmol, 2.0 eq) and 3^{rd}*t*-Bu-Xphos-Pd (17.8 mg, 0.02 mmol, 0.1 eq). The reaction mixture was stirred for 2 h at 80 °C under N₂ protection. The solid was filtered out and filtrate was concentrated, and the residue was purified by Prep-TLC to afford the Example 2j (50 mg, 53% yield) as a yellow solid.

LCMS [M+1] ⁺ =469.3.

### Step 8: Example 2

To a solution of Example 2j (50 mg,0.10 mmol, 1.0 eq) in DCM (2 mL) was added HCl/dioxane (1mL, 4M in THF) dropwise at 0°C. The reaction mixture was stirred for 2 h at r.t. After completion, the reaction mixture was concentrated and the residue was purified by Prep-HPLC to give the desired product Example 2 (17.0 mg, 46% yield) as a light yellow solid. LCMS [M+1] ⁺ = 369.2. ¹H NMR (300 MHz, DMSO-*d*₆) δ 8.91 (s, 1H), 8.32 (d, 1H), 8.22 (brs, 1H), 8.12 (s, 1H), 7.82 (q, 1H), 7.00 (d, 1H), 6.92 (dd, 1H), 5.95 (s, 1H), 4.50 (s, 2H), 3.88 (s, 3H), 3.57 (t, 2H),3.44-3.32 (m, 2H), 2.92 (d, 3H).

### Example 3: General Procedure for synthesis of compound Example 3

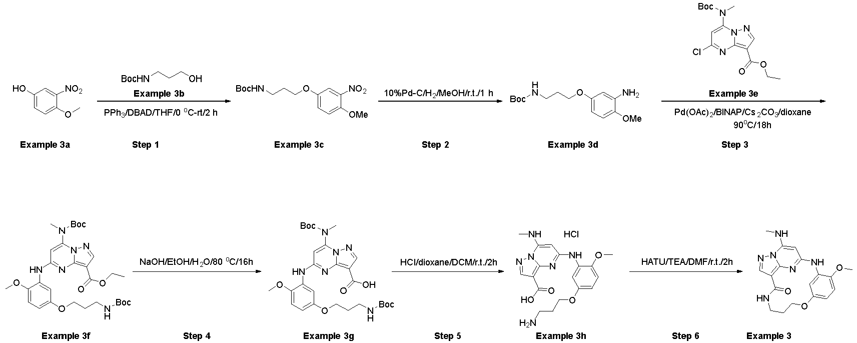

### Step 1: Example 3c

To a solution of Example 3a (2.0 g, 11.69 mmol, 1.0 eq) and Example 3b (2.45 g, 14.03 mmol,1.2 eq) in dry THF (20 mL) were added PPh₃ (3.69 g, 14.03 mmol,1.2 eq) and DBAD (3.22 g, 14.03 mmol,1.2 eq) at 0 °C under N₂, which was stirred for 2 h at r.t. The solvent was removed under vacuum, and the residue was purified by silica gel flash column chromatography to give the desired product Example 3c (2.5 g, 64.9% yield) as a white solid. LCMS [M+1]⁺ = 327.3

### Step 2: Example 3d

To a solution of Example 3c (1.0 g, 3.06 mmol, 1.0 eq) in MeOH (10 mL) was added 10% Pd/C (100 mg) under N₂ protection. The suspension was degassed under vacuum and purged with H₂ three times. The mixture was stirred at r.t. for 1 h under H₂ balloon. The suspension was filtered through a Celite pad and the filter cake was washed with MeOH. The combined filtrates were concentrated in *vacuo* to give the desired product Example 3d (900 mg, 99.1% yield) as colorless oil. LCMS [M+1]⁺ = 297.3

### Step 3: Example 3f

To a mixture of Example 3d (100 mg, 0.28 mmol, 1.0 eq), Example 3e (108.7 mg, 0.40 mmol, 1.5 eq, from Example **1i)** and Cs₂CO₃ (183.6 mg, 0.56 mmol, 2.0 eq) in dioxane (5 mL) were added Pd(OAc)₂ (6.4 mg, 0.028 mmol, 0.1 eq), BINAP (35.1 mg, 0.056 mmol, 0.2 eq). The mixture was degassed with N₂ three times, and stirred for 18 h at 90°C. The reaction was concentrated in *vacuo.* The residue was purified by silica gel flash column chromatography to give the desired product Example 3f (130 mg, 75.9% yield) as a light brown solid. LCMS [M+1]⁺ = 615.4

### Step 4: Example 3g

To a solution of Example 3f (130 mg, 1.78 mmol, 1.0 eq) in EtOH (30 mL) and H₂O (10 mL) was added NaOH (12.7 mg, 1.5mmol, 1.0 eq) at 0 °C. The mixture was stirred for 16 h at 80°C. The solvent was removed to afford the crude product Example 3g (160 mg, quant.) as a white solid. LCMS [M+1]⁺ = 587.4

### Step 5: Example 3h

To a solution of Example 3g (160 mg, 0.27 mmol,1.0 eq) in MeOH (2 mL) was added HCl/dioxane (1.0 mL, 4M in dioxane), which was stirred at r.t. for 2 h. The mixture was concentrated, and the residue was treated with EtOAc (30 mL) to give the crude product Example 3h (150 mg, quant.) as a white solid. LCMS [M+1]⁺ = 387.4.

### Step 6: Example 3

To a solution of Example 3h (crude, 135 mg, 0.30 mmol, 1.0 eq), DIEA (196.7 mg, 1.52 mmol, 5.0 eq) in DMF (10 mL) was added HATU (138.6 mg, 0.37 mmol, 1.2 eq). The mixture was stirred at r.t. for 1 h. EtOAc (40 mL)was added to the reaction mixture, which was washed with brine (20 mL*2), dried over Na₂SO₄ and concentrated. The residue was purified by Prep-HPLC to afford the desired product Example 3 (3.3 mg, 3.1% yield) as a white solid. LCMS [M+1]⁺ = 369.1. ¹H NMR (300 MHz, DMSO-*d*₆) δ 8.87 (s, 1H), 8.44 (s, 1H), 8.25-8.11 (m, 2H), 7.88 (s, 1H), 6.98 (s, 1H), 6.51 (s, 1H), 6.07 (s, 1H), 4.32-4.21 (m, 2H), 3.84 (s, 3H), 3.22-3.15 (m, 2H), 2.92 (s, 3H), 2.02-1.81(m, 2H).

### Example 4: General Procedure for synthesis of compound Example 4

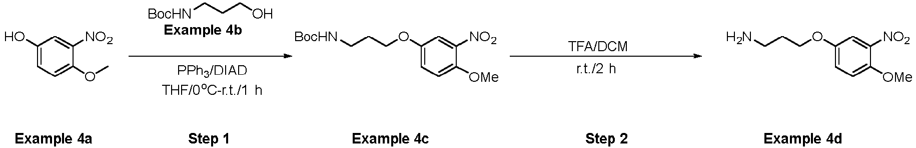

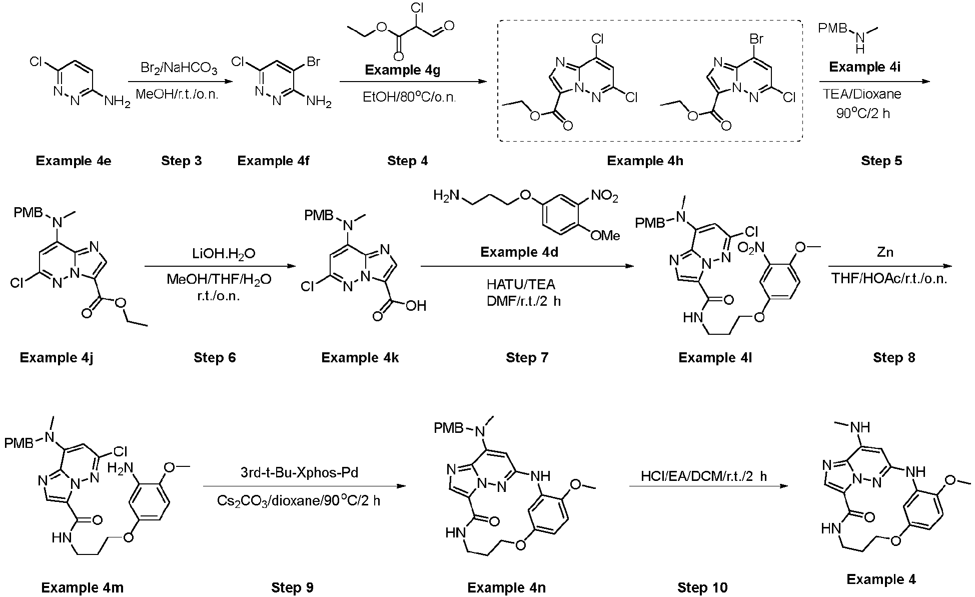

### Step 1: Example 4c

To a solution of Example 4a (1 g, 5.92 mmol) in THF (10 mL) were added Example 4b (1.04 g, 5.92 mmol) and PPh₃ (1.86 g, 7.1 mmol). The mixture was cooled to 0°C and DIAD (1.4 g, 7.1 mmol) was added dropwise. The resulting mixture was stirred at room temperature for 1 h under N₂. The reaction mixture was extracted with EtOAc (50 mL*2). The combined organic phase was washed with brine, dried over Na₂SO₄, filtrated and the filtrate was concentrated under reduced pressure. The residue was purified by silica gel chromatography to give Example 4c (3 g, crude) as yellow oil.

LCMS [M+1-100]⁺ = 227.1

### Step 2: Example 4d

To a solution of Example 4c (crude, 3 g, 9.2 mmol) in DCM (20 mL) was added TFA (10 mL). The mixture was stirred at room temperature for 2 h. The reaction mixture was concentrated. The residue was extracted with DCM (50 mL*2) and H₂O. The aqueous layer was alkalization with NaHCO₃ and extracted with DCM (50 mL*2). The combined organic phase was washed with brine, dried over Na₂SO₄, filtrated and the filtrate was concentrated under reduced pressure to give Example 4d (700 mg, yield 34%) as yellow oil. LCMS [M+1] ⁺ = 227.1. ¹H NMR (400 MHz, Chloroform-d) δ 7.39 (d, 1H), 7.10 (dd, 1H), 7.01 (d, 1H), 4.04 (t, 2H), 3.90 (s, 3H), 2.91 (t, 2H), 1.92 (p, 2H).

### Step 3: Example 4f

To a solution of Example 4e (10 g, 77.5 mmol) in MeOH (100 mL) was added NaHCO₃ (13 g, 155.0 mmol) at 0°C. Then Br₂ (18.6 g, 116.3 mmol) was added dropwise and the resulting mixture was stirred at room temperature overnight. One half of the volume of solvent was removed under reduced pressure. The remaining was poured into ice water. The solid formed was collected and dried to give Example 4f (14.5 g, yield 90%) as a red solid. LCMS [M+1] ⁺ = 209.9. ¹H NMR (400 MHz, DMSO-*d*₆) δ7.98 (s, 1H), 6.96 (s, 2H).

### Step 4: Example 4h

To a solution of Example 4f (14.5 g, 69.7 mmol) in EtOH (100 mL) was added Example 4g (16.7 g, 111.5 mmol). The mixture was stirred at 80°C overnight under N₂. The reaction mixture was concentrated. The residue was purified by silica gel chromatography to give Example 4h (7 g, yield 39%) (Brominated 36% & Chlorinated 64%) as a yellow solid. LCMS [M+1] ⁺ = 260.0/306.0. ¹H NMR (400 MHz, Chloroform-d) δ 8.37 (d, 1H), 7.57 (s, 0.36 H), 7.38 (s, 0.64H), 4.46 (q, 2H), 1.43 (t, 3H).

### Step 5: Example 4j

To a solution of Example 4h (640 mg, 2.46 mmol) in dioxane (6 mL) were added Example 4i (409 mg, 2.71 mmol) and TEA (497 mg, 4.92 mmol). The mixture was stirred at 90°C for 2 h under N₂. The reaction mixture was concentrated to provide a sludge that was triturated with H₂O (5 mL) to provide a solid which was filtered, rinsed with H₂O and then collected with DCM (20 mL). The solution was dried over Na₂SO₄, filtrated and the filtrate was concentrated under reduced pressure to give Example 4j (880 mg, yield 82%) as a yellow solid. LCMS [M+1] ⁺ = 375.1. ¹H NMR (400 MHz, Chloroform-d) δ 8.10 (s, 1H), 7.15 (d, 2H), 6.85 (d, 2H), 6.10 (s, 1H), 5.48 (s, 2H), 4.43 (q, 2H), 3.78 (s, 3H), 3.16 (s, 3H), 1.41 (t, 3H).

### Step 6: Example 4k

To a solution of Example 4j (680 mg, 1.81 mmol) in THF/MeOH/H₂O (9 mL/9 mL/6 mL) was added LiOH.H₂O (305 mg, 7.25 mmol). The mixture was stirred at room temperature overnight. The THF/MeOH were removed in vacuum, the resulting solution was adjusted to pH = 4 using 1M HCl. The mixture was extracted with DCM (30 mL*2). The combined organic phase was washed with brine, dried over Na₂SO₄, filtrated and the filtrate was concentrated under reduced pressure to give Example 4k (760 mg, yield 93%) as a yellow solid. LCMS [M+1] ⁺ = 347.1.

### Step 7: Example 4l

To a solution of Example 4k (660 mg, 1.9 mmol) in DMF (6 mL) were added Example 4d (430 mg, 1.9 mmol), TEA (576 mg, 5.7 mmol) and HATU (867 mg, 2.28 mmol). The mixture was stirred at room temperature for 2 h under N₂. The reaction mixture was extracted with EtOAc (30 mL*2). The combined organic phase was washed with brine, dried over Na₂SO₄, filtrated and the filtrate was concentrated under reduced pressure. The residue was purified by silica gel chromatography to give Example 4l (960 mg, yield 87%) as a yellow solid. LCMS [M+1] ⁺ = 555.2.

### Step 8: Example 4m

To a solution of Example 4l (30 mg, 0.054 mmol) in THF/HOAc (0.5 mL/0.05 mL) was added Zn (35 mg, 0.54 mmol). The mixture was stirred at room temperature overnight. The reaction mixture was alkalization with a.q NaHCO₃ and extracted with DCM (10 mL*2). The combined organic phase was washed with brine, dried over Na₂SO₄, filtrated and the filtrate was concentrated under reduced pressure to give Example 4m (30 mg, crude) as yellow oil. LCMS [M+1] ⁺ = 525.2.

### Step 9: Example 4n

To a solution of Example 4m (30 mg, 0.06mmol) in dioxane (2 mL) were added Cs₂CO₃ (37 mg, 0.11 mmol) and 3^{rd}-*t*Bu-Xphos-Pd (5 mg, 0.006 mmol). The mixture was stirred at 90°C for 2 h under N₂. The reaction mixture was extracted with DCM (10 mL*2). The combined organic phase was washed with brine, dried over Na₂SO₄, filtrated and the filtrate was concentrated under reduced pressure to give Example 4n (40 mg, crude) as yellow oil. LCMS [M+1] ⁺ = 489.2.

### Step 10: Example 4

To a solution of Example 4n (crude, 0.06 mmol) in DCM (1 mL) was added HCl/EtOAc (0.3 mL). The mixture was stirred at room temperature for 2 h. The mixture was concentrated under reduced pressure. The residue was purified by prep-HPLC to give Example 4 (5.1 mg, yield 23%) as a yellow solid. LCMS [M+1]⁺ = 369.1. ¹H NMR (400 MHz, Chloroform-d) δ 8.82 (s, 1H), 8.10-8.07 (m, 2H), 7.00 (s, 1H), 6.87 (d, 1H), 6.65 (s, 1H), 6.57 (dd, 1H), 5.66 (s, 1H), 4.33 (t, 2H), 3.91 (s, 3H), 3.50-3.44 (m, 2H), 3.06 (d, 3H), 2.12-2.03 (m, 2H).

### Example 5:

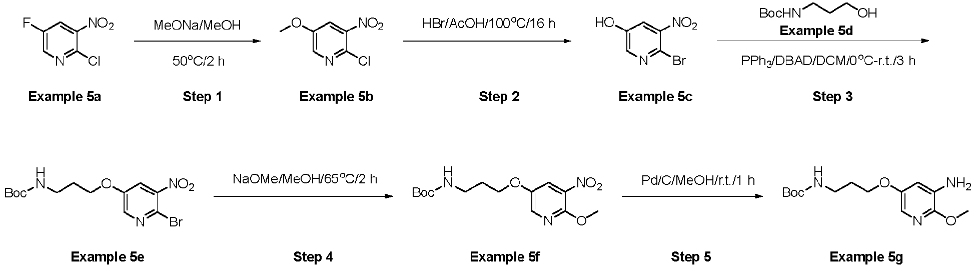

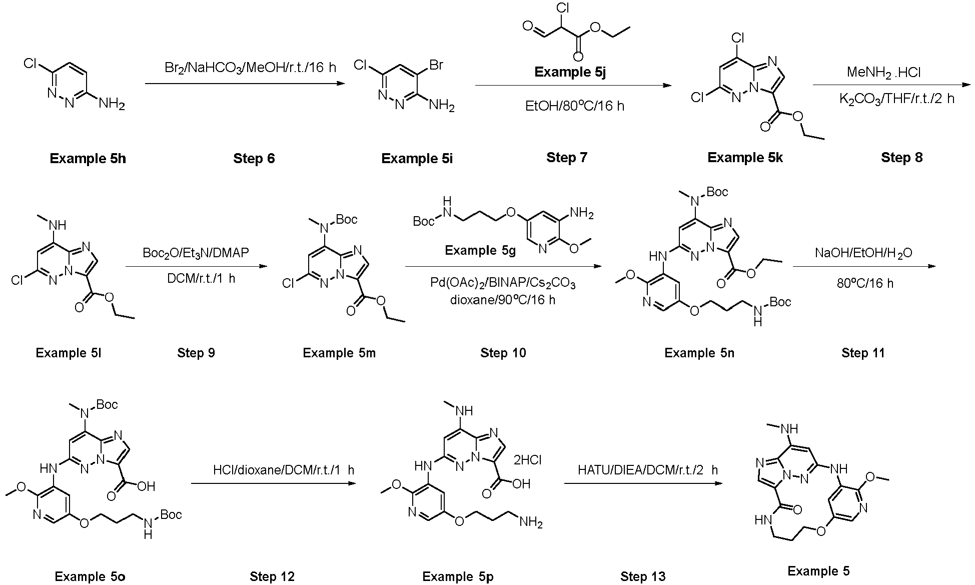

### Step 1: Example 5b

To a solution of **Example 5a** (10.0 g, 56.8 mmol, 1.0 eq) in MeOH (50 mL) was added NaOMe (4.6 g, 85.2 mmol, 1.5 eq) at 0 °C. The reaction mixture was stirred at 50 °C for 2 h. The mixture was concentrated in *vacuo.* The residue was purified by silica gel flash column chromatography to afford the desired product **Example 5a** (1.5 g, yield 14.1%) as a yellow solid. LCMS [M+1] ⁺ = 189.1.

### Step 2: Example 5c

**The** solution of **Example 5b** (1.5g, 13.58 mmol, 1.0 eq) in HBr/AcOH (20 mL) was stirred at 100°C for 16 h. The reaction mixture was concentrated in *vacuo.* The residue was diluted with H₂O (20 mL) and basified with saturated NaHCO₃ aqueous solution to pH ~8. The aqueous was extracted with EtOAc (50 mL*3). The combined organic layer was washed with brine, dried over Na₂SO₄ and concentrated in *vacuo.* The residue was purified by silica gel flash column chromatography to afford the desired product **Example 5c** (1.0 g, yield 57.5%) as a yellow solid. LCMS [M+1] ⁺ = 219.1.

### Step 3: Example 5e

To a solution of **Example 5c** (900 mg, 4.13 mmol, 1.0 eq) and **Example 5d** (867 mg, 4.95 mmol,1.2 eq) in dry DCM (20 mL) was added PPh₃ (1.3 g, 4.95 mmol,1.2 eq), followed by DBAD (1.13 g, 4.95 mmol,1.2 eq) at 0 °C under N₂. The reaction mixture was stirred for 3 h at r.t. The solvent was removed under vacuum, and the residue was purified by silica gel flash column chromatography to give the desired product **Example 5e** (950 mg, yield 61.4%) as a yellow solid. LCMS [M+1] ⁺ = 376.2.

### Step 4: Example 5f

To a solution of **Example 5e** (950 mg, 2.54 mmol, 1.0 eq) in MeOH (20 mL) was added NaOMe (412 mg, 7.62 mmol, 3.0 eq). The reaction mixture was stirred at 65°C for 2 h. The mixture was concentrated in *vacuo.* The residue was purified by silica gel flash column chromatography to afford the product **Example 5f** (700 mg, yield 84.5%) as a yellow solid. LCMS [M+1] ⁺ = 328.3.

### Step 5: Example 5g

To a solution of **Example 5f** (650 mg, 1.98 mmol, 1.0 eq) in MeOH (20 mL) was added 10% Pd/C (60 mg) under N₂ protection. The mixture was degassed with H₂ three times and stirred at r.t. for 1 h under H₂ balloon. The solid was filtered. The filtrate was concentrated in *vacuo* to give the desired product **Example 5g** (550 mg, yield 93.2%) as colorless oil. LCMS [M+1] ⁺ = 298.3.

### Step 6: Example 5i

To a mixture of **Example 5f** (5.0 g, 38.75 mmol, 1.0 eq) and NaHCO₃ (9.76 g, 116.2 mmol, 3.0 eq) in MeOH (30 mL) was added Br₂ (7.4 g, 46.51 mmol, 1.2 eq) dropwise at 0°C. After addition, it was warmed to r.t. and stirred for 16 h. The reaction mixture concentrated in *vacuo.* The residue was purified by silica gel flash column chromatography to afford the desired product **Example 5i** (3.5 g, yield 43.6%) as a yellow solid. LCMS [M+1] ⁺ = 208.1.

### Step 7: Example 5k

To a solution of **Example 5i** (3.5 g, 16.9 mmol, 1.0 eq) in EtOH (50 mL) was added **Example 5j** (5.07 g, 33.8 mmol, 2.0 eq), which was stirred at 80°C for 16 h. The reaction mixture concentrated in *vacuo.* The residue was purified by silica gel flash column chromatography to give the desired product **Example 5k** (1.2 g, yield 34.2%) as a white solid. LCMS [M+1] ⁺ = 260.1.

### Step 8: Example 5l

To a mixture of **Example 5k** (1.2 g, 4.61mmol, 1.0 eq) and K₂CO₃(1.08 g, 13.8 mmol, 3.0 eq) in THF (20 mL) was added methanamine hydrochloride (467 mg, 6.91 mmol, 1.5 eq), which was stirred at r.t. for 2 h, The reaction mixture concentrated in *vacuo.* The residue was purified by silica gel flash column chromatography to give the desired product **Example 5l** (1.05 g, yield 87.5%) as a yellow solid. LCMS [M+1] ⁺ = 255.2.

### Step 9: Example 5m

To a solution of **Example 5l** (1.05 g, 3.92 mmol, 1.0 eq), Et₃N(1.19 g, 11.76 mmol, 3.0 eq) and DMAP (47.5 mg, 0.39 mmol, 0.1 eq) in DCM (15 mL) was added Boc₂O (1.27 g, 5.88 mmol, 1.5 eq) at 0°C, which was stirred for 1 h at room temperature. The reaction mixture concentrated in *vacuo.* The residue was purified by silica gel flash column chromatography to give the desired product **Example 5m** (1.1 g, yield 75.3%) as a white solid. LCMS [M+1] ⁺ = 355.2.

### Step 10: Example 5n

To a mixture of **Example 5m** (350 mg, 0.99 mmol, 1.0 eq), **Example 5g** (352 mg, 1.18 mmol, 1.2 eq) and Cs₂CO₃ (643 mg, 20.0 mmol, 2.0 eq) in dioxane (10 mL) were added Pd(OAc)₂ (22 mg, 0.099 mmol, 0.1 eq) and BINAP (134 mg, 0.198 mmol, 0.2 eq). The mixture was degassed with N₂three times, and then heated to 90°C for 16 h. The reaction was concentrated in *vacuo.* The residue was purified by silica gel flash column chromatography to give the desired product **Example 5n** (290 mg, yield 47.7%) as a light brown solid. LCMS [M+1]⁺ = 616.4.

### Step 11: Example 5o

To a solution of **Example 5n** (280 mg, 0.46 mmol, 1.0 eq) in EtOH (2.5 mL) and H₂O (0.8 mL) was added NaOH (36.5 mg, 0.91 mmol, 2.0 eq) at 0°C. The mixture was heated to 80°C and stirred at for16 h. The reaction mixture was concentrated in *vacuo* to afford the crude product **Example 5o** (360 mg, crude, quant.) as a white solid. LCMS [M+1] ⁺ = 588.4.

### Step 12: Example 5p

To a solution of **Example 5o** (350 mg, 0.596 mmol, 1.0 eq) in DCM (2 mL) was added HCl/dioxane (1.0 mL, 4 M in dioxane), which was stirred at r.t. for 1 h. The mixture was concentrated in *vacuo* and treated with EtOAc (30 mL) to give the crude product **Example 5p** (160 mg, yield 58.4%) as a white solid. LCMS [M+1] ⁺ = 388.4.

### Step 13: Example 5

To a solution of **Example 5p** (160 mg, 0.35 mmol, 1.0 eq) and DIEA (135 mg, 1.04 mmol, 3.0 eq) in DMF (5 mL) was added HATU (199 mg, 0.52 mmol, 1.5 eq). The mixture was stirred at r.t. for 2 h. EtOAc (10 mL) was added to the reaction mixture and washed with brine (10 mL*2). The organic layer was concentrated in *vacuo.* The residue was purified by Prep-HPLC to afford the desired product **Example 5** (4.3 mg, yield 3.3%) as a white solid. LCMS [M+1] ⁺ = 370.2. ¹H NMR (300 MHz, DMSO-*d*₆) δ 8.71 (s, 1H), 8.63 (d, 1H), 8.60-8.57 (m, 1H), 7.83 (s, 1H), 7.58-7.55 (m, 1H), 7.43 (d, 1H), 6.33 (s, 1H), 4.33-4.29 (m, 2H), 3.94 (s, 3H), 3.27-3.26 (m, 2H), 2.88 (d, 3H), 1.95-1.86 (m, 2H).

### Example 6:

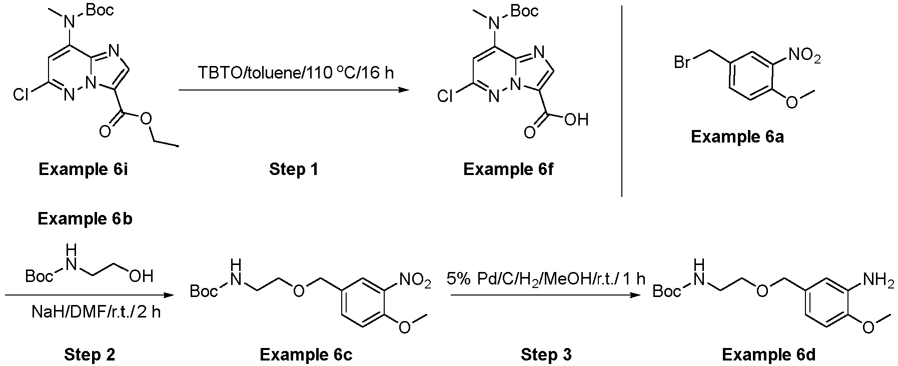

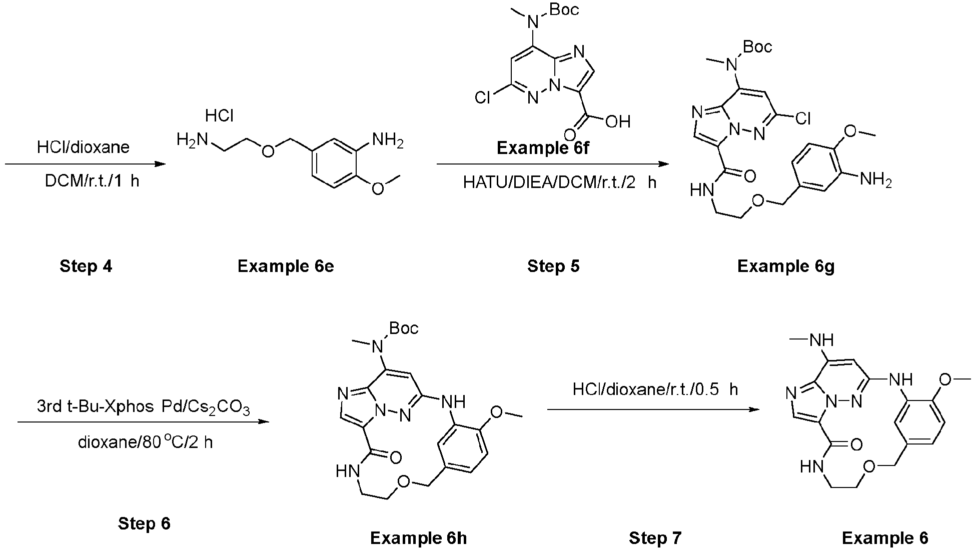

### Step 1: Example 6f

To a solution of **Example 6i** (2.0 g, 5.6 mmol, 1.0 eq) in toluene (20 mL) was added TBTO (6.7 g, 11.2 mmol, 2.0 eq). The mixture was heated to 110°C and stirred for 16 h. The mixture was concentrated in *vacuo.* The crude product was purified by silica gel flash column chromatography to afford the desired product **Example 6f** (1.7 g, yield 88.5%) as a yellow solid. LCMS [M+1] ⁺ =327.2.

### Step 2: Example 6c

To a solution of **Example 6b** (1.18 g, 7.34 mmol, 1.2 eq) in DMF (10 mL) was added NaH (539 mg, 60% in mineral oil, 13.5 mmol, 2.2 eq) in portions at 0°C. After stirring for 0.5 h, a solution of **Example 6a** (1.5 g, 6.12 mmol, 1.0 eq) in DMF (20 mL) was added dropwise. The reaction mixture was stirred at r.t. for 2 h. The reaction was quenched with saturated NH₄Cl aqueous (50 mL) at 0°C and extracted with EtOAc (100 mL*3). The combined organic layer was washed with brine (50 mL*3), dried over Na₂SO₄ and concentrated in *vacuo.* The crude product was purified by silica gel flash column chromatography to afford the desired product **Example 6c** (1.1 g, yield 55.2%) as a yellow solid. LCMS [M+1] ⁺ =327.3.

### Step 3: Example 6d

**Example 6c** (500 mg, 1.53 mmol, 1.0 eq) was dissolved with MeOH (10 mL) and 5% Pd/C (100 mg) was added under N₂ protection. The system was evacuated and then refilled with hydrogen. The mixture solution was stirred for 1 h at r.t. under H₂ balloon. The reaction mixture was filtered and the filtrate was concentrated to afford the desired product **Example 6d** (450mg, yield 99.3%) as colorless oil. LCMS [M+1] ⁺ =297.3.

### Step 4: Example 6e

To a solution of **Example 6d** (450 mg, 1.52 mmol, 1.0 eq) in DCM (10 mL) was added HCl/dioxane (3 mL, 4M). The reaction mixture was stirred for 1 h at r.t. The reaction solution was concentrated in *vacuo* to afford the desired product **Example 6e** (300 mg, yield 85.2%) as a white solid. LCMS [M+1] ⁺ =197.3.

### Step 5: Example 6g

To a solution of **Example 6f** (320 mg, 0.98 mmol, 1.0 eq) in DCM (15 mL) were added DIEA (760 mg, 5.88 mmol, 6.0 eq) and HATU (448 mg, 1.17 mmol, 1.2 eq). After stirred for 0.5 h, **Example 6e** (316 mg, 1.17 mmol, 1.2 eq) was added. The reaction solution was stirred for 2 h at r.t. The solvent was removed, and the residue was purified by silica gel flash column chromatography to afford the desired product **Example 6g** (220 mg, yield 44.4%) as a yellow solid. LCMS [M+1] ⁺ =505.4.

### Step 6: Example 6h

To a solution of **Example 6g** (170 mg, 0.33 mmol, 1.0 eq) in dioxane (10 mL) were added Cs₂CO₃ (219 mg, 0.67 mmol, 2.0 eq) and 3^{rd}-t-Bu-Xphos Pd (30 mg, 0.033 mmol, 0.1 eq). The reaction mixture was stirred for 2 h at 80°C under N₂. The reaction solution was concentrated in *vacuo.* The crude product was purified by Prep-TLC to afford the desired product **Example 6h** (110 mg, yield 69.6%) as a yellow solid. LCMS [M+1] ⁺ =469.4.

### Step 7: Example 6

To a solution of **Example 6h** (110 mg, 0.17mmol, 1.0 eq) in DCM (5 mL) was added HCl/dioxane (1 mL, 4 M in dioxane) at 0 °C. The solution was stirred for 0.5 h at r.t. and then concentrated. The crude product was dissolved in MeOH and Na₂CO₃(aq.) was added to basified pH ~8. The mixture was concentrated and the residue was purified by prep-TLC to afford the desired product **Example 6** (55 mg, yield 63.6%) as an off-white solid. LCMS [M+1] ⁺ =369.4. ¹H NMR (300 MHz, DMSO-*d*₆) δ 8.76 (brs, 1H), 8.42 (s, 1H), 8.07 (d, 1H), 7.81 (s, 1H), 7.45-7.37 (m, 1H), 7.01 (d, 1H), 6.90 (dd, 1H), 6.22 (s, 1H), 4.50 (s, 2H), 3.88 (s, 3H), 3.57-3.54 (m, 2H), 3.45-3.37 (m, 2H), 2.89 (d, 3H).

### Example 7:

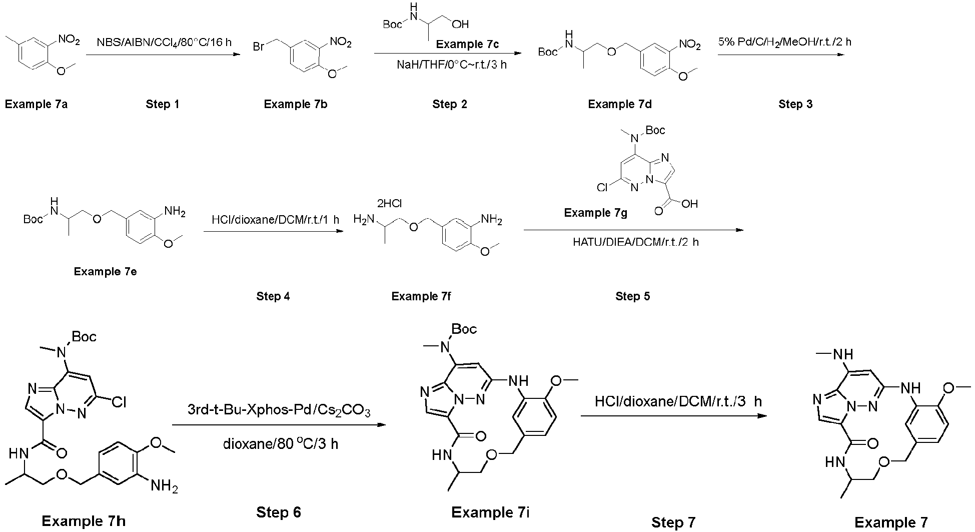

### Step 1: Example 7b

To a mixture of **Example 7a** (21.0 g, 0.126 mol) in CCl₄ (400 mL) were added NBS (23.5 g, 0.132 mol) and AIBN (4.1 g, 0.025 mol). The reaction mixture was stirred at 80°C for 16 h. After cooled to room temperature, the solvent was removed, and the residue was purified by silica gel flash column chromatography to afford the product **Example 7b** (18.5g, yield 59.8%) as a yellow solid.

### Step 2: Example 7d

To a solution of **Example 7c** (2.13 g, 12.2 mmol)in THF (50 mL) was added NaH (0.81 g, 60% in mineral oil, 20.3 mmol) in portions at 0°C. The mixture was stirred for 10 min at the same temperature, then **Example 7b** (2.0 g, 8.1 mmol) in THF was added dropwise. The reaction mixture was stirred at r.t. for 3 h. The mixture was quenched with saturated NH₄Cl aqueous solution (50 mL) and extracted with EtOAc (50 mL*2). The combined organic layer was washed with brine, dried over anhydrous Na₂SO₄ and concentrated in *vacuo.* The residue was purified by silica gel flash column chromatography to afford the product **Example 7d** (1.1 g, yield 39.8) as yellow oil. LCMS [M+1-100] ⁺ = 241.2.

### Step 3: Example 7e

To a solution of **Example 7d** (1.0 g, 2.94 mmol) in MeOH (50 mL) was added 5% Pd/C (100 mg) under N₂ protection. The suspension was degassed under vacuum and purged with H₂ for 3 times. The mixture was stirred at r.t. for 2 h under H₂ balloon. The solid was filtered out, and the filtrate was concentrated to afford the product **Example 7e** (900 mg, yield 98.8%) as yellow oil. LCMS [M+Na]⁺ = 333.4.

### Step 4: Example 7f

To a solution of **Example 7e (500** mg, 1.6 mmol) in DCM (10 mL) was added HCl/dioxane (2 mL,4M in dioxane, 8.0 mmol). The reaction mixture was stirred at r.t. for 1 h. The mixture was concentrated to afford the product **Example 7f** (480 mg, crude, quant.) as yellow oil. LCMS [M+1] ⁺ = 211.2.

### Step 5: Example 7h

To a solution of **Example 7g** (324 mg, 0.99 mmol, from **Example 6f)** in DCM (20 mL) were added DIEA (1.0 g, 7.95 mmol) and HATU (415mg, 1.1 mmol). After stirring for 10 min, **Example 7f** (450 mg, 2.14 mmol) was added to the mixture. The reaction mixture was stirred for 2 h at room temperature. After the reaction was completed, the solvent was removed and the crude was purified by silica gel chromatography to give the desired product **Example 7h** (200 mg, yield 24.3%) as a yellow solid. LCMS [M+1] ⁺ =519.2.

### Step 6: Example 7i

To a solution of **Example 7h** (200 mg, 0.39 mmol) in dioxane (10 mL) were added Cs₂CO₃ (251 mg, 0.77 mmol) and 3rd-*t*-Bu-Xphos-Pd (34 mg, 0.04 mmol). The reaction mixture was stirred at 80°C for 3 h under N₂. After cooled to room temperature, the solvent was removed, and the residue was purified by silica gel flash column chromatography to afford the product **Example 7i** (105 mg, yield 56.4%) as a yellow solid. LCMS [M+1] ⁺ = 483.2.

### Step 7: Example 7

To a solution of **Example 7i** (100mg, 0.2 mmol) in DCM (5 mL) was added HCl/dioxane (1.0 mL,4M in dioxane, 4.0 mmol). The reaction mixture was stirred at r.t. for 3 h and then concentrated in vacuum. The residue was dissolved in MeOH (5 mL) and basified with NaHCO₃ until pH~8. DCM (100 mL) was added to the mixture. The mixture was filtered through a silica gel column. The filtrate was concentrated to give the desired product **Example 7** (38.0 mg, yield 47.9%) as a white solid. LCMS [M+1] ⁺ = 383.3. ¹H NMR (300 MHz, DMSO-*d₆*) δ 8.78 (d, 1H), 8.44 (s, 1H), 8.15 (d, 1H), 7.81 (s, 1H), 7.41 (d, 1H), 7.02 (d, 1H), 6.89 (dd, 1H), 6.22 (s, 1H), 4.65 (d, 1H), 4.38 (d, 1H), 4.05 - 3.94 (m,1H), 3.89 (s, 3H), 3.48 (dd, 1H), 3.29 - 3.22 (m, 1H), 2.88 (d, 3H), 1.14 (d, 3H).

### Example 8:

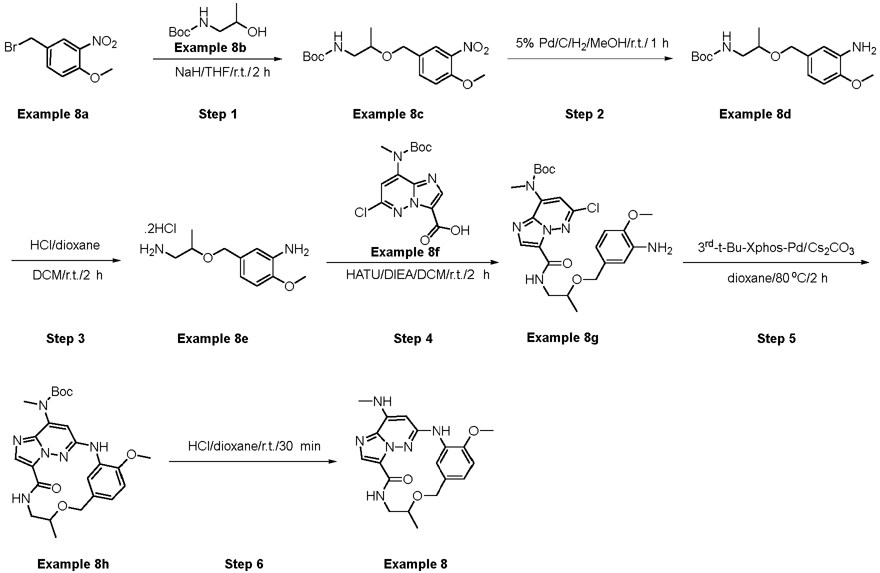

### Step 1: Example 8c

To a solution of **Example 8b** (2.13 g, 12.20 mmol, 1.5 eq) in THF (50 mL) was added NaH (813 mg, 60% in mineral oil, 20.33 mmol, 2.5 eq) in portions at 0°C. After stirred for 30 min, to the above solution was added a solution of **Example 8a** (2.0 g, 8.13 mmol, 1.0 eq) in THF (10 mL). The reaction mixture was stirred at r.t. for 2 **h. The reaction** was quenched with saturated NH₄Cl aqueous solution (25 mL) at 0°C and extracted with EtOAc (50 mL*3). The combined organic layer was washed with brine, dried over Na₂SO₄ and concentrated. The crude product was purified by silica gel flash column chromatography to afford the desired product **Example 8c** (980mg, yield 35.4%) as a yellow solid. LCMS [M+1] ⁺ = 341.3.

### Step 2: Example 8d

**Example 8c** (980 mg, 2.88 mmol, 1.0 eq) was dissolved in MeOH (20 mL), 5% Pd/C (500 mg) was added under N₂ protection. The suspension was evacuated and then refilled with hydrogen for three times. The mixture was stirred for 1 h at r.t. under H₂ balloon. The solid was filtered out and the filtrate was concentrated to afford the desired product **Example 8d** (935 mg, crude, quant.) as a brown solid. LCMS [M+1] ⁺ = 311.4.

### Step 3: Example 8e

To a solution of **Example 8d** (835 mg, 2.69 mmol, 1.0 eq) in DCM (12 mL) was added HCl/dioxane (3 mL, 4M in dioxane). The reaction mixture was stirred at r.t. for 2 h. The solvent was concentrated under vacuum to give a crude product **Example 8e** (980 mg, crude, quant) as a yellow solid. LCMS [M+1] ⁺ =211.3

### Step 4: Example 8g

To a solution of **Example 8f** (300 mg, 0.92 mmol, 1.0 eq, from **Example 6f)** in DCM (10 mL) were added DIEA (947 mg, 7.34 mmol, 8.0 eq) and HATU (383 mg, 1.01 mmol, 1.1 eq). After stirring for 30 min, **Example 8e** (340 mg, 1.38 mmol, 1.5 eq) was added to the solution. The reaction was stirred for 2 h at r.t. The solvent was concentrated, and the residue was purified by silica gel flash column chromatography to afford the desired product **Example 8g** (160 mg, yield 33.6%) as yellow oil. LCMS [M+1] ⁺ =519.3.

### Step 5: Example 8h

To a solution of **Example 8g** (150 mg, 0.29 mmol, 1.0 eq) in dioxane (10 mL) were added Cs₂CO₃ (188 mg, 0.58 mmol, 2.0 eq) and 3^{rd}-t-Bu-Xphos-Pd (27 mg, 0.029 mmol, 0.1 eq). The reaction mixture was stirred for 2 h at 80°C under N₂. The reaction solution was filtered and the filtrate was concentrated. The crude product was purified by prep-TLC to afford the desired product **Example 8h** (90 mg, yield 64.5%) as a yellow solid. LCMS [M+1] ⁺ =483.4.

### Step 6: Example 8

To a solution of **Example 8h** (80 mg, 0.17 mmol, 1.0 eq) in DCM (3 mL) was added HCl/dioxane (1 mL, 4 M in dioxane) at 0°C. The reaction was stirred for 30 min at r.t. and then concentrated. The crude product was dissolved in MeOH, Na₂CO₃(excess) was added and stirred at r.t. for 10 min. The solid was filtered out, the filtrate was concentrated. The residue was purified by silica gel column chromatography to afford the desired product **Example 8** (40.0 mg, yield 63.1%) as an off-white solid. LCMS [M+1] ⁺ =383.3. ¹H NMR (300 MHz, DMSO-*d*₆) δ 8.73 (s, 1H), 8.42 (s, 1H), 8.10 (d, 1H), 7.81 (s, 1H), 7.40 (d, 1H), 7.00 (d, 1H), 6.92 (dd, 1H), 6.21 (s, 1H), 4.64 (d, 1H), 4.42 (d, 1H), 3.88 (s, 3H), 3.65 - 3.49 (m, 1H), 3.42 - 3.35 (m, 1H), 3.27 - 3.14 (m, 1H), 2.89 (d, 3H), 1.20 (d, 3H).

### Example 9:

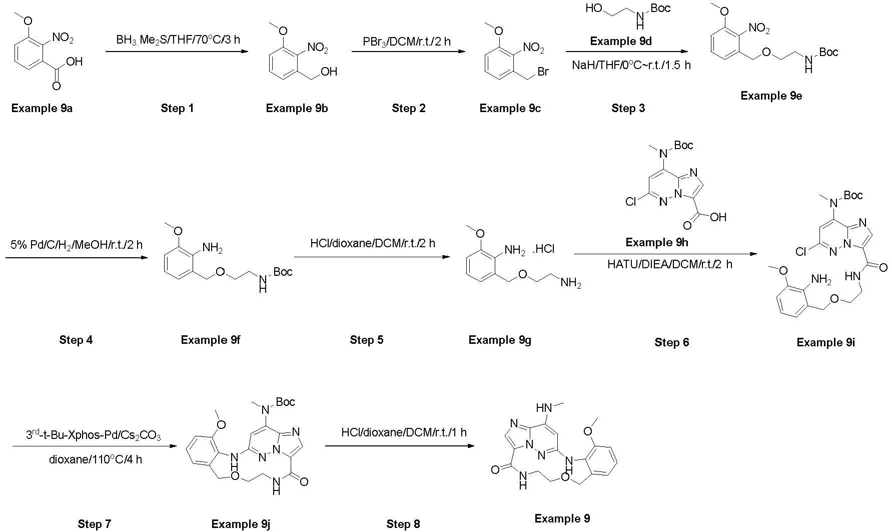

### Step 1: Example 9b

To a solution of **Example 9a** (10.0 g, 50.8 mmol, 1.0 eq) in dry THF (100 mL) was added BH₃.Me₂S (6.1 mL, 10M in DMS, 61.0 mmol, 1.2 eq) dropwise at r.t. The solution was stirred for 3 h at 70 °C. After cooled to room temperature, 3M HCl aqueous solution was added dropwise into the reaction solution until effervescence was no longer observed. The resulting mixture was extracted with EtOAc (100 mL*3). The combined organic layer was washed with saturated Na₂CO₃ aqueous, followed by brine, dried over Na₂SO₄, and concentrated in vacuum to afford the product **Example 9b** (8.7 g, yield 94%) as an off-white solid. LCMS [M-18+1] ⁺ = 166.2

### Step 2: Example 9c

To a solution of **Example 9b** (2.6 g, 14.2mmol, 1.0 eq) in dry DCM (60 mL) was added PBr₃ (7.7 g, 28.4 mmol, 2.0 eq) dropwise, which was stirred for 2 h at r.t. The reaction was diluted with DCM (100 mL), and Na₂CO₃ aqueous solution was added to the solution until a neutral pH was obtained. The resulting mixture was extracted with DCM (100 mL*2). The combined organic layer was washed with brine, dried over Na₂SO₄, and concentrated in vacuum to give the product **Example 9c** (3.3 g, yield 95%) as an off-white solid. LCMS [M+1] ⁺ = 246.1.

### Step 3: Example 9e

To a solution of **Example 9d** (1.47 g, 9.15 mmol, 1.5 eq) in dry THF (10 mL) was added NaH (610 mg, 60% in mineral oil, 15.25 mmol, 2.5 eq) in portions at 0 °C, which was stirred for 30 min. Then a solution of **Example 9c** (1.50 g, 6.1 mmol, 1.0 eq) in THF (5 mL) was added dropwise. The mixture was stirred for 1 h at r.t., then quenched with water (15 mL), extracted with EtOAc (30 mL*2). The combined organic layer was washed with brine, dried over Na₂SO₄, concentrated in vacuum, and the residue was purified by silica gel flash column chromatography to give the desired product **Example 9e** (1.1g, yield 55%) as yellow oil. LCMS [M+1] ⁺ = 327.3.

### Step 4: Example 9f

To a solution of **Example 9e** (1.1 g, 3.4 mmol, 1.0 eq) in MeOH (25 mL) was added 5% Pd/C (200 mg) under N₂ protection, the suspension was degassed under vacuum and purged with H₂ for three times. The mixture was stirred for 2 h at r.t. under H₂ balloon. The solid was filtered out, and the filtrate was concentrated to give the desired product **Example 9f** (950 mg, yield 94%) as yellow oil. LCMS [M+1] ⁺ = 297.3.

### Step 5: Example 9g

To a solution of **Example 9f** (400 mg, 1.35 mmol, 1.0 eq) in DCM (10mL) was added HCl/dioxane (4M in dioxane, 2 mL). The solution was stirred for 2 h at r.t. and then concentrated to give the product (650 mg, crude, quant.) as yellow oil. LCMS [M+1] ⁺ = 197.3

### Step 6: Example 9i

To a solution of **Example 9h** (250 mg, 0.77 mmol, 1.0 eq, from **Example 6f)** and DIEA (695.3 mg, 5.39 mmol, 7.0 eq) were added HATU (352 mg, 0.92 mmol, 1.2 eq), which was stirred for 10 min at room temperature. Then **Example 9g** (452 mg, 2.31 mmol, 3.0 eq) was added. The mixture was stirred for 2 h at r.t., and the solvent was removed. The residue was purified by silica gel flash column chromatography to give the desired product **Example 9i** (280 mg, yield 72%) as yellow oil. LCMS [M+1] ⁺ = 505.3.

### Step 7: Example 9j

To a solution of **Example 9i** (100 mg, 0.20 mmol, 1.0 eq) in dioxane (5 mL) was added Cs₂CO₃ (130 mg, 0.40 mmol, 2.0 eq) and 3^{rd}-t-Bu-Xphos-Pd (17.4 mg, 0.02 mmol, 0.1 eq). The reaction mixture was stirred for 4 h at 110°C under N₂. After cooled to room temperature, the reaction solution was filtered and the filtrate was concentrated. The crude product was purified by prep-TLC to afford the desired product **Example 9j** (30 mg, yield 32%) as a yellow solid. LCMS [M+1] ⁺ =469.2.

### Step 8: Example 9

To a solution of **Example 9j** (20 mg, 0.043 mmol, 1.0 eq) in DCM (6 mL) was added HCl/dioxane (2 mL, 4M in dioxane), which was stirred for 1 h at r.t. and then concentrated. The residue was diluted with MeOH (5 mL), and K₂CO₃ (excess) was added. The mixture was stirred for 30 min at r.t. The solid was filtered out, the filtrate was concentrated and the residue was purified by Prep-TLC to give the desired product **Example 9** (10.5 mg, yield 66%) as a white solid. LCMS [M+1] ⁺ =369.3. ¹H NMR (300 MHz, DMSO-*d₆*) δ 9.88 (s, 1H), 7.78 (s,1H), 7.58 (s,1H), 7.48 (d, 1H), 7.26 (t, 1H), 7.16 (d, 1H), 7.03 (d, 1H), 6.08 (s, 1H), 4.87(s, 2H), 3.80 (s, 5H), 3.63 -3.53 (m, 2H), 2.92 (d, 3H).

### Example 10:

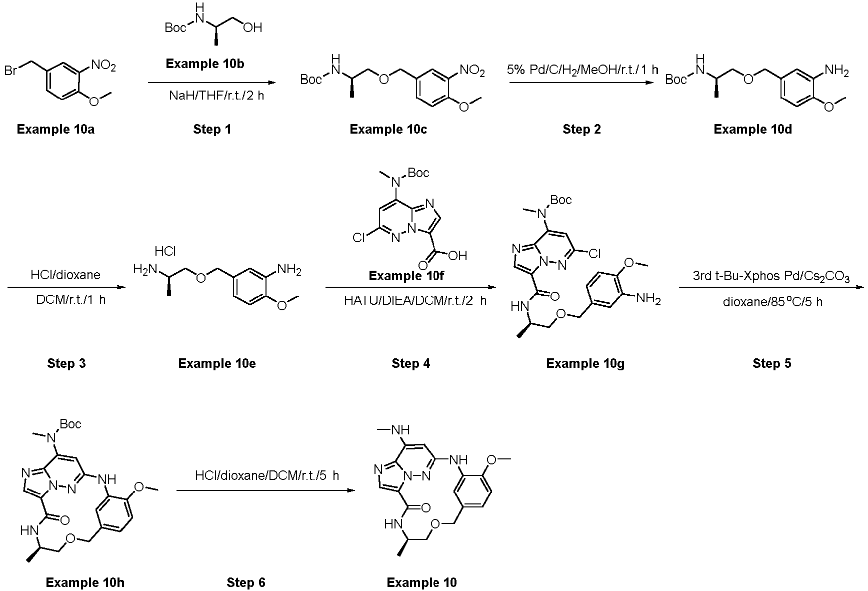

### Step 1: Example 10c

To a solution of **Example 10b** (1.85 g, 10.6 mmol) in THF (20 mL) was added NaH (718 mg, 60% in mineral oil, 17.9 mmol) in portions at 0 °C. After stirring for 0.5 h, a solution of **Example 10a** (2.0 g, 8.16 mmol, from **Example 7b)** in THF (10 mL) was added dropwise. The reaction mixture was stirred for 2 h at r.t. The reaction was quenched with saturated NH₄Cl aqueous (50 mL) at 0°C and extracted with EtOAc (100 mL*3). The combined organic layer was washed with brine (50 mL*3), dried over Na₂SO₄ and concentrated in *vacuo.* The crude product was purified by silica gel flash column chromatography to afford the desired product **Example 10c (2.5** g, yield 89.9%) as a yellow solid. LCMS [M+1] ⁺ = 341.3.

### Step 2: Example 10d

**Example 10c** (2.5 g, 7.35 mmol) was dissolved in MeOH (30 mL),and 5% Pd/C (250 mg) was added under N₂ protection. The system was evacuated and then refilled with hydrogen for three times. The mixture solution was stirred for 1 h at r.t. under H₂ balloon. The reaction mixture was filtered and the filtrate was concentrated to afford the desired product **Example 10d** (1.5 g, yield 65.8%) as colorless oil. LCMS [M+1] ⁺ = 311.3.

### Step 3: Example 10e

To a solution of **Example 10d** (1.0 g, 3.22mmol) in DCM (15 mL) was added HCl/dioxane (2 mL, 4M in dioxane, 8 mmol). The reaction mixture was stirred for 1 h at r.t. The reaction solution was concentrated in *vacuo* to afford the desired product **Example 10e** (700 mg, yield 79.3%) as a white solid. LCMS [M+1] ⁺ =211.2.

### Step 4: Example 10g

To a solution of **Example 10f** (519 mg, 1.84 mmol, from **Example 6f) in** DCM (10 mL) were added DIEA (950 mg, 7.38 mmol) and HATU (559 mg, 1.47 mmol). After stirred for 0.5 h, **Example 10e** (400mg, 1.23 mmol) was added. The reaction solution was stirred for 2 h at r.t. The solvent was removed and the residue was purified by silica gel flash column chromatography to afford the desired product **Example 10g** (210 mg, yield 32.9%) as a yellow solid. LCMS [M+1] ⁺ = 519.3.

### Step 5: Example 10h

To a solution of **Example 10g** (195 mg, 0.38mmol) in dioxane (30 mL) were added Cs₂CO₃ (245 mg, 0.75mmol) and 3^{rd}-*t*-Bu-Xphos-Pd(33 mg, 0.04 mmol). The reaction mixture was stirred at 85°C for 5 h under N₂. After cooled to room temperature, the solvent was removed, and the residue was purified by silica gel flash column chromatography to afford the product **Example 10h** (95 mg, yield 52.3%) as a yellow solid. LCMS [M+1] ⁺ = 483.2.

### Step 6: Example 10

To a solution of **Example 10h** (95 mg, 0.2 mmol) in DCM (5 mL) was added HCl/dioxane(1 mL,4M in dioxane, 4 mmol). The reaction mixture was stirred at r.t. for 5 h and then concentrated in vacuum. The residue was dissolved in MeOH (5 mL), and basified with NaHCO₃ (pH = 8). DCM (100 mL) was added to the mixture and the solid was filtered out. The filtrate was concentrated to give the desired product **Example 10** (50.0 mg, yield 66.4%) as a white solid. LCMS [M+1] ⁺ = 383.3. ¹H NMR (300 MHz, DMSO-*d₆*) δ 8.76 (d, 1H), 8.45 (s, 1H), 8.15 (d, 1H), 7.82 (s, 1H), 7.41 (d, 1H), 7.01 (d, 1H), 6.88 (dd, 1H), 6.23 (s, 1H), 4.62 (d, 1H), 4.35 (d, 1H), 4.05 - 3.93 (m, 1H), 3.89 (s, 3H), 3.54 - 3.46 (m, 1H), 3.25 (t, 1H), 2.88 (d, 3H), 1.12 (d, 3H).

### Example 11:

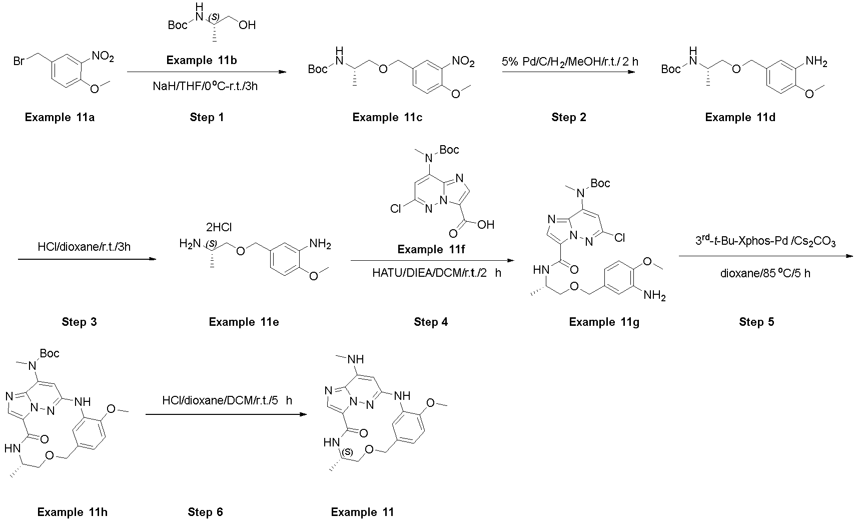

### Step 1: Example 11c

To a solution of **Example 11b** (2.63 g, 15 mmol) in THF (50 mL) was added NaH (1.0 g, 60% in mineral oil, 25 mmol) in portions at 0°C. After stirring for 10 min, a solution of **Example 11a** (2.46 g, 10 mmol, from **Example 7b)** in THF (10 mL) was added dropwise. The reaction mixture was stirred at r.t. for 3 h. The reaction was quenched with saturated NH₄Cl aqueous (20 mL) at 0°C and extracted with EtOAc (50mL), dried over Na₂SO₄ and concentrated in *vacuo.* The crude product was purified by silica gel flash column chromatography to afford the desired product **Example 11c** (2.6 g, yield 76.5%) as a yellow solid. LCMS [M+1] ⁺ =341.3.

### Step 2: Example 11d

**Example 11c** (1.5 g, 4.4 mmol) was dissolved in MeOH (30 mL), and then 5% Pd/C (150 mg) was added under N₂ protection. The system was evacuated and then refilled with hydrogen for three times. The mixture solution was stirred for 2 h at r.t. under H₂ balloon. The reaction mixture was filtered and the filtrate was concentrated to afford the desired product **Example 11d** (1.35 g, yield 98.7%) as yellow oil. LCMS [M+1] ⁺ =311.3.

### Step 3: Example 11e

To a solution of **Example 11d** (600 mg, 1.9 mmol) in DCM (6 mL) was added HCl/dioxane (2 mL,4M in dioxane, 8 mmol). The reaction mixture was stirred at r.t. for 3 h. The reaction solution was concentrated in *vacuo* to afford the desired product **Example 11e** (580 mg, crude, quant.) as yellow oil. LCMS [M+1] ⁺ =211.2.

### Step 4: Example 11g

To a solution of **Example 11f** (418 mg, 1.3 mmol, from **Example 6f)** in DCM (30 mL) were added DIEA (1.3 g, 10.3 mmol) and HATU (730 mg, 1.9 mmol). After stirring for 0.5 h, **Example 11e** (580 mg, 2.0 mmol) was added. The reaction mixture was stirred for 2 h at r.t. The solvent was removed, and the residue was purified by silica gel flash column chromatography to afford the desired product **Example 11g** (240 mg, yield 36.1%) as a yellow solid. LCMS [M+1]⁺ =519.3.

### Step 5: Example

To a solution of **Example 11g** (240 mg, 0.46 mmol) in dioxane (10 mL) were added Cs₂CO₃ (302 mg, 0.92 mmol) and 3^{rd}-*t*-Bu-Xphos-Pd(4l mg, 0.05 mmol). The reaction mixture was stirred at 85°C for 5 h under N₂. After cooled to room temperature, the solvent was removed, and the residue was purified by silica gel flash column chromatography to afford the product **Example 11h** (140 mg, yield 62.7%) as a yellow solid.

LCMS [M+1] ⁺ = 483.2.

### Step 6: Example 11

To a solution of **Example 11h** (140 mg, 0.29 mmol) in DCM (5 mL) was added HCl/dioxane (1 mL, 4M in dioxane, 4 mmol). The reaction mixture was stirred at r.t. for 5 h and then concentrated in vacuum. The residue was dissolved in MeOH (5 mL) and basified with NaHCO₃ (pH = 8). DCM (100 mL) was added to the mixture. The solid was filtered out, and the filtrate was concentrated to give the desired product **Example 11** (70.0 mg, 66.4% yield) as an off-white solid. LCMS [M+1] ⁺ = 383.3. ¹H NMR (300 MHz, DMSO-*d₆*) δ 8.76 (d, 1H), 8.45 (s, 1H), 8.15 (d, 1H), 7.82 (s, 1H), 7.43 (d, 1H), 7.01 (d, 1H), 6.89 (dd, 1H), 6.23 (s, 1H), 4.64 (d, 1H), 4.37 (d, 1H), 4.05 - 3.92 (m,1H), 3.89 (s, 3H), 3.46 (dd, 1H), 3.25 (t, 1H), 2.89 (d, 3H), 1.13 (d, 3H).

### Example 12:

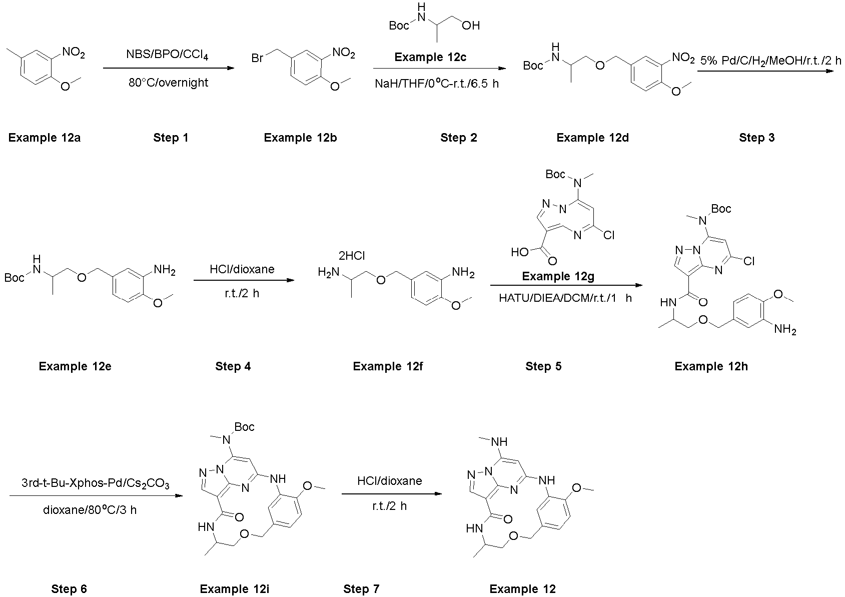

### Step 1: Example 12b

To a solution of **Example 12a (30.0** g, 179 mmol) in CCl₄ (150 mL) were added BPO (4.4 g, 17.9 mmol), NBS (38.15 g, 216 mmol), which was stirred at 80°C overnight. After cooling, the mixture was then diluted by DCM, washed by water, dried over Na₂SO₄, and concentrated under reduced pressure to give **Example 12b** (37.0 g, yield 84.4%) as a yellow solid, which was used for the next step without purification. LCMS [M+1]⁺ = 246.0. ¹H NMR (400 MHz, Chloroform-d) δ 7.87 (d, 1H), 7.57 (dd, 1H), 7.07 (d, 1H), 4.46 (s, 2H), 3.96 (d, 3H).

### Step 2: Example 12d

To a solution of **Example 12b** (2.46 g, 10.0 mmol) in THF (20 mL) was added NaH (400 mg, 60% in mineral oil, 10.0 mmol) at 0°C, which was stirred for 0.5 h. Then **Example 12c** (1.75 g, 10.0 mmol) was added, and the resulting mixture was stirred at r.t. for 6 h. The mixture was quenched by NH₄Cl aq, extracted by EtOAc, dried over Na₂SO₄, and concentrated under reduced pressure. The residue was purified by silica gel column chromatography to give **Example 12d** (3.3 g, yield 96.8%) as a yellow solid. LCMS [M+1-100]⁺ = 241.1.

### Step 3: Example 12e

A suspension of **Example 12d** (688 mg, 2.0 mmol) and 10% Pd/C (34 mg) in MeOH (10 mL) was stirred at r.t. for 2 h under H₂ balloon. The suspension was filtered, and the filtrate was concentrated under reduced pressure to give **Example 12e** (640 mg, crude yield 103%) as a yellow solid, which was used for the next step without purification.

### Step 4: Example 12f

To a solution of **Example 12e** (400 mg, 1.3 mmol) in dioxane (2 mL) was added HCl/dioxane (1.0 mL, 4M in dioxane), which was stirred at r.t. for 2 h. The mixture was concentrated, and the residue was treated with EtOAc (30 mL) to give the crude product **Example 12f** (340 mg, crude yield 124%) as a white solid, which was used for the next step without purification.

### Step 5: Example 12h

To a solution of **Example 12f** (340 mg, 0.65 mmol), **Example 12g** (423 mg, 1.3 mmol, from **Example 6f),** and TEA (810 mg, 8.1 mmol) in DCM (10 mL) was added HATU (616 mg, 1.62mmol). The mixture was stirred at r.t. for 1 h. EtOAc (40 mL) was added to the reaction mixture, which was washed with brine (20 mL*2), dried over Na₂SO₄ and concentrated. The residue was purified by silica gel column chromatography to afford the desired product **Example 12h** (500 mg, yield 59%) as a white solid. LCMS [M+1]⁺ = 519.2.

### Step 6: Example 12i

To a mixture of **Example 12h** (500 mg, 0.97 mmol,), Cs₂CO₃ (652 mg, 2.0 mmol) in dioxane (10 mL) was added 3rd-t-Bu-Xphos-Pd (89 mg, 0.1 mmol). The mixture was degassed with N₂three times, and stirred for 3 h at 80°C. The mixture was diluted by DCM, washed by water, dried over Na₂SO₄, and concentrated under reduced pressure to give **Example 12i** (450 mg, crude yield 93.3%) as a white solid, which was used for the next step without purification. LCMS [M+1]⁺ = 483.3

### Step 7: Example 12

To a solution of **Example 12i** (200 mg, 0.42 mmol) in dioxane (2 mL) was added HCl/dioxane (1.0 mL, 4M in dioxane), which was stirred at r.t. for 2 h. The mixture was concentrated, and the residue was purified by Prep-HPLC to afford the desired product **Example 12** (4.9 mg, yield 3.0%) as a white solid. LCMS [M+1]⁺ = 383.3. ¹H NMR (400 MHz, DMSO-*d₆*) δ 8.90 (s, 1H), 8.32 (s, 1H), 8.22 (d, 1H), 8.08 (s, 1H), 7.80 (d, 1H), 6.97 (d, 1H), 6.88 (d, 1H), 5.91 (s, 1H), 4.54 (d, 1H), 4.37 (d, 1H), 3.85 (s, 3H), 3.44 (d, 1H), 2.89 (d, 3H), 1.11 (d, 3H).

### Example 13:

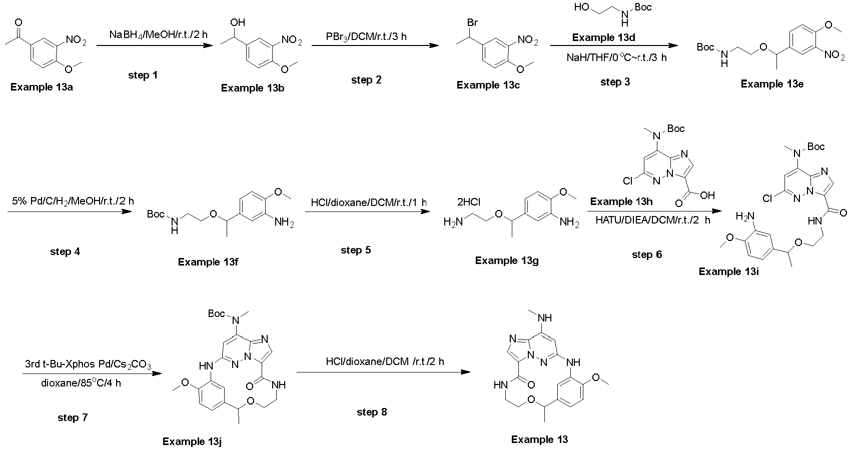

### Step 1: Example 13b

To a solution **of Example 13a** (10.0 g, 0.05 mol) in MeOH (150 mL) was added NaBH₄ (4.87 g, 0.13 mol) in portions. The reaction mixture was stirred at r.t. for 2 h. The solvent was removed and the residue was purified by silica gel flash column chromatography to afford the product **Example 13b** (8.5 g, yield 84.1%) as yellow oil. LCMS [M+1] ⁺ = 198.2.

### Step 2: Example 13c

To a solution of **Example 13b** (1.97 g, 10.0 mmol) in DCM (50 mL) was added PBr₃ (5.4 g, 20.0 mmol). The reaction mixture was stirred at r.t. for 3 h. The mixture was diluted with DCM (100 mL) and washed with saturated NaHCO₃ aqueous (50 mL*2). The organic layer dried over anhydrous Na₂SO₄ and concentrated in *vacuo* to afford the product **Example 13c** (2.3 g, yield 88.5%) as yellow oil.

### Step 3: Example 13e

To a solution of **Example 13d** (2.0 g, 12.5 mmol) in THF (50 mL) was added NaH (0.5 g, 60% in mineral oil, 12.5 mmol) in portions at 0°C. The mixture was stirred for 10 min at the same temperature, then **Example 13c** (1.3 g, 5.0 mmol) in THF was added dropwise. The reaction mixture was stirred at r.t. for 3 h. The mixture was quenched with saturated NH₄Cl (30 mL) and extracted with EtOAc (50 mL). The organic layer was dried over anhydrous Na₂SO₄ and concentrated in *vacuo.* The residue was purified by silica gel flash column chromatography to afford the product **Example 13e** (810 mg, yield 47.6%) as yellow oil. LCMS [M+1-100] ⁺ = 241.2.

### Step 4: Example 13f

**Example 13e** (800 mg, 2.4 mmol) was mixed with MeOH (30 mL), and 5% Pd/C (150 mg) was added under N₂ protection. The system was evacuated and then refilled with hydrogen for three times. The mixture was stirred for 2 h at r.t. under H₂ balloon. The reaction mixture was filtered and the filtrate was concentrated. The residue was purified by silica gel flash column chromatography to afford the product **Example 13f** (420 mg, yield 57.6%) as yellow oil. LCMS [M+1]⁺ =311.3.

### Step 5: Example 13g

To a solution of **Example 13f** (400 mg, 1.29 mmol) in DCM (5 mL) was added HCl/dioxane (2 mL, 4M in dioxane, 8 mmol). The reaction mixture was stirred for 1 h at r.t. The reaction solution was concentrated in *vacuo* to afford the desired product **Example 13g** (360 mg, yield 98.6%) as yellow oil. LCMS [M+1]⁺ =211.2.

### Step 6: Example 13i

To a solution of **Example 13h** (238 mg, 0.7 mmol, from **Example 6f)** in DCM (20 mL) were added DIEA (752 mg, 5.8 mmol) and HATU (443 mg, 1.2 mmol). The solution was stirred for 0.5 h, then **Example 13g** (330 mg, 1.2 mmol) was added. The reaction solution was stirred for 2 h at r.t. The solvent was removed, and the residue was purified by silica gel flash column chromatography to afford the desired product **Example 13i** (41 mg, yield 10.8%) as a yellow solid. LCMS [M+1]⁺ =519.3.

### Step 7: Example 13j

To a solution of **Example 13i** (41 mg, 0.08 mmol) in 1,4-dioxane (10 mL) were added Cs₂CO₃ (51 mg, 0.16 mmol) and 3rd-*t*-Bu-Xphos-Pd (7 mg, 0.01 mmol). The reaction mixture was stirred at 85°C for 4 h under N₂. After cooled to room temperature, the solvent was removed, and the residue was purified by Prep-TLC to afford the product **Example 13j** (25 mg, yield 65.5%) as a yellow solid. LCMS [M+1] ⁺ = 483.2.

### Step 8: Example 13

To a solution of **Example 13j** (25 mg, 0.05 mmol) in DCM (3 mL) was added HCl/dioxane (0.2 mL, 4M in dioxane, 0.8 mmol). The reaction mixture was stirred at r.t. for 2 h and then concentrated in vacuum. The residue was dissolved in MeOH (5 mL), Then pH valve was adjusted to 8 with saturated NaHCO₃ aqueous. The solvent was removed, and the residue was purified by Prep-TLC to afford the product **Example 13** (11.8 mg, yield 59.6%) as an off-white solid. LCMS [M+1] ⁺ = 383.3. ¹H NMR (300 MHz, DMSO-*d*₆) δ 8.80 (s, 1H), 8.42 (s, 1H), 8.03 (d, 1H), 7.80 (s, 1H), 7.41 (d, 1H), 7.00 (d, 1H), 6.90 (dd, 1H), 6.21 (s, 1H), 4.45 (q, 1H), 3.88 (s, 3H), 3.75-3.71 (m, 1H), 3.53-3.43 (m, 1H), 3.42-3.37 (m, 1H), 3.26-3.23 (m, 1H), 2.89 (d, 3H), 1.30 (d, 3H).

### Example 14:

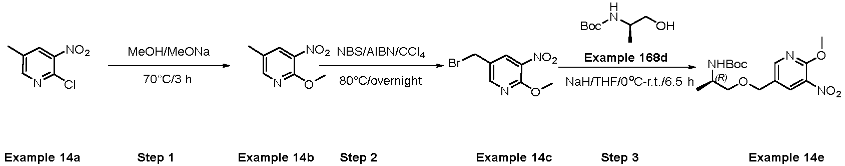

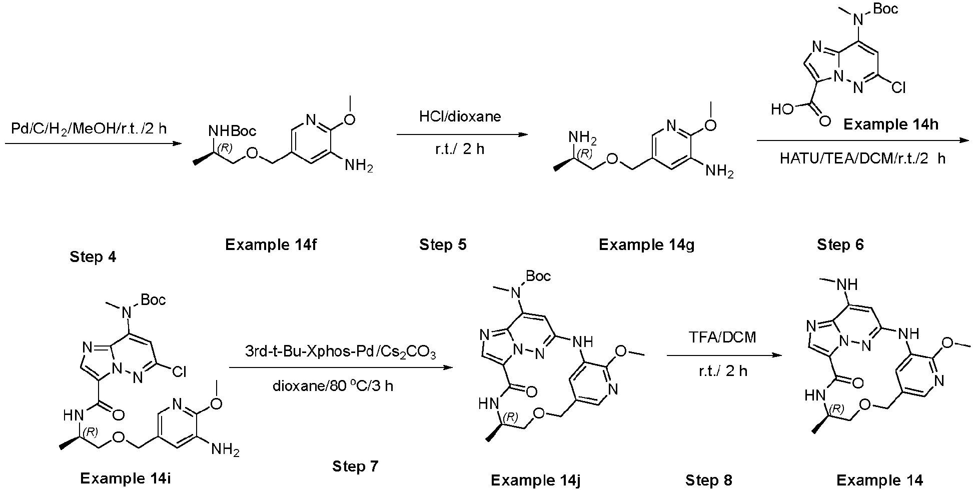

### Step 1: Example 14b

A solution of **Example 14a** (15.0 g, 87.2 mmol) and MeONa (14.1 g, 261.6 mmol) in MeOH (100 mL) was stirred at 70°C for 3 h. The mixture was concentrated under reduced pressure, and then diluted by water, which was then extracted by EtOAc, dried over anhydrous Na₂SO₄, and concentrated to afford crude product **Example 14b** (13.4 g, yield: 92.2%) as a yellow solid. The residue was used in the next step directly without further purification. LCMS [M+1] ⁺ = 169.1.

### Step 2: Example 14c

To a solution of **Example 14b** (5.0 g, 29.8 mmol) in CCl₄ (150 mL) were added BPO (720 mg, 2.98 mmol), and NBS (5.3 g, 29.8 mmol). The reaction mixture was stirred at 80°C overnight, and then diluted by DCM, washed by water, dried over anhydrous Na₂SO₄. After filtration, the filtrate was concentrated under reduced pressure, which was then purified by silica gel column chromatography to give **Example 14c** (5.7 g, yield: 77.6%) as a yellow solid. LCMS [M+1] ⁺ = 247.0

### Step 3: Example 14e

To a solution of **Example 14d** (2.1 g, 12.1 mmol) in THF (40 mL) was added NaH (1.46 g, 36.4 mmol) at 0°C. The reaction mixture was warmed to room temperature and stirred at r.t. for 0.5 h. Then **Example 14c** (3.0 g, 12.1 mmol) was added. The mixture was stirred at r.t. for 6 h, which was then quenched by aq. NH₄Cl, extracted by EtOAc, and dried over anhydrous Na₂SO₄. After filtration, the filtrate was concentrated under reduced pressure, which was then purified by silica gel column chromatography to give **Example 14e** (1.0 g, yield: 24.4%) as a yellow solid. LCMS [M-174] ⁺ = 167.1.

### Step 4: Example 14f

A solution of **Example 14e** (1.0 g, 2.93 mmol) and Pd/C (200 mg) in MeOH (5 mL) was stirred at r.t. for 2 h under 1 atm of H₂. After filtration, the filtrate was concentrated under reduced pressure to afford **Example 14f** (850 mg, yield: 93.2%) as a yellow solid, which was used in next step directly. LCMS [M-174] ⁺ = 137.1

### Step 5: Example 14g

To a solution of **Example 14f** (800 mg crude, 1.3 mmol) in DCM (4 mL) was added TFA (1.0 mL), which was stirred at r.t. for 2 h. The mixture was concentrated to give the crude product **Example 14g** (700 mg, crude, yield: quant.) as black oil. LCMS [M-74] ⁺ = 137.1.

### Step 6: Example 14i

To a solution of **Example 14g** (30 mg, 0.1 mmol), **Example 14h** (33 mg, 0.1 mmol, from **Example 6f),** TEA (202 mg, 1.0 mmol) in DCM (2 mL) was added HATU (38 mg, 0.1 mmol). The reaction mixture was stirred at r.t. for 2 h. Then EtOAc (40 mL) was added to the reaction mixture, which was washed with brine (20 mL*2), dried over anhydrous Na₂SO₄ and concentrated. The residue was purified by silica gel column chromatography to afford the desired product **Example 14i** (32 mg, yield: 62%) as a brown solid. LCMS [M+1] ⁺ = 520.2.

### Step 7: Example 14j

To a mixture of **Example 14i** (32 mg, 0.06 mmol,), Cs₂CO₃ (30 mg, 0.09 mmol) in dioxane (2 mL) was added 3rd-t-Bu-Xphos-Pd (5.5 mg, 0.006 mmol). The mixture was degassed with N₂three times, and stirred for 3 h at 80°C. Then the reaction mixture diluted by DCM, washed by water, dried over anhydrous Na₂SO₄, and then concentrated under reduced pressure to afford crude **Example 14j** (50 mg, crude, yield: quant.) as a white solid, which was used in next step without further purification. LCMS [M+1]⁺ = 484.2

### Step 8: Example 14

To a solution of **Example 14j** (50 mg, 0.1 mmol) in DCM (4 mL) was added TFA (1.0 mL), which was stirred at r.t. for 2 h. The mixture was concentrated, and the residue was purified by Prep-HPLC to afford the desired product **Example 14** (4.5 mg, yield: 31.4%) as a white solid. LCMS [M+1]⁺ = 384.2. ¹H NMR (400 MHz, Chloroform-*d*) δ 8.58 (s, 1H), 8.53 (s, 1H), 8.08 (s, 1H), 7.67 (s, 1H), 6.69 (s, 1H), 5.67 (s, 1H), 4.68 (d, 1H), 4.45 (d, 1H), 4.24 (br,1H), 4.05 (s, 3H), 3.57 - 3.54 (m, 1H), 3.39 - 3.34 (m, 1H), 3.03 (d, 3H),1.25 (d, 3H).

### Example 15:

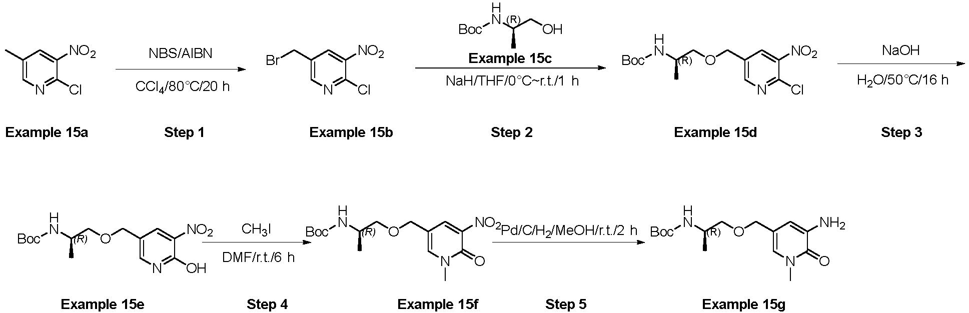

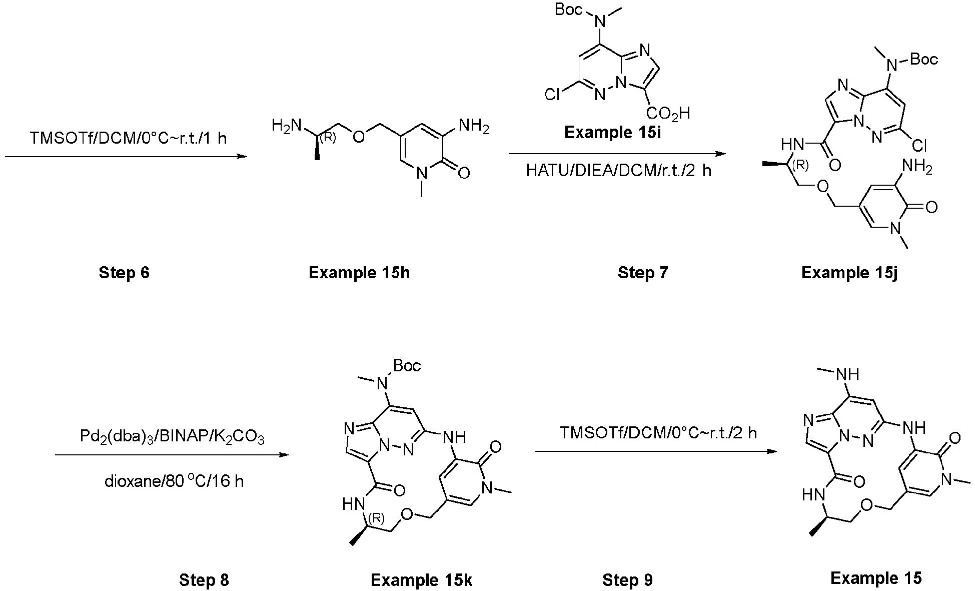

### Step 1: Example 15b

To a solution of **Example 15a** (15.0 g, 87.2 mmol) in CCl₄ (500 mL) were added NBS (31.0 g, 174.4 mmol) and AIBN (2.86 g, 17.4 mmol). The reaction mixture was stirred at 80°C for 20 h under N₂. After filtration, the filtrate was concentrated, and the residue was purified by silica gel flash column chromatography to afford the product **Example 15b** (7.5 g, yield: 34.2%) as yellow oil. LCMS [M+1] ⁺ = 252.9.

### Step 2: Example 15d

To a solution of **Example 15c** (5.8 g, 33.4 mmol) in THF (250 mL) was added NaH (1.3 g, 60% in mineral oil, 33.4 mmol) in portions at 0°C. The mixture was stirred for 5 min at the same temperature, then **Example 15b** (7.0 g, 27.8 mmol) in THF was added dropwise. The reaction mixture was stirred at r.t. for 1 h. After the solvent was concentrated, the residue was purified by silica gel flash column chromatography to afford the product **Example 15d** (2.6 g, yield: 30.0%) as yellow oil. LCMS [M+1] ⁺ = 346.2.

### Step 3: Example 15e

To a mixture of **Example 15d** (2.5 g, 7.2 mmol) in H₂O (50 mL) was added NaOH (1.2 g, 28.9 mmol). The mixture was stirred at 50°C for 16 h. After cooled to room temperature, the reaction solution was concentrated in vacuo to afford the desired product **Example 15e (3.7** g, crude, yield: quant.) as a yellow solid. LCMS [M+1] ⁺ = 328.3.

### Step 4: Example 15f

To a solution of **Example 15e** (3.7 g, crude, 7.2 mmol) in DMF (50 mL) was added CH₃I (2.4 g,17.0 mmol). The reaction mixture was stirred at r.t. for 6 h. After concentration, the residue was diluted with EtOAc (100 mL), washed with H₂O (100 mL), dried over anhydrous Na₂SO₄ and concentrated. The residue was purified by silica gel flash column chromatography (THF/Petroleum Ether = 4/1) to afford the product **Example 15f** (860 mg, yield: 35.0% for 2 steps) as yellow oil. LCMS [M+1] ⁺ = 342.2.

### Step 5: Example 15g

**Example 15f** (820 mg, 2.4 mmol) was dissolved in MeOH (20 mL) and then Pd/C (80 mg) was added in portions under N₂ protection. The mixture was degassed under vacuum and purged with H₂ for three times. The mixture was stirred for 2 h at r.t. under H₂ balloon. The solid was filtered off and the filtrate was concentrated. The residue was purified by silica gel flash column chromatography to afford the product **Example 15g** (380 mg, yield: 50.8%) as yellow oil. LCMS [M+1] ⁺ = 312.2.

### Step 6: Example 15h

To a solution of **Example 15g** (370 mg, 1.2 mmol) in DCM (10 mL) was added TMSOTf (396 mg, 1.8 mmol) at 0°C. The reaction mixture was stirred at r.t. for 1 h. The solvent was concentrated in vacuo to afford the desired product **Example 15h** (430 mg, crude) as yellow oil. LCMS [M+1] ⁺ = 212.2.

### Step 7: Example 15j

To a solution of **Example 15i** (260 mg, 0.8 mmol, from **Example 6f)** in DCM (20 mL) were added DIEA (411 mg, 3.2 mmol) and HATU (303 mg, 0.8 mmol). The mixture was stirred for 5 min, and then **Example 15h** (420 mg, crude, 2.0 mmol) was added. The resulting mixture was stirred for 2 h at r.t. The solvent was removed and the residue was purified by silica gel flash column chromatography to afford the desired product **Example 15j** (200 mg, 30.6% yield) as a yellow solid. LCMS [M+1]⁺ =520.2.

### Step 8: Example 15k

To a solution of **Example 15j** (190 mg, 0.37 mmol) in dioxane (20 mL) were added K₂CO₃ (101 mg, 0.73 mmol), BINAP (228 mg, 0.37 mmol) and Pd₂(dba)₃CHCl₃ (189 mg, 0.18 mmol). The reaction mixture was stirred at 80°C for 16 h under N₂. After cooled to room temperature, the solvent was removed, and the residue was purified by Pre-TLC to afford the product **Example 15k** (50 mg, 28.3% yield) as a yellow solid. LCMS [M+1] ⁺ = 484.4.

### Step 9: Example 15

To a solution of **Example 15k** (45 mg, 0.09 mmol) in DCM (5 mL) was added TMSOTf (41 mg, 0.02 mmol) at 0°C. The reaction mixture was stirred at r.t. for 2 h. The reaction solution was concentrated in vacuo and the residue was purified by Pre-TLC to afford the product **Example 15** (15.3 mg, yield: 42.9%) as an off-white solid. LCMS [M+1] ⁺ = 384.3. ¹H NMR (300 MHz, DMSO-*d*₆) δ 8.73 - 8.63 (m, 2H), 8.10 (d, 1H), 7.83 (s, 1H), 7.46 (q, 1H), 7.33 (d, 1H), 6.34 (s, 1H), 4.47 (d, 1H), 4.23 (d, 1H), 4.05 - 4.03 (m, 1H), 3.53 (s, 3H), 3.48(d, 1H),3.40 (d, 1H), 2.87 (d, 3H), 1.13 (d, 3H).

### Example 16

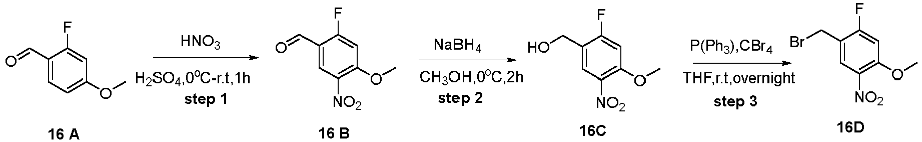

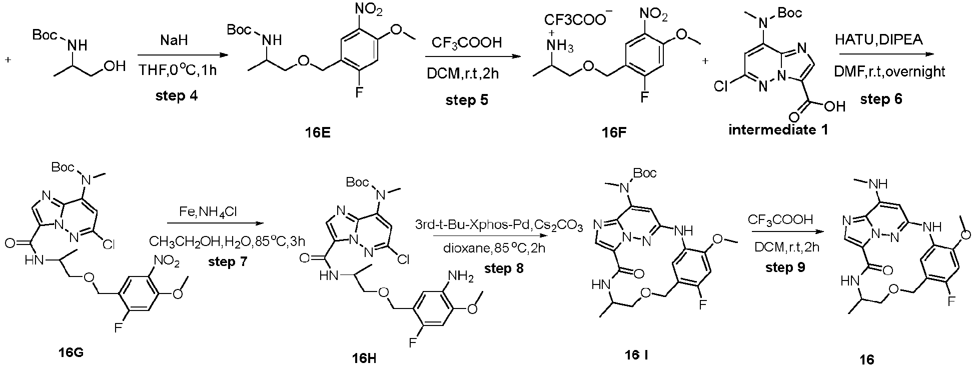

### Step 1: 2-fluoro-4-methoxy-5-nitro-benzaldehyde (16B)

2-fluoro-4-methoxy-benzaldehyde **(16A)** (5 g, 32.46 mmol) was dissolved in concentrated sulfuric acid (30 mL) and cooled to -10°C. Concentrated nitric acid (2.1 mL) in concentrated sulfur acid (4 mL) was added dropwise over 20 min. After an additional hour of stirring at below -10°C, the mixture was poured into crushed ice. the precipitate was collected by filtration and partitioned between dichloromethane (40 mL) and saturated sodium hydrogen carbonate (30 mL). The organic layer was dried (Na₂SO₄) and evaporated in vacuo to give the title compound **(16 B)** (5.2g, 80.50%) as a cream solid. LC-MS (ESI): m/z =200.1 [M+H]⁺.

### Step 2: (2-fluoro-4-methoxy-5-nitro-phenyl)methanol (16C)

Sodium borohydride (0.304 g,8.04 mmol) was added portionwise to a stirring solution of 2-fluoro-4-methoxy-5-nitro-benzaldehyde (**16B**)(0.8 g,4.02 mmol) in methanol (10mL) at 0°C. After 2 hours, the methanol was removed in vacuo. The residue was treated with cold water and extracted with dichloromethane. The combined organic layer was washed with brine, dried (Na₂SO₄) and then evaporated in vacuo to give the title compound **(16C)** as a crude solid (0.79 g, 97.77%). LC-MS (ESI): m/z =202.1 [M+H]⁺

### Step 3: 1-(bromomethyl)-2-fluoro-4-methoxy-5-nitro-benzene (16D)

Carbon tetrabromide (2.64 g,7.96 mmol) in anhydrous diethyl ether (5 mL) was added dropwise to a stirred solution of (2-fluoro-4-methoxy-5-nitro-phenyl)methanol **(16C)** (0.8 g,3.98 mmol) and triphephosphine (2.08 g,7.96 mmol) in anhydrous diethyl ether (15 mL). The mixture was stirred overnight before it was concentrated. chromatography with ethyl acetate in hexane (0-10%) gave the title compound **(16D)** as pale yellow solid (0.69 g, 66.34%). LC-MS (ESI): m/z =264.1 [M+H]⁺

### Step 4 : tert-butyl N-[2-[(2-fluoro-4-methoxy-5-nitro-phenyl)methoxy]-1-methyl-ethyl] carbamate (16E)

Sodium hydride (105 mg, 2.62 mmol) was added portionwise to a stirred solution of tert-butyl N-(2-hydroxy-1-methyl-ethyl)carbamate (0.46 g, 2.62 mmol) in THF (15 mL) at 0°C, the mixture was stirred at 0°C for 10 min. then 1-(bromomethyl)-2- fluoro-4-methoxy-5-nitro-benzene (**16D**)(0.69 g,2.62 mmol) was added to the mixture at 0°C, after 30 min, The mixture was treated with cold water and extracted with ethyl acetate. The combined organic layer was washed with brine, dried (Na₂SO₄) and then the residue was purified by flash chromatography to afford the title compound (**16E**)(0.1 g, 10.65%) as a brown solid. LC-MS (ESI): m/z =381.1 [M+23]⁺. ¹H NMR (400 MHz, CDCl₃) δ 8.02 (d, 1H), 6.78 (d, 1H), 4.60 (s, 1H), 4.53 (q, 2H), 3.96 (s, 3H), 3.91- 3.83(m, 1H), 3.49- 3.43 (m, 2H), 1.44 (s, 9H), 1.18 (d, 3H).

### Step 5 : [2-[(5-amino-2-fluoro-4-methoxy-phenyl)methoxy]-1-methyl-ethyl]ammonium;2,2,2-trifluoroacetate (16F)

Trifluoroacetic acid (1 mL) was added to a solution of N-[2-[(2-fluoro-4-methoxy- 5-nitrophenyl)methoxy]-1-methyl-ethyl]carbamate(16E)(0.1 g, 0.28mmol) in DCM (3 mL), The mixture was stirred 2 h, The mixture solution was evaporated to dryness, then the title compound **(16F)** (0.1 g. 100%) was obtained as brown liquid, which was used in the next step without further purification. LC-MS (ESI): m/z =259.2 [M+H]⁺

### Step 6 : tert-butyl N-[6-chloro-3-[[2-[(2-fluoro-4-methoxy-5-nitro-phenyl)methoxy]-1-methyl-ethyl] carbamoyl]imidazo[1,2-b]pyridazin-8-yl]carbamate (16G)

[2-[(5-amino-2-fluoro-4-methoxy-phenyl)methoxy]-1-methyl-ethyl]ammonium;2,2,2-trifluoroacetate (**16F**)(0.1g, 0.27 mmol) was dissolved in DMF (5 mL), HATU (0.153 g, 0.4 mmol), DIPEA( 0.07 g, 0.54 mmol) and **intermediate 1** (0.09 g, 0.27 mmol) were added to the solution in room temperature. After 18 h, the solution mixture was diluted with EA (30 mL), washed with water (2× 30 mL) and brine (30 mL), dried with Na₂SO₄ and concentrated. The crude product was purified by flash chromatography (PE/EA = 3:1) to afford the title compound **(16G)** (0.06 g, 39.47%) as a white solid. LC-MS (ESI): m/z =567.2 [M+H]⁺

### Step 7 : tert-butyl N-[3-[[2-[(5-amino-2-fluoro-4-methoxy-phenyl)methoxy]-1-methylethyl]carbamoyl]-6-chloro-imidazo[1,2-b]pyridazin-8-yl]-N-methyl-carbamate (H)

tert-butyl N-[6-chloro-3-[[2-[(2-fluoro-4-methoxy-5-nitro-phenyl)methoxy] -1-methylethyl]carbamoyl]imidazo[1,2-b]pyridazin-8-yl]carbamate **(16G)** (0.06 g, 0.1 mmol) was dissolved in ethanol (9 mL) and H₂O (3 mL),Fe powder (60 mg,1.06 mmol) and NH₄Cl(34 mg,0.64 mmol) were added to solution, then the reaction mixture heated to 85 °C for 3 h, After cooling to room temperature, reaction filtered, filtrate was removed in vacuo. The residue was purified by flash chromatography to afford the title compound **(16H)** (0.044 g, 78.57%) as a white solid. LC-MS (ESI): m/z =537.1 [M+H]⁺

### Step 8 : tert-butyl (E)-(3⁴-fluoro-3⁶-methoxy-7-methyl-9-oxo-5-oxa-2,8-diaza-1(6,3)-imidazo[1,2-b]pyridazina-3(1,3)-benzenacyclononaphane-1⁸-yl)(methyl)carbamate (161)

To a solution of **(16H)** (44 mg, 0.082 mmol) in 1,4-dioxane ( 20 mL) were added Cs₂CO₃ (80 mg, 0.25 mmol) and 3rd-*t*-Bu-Xphos-Pd (30 mg). The reaction mixture was stirred at 85°C for 2 h under N₂. After cooled to room temperature, the solvent was removed, and the residue was purified by silica gel flash column chromatography to afford the product **(161)** (22 mg,53.65%) as a white solid. LC-MS (ESI): m/z =501.3 [M+H]⁺

### Step 9 : tert-butyl (E)-(3⁴-fluoro-3⁶-methoxy-7-methyl-9-oxo-5-oxa-2,8-diaza-1(6,3)-imidazo[1,2-b]pyridazina-3(1,3)-benzenacyclononaphane-1⁸-yl)(methyl)carbamate (16)

A solution of (16I) (22 mg, 0.044 mmol) and trifluoroacetic acid (0.5 mL) in DCM (4 mL) was stirred at room temperature for 2 h. Solvent was evaporated, and the crude product was partitioned between water and DCM. The aqueous layer was basified with NaHCO₃ and extracted with DCM. Combined organic layers were washed with brine, dried over sodium sulfate, filtered, and evaporated, the residue was purified by silica gel flash column chromatography to afford the product 16 (5 mg, 28.57%) as a white solid. 1H NMR (400 MHz, CDCl₃) δ 8.71 (d, 1H), 8.28 (d, 1H), 8.07 (s, 1H), 6.74 - 6.60 (m, 3H), 5.64 (s, 1H), 4.69 - 2.59 (m, 2H), 4.28 - 4.21 (m, 1H), 3.93 (d, 3H), 3.57 (dd, 1H), 3.43 - 3.37 (m, 1H), 3.05 (d, 3H), 1.29 (d, 3H). LC-MS (ESI): m/z =401.2 [M+H]⁺

### Example 17

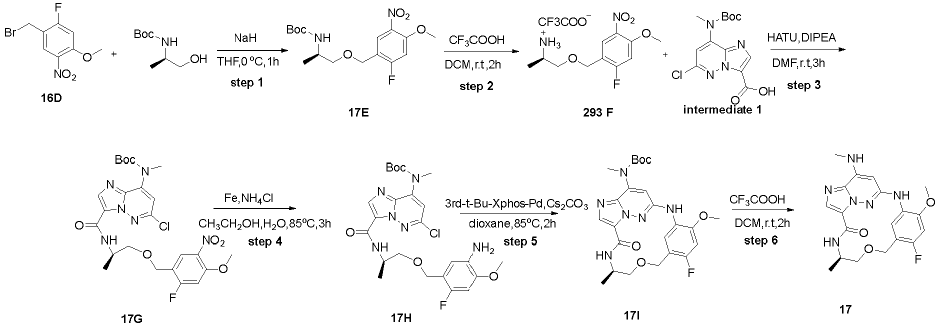

### Step 1 : tert-butyl N-[(1R)-2-[(2-fluoro-4-methoxy-5-nitro-phenyl)methoxy]-1-methylethyl]carbamate (17E)

Sodium hydride (228 mg, 5.70 mmol) was added portionwise to a stirred solution of tert-butyl N-[(1R)-2-hydroxy-1-methyl-ethyl]carbamate (1 g, 5.70 mmol) in THF (30 mL) at 0°C, the mixture was stirred at 0°C for 10 min then 1-(bromomethyl)-2- fluoro-4-methoxy-5-nitro-benzene (**16D**)(1.5 g,5.70 mmol) was added to the mixture at 0°C,after 30 min, The mixture was treated with cold water and extracted with ethyl acetate. The combined organic layer was washed with brine, dried (Na₂SO₄) and then the residue was purified by flash chromatography to afford the title compound **(17E)** (0.57 g, 27.94%) as a white solid. LC-MS (ESI): m/z =359.1 [M+H]⁺

### Step 2 : [(1R)-2-[(2-fluoro-4-methoxy-5-nitro-phenyl)methoxy]-1-methyl-ethyl]ammonium;2,2,2-trifluoroacetate (17F)

Trifluoroacetic acid (3 mL) was added to a solution of tert-butyl N-[(1R)-2-[(2-fluoro-4-methoxy-5-nitro-phenyl)methoxy]-1-methyl-ethyl]carbamate **(17E)**(0.57 g, 1.53 mmol) in DCM (8 mL), The mixture was stirred overnight, The mixture solution was evaporated to dryness, then the title compound **(17F)** (0.54 g. 91.21%) was obtained as brown liquid, which was used in the next step without further purification. LC-MS (ESI): m/z =259.2 [M+H]⁺

### Step 3 : tert-butyl N-[6-chloro-3-[[(1R)-2-[(2-fluoro-4-methoxy-5-nitro-phenyl)methoxy]-1-methylethyl]carbamoyl]imidazo[1,2-b]pyridazin-8-yl]carbamate (17G)

[(1R)-2-[(2-fluoro-4-methoxy-5-nitro-phenyl)methoxy]-1-methyl-ethyl]ammonium;2,2,2-trifluoroacetate **(17F)** (0.54 g, 1.45 mmol) was dissolved in DMF (15 mL),HATU (0.827 g, 2.17 mmol), DIPEA(0.374 g, 2.9 mmol) and **intermediate 1**(0.473 g, 1.45 mmol) were added to the solution in room temperature. After 18h, the solution mixture was diluted with EA (50 mL), washed with water (2×50 mL) and brine (50 mL), dried with Na₂SO₄ and concentrated. The crude product was purified by flash chromatography to afford the title compound **(17G)** (0.512 g, 62.36%) as a white solid. LC-MS (ESI): m/z =567.2 [M+H]⁺

### Step 4 : tert-butyl N-[3-[[(1R)-2-[(5-amino-2-fluoro-4-methoxy-phenyl)methoxy]-1-methylethyl]carbamoyl]-6-chloro-imidazo[1,2-b]pyridazin-8-yl]carbamate (17H)

tert-butyl N-[6-chloro-3-[[(1R)-2-[(2-fluoro-4-methoxy-5-nitro-phenyl)methoxy]-1-methylethyl]carbamoyl]imidazo[1,2-b]pyridazin-8-yl]carbamate (**17G**)(0.512 g, 0.9 mmol) was dissolved in ethanol (50 mL) and H₂O (15 mL), Fe (506 mg, 9.04 mmol) and NH₄Cl(290 mg, 5.42 mmol) were added, then the reaction mixture heated to 85 °C for 3 h, After cooling to room temperature, filtered, filtrate was removed in vacuo. The residue was purified by flash chromatography (PE/EA = 3:1) to afford the title compound **(17H)**(0.44 g, 90.90%) as a white solid. LC-MS (ESI): m/z =537.1 [M+H]⁺

### Step 5 : tert-butyl ((7R,E)-3⁴-fluoro-3⁶-methoxy-7-methyl-9-oxo-5-oxa-2,8-diaza-1(6,3)-imidazo[1,2-b]pyridazina-3(1,3)-benzenacyclononaphane-1⁸-yl)(methyl)carbamate (171)

To a solution of **(17H)** (440 mg, 0.82 mmol) in 1,4-dioxane (80 mL) was added Cs₂CO₃ (802 mg, 2.46 mmol) and 3rd-*t*-Bu-Xphos-Pd (280 mg). The reaction mixture was stirred at 80°C for 2 h under N₂. After cooled to room temperature, the solvent was removed, and the residue was purified by silica gel flash column chromatography to afford the desired product **(17 I)** (230 mg , 56.09%) as a white solid. LC-MS (ESI): m/z =501.3 [M+H]⁺.

### Step 6 : (E)-3⁴-fluoro-3⁶-methoxy-7-methyl-1⁸-(methylamino)-5-oxa-2,8-diaza-1(6,3)-imidazo[1,2-b]pyridazina-3(1,3)-benzenacyclononaphan-9-one (17)

A solution of **(17)** (230 mg, 0.46 mmol) and trifluoroacetic acid (2 mL) in DCM (10 mL) was stirred at room temperature for 2 h. Solvent was evaporated, and the crude product was partitioned between water and DCM. The aqueous layer was quenched with NaHCO₃ and extracted with DCM. Combined organic layers were washed with brine, dried over sodium sulfate, filtered, and evaporated, the residue was purified by silica gel flash column chromatography (PE/EA = 2:1) to afford the product **17** (65mg, 35.32%) as a white solid. ¹H NMR (400 MHz, CDCl₃) δ 8.74 (d, 1H), 8.30 (d, 1H), 8.05 (s, 1H), 6.70 - 6.55 (m, 2H), 6.21 (s, 1H), 5.59 (s, 1H), 4.72 - 4.55 (m, 2H), 4.31 - 4.21 (m, 1H), 3.92 (s, 3H), 3.59- 3.55 (dd, 1H), 3.44 - 3.38 (m, 1H) ,3.04 (d, 3H), 1.29 (d, 3H). LC-MS (ESI): m/z =401.2 [M+H]⁺.

### Example 18

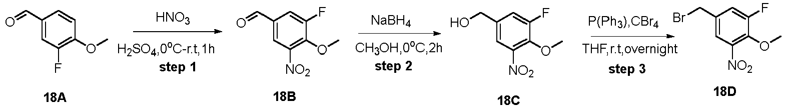

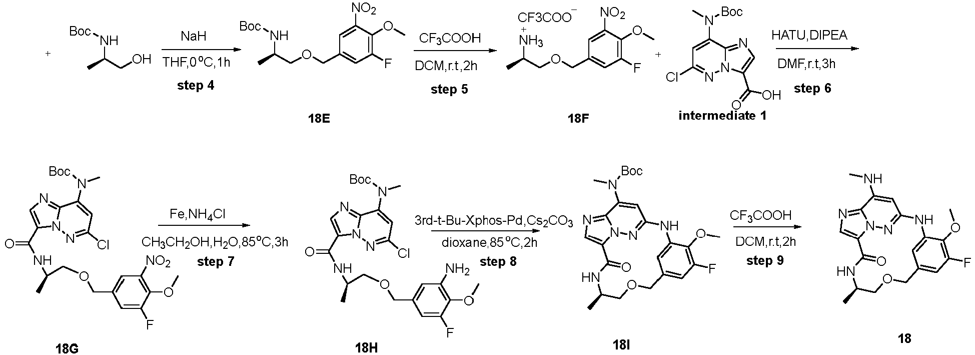

### Step 1: 3-fluoro-4-methoxy-5-nitro-benzaldehyde (18B)

3-fluoro-4-methoxy-benzaldehyde **(18A)** (3.6 g, 23.37 mmol ) was dissolved in concentrated sulfuric acid (30 mL) and cooled to -10°C. Concentrated nitric acid (2.5 mL) in concentrated sulfur acid (4 mL) was added dropwise over 20 min. After an additional hour of stirring at below -10°C, the mixture was poured into crushed ice. the precipitate was collected by filtration and partitioned between dichloromethane (40 mL) and saturated sodium hydrogen carbonate (30mL). The organic layer was dried (Na₂SO₄) and evaporated in vacuo to give the title compound (18B) (2.5 g, 53.76%) as an oil. LC-MS (ESI): m/z =200.1 [M+H]⁺.

### Step 2: (3-fluoro-4-methoxy-5-nitro-phenyl)methanol (18C)

To a stirring solution of 3-fluoro-4methoxy-5-nitro-benzaldehyde (1 g,5.02 mmol) in methanol (20 mL) was added sodium borohydride (0.38 g,10.04 mmol) portionwise at 0°C. After 2 hours, the methanol was removed in vacuo. The residue was treated with cold water and extracted with dichloromethane. The combined organic layer was washed with brine, dried (Na₂SO₄) and then evaporated in vacuo to give the title compound (**18C**)as a crude solid(1 g,99.0%). LC-MS (ESI): m/z =202.1 [M+H]⁺.

### Step 31-(bromomethyl)-3-fluoro-4-methoxy-5-nitro-benzene (18D).

To a solution of (3-fluoro-4-methoxy-5-nitro-phenyl)methanol (1 g, 4.97 mmol) and triphephosphine (2.61g, 9.95 mmol) in anhydrous diethyl ether (30 mL) was added carbon tetrabromide (3.3 g, 9.95 mmol) in anhydrous diethyl ether (5 mL) dropwise. The mixture was stirred overnight before it was concentrated down to a sticky oil. Silica gel chromatography gave the title compound **(18D)** as a pale yellow solid (0.95g, 73.07%). LC-MS (ESI): m/z =264.1 [M+H]⁺

### Step 4 : tert-butyl N-[(1R)-2-[(3-fluoro-4-methoxy-5-nitro-phenyl)methoxy]-1-methylethyl]carbamate (18 E)

To a stirred solution of tert-butyl N-(2-hydroxy-1-methyl-ethyl)carbamate (0.63 g, 3.61 mmol) in THF (15 mL) was added sodium hydride (144 mg, 3.61 mmol) portionwise at 0°C, the mixture was stirred at 0°C for 10 min. then 1-(bromomethyl)-3- fluoro-4-methoxy-5-nitro-benzene (**18D**)(0.95 g, 3.61 mmol) was added to the mixture at 0°C, after 30 min, the mixture was quenched with cold water and extracted with ethyl acetate. The combined organic layer was washed with brine, dried (Na₂SO₄) and then the residue was purified by flash chromatography to afford the title compound (**18E**)(0.63 g, 48.83%) as a brown solid. LC-MS (ESI): m/z =359.1 [M+H]⁺.

### Step 5 : [(1R)-2-[(3-fluoro-4-methoxy-5-nitro-phenyl)methoxy]-1-methyl-ethyl]ammonium;2,2,2-trifluoroacetate (18F)

Trifluoroacetic acid (1.5 mL) was added to a solution of N-[2-[(3-fluoro-4-methoxy- 5-nitrophenyl)methoxy]-1-methyl-ethyl]carbamate **(18E)** (0.63g, 1.76 mmol) in DCM(5 mL), The mixture was stirred 2 h, The mixture solution was evaporated to dryness, then the title compound **(18F)** (0.6 g. 91.46%) was obtained as brown liquid, which was used in the next step without further purification. LC-MS (ESI): m/z =259.2 [M+H]⁺

### Step 6 : tert-butyl N-[6-chloro-3-[[(1R)-2-[(3-fluoro-4-methoxy-5-nitro-phenyl)methoxy]-1-methylethyl]carbamoyl]imidazo[1,2-b]pyridazin-8-yl]-N-methyl-carbamate (18G)

[(1R)-2-[(3-fluoro-4-methoxy-5-nitro-phenyl)methoxy]-1-methyl-ethyl]ammonium;2,2,2-trifluoroacetate (18F)(0.6 g, 1.6 mmol) was dissolved in DMF (10 mL),HATU (0.91g, 2.41mmol), DIPEA(0.41g, 3.2 mmol) and **intermediate** 1(0.52 g, 1.6 mmol) were added to the solution in room temperature. After 18h, the solution mixture was diluted with EA (50 mL), washed with water (2×50 mL) and brine (50 mL), dried with Na₂SO₄ and concentrated. The crude product was purified by flash chromatography to afford the title compound **(18G)** (545 mg, 59.89%) as a white solid. LC-MS (ESI): m/z =567.2 [M+H]⁺

### Step 7 : tert-butyl N-[3-[[(1R)-2-[(3-amino-5-fluoro-4-methoxy-phenyl)methoxy]-1-methylethyl]carbamoyl]-6-chloro-imidazo[1,2-b]pyridazin-8-yl]carbamate (18H)

tert-butyl N-[6-chloro-3-[[2-[(2-fluoro-4-methoxy-5-nitro-phenyl)methoxy]-1-methylethyl]carbamoyl]imidazo[1,2-b]pyridazin-8-yl]carbamate(**18G**) (545 mg, 0.96 mmol) was dissolved in ethanol (45 mL) and H₂O (15 mL), iron powder (540 mg,9.62 mmol) and NH₄Cl(310 mg,5.77 mmol) were added to solution, then the reaction mixture heated to 85 °C for 3 h, After cooling to room temperature, reaction filtered, filtrate was removed in vacuo, The residue was purified by flash chromatography (PE/EA = 2:1) to afford the title compound **(18H)**(450 mg, 87.2%) as a white solid. LC-MS (ESI): m/z =537.1 [M+H]⁺

### Step 8 : tert-butyl ((7R,E)-3⁵-fluoro-3⁶-methoxy-7-methyl-9-oxo-5-oxa-2,8-diaza-1(6,3)-imidazo[1,2-b]pyridazina-3(1,3)-benzenacyclononaphane-1⁸-yl)(methyl)carbamate(18I)

To a solution of **(18H)** (450 mg, 0.84 mmol) in 1,4-dioxane (100 mL) were added Cs₂CO₃ (820 mg, 2.51 mmol) and 3rd-*t*-Bu-Xphos-Pd (250 mg). The reaction mixture was stirred at 80°C for 2 h under N₂. After cooled to room temperature, the solvent was removed, and the residue was purified by silica gel flash column chromatography to afford the product **(181)** (220 mg, 52.50%) as a white solid. LC-MS (ESI): m/z =501.3 [M+H]⁺.

### Step 9 : (7R,E)-3⁵-fluoro-3⁶-methoxy-7-methyl-1⁸-(methylamino)-5-oxa-2,8-diaza-1(6, 3)-imidazo[1, 2-b]pyridazina-3(1, 3)-benzenacyclononaphan-9-one (18)

A solution of **(181)** (220 mg, 0.44 mmol) and trifluoroacetic acid (1 mL) in DCM (5 mL) was stirred at room temperature for 2 h. Solvent was evaporated, and the crude product was partitioned between water and DCM. The aqueous layer was basified with NaHCO₃ and extracted with DCM. Combined organic layers were washed with brine, dried over sodium sulfate, filtered, and evaporated, the residue was purified by silica gel flash column chromatography to afford the product 18 (71 mg, 40.34%) as a white solid. ¹H NMR (400 MHz, CDCl₃) δ 8.76 (d, 1H), 8.20 (s, 1H), 8.07 (s, 1H), 6.87 (s, 1H), 6.63 - 6.58 (m, 1H), 6.40 (s, 1H), 5.63 (s, 1H), 4.67 (d, 1H), 4.38 (d, 1H), 4.30 - 4.20 (m, 1H), 4.05 (d, 3H), 3.60 - 3.56 (m, 1H), 3.48 - 3.38 (m, 1H), 3.06 (d, 3H), 1.29 (d, 3H). LC-MS (ESI): m/z =401.2 [M+H]⁺.

### Example 19

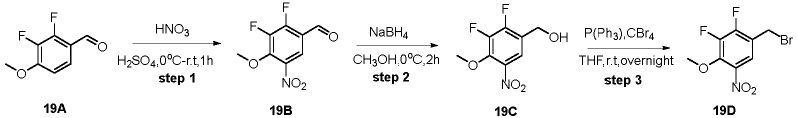

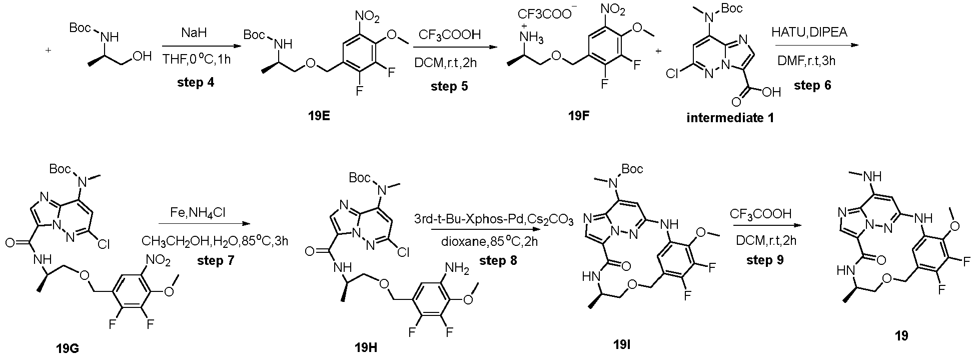

### Step 1: 2,3-difluoro-4-methoxy-5-nitro-benzaldehyde (19B)

2, 3-difluoro-4-methoxy-benzaldehyde **(19A)** (3 g,17.43 mmol ) was dissolved in concentrated sulfuric acid (18 mL) and cooled to -10°C. Concentrated nitric acid (1.5 mL) in concentrated sulfur acid (3 mL) was added dropwise over 10 min. After an additional hour of stirring at below -10°C, the mixture was poured into crushed ice. the precipitate was collected by filtration and partitioned between dichloromethane (30 mL) and saturated sodium hydrogen carbonate (30 mL). The organic layer was dried (Na₂SO₄) and evaporated in vacuo to give the title compound **(19B)** (3.1g, 82.01%) as a white solid. LC-MS (ESI): m/z =218.1 [M+H]⁺

### Step 2: (2,3-difluoro-4-methoxy-5-nitro-phenyl)methanol (19C)

Sodium borohydride (1.08 g,28.56 mmol) was added portionwise to a stirring solution of 2,3-difluoro-4-methoxy-5-nitro-benzaldehyde **(19B)** (3.1g,14.28 mmol) in methanol (60 mL) at 0°C. After 2 hours, the methanol was removed in vacuo. The residue was treated with cold water and extracted with dichloromethane. The combined organic layer was washed with brine, dried (Na₂SO₄) and then evaporated in vacuo to give the title compound **(19C)** as a little yellow solid(2.8 g, 89.51%). LC-MS (ESI): m/z = 220.1 [M+H]⁺

### Step 3: 1-(bromomethyl)-2,3-difluoro-4-methoxy-5-nitro-benzene (19D)

Carbon tetrabromide (8.47 g,25.56 mmol) in anhydrous diethyl ether (30 mL) was added dropwise to a stirred solution of (2,3-difluoro-4-methoxy-5-nitro-phenyl)methanol **(19C)** (2.8 g,12.78 mmol) and triphephosphine (6.7 g,25.56 mmol) in anhydrous diethyl ether (100 mL). The mixture was stirred overnight before it was concentrated down to a sticky oil. Silica gel chromatography with ethyl acetate in hexane (0-10%) gave the title compound **(19D)** as a pale yellow solid (2.12 g, 59.05%). LC-MS (ESI): m/z =281.9 [M+H]⁺

### Step 4: tert-butyl N-[(1R)-2-[(2,3-difluoro-4-methoxy-5-nitro-phenyl)methoxy]-1-methylethyl]carbamate (19E)

Sodium hydride (92 mg, 2.31mmol) was added portionwise to a stirred solution of tert-butyl N-[(1R)-2-hydroxy-1-methyl-ethyl]carbamate (0.405 g, 2.31 mmol) in THF (15 mL) at 0°C, the mixture was stirred at 0°C for 10 min. then 1-(bromomethyl)-2,3-difluoro-4-methoxy-5-nitro-benzene **(19D)** (0.65 g, 2.31 mmol) was added to the mixture at 0°C, after 10 min, The mixture was treated with cold water and extracted with ethyl acetate. The combined organic layer was washed with brine, dried (Na₂SO₄) and then the residue was purified by flash chromatography to afford the title compound (19E)(0.3 g, 34.48%) as a brown solid. LC-MS (ESI): m/z =377.1 [M +H]⁺

### Step 5: [(1R)-2-[(2,3-difluoro-4-methoxy-5-nitro-phenyl)methoxy]-1-methylethyl]ammonium;2,2,2-trifluoroacetate (19F)

Trifluoroacetic acid (1.5 mL) was added to a solution of tert-butyl N-[(1R)-2-[(2,3-difluoro-4-methoxy-5-nitro-phenyl)methoxy]-1-methyl-ethyl] carbamate (**19E**)(0.3 g, 0.8 mmol) in DCM(5 mL), The mixture was stirred 2 h, the mixture solution was evaporated to dryness, then the title compound(19F) (0.28 g. 90.03%) was obtained as brown liquid, which was used in the next step without further purification. LC-MS (ESI): m/z =277.2 [M+H]⁺

### Step 6: tert-butyl N-[6-chloro-3-[[(1R)-2-[(2,3-difluoro-4-methoxy-5-nitro-phenyl)methoxy]-1-methyl-ethyl]carbamoyl]imidazo[1,2-b]pyridazin-8-yl]carbamate (19G)

[(1R)-2-[(2,3-difluoro-4-methoxy-5-nitro-phenyl)methoxy]-1-methyl-ethyl]ammonium;2,2,2-trifluoroacetate(**19F**)(0.28 g, 0.71 mmol) was dissolved in DMF (5 mL), HATU (0.41 g, 1.07 mmol), DIPEA(0.185 g, 1.43 mmol) and **intermediate 1**(0.24 g, 0.71 mmol) were added to the solution in room temperature. After 18 h, the solution mixture was diluted with EA (50 mL), washed with water (2×50 mL) and brine (50 mL), dried with Na₂SO₄ and concentrated. The crude product was purified by flash chromatography (PE/EA = 2:1) to afford the title compound **(19G)** (0.18 g, 42.95%) as a white solid. LC-MS (ESI): m/z =585.2 [M+H]⁺

### Step 7: tert-butyl N-[3-[[(1R)-2-[(5-amino-2,3-difluoro-4-methoxy-phenyl)methoxy]-1-methylethyl]carbamoyl]-6-chloro-imidazo[1,2-b]pyridazin-8-yl]carbamate (19H)

tert-butylN-[6-chloro-3-[[(1R)-2-[(2,3-difluoro-4-methoxy-5-nitro-phenyl)methoxy]-1-methylethyl]carbamoyl]imidazo[1,2-b]pyridazin-8-yl]carbamate **(19G)** (0.18 g, 0.3 mmol) was dissolved in ethanol (30 mL) and H₂O (10 mL), Fe powder (172 mg,3.08 mmol) and NH₄Cl (100 mg,1.85mmol) were added to solution, then the reaction mixture heated to 85 °C for 3 h, After cooling to room temperature, reaction filtered, filtrate was removed in vacuo, The residue was purified by flash chromatography to afford the title compound **(19H)** (80 mg, 47.05%) as a white solid. LC-MS (ESI): m/z =555.2 [M+H]⁺

### Step 8: tert-butyl ((7R,E)-3⁴,3⁵-difluoro-36-methoxy-7-methyl-9-oxo-5-oxa-2,8-diaza-1(6,3)-imidazo[1,2-b]pyridazina-3(1,3)-benzenacyclononaphane-1⁸-yl)(methyl)carbamate (191)

To a solution of **(19H)** (80 mg, 0.14 mmol) in 1,4-dioxane (40 mL) were added Cs₂CO₃ (141 mg, 0.43 mmol) and 3rd-*t*-Bu-Xphos-Pd (50 mg). The reaction mixture was stirred at 80°C for 2 h under N₂. After cooled to room temperature, the solvent was removed, and the residue was purified by silica gel flash column chromatography to afford the product **(191)** (42 mg, 47.05%) as a white solid. LC-MS (ESI): m/z =519.3 [M+H]⁺

### Step 9: (7R,E)-3⁴,3⁵-difluoro-3⁶-methoxy-7-methyl-1⁸-(methylamino)-5-oxa-2,8-diaza-1(6,3)-imidazo[1,2-b]pyridazina-3(1,3)-benzenacyclononaphan-9-one

A solution **of 19I** (42mg, 0.081mmol) and trifluoroacetic acid (0.3 mL) in DCM (4 mL) was stirred at room temperature for 2 h. Solvent was evaporated, and the crude product was partitioned between water and DCM. The aqueous layer was basified with NaHCO₃ and extracted with DCM. Combined organic layers were washed with brine, dried over sodium sulfate, filtered, and evaporated, the residue was purified by silica gel flash column chromatography to afford the product **19** (12 mg, 36.36%) as a white solid. ¹H NMR (400 MHz, CDCl₃) δ 8.67 (d, 1H), 8.14 (dd, 1H), 8.08 (s, 1H), 6.72 (s, 1H), 6.58 (s, 1H), 5.63 (s, 1H), 4.71 -4.60 (m, 2H), 4.31 -4.21 (m, 1H), 4.10 (d, 3H), 3.61 (dd, 1H), 3.46 - 3.39 (m, 1H), 3.06 (d, 3H), 1.31 (d, 3H). LC-MS (ESI): m/z =419.2[M+H]⁺.

### Example 20

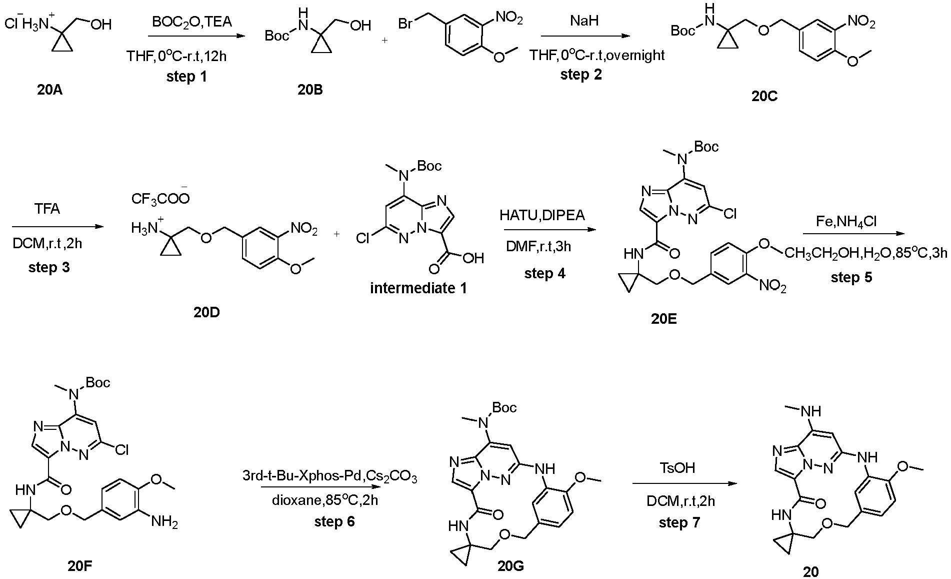

### Step 1: tert-butyl N-[1-(hydroxymethyl)cyclopropyl]carbamate (20B)

Triethylamine (4.93 g,48.76 mmol) was added dropwise to a stirred solution of (1-aminocyclopropyl)methanol **hydrochloride(20A)** (2 g,16.25 mmol) in THF (50 mL) at 0°C. After 10 min of stirring, di-tert-butyl dicarbonate (7.09 g,32.50mmol) in THF (5 mL) was added dropwise to the mixture at 0°C. The mixture was stirred overnight in room temperature, the solvent was removed in vacuo. The residue was diluted with ethyl acetate (60 mL), washed with water (2×60 mL) and brine (50 mL), dried with Na₂SO₄ and concentrated. then the title compound **(20B)** (3g. 100%) was obtained as white solid, which was used in the next step without further purification. ¹H NMR (400 MHz, CDCl₃) δ 5.05 (s, 1H), 3.59 (s, 2H), 2.40 (s, 1H), 1.44 (s, 9H), 0.83 (m, 4H).

### Step 2: tert-butyl N-[1-[(4-methoxy-3-nitro-phenyl)methoxymethyl]cyclopropyl]carbamate (20C)

Sodium hydride (480 mg, 12.02 mmol) was added portionwise to a stirred solution of tert-butyl N-[1-(hydroxymethyl)cyclopropyl]carbamate **(20B)**(1.5 g,8.01 mmol) in THF (60 mL) at 0°C, the mixture was stirred at 0°C for 30 min. then 4-(bromomethyl)-1-methoxy-2-nitrobenzene (1.96 g,8.01 mmol) was added to the mixture at 0°C, The mixture was stirred overnight in room temperature, the mixture was treated with cold water (80 mL) and extracted with ethyl acetate( 2×100 mL). The combined organic layer was washed with brine, dried with Na₂SO₄ and concentrated, then the residue was purified by flash chromatography to afford the title compound (**20C)**(2.4 g,85.40%) as a little yellow solid. ¹H NMR (400 MHz, CDCl₃) δ 7.76 (d, 1H), 7.46 (dd, 1H), 7.02 (d, 1H), 5.13 (s, 1H), 4.46 (s, 2H), 3.89 (s, 3H), 3.44 (s, 2H), 1.34 (s, 9H), 0.81 - 0.66 (m, 4H).

### Step 3: [1-[(4-methoxy-3-nitro-phenyl)methoxymethyl]cyclopropyl]ammonium;2,2,2-trifluoroacetate (20D)

Trifluoroacetic acid (1.5 mL) was added to a solution of tert-butyl N-[1-[(4-methoxy-3-nitrophenyl)methoxymethyl]cyclopropyl]carbamate(**20C**) (0.55 g, 1.56 mmol) in DCM(5 mL), The mixture was stirred overnight, The mixture solution was evaporated to dryness, then the title compound(**20D**)

(0.54 g. 94.57%) was obtained as brown liquid, which was used in the next step without further purification. LC-MS (ESI): m/z =253.2[M+H]⁺

### Step 4: tert-butyl N-[6-chloro-3-[[1-[(4-methoxy-3-nitrophenyl)methoxymethyl]cyclopropyl]carbamoyl]imidazo[1,2-b]pyridazin-8-yl]-N-methyl-carbamate (20E)

[1-[(4-methoxy-3-nitro-phenyl)methoxymethyl]cyclopropyl]ammonium;2,2,2-trifluoroacetate(**20D**)(0.54g, 1.47 mmol) was dissolved in DMF(10 mL),HATU(0.84 g, 2.21 mmol), DIPEA(0.38 g, 2.95 mmol) and **intermediate 1**(0.1g, 1.47 mmol)were added to the solution in room temperature. After 18h, the solution mixture was diluted with EA (50 mL), washed with water (2×50 mL) and brine (50 mL), dried with Na₂SO₄ and concentrated. The crude product was purified by flash chromatography to afford the title compound **(20E)** (0.52 g, 62.95%) as a pale solid. LC-MS (ESI): m/z =561.3[M+H]⁺

### Step 5: tert-butyl N-[3-[[1-[(3-amino-4-methoxy-phenyl)methoxymethyl]cyclopropyl]carbamoyl]-6-chloro-imidazo[1,2-b]pyridazin-8-yl]-N-methyl-carbamate (20F)

tert-butyl N-[6-chloro-3-[[1-[(4-methoxy-3-nitro phenyl)methoxymethyl]cyclopropyl]carbamoyl]imidazo[1,2-b]pyridazin-8-yl]-N-methyl-carbamate **(20E)** (0.52 g, 0.93 mmol) was dissolved in ethanol (60 mL) and H₂O (15 mL), iron powder (520 mg,9.28 mmol) and NH₄Cl (0.3 g, 5.57mmol) were added to solution, then the reaction mixture heated to 85 °C for 3 h, After cooling to room temperature, reaction filtered, filtrate was removed in vacuo. The residue was purified by flash chromatography (PE/EA = 2:1) to afford the title compound (20F)(0.32 g, 65.04%) as a white solid. LC-MS (ESI): m/z =531.3[M+H]⁺

### Step 6: tert-butyl (E)-(6'-methoxy-9'-oxospiro[cyclopropane-1,7'-5-oxa-2,8-diaza-1(6,3)-imidazo[1,2-b]pyridazina-3(1,3)-benzenacyclononaphan]-8'-yl)(methyl)carbamate (20G)

To a solution of **(20F)** (320 mg, 0.6 mmol) in 1,4-dioxane (40 mL) were added Cs₂CO₃ (590 mg, 1.81 mmol) and 3rd-*t*-Bu-Xphos-Pd (180 mg). The reaction mixture was stirred at 85°C for 2 h under N₂. After cooled to room temperature, the solvent was removed, and the residue was purified by silica gel flash column chromatography to afford the product **(20G)** (175mg, 58.72%) as a white solid. LC-MS (ESI): m/z =495.3[M+H]⁺

### Step 7: (E)-6'-methoxy-8'-(methylamino)spiro[cyclopropane-1,7'-5-oxa-2,8-diaza-1(6,3)-imidazo[1,2-b]pyridazina-3(1,3)-benzenacyclononaphan]-9'-one (20H)

A solution **of 20G** (175mg, 0.35 mmol) and p-Toluenesulfonic acid monohydrate (101 mg, 0.53 mmol) in DCM (4 mL) was stirred at room temperature for 2h. Solvent was evaporated, and the crude product was partitioned between water and DCM. The aqueous layer was basified with NaHCO₃ and extracted with DCM. Combined organic layers were washed with brine, dried over sodium sulfate, filtered, and evaporated, the residue was purified by silica gel flash column chromatography to afford the product 20 (35 mg, 25.17%) as a white solid. ¹H NMR (400 MHz, CDCl₃) δ 8.99 (s, 1H), 8.36 (d, 1H), 7.97 (s, 1H), 6.90 - 6.73 (m, 3H), 6.34 (s, 1H), 5.63 (s, 1H), 4.60 (s, 2H), 3.93 (s, 3H), 3.49 (s, 2H), 3.04 (d, 3H), 1.82 (q, 2H), 0.68 (q, 2H). LC-MS (ESI): m/z =395.2[M+H]⁺

### Example 21:

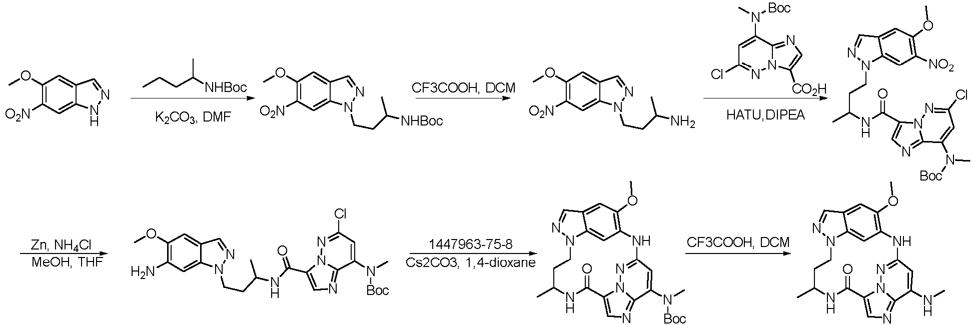

### Step 1: tert-butyl (4-(5-methoxy-6-nitro-1H-indazol-1-yl)butan-2-yl)carbamate

A solution of 5-methoxy-6-nitro-1H-indazole (1.0 g, 5.18 mmol), tert-butyl (4-bromobutan-2-yl)carbamate (1.7 g, 6.73 mmol) and K₂CO₃ (1.4 g, 10.36 mmol) in DMF (30 mL) stirred at 60 °C overnight. After reaction completed, the reaction was cooled in an ice bath and was diluted with EA (100 mL) and the solution was extracted with water (3 x 20 mL). The combined organic layers were dried over Na₂SO₄, filtered and concentrated under reduced pressure to afford the crude product which was purified by column chromatography (PE/EtOAc=3/1) to give the desired product (0.8 g, 42%) as a white solid. LM-MS: m/z =365.4[M+H]⁺

### Step 2: 4-(5-methoxy-6-nitro-1H-indazol-1-yl)butan-2-amine

A solution of tert-butyl (4-(5-methoxy-6-nitro-1H-indazol-1-yl)butan-2-yl) carbamate (0.7 g, 1.92 mmol) and trifluoroacetic acid (0.5 mL) in DCM (4 mL) was stirred at room temperature for 2h. Solvent was evaporated, and the crude product was partitioned between water and DCM. The aqueous layer was quenched with NaHCO₃ and extracted with DCM. Combined organic layers was washed with brine, dried over sodium sulfate, filtered, and evaporated to give the crude product which was purified by column chromatography (PE/EtOAc=3/1) to give the desired product (0.4 g, 79%) as a white solid. LM-MS: m/z =265.3[M+H]⁺

### Step 3: tert-butyl (6-chloro-3-((4-(5-methoxy-6-nitro-1H-indazol-1-yl)butan-2-yl) carbamoyl)imidazo[1,2-b]pyridazin-8-yl)(methyl)carbamate

4-(5-methoxy-6-nitro-1H-indazol-1-yl)butan-2-amine (0.2 g, 0.76 mmol) was dissolved in DCM (5 mL), HATU (0.58 g, 1.515 mmol), DIPEA (0.15 g, 1.14 mmol) and 8-((tert-butoxycarbonyl)(methyl)amino)-6-chloroimidazo[1,2-b]pyridazine-3 -carboxylic acid (0.25 g, 0.76 mmol) were added to the solution in room temperature. The mixture was stirred at r.t. for 1 h, then diluted with EA (20 mL), washed with water (10 mL) and brine (10 mL), dried with Na₂SO₄ and concentrated. The crude product was purified by flash chromatography (PE/EtOAc=3/1) to afford the title compound (0.3 g, 69%) as a white solid. LM-MS: m/z =574.0[M+H]⁺

### Step 4: tert-butyl (3-((4-(6-amino-5-methoxy-1H-indazol-1-yl)butan-2-yl)carbamoyl) -6-chloroimidazo[1,2-b]pyridazin-8-yl)(methyl)carbamate

### The tert-butyl (6-chloro-3-((4-(5-methoxy-6-nitro-1H-indazol-1-yl)butan-2-yl)

carbamoyl)imidazo[1,2-b]pyridazin-8-yl)(methyl)carbamate (0.3 g, 0.52 mmol) and NH₄Cl (330 mg, 6.24 mmol) was dissolved in MeOH (5 mL) at r.t., Zn powder ( 405 mg, 6.24 mmol) were added to the solution, then the reaction mixture was stirred at room temperature for 1 h. After reaction completed, reaction filtered, filtrate was removed in vacuo. The residue was purified by flash chromatography to afford the title compound (0.27 g, 96 %) as a white solid. LM-MS: m/z =543.0[M+H]⁺

### Step 5: tert-butyl (E)-(3⁵-methoxy-6-methyl-8-oxo-3¹H-2,7-diaza-1(6,3) -imidazo[1,2-b]pyridazina-3(6,1)-indazolacyclooctaphane-1⁸-yl)(methyl)carbamate

### To a solution of tert-butyl (3-((4-(6-amino-5-methoxy-1H-indazol-1-yl)

butan-2-yl)carbamoyl)-6-chloroimidazo[1,2-b]pyridazin-8-yl)(methyl)carbamate (270 mg, 0.5 mmol) in 1,4-dioxane (20 mL) was added Cs₂CO₃ (325 mg, 1.0 mmol) and 3rd-*t*-Bu-Xphos-Pd (120 mg). The reaction mixture was stirred at 80°C for 3 h under N₂. After cooled to room temperature, reaction filtered, the solvent was removed, and the residue was purified by silica gel flash column chromatography to afford the desired compound (150 mg, 59%) as a white solid. LM-MS: m/z =507.7[M+H]⁺

### Step6: (E)-3⁵-methoxy-6-methyl-1⁸-(methylamino)-3¹H-2,7-diaza-1(6,3)-imidazo [1,2-b]pyridazina-3(6,1)-indazolacyclooctaphan-8-one

### A solution of tert-butyl (E)-(3⁵-methoxy-6-methyl-8-oxo-3¹H-2,7-diaza-1(6,3)

-imidazo[1,2-b]pyridazina-3(6,1)-indazolacyclooctaphane-1⁸-yl)(methyl)carbamate (0.15 g, 0.3 mmol) and trifluoroacetic acid (0.2 mL) in DCM (4 mL) was stirred at room temperature for 2 h. Solvent was evaporated, and the crude product was partitioned between water and DCM. The aqueous layer was quenched by NaHCO₃ and extracted with DCM. Combined organic layers were washed with brine, dried over sodium sulfate, filtered. The filtrate was purified directly by prep-HPLC to give the desired product 21 (125 mg, yield: 42%) as a brow solid. LM-MS: m/z =407.5[M+H]⁺. ¹H NMR (400 MHz, CD₃OD) δ 8.88 (s, 1H), 7.93 (s, 1H), 7.85 (s, 1H), 7.22 (s, 1H), 6.19 (s, 1H), 4.61 - 4.48 (m, 3H), 4.00 (s, 3H), 3.04 (s, 3H), 2.14 (d, 2H), 1.01 (d, 3H).

### Example 22

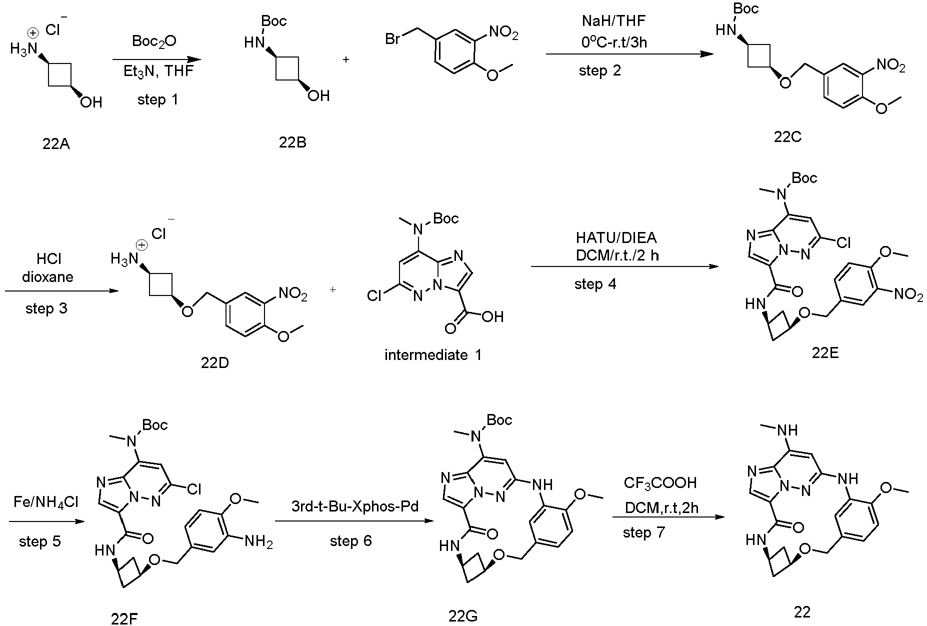

### Step 1: tert-butyl ((1s,3s)-3-hydroxycyclobutyl)carbamate (22B)

Triethylamine (4.93g, 48.76 mmol) was added dropwise to a stirred solution of (1s,3s)-3-hydroxycyclobutan-1-aminium chloride **(22A)** (2g, 16.25mmol) in THF (50 mL) at 0°C. After 10 min of stirring, ditertbutyl dicarbonate (7.09g, 32.50mmol) in THF (5 mL) was added dropwise to the mixture at 0°C. The mixture was stirred overnight in room temperature, the solvent was removed in vacuo. The residue was diluted with ethyl acetate (60 mL), washed with water (2×60 mL) and brine (50 mL), dried with Na₂SO₄ and concentrated. then the title compound **(22B)** (3g. 100%) was obtained as colorless oil, which was used in the next step without further purification. LC-MS (ESI): m/z =188.1 [M+H]⁺

### Step 2: tert-butyl ((1s,3s)-3-((4-methoxy-3-nitrobenzyl)oxy)cyclobutyl)carbamate (22C)

To a stirred solution of tert-butyl ((1s,3s)-3-hydroxycyclobutyl)carbamate **(22B)** (1.5g,8.02mmol) in THF (60 mL) was added sodium hydride (577mg, 14.43mmol) portionwise at 0°C, the mixture was stirred at 0°C for 30 min. Then 4-(bromomethyl)-1-methoxy-2-nitrobenzene (0.69g, 2.62mmol) was added to the mixture at 0°C, The mixture was stirred overnight in room temperature, the mixture was treated with cold water (80 mL) and extracted with ethyl acetate( 2×100 mL). The combined organic layer was washed with brine, dried with Na2SO4 and concentrated, then the residue was purified by flash chromatography to afford the title compound **(22C)** (0.2 g, 21.30%) as a white solid. LC-MS (ESI): m/z =353.1 [M+H]⁺

### Step 3: (1s,3s)-3-((4-methoxy-3-nitrobenzyl)oxy)cyclobutan-1-aminium chloride (22D)

To a solution of tert-butyl ((1s,3s)-3-((4-methoxy-3-nitrobenzyl)oxy) cyclobutyl)carbamate **(222C)** (0.55g, 2.79mmol) in DCM (5 mL) was added trifluoroacetic acid (1.5 mL), The mixture was stirred overnight at r.t., The mixture solution was evaporated to dryness, then the title compound **(22D)** (0.39g. 100%) was obtained as white solid, which was used in the next step without further purification. LC-MS (ESI): m/z =253.1 [M+H]⁺

### Step 4: tert-butyl tert-butyl (6-chloro-3-(((1s,3s)-3-((4-methoxy-3-nitrobenzyl) oxy)cyclobutyl)carbamoyl)imidazo[1,2-b]pyridazin-8-yl)(methyl)carbamate (22E)

(1s,3s)-3-((4-methoxy-3-nitrobenzyl)oxy)cyclobutan-1-aminium chloride **(22D)** (200 mg, 0.79 mmol) was dissolved in DMF(5 mL), HATU(360 mg, 0.94 mmol), DIPEA(180mg, 1.18mmol) and intermediate 1(257mg, 0.79mmol)were added to the solution in room temperature. The mixture was stirred at r.t. for 18h, then diluted with EA (50 mL), washed with water (2×50 mL) and brine (50 mL), dried with Na₂SO₄ and concentrated. The crude product was purified by flash chromatography (PE/EA = 2:1) to afford the title compound **(22E)** (400 mg, 90.00%) as a white solid. LC-MS (ESI): m/z =561.2 [M+H]⁺

### Step 5: tert-butyl (6-chloro-3-(((1s,3s)-3-((4-methoxy-3-nitrobenzyl)oxy)cyclobutyl) carbamoyl)imidazo[1,2-b]pyridazin-8-yl)(methyl)carbamate (22F)

(6-chloro-3-(((1s,3s)-3-((4-methoxy-3-nitrobenzyl)oxy)cyclobutyl)carbamoyl) imidazo[1,2-b]pyridazin-8-yl)(methyl)carbamate **(22E)** (280 mg, 0.5 mmol) was dissolved in ethanol (9 mL) and H₂O (3 mL), iron powder (560 mg,10 mmol) and NH₄Cl (530 mg,10 mmol) were added to solution, then the reaction mixture heated to 85 °C for 3 h, After cooling to room temperature, reaction filtered, filtrate was removed in vacuo. The residue was purified by flash chromatography to afford the title compound **(22H)** (200 mg, 75.47%) as a white solid. LC-MS (ESI): m/z =531.2 [M+H]⁺.

### Step 6: tert-butyl ((6¹s,6³s,E)-3⁶-methoxy-8-oxo-5-oxa-2,7-diaza-1(6,3)-imidazo [1,2-b]pyridazina-3(1,3)-benzena-6(1,3)-cyclobutanacyclooctaphane-18-yl)(methyl) carbamate (22G)

To a solution of (6-chloro-3-(((1s,3s)-3-((4-methoxy-3-nitrobenzyl)oxy)cyclobutyl) carbamoyl)imidazo[1,2-b]pyridazin-8-yl)(methyl)carbamate **(22F)** (265 mg, 0.5 mmol) in 1,4-dioxane (10 mL) were added Cs₂CO₃ (326 mg, 1.0 mmol) and 3rd-*t*-Bu-Xphos-Pd (35 mg, 0.04 mmol). The reaction mixture was stirred at 80°C for 3 h under N₂. After cooled to room temperature, the solvent was removed, and the residue was purified by silica gel flash column chromatography to afford the product Compound **(22G)** (50mg, 20.24%) as a white solid. LC-MS (ESI): m/z =495.1 [M+H]⁺

### Step 7: 6¹s,6³s,E)-36-methoxy-1⁸-(methylamino)-5-oxa-2,7-diaza-1(6,3)-imidazo[1,2-b]pyridazina-3(1,3)-benzena-6(1,3)-cyclobutanacyclooctaphan-8-one (22)

A solution of tert-butyl tert-butyl ((6¹s,6³s,E)-3⁶-methoxy-8-oxo-5-oxa- 2,7-diaza-1(6,3)-imidazo[1,2-b]pyridazina-3(1,3)-benzena-6(1,3)-cyclobutanacyclooctaphane-18-yl)(methyl) carbamate **(22G)** (50mg, 1.02 mmol) and p-Toluenesulfonic acid monohydrate (50mg, 0.29mol) in DCM (4 mL) was stirred at room temperature for 2h. Solvent was evaporated, and the crude product was partitioned between water and DCM. The aqueous layer was basified with NaHCO₃ and extracted with DCM. Combined organic layers and evaporated, the residue was purified by TLC to afford **22** (2 mg, 5.01%). LC-MS (ESI): m/z =395.1 [M+H]⁺. ¹H NMR (400 MHz, DMSO-d6) δ 9.15 (d, 1H), 8.29 (s, 1H), 8.18 (d, 1H), 7.82 (s, 1H), 7.38 (d, 1H), 7.00 (d, 1H), 6.85 (dd, 1H), 6.23 (d, 1H), 4.50 (s, 2H), 4.34 (dd, 1H), 4.21 (s, 1H), 3.88 (s, 3H), 2.89 (d, 3H), 2.72 - 2.65 (m, 2H), 1.75 (d, 2H).

### Example 24:

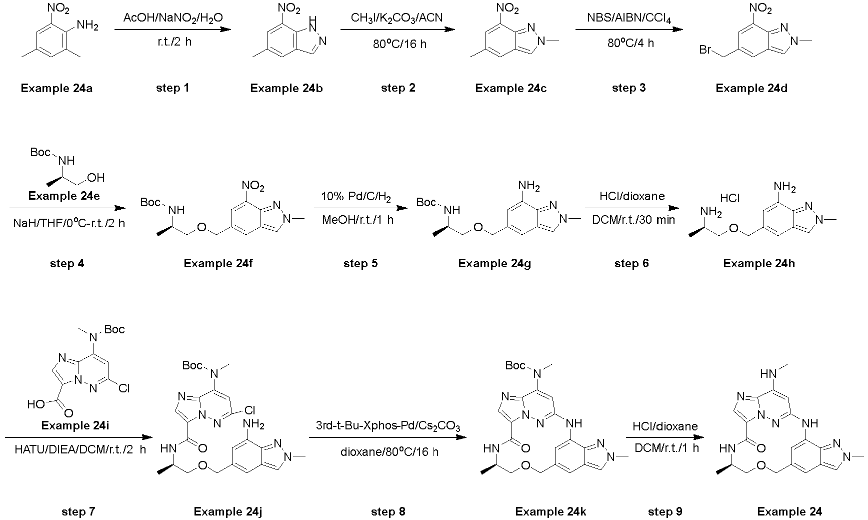

### Step 1: Example 24b

To a solution of **Example 24a** (10.0 g, 60.24 mmol, 1.0 eq) in AcOH (150 mL) was added a solution of NaNO₂ (4.99 g, 72.29 mmol, 1.2 eq) in H₂O (10 mL) dropwise at r.t. The reaction mixture was stirred for 2 h at r.t. Then, water (100 mL) was added to the mixture, which was stirred for 30 min. The precipitated solid was collected by filtration, which was washed with H₂O and MTBE. The crude product was purified by silica gel flash column chromatography to afford the desired product **Example 24b** (3.9 g, 36.6% yield) as a yellow solid. LCMS [M+1]⁺ = 178.2.

### Step 2: Example 24c

To a solution of **Example 24b** (3.9 g, 22.03 mmol, 1.0 eq) in CH₃CN (50 mL) was added CH₃I (15.6 g, 110.17 mmol, 5.0 eq) and K₂CO₃ (7.6 g, 55.08 mmol, 2.5 eq). The reaction mixture was stirred for 16 h at 80°C. The solvent was concentrated, and the crude was purified by silica gel flash column chromatography to afford the desired product **Example 24c** (3.17 g, 75.3% yield) as a brown solid and its isomer (200 mg) as a brown solid. LCMS [M+1]⁺ = 192.2. **Example 24c:** ¹H NMR (300 MHz, DMSO-*d*₆) δ 8.21 (d, 1H), 8.05 (s,1H), 7.79 (s,1H), 4.33 (s, 3H), 2.51 (s, 3H). **Isomer:** ¹H NMR (300 MHz, DMSO-*d*₆) δ 8.04 (s,1H), 7.96 (s, 1H), 7.79 (s,1H), 4.23 (s, 3H), 2.51 (s, 3H).

### Step 3: Example 24d

A solution of **Example 24c** (500 mg, 2.62 mmol, 1.0 eq) in CCl₄ (12 mL) was heated to 80°C, to which NBS (559 mg, 3.14 mmol, 1.2 eq) and AIBN (429 mg, 2.62 mmol, 1.0 eq) were added. The reaction mixture was stirred for 4 h at 80°C. After cooling to room temperature, the reaction mixture was concentrated, and the crude was purified by silica gel flash column chromatography to afford the desired product **Example 24d** (577 mg, 81.7% yield) as a yellow solid. ¹H NMR (300 MHz, DMSO-*d*₆) δ 8.78 (s, 1H), 8.39 (d, 1H), 8.36 (d, 1H), 4.95 (s, 2H), 4.28 (s, 3H).

### Step 4: Example 24f

To a solution of **Example 24e** (681 mg, 3.89 mmol, 2.0 eq) in THF (10 mL) was added NaH (117 mg, 60% in mineral oil, 2.92 mmol, 1.5 eq) at 0°C. After stirring for 30 min, **Example 24d** (525 mg, 1.94 mmol, 1.0 eq) was added to the mixture, which was stirred for another 2 h at r.t. The mixture was quenched with saturated aqueous NH₄Cl and extracted with EtOAc (30 mL*3). The combined organic layers were washed with brine, dried over Na₂SO₄ and concentrated. The residue was purified by silica gel flash column chromatography to afford the desired product **Example 24f (306** mg, 43.2% yield) as a yellow solid. LCMS [M+1-56]⁺ = 309.2.

### Step 5: Example 24g

**Example24f** (306 mg, 0.84 mmol, 1.0 eq) was dissolved in MeOH (10 mL), and 10% Pd/C (200 mg) was added slowly in portions under N₂ protection. The system was evacuated and then refilled with hydrogen. The mixture solution was stirred for 1 h at r.t. under H₂ balloon. The reaction mixture was filtered through a Celite pad and the filtrate was concentrated. The residue was purified by prep-TLC to afford the desired product **Example 24g** (150 mg, 53.3% yield) as a light yellow solid. LCMS [M+1+22]⁺ = 357.3.

### Step 6: Example 24h

To a solution of **Example 24g** (130 mg, 0.39 mmol, 1.0 eq) in DCM (6 mL) was added HCl/dioxane (2 mL, 4 M in dioxane). The reaction solution was stirred for 0.5 h at r.t. and concentrated to afford the desired product **Example 24h** (240 mg, crude) as a yellow solid. LCMS [M+1]⁺ = 235.3.

### Step 7: Example 24j

To a solution of **Example 24i** (173 mg, 0.53 mmol, 1.0 eq) in DCM (10 mL) were added DIEA (545 mg, 4.23 mmol, 8.0 eq) and HATU (221 mg, 0.58 mmol, 1.1 eq). After stirring for 30 min, **Example 24h** (215 mg, 0.79 mmol, 1.5 eq) was added. The reaction mixture was stirred for 2 h at r.t. The solvent was removed, and the crude was purified by prep-TLC (EtOAc) to afford the desired product example **24j** (250 mg, 87.0ield) as yellow oil. LCMS [M+1]⁺ = 543.3.

### Step 8: Example 24k

To a solution of **Example 24j** (150 mg, 0.28 mmol, 1.0 eq) in dioxane (20 mL) were added Cs₂CO₃ (180 mg, 0.55 mmol, 2.0 eq) and 3^{rd}-t-Bu-Xphos-Pd (25 mg, 0.028 mmol, 0.1 eq). The reaction mixture was stirred for 16 h at 80°C under N₂. The reaction mixture was concentrated and purified by prep-TLC to afford the desired product **Example 24k** (50 mg, 35.7% yield) as a yellow solid. LCMS [M+1]⁺ = 507.3.

### Step 9: Example 24

To a solution of **Example 24k** (45 mg, 0.089 mmol, 1.0 eq) in DCM (3 mL) was added HCl/dioxane (1 mL, 4 M in dioxane). The reaction solution was stirred for 1 h at r.t., and the solvent was concentrated. The crude product was dissolved in MeOH and Na₂CO₃was added. The mixture was stirred for 10 min at r.t. and then filtered. The filtrate was concentrated and the residue was purified by prep-TLC to afford the desired product **Example 24** (20.0 mg, 55.4% yield) as an off-white solid. LCMS [M+1]⁺ = 407.3. ¹H NMR (300 MHz, DMSO-*d*₆) δ 9.19 (s, 1H), 8.95 (d, 1H), 8.32 (s, 1H), 7.95 (s, 1H), 7.83 (s, 1H), 7.44 (d, 1H), 7.20 (s, 1H), 6.36 (s, 1H), 4.73 (d, 1H), 4.45 (d, 1H), 4.21 (s, 3H), 4.08-3.96 (m, 1H), 3.48 (d, 1H), 3.26-3.20(m, 1H), 2.90 (d, 3H), 1.11 (d, 3H).

### Example 25:

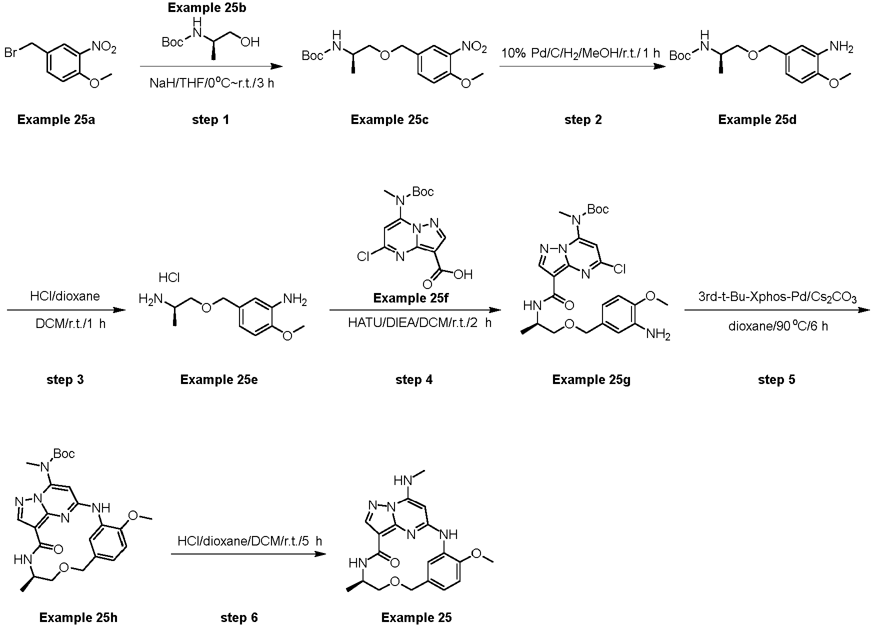

### Step 1: Example 25c

To a solution **of Example 25b** (2.3 **g,** 13.4 mmol) in THF (40 mL) was added NaH (0.9 g, 60% in mineral oil, 22.4 mmol) in portions at 0°C. After stirring for 10 min, a solution of **Example 25a** (2.2 g, 8.9 mmol) in THF (10 mL) was added dropwise. The reaction mixture was stirred for 3 h at r.t. The reaction was quenched with saturated NH₄Cl aqueous solution (20 mL) at 0°C and extracted with EtOAc (50 mL*2). The combined organic layer was dried over Na₂SO₄ and concentrated in *vacuo.* The crude product was purified by silica gel flash column chromatography to afford the desired product **Example 25c** (2.1 g, 69.4% yield) as a yellow solid. LCMS [M+1]⁺ = 341.3.

### Step 2: Example 25d

**Example 25c** (1.1 g, 3.2 mmol) was dissolved in MeOH (50 mL); 10% Pd/C (110 mg) was added in portions under N₂ protection. The system was evacuated and then refilled with hydrogen. The mixture was stirred for 1 h at r.t. under H₂ balloon, and then filtered. The filtrate was concentrated and the crude product was purified by silica gel flash column chromatography to afford the desired product **Example 25d** (850 mg, 85.7% yield) as colorless oil. LCMS [M+1]⁺ = 311.3.

### Step 3: Example 25e

To a solution of **Example 25d** (840 mg, 2.7 mmol) in DCM (5 mL) and MeOH (1 mL) was added HCl/dioxane (1 mL, 4M in Dioxane, 4 mmol). The reaction mixture was stirred for 1 h at r.t. The reaction solution was concentrated in vacuo to afford the desired product **Example 25e** (800 mg, crude) as a white solid. LCMS [M+1]⁺ = 211.2.

### Step 4: Example 25g

To a solution of **Example 25f** (607 mg, 1.9 mmol) in DCM (30 mL) were added DIEA (1.92 g, 14.9 mmol) and HATU (1.06 g, 2.8 mmol). After stirring for 0.5 h, **Example 25e** (790 mg, 2.8 mmol) was added. The reaction mixture was stirred for 2 h at r.t. The solvent was removed and the residue was purified by silica gel flash column chromatography to afford the desired product **Example 25g** (310 mg, 21.4% yield) as a yellow solid. LCMS [M+1]⁺ = 519.3.

### Step 5: Example 25h

To a solution of **Example 25g** (300 mg, 0.6 mmol) in dioxane (50 mL) were added Cs₂CO₃ (377 mg, 1.2 mmol) and 3rd-*t*-Bu-Xphos-Pd (154 mg, 0.2 mmol). The reaction mixture was stirred at 90°C for 6 h under N₂. After cooling to room temperature, the solvent was removed, and the residue was purified by silica gel column chromatography to afford the product **Example 25h** (110 mg, 39.4% yield) as a yellow solid. LCMS [M+1] ⁺ = 483.2.

### Step 6: Example 25

To a solution of **Example 25h** (110 mg, 0.23 mmol) in DCM (5 mL) was added HCl/dioxane (1 mL, 4M in Dioxane, 4 mmol). The reaction mixture was stirred at r.t. for 5 h and then concentrated in vacuum. The residue was dissolved in MeOH (5 mL), and basified with NaHCO₃ (pH = 8). The precipitate was filtered out, and the filtrate was concentrated. The residue was purified by Prep-TLC to afford the desired product **Example 25** (57.3 mg, 65.7% yield) as an off-white solid. LCMS [M+1] ⁺ = 383.2. ¹H NMR (300 MHz, DMSO-*d*₆) δ 8.92 (s, 1H), 8.36 (d, 1H), 8.24 (d, 1H), 8.11 (s, 1H), 7.81 (d, 1H), 6.99 (d, 1H), 6.92 (d, 1H), 5.94 (s, 1H), 4.57 (d, 1H), 4.40 (d, 1H), 3.94 - 3.83 (m, 4H), 3.47 (d, 1H),3.29 - 3.25 (m, 1H), 2.92 (d, 3H), 1.14 (d, 3H).

### Example 26:

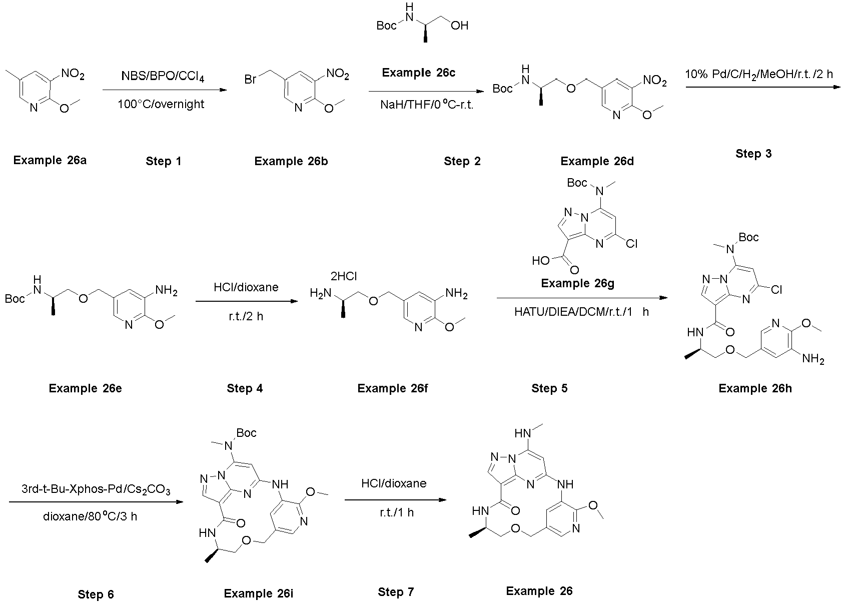

### Step 1: Example 26b

To a solution of **Example 26a** (30.0 g, 179.0 mmol) in CCl₄ (150 mL) were added BPO (4.4 g, 17.9 mmol), NBS (38.2 g, 216.0 mmol). The reaction mixture was stirred at 100°C for overnight, and then diluted by DCM, washed by water, and dried over anhydrous Na₂SO₄. The solution was concentrated under reduced pressure to afford crude residue **Example26b** (37.0 g, yield 84.4%) as a yellow solid, which was used in the next step directly without further purification. LCMS [M+1] ⁺ = 246.0. ¹H NMR (400 MHz, Chloroform-d) δ 7.87 (d, 1H), 7.57 (dd, 1H), 7.07 (d, 1H), 4.46 (s, 2H), 3.96 (d, 3H).

### Step 2: Example 26d

To a solution of **Example 26b** (2.4 g, 9.8 mmol) in THF (20 mL) was added NaH (400 mg, 60% in mineral oil, 10.0 mmol) at 0°C. The reaction mixture was warmed to room temperature and stirred at r.t. for 0.5 h. Then **Example 26c** (1.7 g, 9.7 mmol) was added and the mixture was stirred at r.t. for 6 h. The reaction mixture was quenched by sat. NH₄Cl (aq.), extracted by EtOAc, and dried over anhydrous Na₂SO₄. The solution was concentrated under reduced pressure. The residue was purified by silica gel column chromatography to give **Example26d** (3.3 g, yield: 96.8%) as a yellow solid. LCMS [M+1-100] ⁺ = 241.1.

### Step 3: Example 26e

A solution of **Example 26d** (688 mg, 2.0 mmol) and 10% Pd/C (34 mg) in MeOH (10 mL) was stirred at r.t. for 2 h under 1 atm H₂. The mixture was filtered, and the filtrate was concentrated under reduced pressure. The residue **Example 26e** (640 mg, yield: quant.) was obtained as a yellow solid which was used in the next step directly. LCMS [M-174] ⁺ = 136.1

### Step 4: Example 26f

To a solution of **Example 26e** (crude 550 mg, 1.77 mmol) in DCM (10 mL) was added TFA (2.0 mL), which was stirred at r.t. for 2 h. The mixture was concentrated, and the residue was treated with EtOAc (30 mL) to give the crude product **Example 26f** (340 mg, yield: quant.) as a white solid. LCMS [M-74] ⁺ = 137.1.

### Step 5: Example 26h

To a solution of **Example 26f** (crude 300 mg, 1.42 mmol), **Example 26g** (464 mg, 1.42 mmol) DIPEA (916 mg, 7.1 mmol) in DCM (10 mL) was added HATU (538 mg, 1.42 mmol). The reaction mixture was stirred at r.t. for 1 h. Then EtOAc (40 mL) was added to the reaction mixture, which was washed with brine (20 mL*2), dried over anhydrous Na₂SO₄ and concentrated. The residue was purified by silica gel column chromatography to afford the desired product **Example 26h** (600 mg, yield: 81.4%) as a white solid. LCMS [M+1] ⁺ = 520.2.

### Step 6: Example 26i

To a mixture of **Example 26h** (330 mg, 0.97 mmol), Cs₂CO₃ (652 mg, 2.0 mmol) in dioxane (10 mL) was added 3rd-t-Bu-Xphos-Pd (89 mg, 0.1 mmol). The mixture was degassed with N₂three times, and stirred for 3 h at 80°C. Then the reaction mixture diluted by DCM, washed by water, dried over anhydrous Na₂SO₄, and then concentrated under reduced pressure to afford crude **Example 26i** (400 mg, crude yield >100%) as a white solid, which was used in the next step without further purification. LCMS [M+1] ⁺ = 484.2

### Step 7: Example 26

To a solution of **Example 26i** (400 mg, 0.82 mmol) in DCM (4 mL) was added TFA (1.0 mL), which was stirred at r.t. for 1 h. The mixture was concentrated, and the residue was purified by Prep-HPLC to afford the desired product **Example 26** (18.3 mg, yield 5.8% over two steps) as a white solid. LCMS [M+1] ⁺ = 384.1. ¹H NMR (400 MHz, DMSO-*d*₆) δ 9.11 (s, 1H), 8.60-8.54 (m, 1H), 8.10 (s, 1H), 8.04 (d, 1H), 7.89 (d, 1H), 7.71 (s, 1H), 5.90 (s, 1H), 4.53 (d, 1H), 4.43 (d, 1H), 3.94 (s, 3H), 3.86 (s, 1H), 3.46 (d, 1H), 3.25 (s, 1H), 2.89 (d, 3H), 1.11 (d, 3H).

### Example 27:

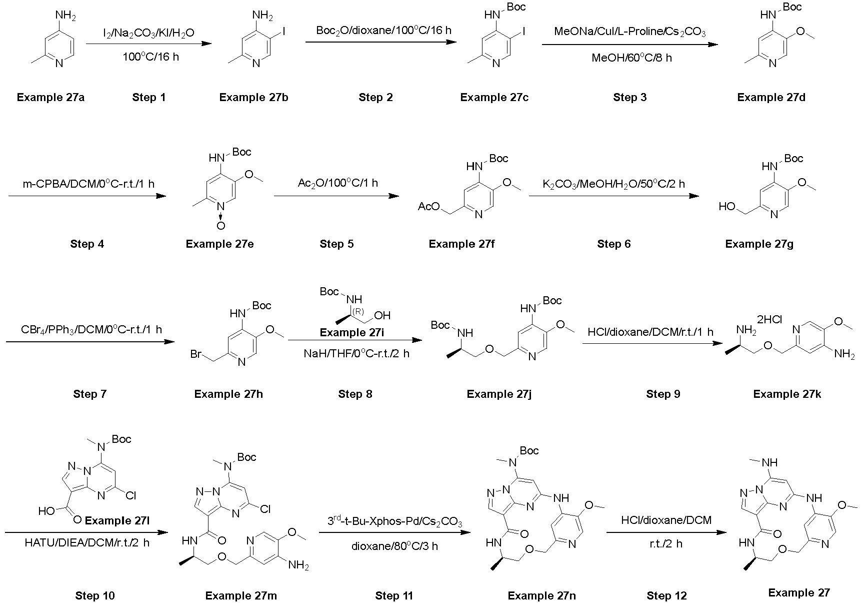

### Step 1: Example 27b

To a solution **of Example27a** (30.0 g, 277.8 mmol, 1.0 eq), Na₂CO₃ (20.6 g, 194.5 mmol, 0.7 eq) in H₂O (150 mL) were added KI (59.9 g, 361.1 mmol, 1.3 eq) and I₂ (56.4 g, 222.2 mmol, 0.8 eq) in H₂O (50 mL) at 100°C, which was stirred for 16 h. After cooling to r.t., the reaction mixture was quenched with Na₂SO₃ (35.0 g, 277.8 mmol, 1.0 eq) and extracted with DCM (300 mL*2). The combined organic layers were washed with brine, dried over Na₂SO₄ and concentrated. The residue was purified by silica gel flash column chromatography to afford the product **Example 27b** (8.4 g, 12.9% yield) as a yellow solid. LCMS [M+1]⁺ = 235.1

### Step 2: Example 27c

To a solution of **Example 27b** (7.0 g, 50.7 mmol, 1.0 eq) in dioxane (50 mL) was added Boc₂O (33.2 g, 152.1 mmol, 3.0 eq), which was stirred for 16 h at 100°C. The reaction mixture was concentrated and the residue was purified by silica gel flash column chromatography to afford the product **Example 27c** (3.5 g, 35% yield) as a white solid. LCMS [M+1]⁺ = 335.1.

### Step 3: Example 27d

To a solution of **Example 27c** (3.5 g, 10.5 mmol, 1.0 eq) and MeONa (2.82 g, 52.25 mmol, 5.0eq) in MeOH (30 mL) were added Cs₂CO₃ (10.2 g, 21.0 mmol, 2.0 eq), CuI (199 mg, 1.05 mmol, 0.1 eq), and L-proline (343 mg, 2.1 mmol, 0.2 eq). The reaction mixture was stirred for 8 h at 60°C under N₂ protection. The reaction mixture was concentrated. The residue was purified by silica gel flash column chromatography to afford the product **Example 27d** (750 mg, 30.1% yield) as a white solid. LCMS [M+1]⁺ = 239.3.

### Step 4: Example 27e

To a solution of **Example 27d** (550 mg, 2.3 mmol, 1.0 eq) in DCM (10 mL) at 0°C (ice-water bath) was added *m*-CPBA (596 mg, 3.45 mmol, 1.5 eq) portionwise. After addition, the reaction was stirred for 1 h at r.t. The solution was quenched with Na₂SO₃ (150 mg, 1.15 mmol, 0.5 eq) and extracted with DCM (30 mL*2). The combined organic layers were washed with brine, dried over Na₂SO₄ and concentrated to afford the product **Example 27e** (550 mg, 93.8% yield) as a yellow solid. LCMS [M+1]⁺ = 255.3.

### Step 5: Example 27f

A solution of **Example 27e** (400 mg, 1.65 mmol, 1.0 eq) in Ac₂O (10 mL) was stirred for 1 h at 100°C. The mixture was concentrated to afford the crude product **Example 27f** (550 mg, quant. yield) as brown oil. LCMS [M+1]⁺ = 297.3.

### Step 6: Example 27g

To a solution of **Example 27f** (450 mg, 1.52 mmol, 1.0 eq) in MeOH (15 mL) and H₂O (5 mL) was added K₂CO₃ (418.2 mg, 3.04 mmol, 2.0 eq) at 0°C. The mixture was stirred for 2 h at 50°C. After the reaction was completed, the mixture was concentrated in vacuo. The residue was purified by silica gel flash column chromatography to afford the desired product **Example 27g** (250 mg, 64.6% yield) as a white solid. LCMS [M+1]⁺ = 255.2.

### Step 7: Example 27h

To a solution of **Example 27g** (230 mg, 0.90 mmol, 1.0 eq), CBr₄ (597 mg, 1.80 mmol, 2.0 eq) in DCM (15 mL) was added PPh₃ (355 mg, 1.35 mmol, 1.5 eq) in DCM (5 mL) at 0°C, which was stirred for 1 h at r.t. under N₂ protection. The mixture was concentrated in vacuo. The residue was purified by silica gel flash column chromatography to afford the product **Example 27h** (150 mg, 52.7% yield) as yellow oil. LCMS [M+1]⁺ = 317.2.

### Step 8: Example 27j

To a solution of **Example 27i** (165.6 mg, 0.94 mmol, 1.5 eq) in THF (5 mL) was added NaH (75.7 mg, 60% in mineral oil, 1.89 mmol, 3.0 eq) in portions at 0°C. After stirring for 0.5 h, a solution of **Example 27h** (200 mg, 0.63 mmol, 1.0 eq) in THF (1 mL) was added dropwise. The reaction mixture was stirred for 1.5 h at r.t. The reaction was quenched with saturated NH₄Cl aqueous solution (10 mL) at 0°C and extracted with EtOAc (20 mL*3). The combined organic layers were washed with brine (10 mL*2), dried over Na₂SO₄ and concentrated in *vacuo.* The crude product was purified by silica gel flash column chromatography to afford the desired product **Example 27j** (105 mg, 40.4% yield) as a light yellow solid. LCMS [M+1]⁺ = 412.4.

### Step 9: Example 27k

To a solution of **Example 27j** (105 mg, 0.25 mmol, 1.0 eq) in DCM (15 mL) was added HCl/dioxane (0.2 mL, 4 mol/L in dioxane) at 0°C. The reaction mixture was stirred for 1 h at r.t. The reaction solution was concentrated in vacuo to afford the desired product **Example** 27k (150 mg, quant. yield) as a white solid. LCMS [M+1]⁺ = 212.3.

### Step 10: Example 27m

To a solution of **Example 27l** (121 mg, 0.37 mmol, 1.0 eq) and DIEA (239 mg, 1.85 mmol, 5.0 eq) in DCM (30 mL) was added HATU (167 mg, 0.44 mmol,1.2 eq). After stirring for 10 min, **Example 27k** (crude 105 mg, 0.37 mmol, 1.0 eq) was added, which was stirred for 2 h at r.t. The mixture was concentrated in vacuo. The residue was purified by silica gel flash column chromatography to afford the product **Example 27m** (65 mg, 33.8% yield) as a white solid. LCMS [M+1]⁺ = 520.3.

### Step 11: Example 27n

To a solution of **Example 27m** (60 mg, 0.12 mmol, 1.0 eq) in dioxane (2 mL) were added Cs₂CO₃ (75.1 mg, 0.24 mmol, 2.0 eq) and 3^{rd} t-Bu-XphosPd (10.2 mg, 0.012 mmol, 0.1 eq). The reaction mixture was stirred for 3 h at 80°C under N₂. The reaction solution was filtered and the filtrate was concentrated in *vacuo.* The crude product was purified by prep-TLC to afford the desired product **Example 27n** (25 mg, 44.7% yield) as a white solid. LCMS [M+1]⁺ = 484.4.

### Step 12: Example 27

To a solution of **Example 27n** (25 mg, 0.052 mmol, 1.0 eq) in DCM (2 mL) was added HCl/dioxane (0.2 mL, 4 mol/L dioxane) at 0°C, which was stirred for 2 h at r.t. The reaction solution was concentrated in *vacuo.* The crude product was dissolved in MeOH, and Na₂CO₃ (excess) was added. The resulting mixture was stirred for 10 min at r.t. and the precipitate was filtered. The filtrate was concentrated, and the residue was purified by pre-TLC to afford the desired product **Example 27** (7.2 mg, 36.4% yield) as a white solid. LCMS [M+1]⁺ = 384.3. ¹H NMR (300 MHz, DMSO-*d*₆) δ 9.31 (s, 1H), 8.67 (s, 1H), 8.25 (d, 1H), 8.20 (d, 1H),8.02-8.04 (m, 1H),6.13 (s, 1H), 4.62 (d, 2H), 4.47 (d, 2H),3.98 (s, 3H), 3.89-3.96 (m, 1H), 3.54-3.62 (m, 1H), 3.32 -3.45 (m, 1H), 2.93 (d, 3H),1.19 (d, 3H).

### Example 28:

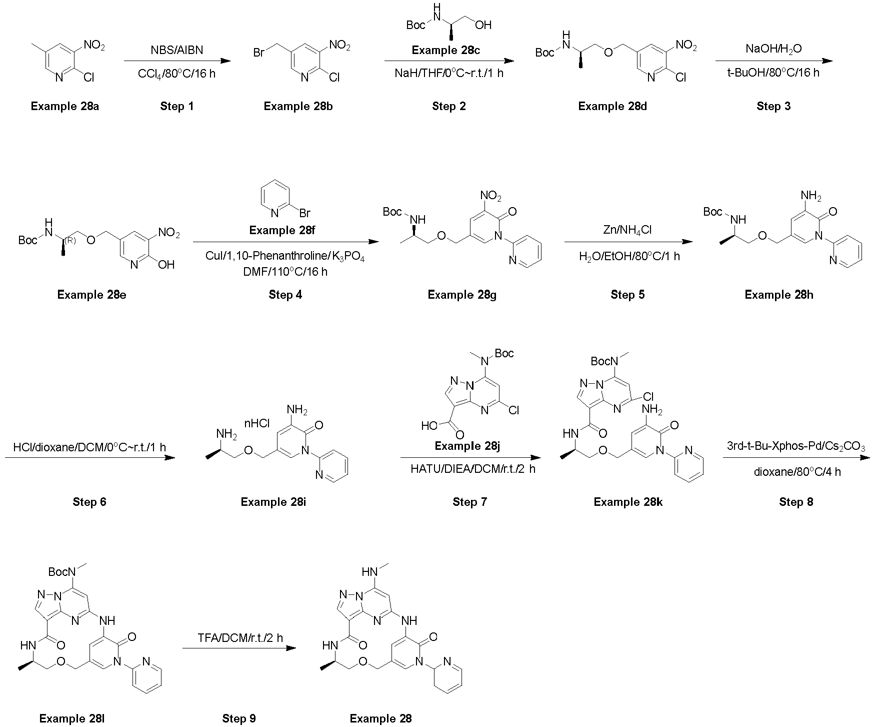

### Step 1: Example 28b

To a solution of **Example 28a** (20.0 g, 0.12 mol, 1.0 eq) in CCl₄ (400 mL) were added NBS (72.4 g, 0.41 mol, 3.5 eq) and AIBN (13.4 g, 0.08 mol, 0.7 eq). The reaction mixture was stirred at 80°C for 16 h. After cooled to room temperature, the solid was filtered out, and the filtrate was concentrated. The residue was purified by silica gel flash column chromatography to afford the product **Example 28b** (15.1 g, 52% yield) as a yellow solid. LCMS [M+1]⁺ = 251.2.

### Step 2: Example 28d

To a solution of **Example 28c** (12.7 g, 72.5 mmol, 1.2 eq) in THF (400 mL) was added NaH (2.9 g, 60% in mineral oil, 72.5 mmol, 1.2 eq) in portions at 0°C. The mixture was stirred for 5 min at the same temperature, then **Example 28b** (15.1 g, 60.4 mmol, 1.0 eq) in THF (50 mL) was added dropwise. The reaction mixture was stirred at r.t. for 1 h. The reaction was quenched with H₂O (100 mL) and extracted with EtOAc (200 mL*3). The combined organic layers were washed with brine, dried over Na₂SO₄ and concentrated. The residue was purified by silica gel chromatography to afford the desired product **Example 28d** (5.2 g, 25% yield) as yellow oil. LCMS [M+1]⁺ = 346.3.

### Step 3: Example 28e

To a solution of **Example 28d (5.1** g, 14.7 mmol, 1.0 eq) in *t*-BuOH/H₂O (100 mL/30 mL) was added NaOH (2.9 g,73.7 mmol, 5.0 eq). The mixture was stirred for 16 h at 80°C. After cooled to r.t., the mixture was acidified with 0.2 M HCl aqueous solution, which was then extracted with mixed solvent of DCM/MeOH (200 mL*3, v/v = 10/1). The combined organic layers were washed with brine, dried over Na₂SO₄ and concentrated. The residue was purified by silica gel chromatography to afford the desired product **Example 28e** (650 mg, 14% yield) as a yellow solid. LCMS [M+1]⁺ = 328.3.

### Step 4: Example 28g

To a solution of **Example 28e** (600 mg, 1.84 mmol, 1.0 eq) and **Example 28f** (580 mg, 3.67 mmol, 2.0 eq) in DMF (12 mL) were added CuI (348.6 mg, 1.84 mmol, 1.0 eq), 1,10-phenanthroline (182 mg, 0.92 mmol, 0.5 eq) and K₃PO₄ (778 mg, 3.67 mmol, 2.0 eq). The mixture was stirred at 110°C for 16 h under N₂. The reaction solution was diluted with EtOAc (100 mL), washed with brine (100 mL*3), dried over Na₂SO₄ and concentrated. The residue was purified by silica gel chromatography to afford the desired product **Example 28g** (120 mg, 16.2% yield) as a yellow solid. LCMS [M+1]⁺ = 405.3.

### Step 5: Example 28h

To a solution of **Example 28g** (115 mg, 0.284 mmol, 1.0 eq) in EtOH (2.2 mL) and water (0.7 mL) were added Zn (92.5 mg, 1.423 mmol, 5.0 eq), and NH₄Cl (76.8 mg, 1.423 mmol, 5.0 eq). The reaction mixture was stirred for 1 h at 80°C. After cooled to room temperature, the mixture was filtered, and the filtrate was concentrated. The crude product was purified by silica gel flash column chromatography to afford the product **Example 28h** (85 mg, 80% yield) as a yellow solid. LCMS [M+1]⁺ = 375.3.

### Step 6: Example 28i

To a solution of **Example 28h** (80 mg, 0.214 mmol, 1.0 eq) in DCM (1 mL) was added HCl/dioxane (0.3 mL, 4M in dioxane), which was stirred at r.t. for 1 h. After the reaction was completed, the solvent was concentrated to give **Example 28i** (70 mg, crude) as a yellow solid. The crude was used next step directly without further purification. LCMS [M+1]⁺ = 275.3.

### Step 7: Example 28k

To a solution of **Example 28j** (57 mg, 0.175 mmol, 0.8 eq) in DCM (1 mL) were added HATU (99.8 mg, 0.263 mmol, 1.2 eq) and DIEA (113 mg, 0.876 mmol, 4.0 eq). The mixture was stirred for 20 min, then **Example 28i** (60 mg, 0.219 mmol, 1.0 eq) was added. The reaction mixture was stirred at r.t. for 2 h. The solution was concentrated in vacuum, the crude was purified by Prep-TLC to afford the product **Example 28k** (70 mg, 69% yield) as a yellow solid. LCMS [M+1]⁺ = 583.3.

### Step 8: Example 28l

To a solution of **Example 28k** (70 mg, 0.12 mmol, 1.0 eq) in dioxane (1 mL) were added Cs₂CO₃ (78 mg, 0.24 mmol, 2.0 eq) and 3^{rd}*t*-Bu-Xphos-Pd (11 mg, 0.012 mmol, 0.1 eq). The reaction mixture was stirred for 4 h at 80°C under N₂ protection. The solid was filtered out, and the filtrate was concentrated. The residue was purified by Prep-TLC to afford the **Example 28l** (45 mg, 69% yield) as a yellow solid. LCMS [M+1]⁺ =547.3.

### Step 9: Example 28

To a solution of **Example 28l** (40 mg, 0.073 mmol, 1.0 eq) in DCM (1 mL) was added TFA (0.3 mL) dropwise at 0°C. The reaction mixture was stirred for 2 h at r.t. After completion, the reaction mixture was concentrated. The crude product was dissolved in MeOH (2 mL) and basified with NaHCO₃. The solid was filtered out and filtrate was concentrated. The residue was purified by Prep-TLC to afford the Example 28 (4.2 mg, 13% yield) as a yellow solid. LCMS [M+1]⁺ = 447.1. ¹H NMR (300 MHz, DMSO-*d₆*)δ 9.19 (s, 1H), 8.64 (d, 1H), 8.48 (d, 1H), 8.22-8.15 (m, 2H), 8.08-8.01 (m, 1H), 7.96-7.90 (m, 1H), 7.86 (d, 1H), 7.59-7.51 (m, 2H), 6.11 (s, 1H), 4.43 (s, 2H), 4.06-3.93 (m, 1H), 3.63-3.48 (m, 2H), 2.92 (d, 3H), 1.19 (d, 3H).

### Example 29:

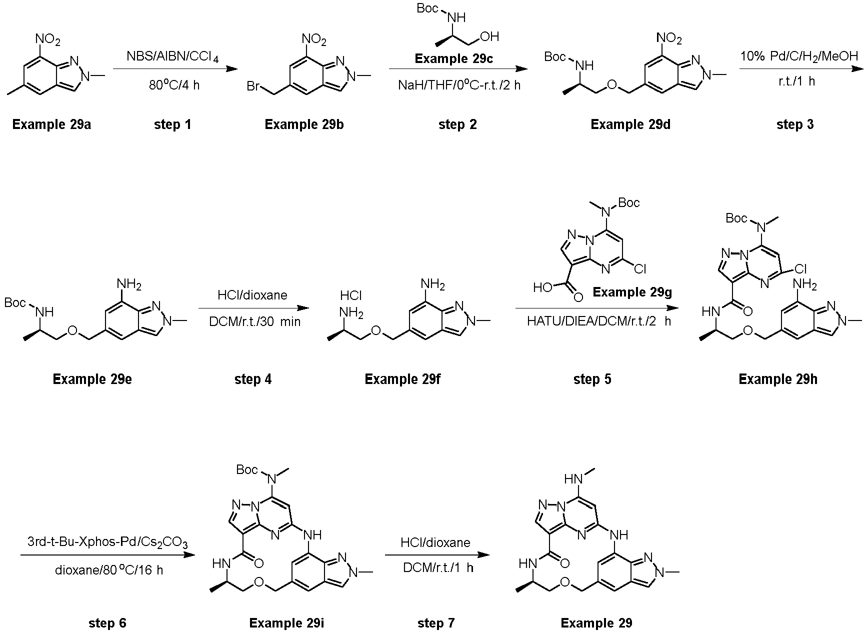

### Step 1: Example 29b

A solution of **Example 29a** (2.40 g, 12.57 mmol, 1.0 eq) in CCl₄ (100 mL) was heated to 80 °C, followed by addition of NBS (2.68 g, 15.08 mmol, 1.2 eq) and AIBN (2.06 g, 12.57 mmol, 1.0 eq). The reaction mixture was stirred for 4 h at 80°C. The reaction solution was concentrated and purified by silica gel flash column chromatography to afford the desired product **Example 29b** (2.16 g, 63.7% yield) as a yellow solid.

### Step 2: Example 29d

To a solution of **Example 29c** (2.80 g, 16.00 mmol, 2.0 eq) in THF (100 mL) was added NaH (480 mg, 60% in mineral oil, 12.00 mmol, 1.5 eq) in portions at 0°C. After stirring for 30 min, **Example 29b** (2.16 g, 8.00 mmol, 1.0 eq) was added, which was stirred for another 2 h at r.t. The reaction was quenched with saturated aqueous NH₄Cl and extracted with EtOAc (100 mL*3). The combined organic layers were washed with brine, dried over Na₂SO₄ and concentrated. The residue was purified by silica gel flash column chromatography to afford the desired product **Example 29d** (1.27 g, 43.6% yield) as a yellow solid. LCMS [M+1-56]⁺ = 309.2.

### Step 3: Example 29e

**Example 29d** (1.27 g, 0.84 mmol, 1.0 eq) was dissolved in MeOH (30 mL), and 10% Pd/C (500 mg) was added in portions under N₂ protection. The system was evacuated and then refilled with hydrogen. The mixture solution was stirred for 1 h at r.t. under H₂ balloon. The reaction mixture was filtered through a Celite pad and the filtrate was concentrated to afford the desired product **Example 29e** (1.14 g, 97.8% yield) as yellow oil. LCMS [M+1+22]⁺ = 357.3.

### Step 4: Example 29f

To a solution of **Example 29e** (1.14 g, 3.41 mmol, 1.0 eq) in DCM (30 mL) was added HCl/dioxane (10 mL, 4 M in dioxane). The reaction solution was stirred for 0.5 h at r.t., and the solvent was removed to afford the crude desired product **Example 29f** (1.64 g, quant. yield) as a yellow solid. LCMS [M+1]⁺ = 235.3.

### Step 5: Example 29h

To a solution of **Example 29g** (300 mg, 0.92 mmol, 1.0 eq) in DCM (15 mL) were added DIEA (947 mg, 7.34 mmol, 8.0 eq) and HATU (383 mg, 1.01 mmol, 1.1 eq). After stirring for 30 min, **Example 29f** (373 mg, 1.38 mmol, 1.5 eq) was added. The reaction solution was stirred for 2 h at r.t. The reaction mixture was concentrated and purified by silica gel flash column chromatography to afford the desired product **Example 29h** (280 mg, 56.2% yield) as a yellow solid. LCMS [M+1]⁺ = 543.3.

### Step 6: Example 29i

To a solution of **Example 29h** (240 mg, 0.44 mmol, 1.0 eq) in dioxane (10 mL) were added Cs₂CO₃ (288 mg, 0.88 mmol, 2.0 eq) and 3rd-t-Bu-Xphos-Pd (39 mg, 0.044 mmol, 0.1 eq). The reaction mixture was stirred for 16 h at 80°C under N₂. The reaction mixture was concentrated and purified by prep-TLC to afford the desired product **Example 29i** (75 mg, 33.5% yield) as a yellow solid. LCMS [M+1]⁺ = 507.3.

### Step 7: Example 29

To a solution of **Example 29i (65** mg, 0.13 mmol, 1.0 eq) in DCM (6 mL) was added HCl/dioxane (3 mL, 4 M in dioxane). The reaction solution was stirred for 1 h at r.t. and concentrated. The crude product was dissolved in MeOH, and Na₂CO₃ solid (excess) was added to the mixture, which was stirred for 10 min at r.t. The mixture was filtered and the filtrate was concentrated. The residue was purified by prep-TLC to afford the desired product **Example 29** (33.8 mg, 64.8% yield) as an off-white solid. LCMS [M+1]⁺ = 407.3. ¹H NMR (300 MHz, DMSO-*d*₆) δ 9.60 (s, 1H), 8.39 (d, 1H), 8.31 (s, 1H), 8.23 (d, 1H), 8.13 (s, 1H), 7.84 (d, 1H), 7.22 (s, 1H), 6.08 (s, 1H), 4.65 (d, 1H), 4.51 (d, 1H), 4.20 (s, 3H), 4.01 - 3.90 (m, 1H), 3.51 (d, 1H), 3.38 (d, 1H), 2.93 (d, 3H), 1.13 (d, 3H).

### Example 30 & Example 31

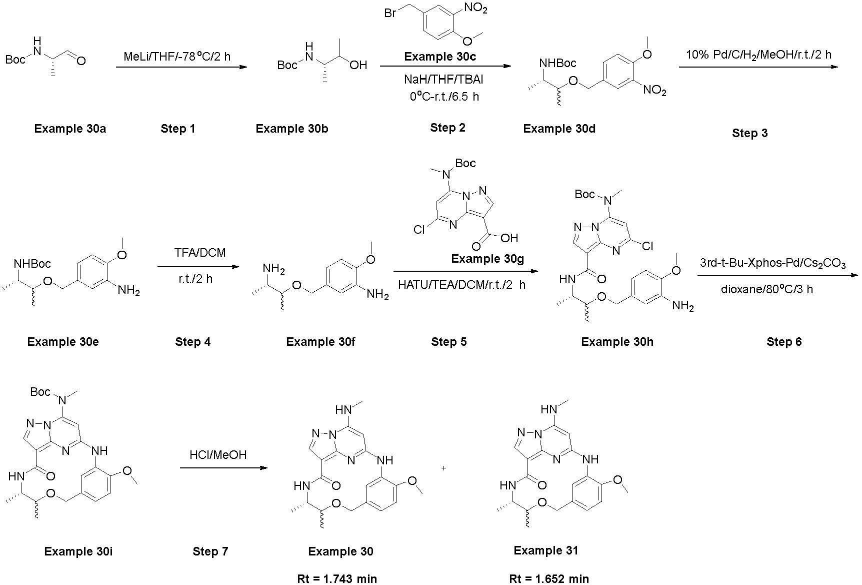

### Step 1: Example 30b

To a solution of **Example 30a** (2.6 g, 15.0 mmol) in THF (30 mL) was drop-wised MeLi (18.7 mL, 1.6 moL/L) at -78°C under N₂ protection. The reaction mixture was stirred at -78°C for 2 h. Then the mixture was quenched by NH₄Cl (aq.), diluted by DCM, washed by water, and dried over anhydrous Na₂SO₄. The solution was concentrated under reduced pressure and the residue was purified by silica gel column chromatography to give **Example 30b** (1.8 g, yield: 63%) as a white solid.

### Step 2: Example 30d

To a solution of **Example 30b** (380 mg, 2.0 mmol), **TBAI** (75 mg, 0.2 mmol) in THF (5 mL) was added NaH (173 mg, 60% in mineral oil, 3.0 mmol) at 0°C. The reaction mixture was warmed to room temperature and stirred for 0.5 h. Then **Example 30c** (492 mg, 2.0 mmol) was added. The mixture was stirred at r.t. for another 6 h. The reaction mixture was quenched by aq. NH₄Cl, extracted by EtOAc, and dried over anhydrous Na₂SO₄. The solution was concentrated under reduced pressure. The residue was purified by silica gel column chromatography to give **Example 30d** (230 mg, yield: 32%) as a yellow solid. LCMS [M+1-100] ⁺ = 255.1.

### Step 3: Example 30e

To a solution of **Example 30d** (230 mg, 0.65 mmol) and 10% Pd/C (50 mg) in MeOH (10 mL) was stirred at r.t. for 2 h under 1 atm H₂. After completion, the mixture was filtered over a Celite, and the filtrate was concentrated under reduced pressure. The residue **Example 30e** (178 mg, yield: quant.) was obtained as a yellow solid, which was used in the next step directly. LCMS [M-188] ⁺ = 137.1

### Step 4: Example 30f

To a solution of **Example 30e** (178 mg, 0.75 mmol) in DCM (4 mL) was added TFA (1.0 mL), which was stirred at r.t. for 2 h. The mixture was concentrated to give the crude product **Example 30f** (138 mg, crude, yield: 82%) as black oil. LCMS [M+1] ⁺ = 225.1.

### Step 5: Example 30h

To a solution of **Example 30f** (138 mg, 0.62 mmol), **Example 30g** (200 mg, 0.62 mmol), and TEA (311 mg, 3.1 mmol) in DCM (5 mL) was added HATU (236 mg, 0.62 mmol). The reaction mixture was stirred at r.t. for 2 h. Then, DCM (40 mL) was added to the reaction mixture, which was washed with brine (20 mL*2), dried over anhydrous Na₂SO₄ and concentrated. The residue was purified by silica gel column chromatography to afford the desired product **Example 30h** (150 mg, yield: 45%) as a brown solid. LCMS [M+1] ⁺ = 533.2

### Step 6: Example 30i

To a mixture of **Example 30h** (150 mg, 0.28 mmol), Cs₂CO₃ (137 mg, 0.42 mmol) in dioxane (2 mL) was added 3rd-t-Bu-Xphos-Pd (25 mg, 0.028 mmol). The mixture was degassed with N₂ three times, and stirred for 3 h at 80°C. Then the reaction mixture diluted by DCM, washed by water, dried over anhydrous Na₂SO₄, and then concentrated under reduced pressure to afford crude **Example 30i** (170 mg, crude, yield: quant.) as a white solid, which was used in the next step without further purification. LCMS [M+1]⁺ = 497.2.

### Step 7: Example 30 & Example 31

To a solution of **Example 30i** (120 mg, crude, 0.34 mmol) in DCM (4 mL) was added TFA (1.0 mL), which was stirred at r.t. for 2 h. The mixture was concentrated, and the residue was purified by Prep-HPLC to afford the desired products:
**Example 30** (6.7 mg, R.T. = 1.743 min, yield: 5%) as a white solid. LCMS [M+1]⁺ = 397.2. ¹H NMR (400 MHz, DMSO-*d*₆) δ 8.81 (s, 1H), 8.54 (s, 1H), 8.15 (d, 1H), 8.10 (s, 1H), 7.77 (d, 1H), 6.89 (q, 2H), 5.98 (s, 1H), 4.58 (d, 1H), 4.31 (d, 1H), 3.84 (s, 3H), 3.81 (d, 1H), 3.73 (d, 1H), 3.28 (s, 1H), 2.88 (d, 3H), 1.17 (d, 3H), 1.08 (d, 3H).
**& Example 31** (3.1 mg, R.T. = 1.652 min, yield: 2%) as a white solid. LCMS [M+1] ⁺= 397.2 ¹H NMR (400 MHz, DMSO-*d*₆) δ 8.94 (s, 1H), 8.33 (s, 1H), 8.15 (s, 1H), 8.07 (s, 1H), 7.79 (s, 1H), 6.92 (d, 2H), 5.85 (s, 1H), 4.56 (d, 1H), 4.39 (d, 1H), 3.84 (s, 3H), 3.59 (s, 1H), 2.88 (d, 3H), 1.12 (d, 3H), 1.03 (d, 3H).

### Example 32 & Example 33

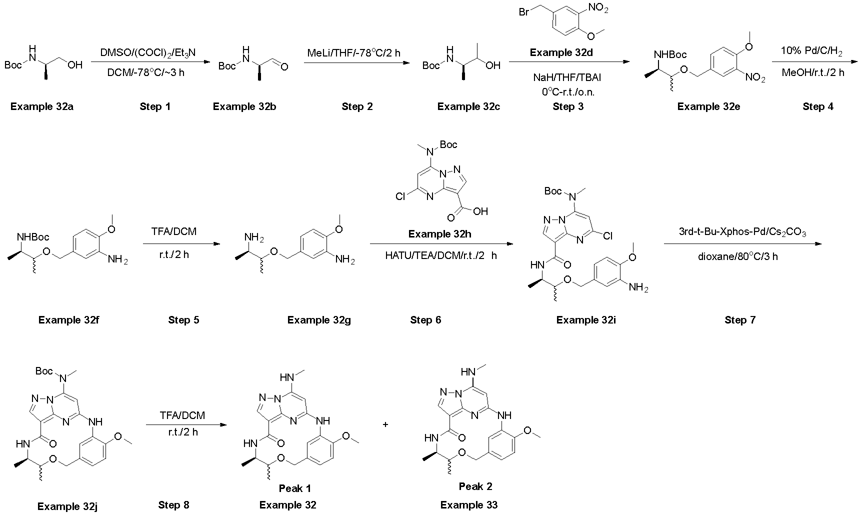

### Step 1: Example 32b

To a solution of DMSO (3.3 mL, 47.9 mmol) in DCM (10 mL) was added (COCl)₂ (3.0 mL, 34.2 mmol) at -78°C, which was stirred at -78°C for 15 min. Then **Example 32a** (3.0 g, 17.1 mmol) in THF (2 mL) was added dropwise, which was stirred at -78°C for 2 h. Then TEA (3.3 mL, 85.6 mmol) was added dropwise and the resulting mixture was stirred at -78°C for 0.5 h. The reaction mixture was quenched by brine, diluted by DCM, washed by water, and dried over anhydrous Na₂SO₄. The solution was concentrated under reduced pressure, which was purified by silica gel column chromatography to give **Example 32b** (2.1 g, yield: 71%) as a white solid.

### Step 2: Example 32c

To a solution of **Example 32b** (1.2 g, 6.9 mmol) in THF (10 mL) was added MeLi (10.8 mL, 17.3 mmol, 1.6 moL/L) dropwise at -78°C under N₂ protection. The reaction mixture was stirred at -78°C for 2 h. Then the reaction mixture was quenched by aq. NH₄Cl, diluted by DCM, washed by water, and dried over anhydrous Na₂SO₄. The solution was concentrated under reduced pressure, which was purified by silica gel column chromatography to give **Example 32c** (600 mg, yield: 46%) as a white solid.

### Step 3: Example 32e

To a solution of **Example 32c** (500 mg, 2.64 mmol), **TBAI** (96 mg, 0.26 mmol) in THF (5 mL) was added NaH (127 mg, 60% in mineral oil, 5.28 mmol) at 0°C in portions. The reaction mixture was warmed to room temperature and stirred at r.t. for 0.5 h. Then **Example 32d** (780 mg, 3.17 mmol) was added. The mixture was stirred at r.t. for overnight. The reaction mixture was quenched by aq. NH₄Cl, and then extracted by EtOAc, and dried over anhydrous Na₂SO₄. The solution was concentrated under reduced pressure and purified by silica gel column chromatography to give **Example 32e** (160 mg, yield: 18%) as a yellow solid. LCMS [M+1-100]⁺ = 255.1.

### Step 4: Example 32f

A mixture of **Example 32e** (160 mg, 0.45 mmol) and 10% Pd/C (20 mg) in MeOH (10 mL) was stirred at r.t. for 2 h under 1 atm of H₂. Then the suspension was filtered, and the organic phase was concentrated under reduced pressure to give crude **Example 32f** (140 mg, yield: quant.) as a yellow solid, which was used in the next step directly. LCMS [M-188]⁺ = 137.1.

### Step 5: Example 32g

To a solution of **Example 32f** (140 mg, 0.43 mmol) in DCM (4 mL) was added TFA (2.0 mL), which was stirred at r.t. for 2 h. The mixture was concentrated to give the crude product **Example 32g** (100 mg crude, yield: quant.) as black oil. LCMS [M+1]⁺ = 225.1.

### Step 6: Example 32i

To a solution of **Example 32g** (97 mg, 0.43 mmol), **Example 32h** (141 mg, 0.43 mmol), TEA (112 mg, 0.86 mmol) in DCM (5 mL) was added HATU (246 mg, 0.65 mmol). The reaction mixture was stirred at r.t. for 2 h. Then DCM (40 mL) was added to the reaction mixture, which was washed with brine (20 mL*2), dried over anhydrous Na₂SO₄ and concentrated. The residue was purified by silica gel column chromatography to afford the desired product **Example 32i** (110 mg, yield: 48%) as a brown solid. LCMS [M+1]⁺ = 533.2.

### Step 7: Example 32j

To a mixture of **Example 32i** (110 mg, 0.21 mmol), Cs₂CO₃ (134 mg, 0.41 mmol) in dioxane (2 mL) was added 3rd-t-Bu-Xphos-Pd (17 mg, 0.021 mmol). The mixture was degassed with N₂ three times, and stirred for 3 h at 80°C. Then the reaction mixture diluted by DCM, washed by water, dried over anhydrous Na₂SO₄, and then concentrated under reduced pressure to afford crude **Example 32j** (100 mg crude, yield: 97%) as a white solid, which was used in the next step without further purification. LCMS [M+1]⁺= 497.2.

### Step 8: Example 32 & Example 33

To a solution of **Example 32j** (100 mg, 0.201 mmol) in DCM (4 mL) was added TFA (1.0 mL), which was stirred at r.t. for 2 h. The mixture was concentrated and neutralized by aq NaHCO₃, extracted by DCM, washed by water, dried over anhydrous Na₂SO₄, and then concentrated under reduced pressure. The residue was purified by Prep-HPLC to afford the desired product **Example 32** (5.7 mg, yield: 16%) as a white solid. As Peak 1: LCMS [M+1]⁺ = 397.2; Rt = 1.609 min. ¹H NMR (400 MHz, DMSO-*d*₆) δ 8.97 (s, 1H), 8.34 (d, 1H), 8.16 (d, 1H), 8.08 (s, 1H), 7.81 (d, 1H), 6.98-6.88 (m, 2H), 5.86 (s, 1H), 4.57 (d, 1H), 4.40 (d, 1H), 3.85 (s, 3H), 3.59 (q, 1H), 3.25-3.15 (m, 1H), 1.13 (d, 3H), 1.04 (d, 3H).
**& Example 33** (5.1 mg, yield: 13%) as a white solid. As Peak 2: LCMS [M+1]⁺ = 397.2; Rt = 1.675 min. ¹H NMR (400 MHz, Methanol-*d*₄) δ 8.64-8.56 (m, 1H), 8.42 (s, 1H), 8.15 (s, 1H), 6.89 (t, 2H), 5.77 (s, 1H), 5.49 (s, 1H), 4.65 (d, 1H), 4.36 (d, 1H), 3.91 (s, 4H), 3.82 (d, 1H), 3.02 (s, 3H), 1.23 (d, 6H).

### Example 34:

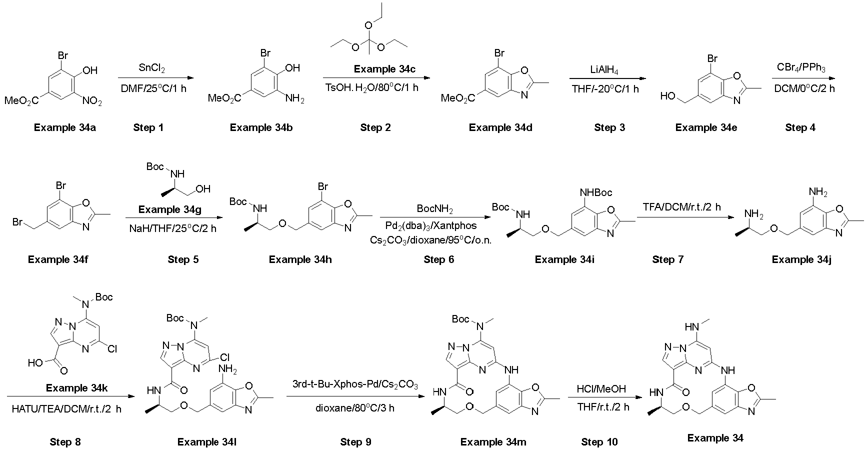

### Step 1: Example 34b

To a solution of **Example 34a** (15.0 g, 54.0 mmol) in DMF (225 mL) was added SnCl₂ (35.8 g, 189.0 mmol). The mixture was degassed with N₂ three times, and stirred for 1 h at 25°C. Then water was added, and the organics were extracted with EtOAc for 3 times. The combined organics were dried over anhydrous Na₂SO₄, and then concentrated under reduced pressure, which was purified by silica gel column chromatography to afford the desired product **Example 34b** (8.1 g, yield: 61%) as a red solid. LCMS [M+1]⁺ = 246.0.

### Step 2: Example 34d

To a solution of **Example 34a** (8.1 g, 32.5 mmol) in **Example 34c** (100 mL) was added TsOH.H₂O (627 mg, 3.3 mmol). The mixture was degassed with N₂ three times, and stirred for 1 h at 80°C. The mixture was concentrated under reduced pressure and purified by silica gel column chromatography to afford the desired product **Example 34d** (5.5 g, yield: 61%) as a yellow solid. LCMS [M+1]⁺ = 269.9.

### Step 3: Example 34e

To a solution of **Example 34d** (5.97 g, 22.10 mmol) in THF (90 mL) was added LiAlH₄ (1.00 g, 26.47 mmol) at -20°C. The mixture was degassed with N₂ three times, and stirred for 1 h at -20°C. The reaction was then quenched by the addition of water (1.2 mL) at -20°C. The resulting solution was diluted with aqueous NaOH solution (15%, 3.6 mL) and EtOAc (1.2 mL) at room temperature. The solids were filtered out. The filtrate was concentrated under reduced pressure and purified by silica gel column chromatography to afford the desired product **Example 34e** (2.31 g, yield: 46%) as a yellow solid. LCMS [M+1]⁺ = 241.9.

### Step 4: Example 34f

To a solution of **Example 34e** (2.31 g, 9.79 mmol) in DCM (45 mL) was added PPh₃ (3.30 g, 12.70 mmol). The mixture was cooled to 0°C. Then a solution of CBr₄ (4.20 g, 12.70 mmol) in DCM (5 mL) was added dropwise. After addition, the reaction mixture was stirred for 2 h at 0°C. The mixture was concentrated under reduced pressure and purified by silica gel column chromatography to afford the desired product **Example 34f** (2.51 g, yield: 82%) as a yellow solid. LCMS [M+1]⁺ = 305.9

### Step 5: Example 34h

To a solution of **Example 34f** (2.20 g, 7.26 mmol), **Example 34g** (1.52 g, 8.59 mmol) in THF (40 mL) was added NaH (348 mg, 60% in mineral oil, 8.59 mmol). The mixture was degassed with N₂ three times, and stirred for 2 h at 25°C. The reaction was then quenched by addition of aqueous NH₄Cl solution (10 mL) and the organics were extracted with EtOAc for 3 times. The combined organics was dried over anhydrous Na₂SO₄, concentrated under reduced pressure and purified by silica gel column chromatography to afford the desired product **Example 34h** (1.20 g, yield: 75%) as yellow oil. LCMS [M+1-100]⁺ = 299.0.

### Step 6: Example 34i

To a solution of **Example 34h** (820 mg, 2.06 mmol), NH₂Boc (480 mg, 4.12 mmol), Cs₂CO₃ (1340 mg, 4.12 mmol) in dioxane (5.0 mL) were added Pd₂(dba)₃ (189 mg, 0.21 mmol), and Xantphos (123 mg, 0.21 mmol). The mixture was degassed with N₂ three times, and stirred for overnight at 95°C. Then the reaction mixture diluted by EtOAc, washed by water, dried over anhydrous Na₂SO₄, and then concentrated under reduced pressure. The residue was purified by silica gel column chromatography to afford the desired product **Example 34i** (520 mg, yield: 58%) as a white solid. LCMS [M+1-100]⁺ = 336.2.

### Step 7: Example 34j

To a solution of **Example 34i** (170 mg, 0.55 mmol) in DCM (5.0 mL) was added TFA (1.0 mL), which was stirred at r.t. for 2 h. The mixture was concentrated to give the crude product **Example 34j** (348 mg crude, quant. yield) as black oil. LCMS [M+1]⁺ =236.1

### Step 8: Example 34l

To a solution of **Example 34j** (348 mg crude, 0.75 mmol), **Example 34k** (245 mg, 0.75 mmol), TEA (755 mg, 7.50 mmol) in DCM (4 mL) was added HATU (284 mg, 0.75 mmol). The reaction mixture was stirred at r.t. for 2 h. Then DCM (40 mL) was added to the reaction mixture, which was washed with brine (20 mL*2), dried over anhydrous Na₂SO₄ and concentrated. The residue was purified by silica gel column chromatography to afford the desired product **Example 34l** (70 mg, yield: 17%) as a brown solid. LCMS [M+1]⁺ = 544.2.

### Step 9: Example 34m

To a mixture of **Example 34l** (70 mg, 0.13 mmol), Cs₂CO₃ (65 mg, 0.20 mmol) in dioxane (2.0 mL) was added 3rd-t-Bu-Xphos-Pd (12 mg, 0.013 mmol). The mixture was degassed with N₂ three times, and stirred for 3 h at 80°C. Then the reaction mixture was diluted by EtOAc, washed by water, dried over anhydrous Na₂SO₄, and then concentrated under reduced pressure to afford crude **Example 34m** (60 mg crude, quant. Yield) as a white solid, which was used in the next step without further purification. LCMS [M+1]⁺ = 508.2

### Step 10: Example 34

To a solution of **Example 34m** (60 mg crude, 0.49 mmol) in THF (1.4 mL) was added MeOH/HCl (2.0 mL, 6.0 moL/L), which was stirred at r.t. for 2 h. The mixture was concentrated, and the residue was purified by Prep-HPLC to afford the desired product **Example 34** (1.2 mg, yield: 3%) as a white solid. LCMS [M+1]⁺ = 408.1. ¹H NMR (400 MHz, DMSO-*d*₆) δ 9.93 (s, 1H), 8.38 (d, 1H), 8.22 (d, 1H), 8.13 (s, 1H), 7.97 (d, 1H), 7.21 (s, 1H), 5.82 (s, 1H), 4.68 (d, 1H), 4.54 (d, 1H), 3.89 (d, 1H), 3.49 (d, 1H), 3.29 (s, 1H), 2.92 (d, 3H), 2.63 (s, 3H), 1.12 (d, 3H).

### Example 35:

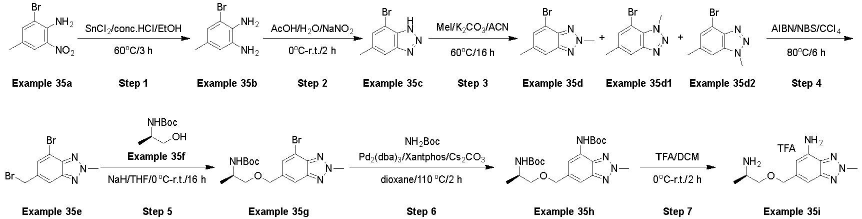

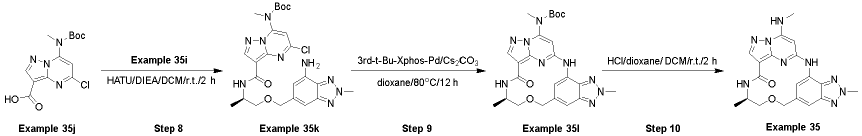

### Step 1: Example 35b

To a solution of **Example 35a** (20.0 g, 86.6 mmol, 1.0 eq) in EtOH (120 mL) and conc. HCl (40 mL) was added SnCl₂ (97.4 g, 433 mmol, 5.0 eq). The reaction mixture was stirred at 60°C for 3 h under N₂. After cooled to room temperature, the mixture was poured into 2M NaOH aqueous solution (750 mL) at 0°C. DCM (800 mL) was added to the mixture, and the white solid was removed by filtration. The organic layer was separated, and the aqueous phase was extracted with DCM (500 mL*2). The combined organic extracts were washed with brine, dried over Na2SO4, and concentrated. The crude was purified by silica gel flash column chromatography to afford the product **Example 35b** (16.8 g, 97% yield) as a yellow solid. LCMS [M+1]⁺ = 201.2.

### Step 2: Example 35c

A solution of **Example 35b** (12.0 g, 59.7 mmol, 1.0 eq) in AcOH (115 mL) and H₂O (33 mL) was cooled to 0°C, followed by the addition of NaNO₂ (4.9 g, 71.6 mmol, 1.2 eq) in water (20 mL). The reaction mixture was stirred for 2 h at r.t. After completion, a gradual formation of a yellow precipitate was observed. The solid was collected by filtration and concentrated to afford the product **Example 35c** (12.5 g, 99% yield) as a yellow solid.

LCMS [M+1]⁺ =212.2.

### Step 3: Example 35d

To a solution of **Example 35c** (12.7 g, 60.0 mmol, 1.0 eq) in ACN (130 mL) were added K₂CO₃ (16.6 g, 120.0 mmol, 2.0 eq) and MeI (25.6 g, 180.0 mmol, 3.0 eq). The reaction mixture was stirred at 60°C for 16 h. After cooled to room temperature, the solid was filtered out and filtrate was concentrated. The crude product was purified by silica gel flash column chromatography to afford the product **Example 35d** (3.2 g, 24% yield, retention time: 1.48 min) as a white solid, **Example 35d1** (2.5 g, 18% yield, retention time: 1.42 min) as a white solid, and **Example 35d2** (3.4 g, 25% yield, retention time: 1.33 min) as a white solid. LCMS [M+1]⁺ = 226.2.

### Step 4: Example 35e

To a solution of **Example 35d** (1.5 g, 6.6 mmol, 1.0 eq) in CCl₄ (30 mL) was added NBS (1.76 g, 9.9 mmol, 1.5 eq) and AIBN (541.2 mg, 3.3 mmol, 0.5 eq). The reaction mixture was stirred at 80°C for 6 h. After cooled to room temperature, the solvent was removed, and the residue was purified by silica gel flash column chromatography to afford the product **Example 35e** (1.5 g, 74% yield) as a white solid. LCMS [M+1]⁺ = 306.2.

### Step 5: Example 35g

To a solution of **Example 35f** (875 mg, 5.0 mmol, 1.5 eq) in THF (20 mL) were added NaH (160 mg, 4.0 mmol, 1.2 eq) in portions at 0°C. The mixture was stirred for 30 min at the same temperature, then **Example 35e** (1.0 g, 3.3 mmol, 1.0 eq) in THF (15 mL) was added dropwise. The reaction mixture was stirred at r.t. for 16 h. The mixture was poured into a saturated aqueous solution of NH₄Cl (50 mL), which was extracted with EtOAc (50 mL*3). The combined organic layers were washed with brine, dried over Na₂SO₄, and concentrated. The crude product was purified by silica gel flash column chromatography to afford the product **Example 35g** (860 mg, 65% yield) as a white solid. LCMS [M+1]⁺ = 399.3.

### Step 6: Example 35h

To a solution of **Example 35g** (860 mg, 2.16 mmol, 1.0 eq) in dioxane (10 mL) were added Cs₂CO₃ (1.4 g, 4.32 mmol, 2.0 eq), NH₂-Boc (505.4 mg, 4.32 mmol, 2.0 eq), Xantphos (250.0 mg, 0.43 mmol, 0.2 eq) and Pd₂(dba)₃.CHCl₃ (227.7 mg, 0.22 mmol, 0.1 eq). The reaction mixture was stirred for 2 h at 110°C under N₂ protection. After cooled to room temperature, the solvent was removed. The crude product was purified by silica gel flash column chromatography to afford the product **Example 35h** (670 mg, 71% yield) as yellow oil. LCMS [M+1]⁺ = 436.4.

### Step 7: Example 35i

To a solution of **Example 35h** (670 mg, 1.54 mmol, 1.0 eq) in DCM (5 mL) was added TFA (2.5 mL) dropwise at 0°C. The reaction mixture was stirred for 2 h at r.t. The solution was concentrated in vacuum to give the crude product **Example 35i** (1.2 g, crude)as yellow oil, which was used to next step directly without purification. LCMS [M+1]⁺ = 236.2.

### Step 8: Example 35k

To a solution of **Example 35j** (817.5 mg, 2.5 mmol, 0.8 eq) in DCM (20 mL) were added HATU (2.28 g, 6.0 mmol, 1.2 eq) and DIEA (5.16 g, 40.0 mmol, 8.0 eq). The mixture was stirred for 20 min, then **Example 35i** (1.2 g, 5.0 mmol, 1.0 eq) was added. The reaction mixture was stirred at r.t. for 2 h. The solution was concentrated in vacuum to give the crude product was purified by silica gel flash column chromatography to afford the product **Example 35k** (260 mg, 19% yield) as a yellow solid. LCMS [M+1]⁺ = 544.4.

### Step 9: Example 35l

To a solution of **Example 35k** (260 mg, 0.48 mmol, 1.0 eq) in dioxane (3 mL) were added Cs₂CO₃ (313.0 mg, 0.96 mmol, 2.0 eq) and 3^{rd}-*t*-Bu-Xphos-Pd (44.1 mg,0.05 mmol, 0.1 eq). The reaction mixture was stirred for 12 h at 80 °C under N₂ protection. The solid was filtered out and filtrate was concentrated. The residue was purified by Prep-TLC to afford the **Example 35l** (75 mg, 31%yield) as a yellow solid. LCMS [M+1]⁺ = 508.3.

### Step 10: Example 35

To a solution of **Example 35l** (75 mg, 0.15 mmol, 1.0 eq) in DCM (3 mL) was added HCl/dioxane (3mL, 4M in dioxane) dropwise at 0°C. The reaction mixture was stirred for 2 h at r.t. After completion, the reaction mixture was concentrated. The crude product was dissolved in MeOH (2 mL), and NaHCO₃ (excess) was added. The mixture was stirred for 20 min at r.t., and then DCM (20 mL) was added. The solid **was** filtered out and filtrate was concentrated. The residue was purified by Prep-TLC to afford the **Example 35** (22.7 mg, 37% yield) as an off-white solid. LCMS [M+1]⁺ = 408.2. ¹H NMR (300 MHz, DMSO-*d*₆) δ 10.05 (s, 1H), 8.47 (s, 1H), 8.36 (d, 1H), 8.17 (s, 1H), 7.96 (d, 1H), 7.41 (d,1H), 6.06 (s, 1H), 4.76 (d, 1H), 4.65 (d, 1H), 4.53 (s, 3H), 3.99-3.95 (m, 1H), 3.59-3.56 (m, 1H), 3.44-3.38 (m, 1H), 2.95 (d, 3H), 1.16 (d, 3H).

### Example 36:

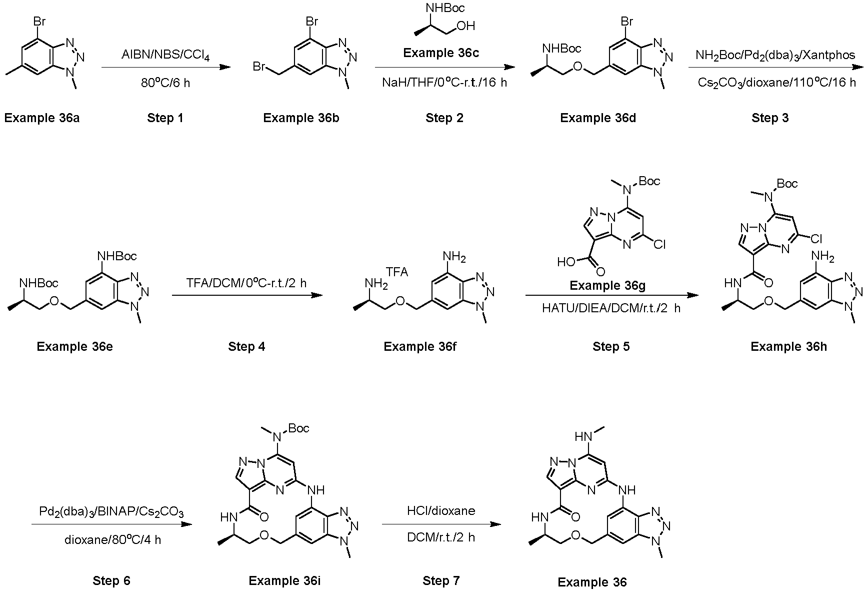

### Step 1: Example 36b

To a solution of **Example 36a** (1.2 g, 5.0 mmol, 1.0 eq) in CCl₄ (12 mL) were added NBS (1.42 g, 8.0 mmol, 1.5 eq) and AIBN (262 mg,1.6 mmol, 0.3 eq). The reaction mixture was stirred at 80°C for 6 h. After cooled to room temperature, the solvent was removed, and the residue was purified by silica gel flash column chromatography (to afford the product **Example 36b** (850 mg, 52% yield) as a yellow solid. LCMS [M+1]⁺ = 306.2.

### Step 2: Example 36d

To a solution of **Example 36c** (2.15 g, 12.0 mmol, 1.5 eq) in THF (25 mL) was added NaH (490 mg, 60% in mineral oil, 12.0 mmol, 1.5 eq) in portions at 0°C. The mixture was stirred for 30 min at the same temperature, then **Example 36b** (2.5 g, 8.0 mmol, 1.0 eq) in THF (20 mL) was added dropwise. The reaction mixture was stirred at r.t. for 16 h. Then, the mixture was poured into a saturated aqueous solution of NH₄Cl (50 mL), which was extracted with EtOAc (50 mL*3). The combined organic layers were washed with brine, dried over Na₂SO₄ and concentrated. The crude product was purified by silica gel flash column chromatography to afford the product **Example 36d** (1.8 g, 55% yield) as an off-white solid. LCMS [M+1]⁺ = 399.3.

### Step 3: Example 36e

To a solution of **Example 36d** (1.8 g, 4.5 mmol, 1.0 eq) in dioxane (36 mL) were added Cs₂CO₃ (2.95 g, 9.0 mmol, 2.0 eq), NH₂-Boc (4.23 g, 36.0 mmol, 8.0 eq), Xantphos (130 mg, 0.23 mmol, 0.05 eq) and Pd₂(dba)₃ (470 mg, 0.45 mmol, 0.1 eq). The reaction mixture was stirred for 16 h at 110°C under N₂ protection. After cooled to room temperature, the solvent was removed. The crude product was purified by silica gel flash column chromatography to afford the product **Example 36e** (1.1 g, 56% yield) as yellow solid. LCMS [M+1] ⁺ = 436.3.

### Step 4: Example 36f

To a solution of **Example 36e** (1.1 g, 3.0 mmol, 1.0 eq) in DCM (11 mL) was added TFA (33 mL) dropwise at 0°C. The reaction mixture was stirred for 2 h at r.t. The solution was concentrated in vacuum to give the crude product **Example 36f** (1.7 g, crude) as brown oil, which was used to next step directly without purification. LCMS [M+1]⁺ =236.4.

### Step 5: Example 36h

To a solution of **Example 36g** (485 mg, 1.489 mmol, 0.7 eq) in DCM (10 mL) were added HATU (808 mg, 2.127 mmol, 1.0 eq) and DIEA (2.2 g, 17.021 mmol, 8.0 eq). The mixture was stirred for 20 min, and then **Example 36f** (500 mg, 2.127 mmol, 1.0 eq) was added. The reaction mixture was stirred at r.t. for 2 h. The solution was concentrated in vacuum, and the crude product was purified by prep-TLC to afford the product **Example 36h** (250 mg, 22% yield) as a yellow solid. LCMS [M+1] ⁺ = 544.3.

### Step6: Example 36i

To a solution of **Example 36h** (250 mg, 0.46 mmol, 1.0 eq) in dioxane (2 mL) were added Cs₂CO₃ (300 mg, 0.921 mmol, 2.0 eq), Pd₂(dba)₃ (47 mg, 0.046 mmol, 0.1 eq) and BINAP (14 mg, 0.023 mmol, 0.05 eq). The reaction mixture was stirred for 4 h at 80°C under N₂ protection. The solid was filtered out and filtrate was concentrated. The residue was purified by Prep-TLC to afford the **Example 36i** (120 mg, 52% yield) as a yellow solid. LCMS [M+1] ⁺ = 508.3.

### Step 7: Example 36

To a solution of **Example 36i** (120 mg, 0.236 mmol, 1.0 eq) in DCM (1.2 mL) was added HCl/dioxane (6 mL, 4M in dioxane) dropwise at 0°C. The reaction mixture was stirred for 2 h at r.t. After completion, the reaction mixture was concentrated. The crude product was treated with MeOH (2 mL), NaHCO₃(excess) was added to the solution, which was stirred for 20 minutes at r.t. Then, DCM (20 mL) was added to the mixture and the solid was filtered out. The filtrate was concentrated, and the residue was purified by Prep-TLC to afford the **Example 36** (56.8 mg, 59% yield) as an off-white solid. LCMS [M+1]⁺ = 408.2. ¹H NMR (300 MHz, DMSO-*d*₆) δ 10.31 (s, 1H), 8.54 (s, 1H), 8.38 (d, 1H), 8.19 (s,1H), 7.98 (d, 1H), 7.33 (s,1H), 6.13 (s, 1H), 4.81 (d, 1H), 4.68 (d, 1H), 4.29 (s, 3H), 3.99-3.95 (m, 1H), 3.99-3.95 (m, 1H), 3.45-3.29 (m, 1H), 2.96 (d, 3H), 1.15 (d, 3H).

### Example 37:

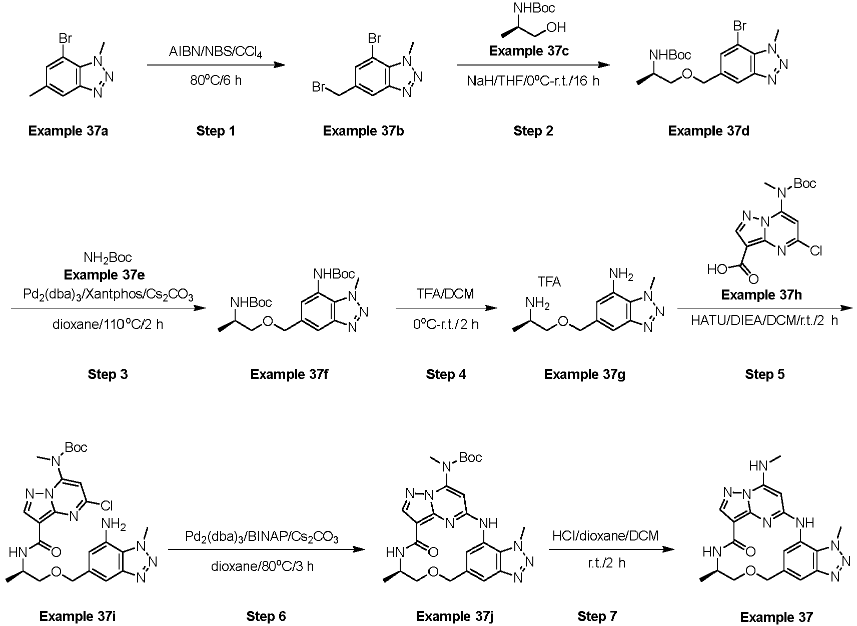

### Step 1: Example 37b

To a solution of **Example 37a** (1.9 g, 8.4 mmol, 1.0 eq) in CCl₄ (20 mL) were added NBS (1.64 g, 9.24 mmol, 1.1 eq) and AIBN (137.8 mg, 0.84 mmol, 0.1 eq). The reaction mixture was stirred at 80°C for 6 h. After cooled to room temperature, the solvent was removed, and the residue was purified by silica gel flash column chromatography to afford the product **Example 37b** (1.9 g, 74% yield) as a white solid. LCMS [M+1]⁺ = 306.2.

### Step 2: Example 37d

To a solution of **Example 37c** (1.6 g, 9.3 mmol, 1.5 eq) in THF (20 mL) was added NaH (372 mg, 60% in mineral oil, 9.3 mmol, 1.5 eq) in portions at 0°C. The mixture was stirred for 30 min at the same temperature, then **Example 37b** (1.9 g, 6.2 mmol, 1.0 eq) in THF (20 mL) was added dropwise. The reaction mixture was stirred at r.t. for 16 h. Then the mixture was poured into a saturated aqueous solution of NH₄Cl (50 mL), which was extracted with EtOAc (50 mL*3). The combined organic layers were washed with brine, dried over Na₂SO₄, and concentrated. The crude product was purified by silica gel flash column chromatography to afford the product **Example 37d** (1.5 g, 61% yield) as a white solid. LCMS [M+1]⁺ = 399.3.

### Step 3: Example 37f

To a solution of **Example 37d** (1.48 g, 3.7 mmol, 1.0 eq) in dioxane (14 mL) were added Cs₂CO₃ (2.4 g, 7.4 mmol, 2.0 eq), **Example 37e** (2.16 g, 18.5 mmol, 5.0 eq), Xantphos (428.5 mg, 0.74 mmol, 0.2 eq) and Pd₂(dba)₃ (383 mg, 0.37 mmol, 0.1 eq). The reaction mixture was stirred for 2 h at 110°C under N₂ protection. After cooled to room temperature, the solvent was removed. The crude product was purified by silica gel flash column chromatography to afford the product **Example 37f** (1.04 g, 64% yield) as yellow oil. LCMS [M+1] ⁺ = 436.3.

### Step 4: Example 37g

To a solution of **Example 37f** (1.04 g, 2.4 mmol, 1.0 eq) in DCM (5 mL) was added TFA (5 mL) dropwise at 0°C. The reaction mixture was stirred for 2 h at r.t. The solution was concentrated in vacuum to give the crude product **Example 37g** (930 mg, crude) as yellow oil, which was used to next step directly without purification. LCMS [M+1]⁺ =236.4

### Step 5: Example 37i

To a solution of **Example 37h** (222 mg, 0.68 mmol, 0.8 eq) in DCM (10 mL) were added HATU (323 mg, 0.85 mmol, 1.0 eq) and DIEA (438.6 mg, 3.4 mmol, 4.0 eq). The mixture was stirred for 20 min, and then **Example 37g** (320 mg, 0.85 mmol, 1.0 eq) was added. The reaction mixture was stirred at r.t. for 2 h. The solution was concentrated in vacuum, and the crude product was purified by silica gel flash column chromatography to afford the product **Example 37i** (180 mg, 39% yield) as a yellow solid. LCMS [M+1]⁺ = 544.3.

### Step6: Example 37j

To a solution of **Example 37i** (100 mg, 0.18 mmol, 1.0 eq) in dioxane (2 mL) were added Cs₂CO₃ (117.4 mg, 0.36 mmol, 2.0 eq), Pd₂(dba)₃ (18.6 mg, 0.018 mmol, 0.1 eq) and BINAP (22.4 mg, 0.036 mmol, 0.2 eq). The reaction mixture was stirred for 3 h at 80°C under N₂ protection. The solid was filtered out and the filtrate was concentrated. The residue was purified by Prep-TLC to afford the **Example 37j** (80 mg, 88% yield) as a yellow solid. LCMS [M+1] ⁺ =508.3.

### Step 7: Example 37

To a solution of **Example 37j** (80 mg, 0.157 mmol, 1.0 eq) in DCM (2 mL) was added HCl/dioxane (2 mL, 4M in dioxane) dropwise at 0°C. The reaction mixture was stirred for 2 h at r.t. After completion, the reaction mixture was concentrated. The crude product was treated with MeOH (2 mL), NaHCO₃(excess) was added to the solution, the mixture was stirred for 20 min at r.t., then DCM (20 mL) was added. The solid was filtered out and filtrate was concentrated. The residue was purified by Prep-TLC to afford the **Example 37** (30.5 mg, 48% yield) as an off-white solid. LCMS [M+1] ⁺ = 408.2. ¹H NMR (300 MHz, DMSO-*d*₆) δ 9.55 (s, 1H), 8.21 (s, 1H), 8.14 (s, 1H), 8.09 (d, 1H), 7.99 (d, 1H), 7.74 (d, 1H), 5.78 (s, 1H), 4.76 (d, 1H), 4.58 (d, 1H), 4.53 (s, 3H), 3.86-3.84 (m, 1H), 3.44- 3.39 (m, 1H), 3.16-3.09 (m, 1H), 2.98 (d, 3H), 1.10 (d, 3H).

### Example 38:

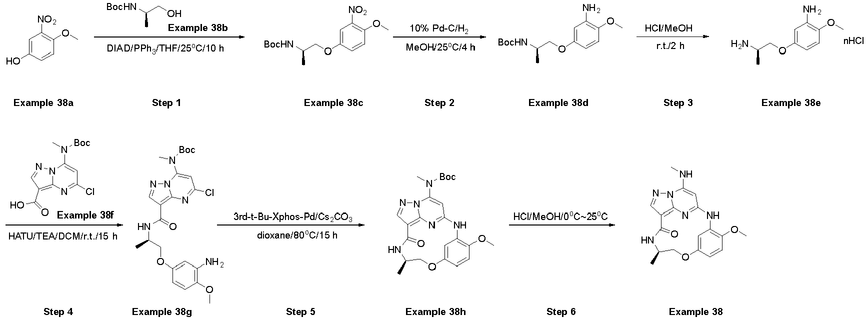

### Step 1: Example 38c

To a solution of **Example 38a** (5.08 g, 30.0 mmol), **Example 38b** (6.76 g, 38.6 mmol), PPh₃ (10.1 g, 38.5 mmol) in THF (100 mL) was added DIAD (8.30 g, 41.0 mmol). The reaction mixture was stirred at 25°C for 10 h. Then EtOAc (400 mL) was added to the reaction mixture, which was washed with brine (100 mL*2), dried over anhydrous Na₂SO₄ and concentrated. The residue was purified by silica gel column chromatography to give the desired product **Example 38c** (4.8 g, yield: 49%) as a yellow solid. LCMS [M+1]⁺ = 327.15

### Step 2: Example 38d

To a mixture of **Example 38c** (4.8 g, 14.7 mmol) in EtOH (79 mL) was added 10% Pd-C (500 mg). The mixture was stirred for 4 h at 25°C under H₂ atmosphere. The mixture was then concentrated under reduced pressure to afford crude **Example 38d** (4.44 g crude, yield: quant.) as a yellow solid. The residue was used in the next step without further purification. LCMS [M+1]⁺ = 297.18.

### Step 3: Example 38e

A solution of **Example 38d** (2.0 g, 6.76 mmol) in HCl-MeOH (26 mL, 3N) was stirred at r.t. for 2 h. The mixture was then concentrated under reduced pressure to afford crude **Example 38e** (1.6 g crude, yield: quant.) as a white solid. The residue was used at next step without further purification. LCMS [M+1]⁺ = 197.12.

### Step 4: Example 38g

To a solution of **Example 38e** (707 mg, 3.6 mmol), **Example 38f** (1.4 g, 4.3 mmol), TEA (935 mg, 9.3 mmol) in DCM (12 mL) was added HATU (2.25 g, 5.9 mmol), which was stirred at r.t. for 15 h. Then EtOAc (400 mL) was added to the reaction mixture, which was washed with brine (100 mL*2), dried over anhydrous Na₂SO₄ and concentrated. The residue was purified by silica gel column chromatography to give the desired product **Example 38g** (428 mg, yield:24% over 3 steps) as a white solid. LCMS [M+1]⁺ = 506.19.

### Step 5: Example 38h

To a solution of **Example 38g** (144 mg, 0.29 mmol), Cs₂CO₃ (291 mg, 0.89 mmol) in dioxane (3 mL) was added 3rd-t-Bu-Xphos-Pd (16 mg, 0.02mmol), which was stirred at 80°C for 15 h under N₂ atmosphere. Then EtOAc (400 mL) was added to the reaction mixture, which was washed with brine (100 mL*2), dried over anhydrous Na₂SO₄ and concentrated. The residue was purified by silica gel column chromatography to give the desired product **Example 38h** (24 mg, yield: 18%) as a white solid. LCMS [M+1]⁺ = 469.22

### Step 6: Example 38

A solution of **Example 38h** (24 mg, 0.05 mmol) in HCl-MeOH (2 mL, 3N) was stirred at 0°C in an ice bath and then warmed up to 25°C for 6 h. Then EtOAc (400 mL) was added to the reaction mixture, which was washed with brine (100 mL*2), dried over anhydrous Na₂SO₄ and concentrated. The residue was purified by silica gel column chromatography to give **Example 38** (13 mg, yield 63%) as a white solid. LCMS [M+1]⁺ = 369.16. ¹H NMR (400 MHz, Chloroform-d) δ 8.24 (s, 1H), 7.05 (s, 1H), 6.85 (d, 1H), 6.75 (dd, 1H), 5.39 (s, 1H), 4.46-4.29 (m, 3H), 3.86 (s, 3H), 3.08 (d, 3H), 1.35 (d, 3H).

### Reference Example 39:

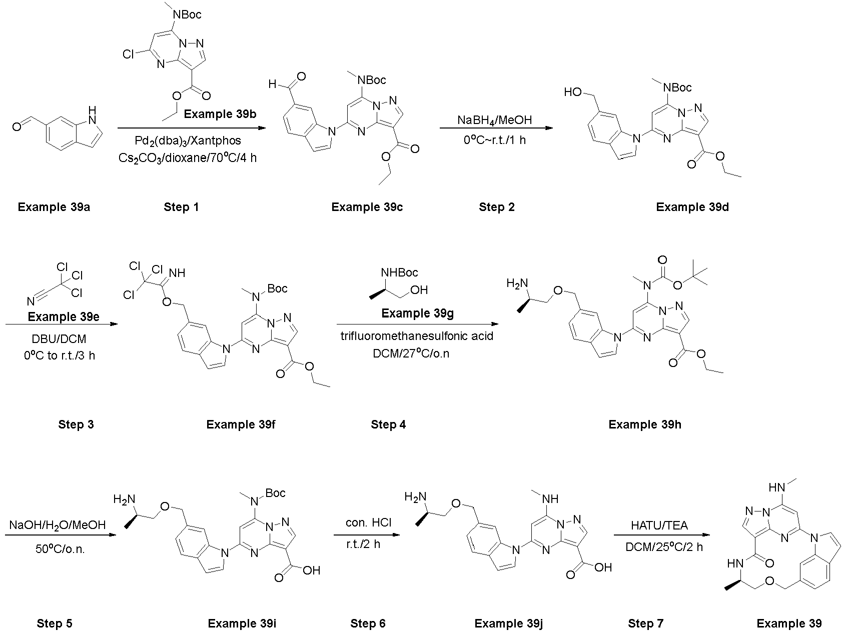

### Step 1: Example 39c

To a mixture of **Example 39a** (1.45 g, 10.0 mmol), **Example 39b (3.55** g, 10.0 mmol), Pd₂ (dba)₃ (558 mg, 0.5 mmol), Xantphos (298 mg, 0.5 mmol), Cs₂CO₃ (4.88 g, 15.0 mmol) in dioxane (30 mL) was degassed with N₂ three times, and stirred for 4 h at 70°C. Then the reaction mixture was cooled to r.t., diluted by DCM, washed by water, dried over anhydrous Na₂SO₄, and then concentrated under reduced pressure, which was purified by silica gel column chromatography to give **Example 39c** (3.0 g, yield: 70%) as a white solid. LCMS [M+1]⁺ = 464.1. ¹H NMR (400 MHz, Chloroform-*d*) δ 10.21 (s, 1H), 9.64 (s, 1H), 8.56 (s, 1H), 7.91-7.85 (m, 2H), 7.75 (d, 1H), 7.08 (s, 1H), 6.88 (d, 1H), 4.54 (q, 2H), 4.11 (q, 2H), 3.46 (s, 3H), 2.04 (s, 3H), 1.58 (s, 3H), 1.43 (s, 12H), 1.24 (d, 3H).

### Step 2: Example 39d

To a solution of **Example 39c** (3.1 g, 6.7 mmol) in MeOH (20 mL) was added NaBH₄ (254 mg, 6.7 mol) at 0°C. The reaction mixture was stirred at r.t. for 1 h, which was then quenched by water, diluted by DCM, washed by brine, and dried over anhydrous Na₂SO₄. The solution was concentrated under reduced pressure and purified by silica gel column chromatography to give **Example 39d** (2.8 g, yield: 90%) as a white solid. LCMS [M+1-17]⁺ = 448.2. ¹H NMR (400 MHz, Chloroform-*d*) δ 9.26 (s, 1H), 8.50 (s, 1H), 7.62 (t, 2H), 7.30 (d, 1H), 7.02 (s, 1H), 6.78 (s, 1H), 4.92 (s, 2H), 4.77 (s, 1H), 4.49 (q, 2H), 3.44 (s, 3H), 1.46 (d, 3H), 1.41 (s, 9H).

### Step 3: Example 39f

To a solution of **Example 39d** (2.70 g, 5.8 mmol), **Example 39e** (2.52 g, 17.5 mmol) in DCM (20 mL) was added DBU (2.64 g, 17.5 mmol) at 0°C. The reaction mixture was warmed to room temperature and stirred for 3 h. The mixture was concentrated under reduced pressure and purified by silica gel column chromatography to give **Example 39f** (2.3 g, yield: 65%) as a brown solid.

### Step 4: Example 39h

To a solution of **Example 39f** (2.30 g, 3.79 mmol), **Example 39g** (795 mg, 4.54 mmol) in DCM (20 mL) was added CF₃SO₃H (285 mg, 1.9 mmol) in DCM (20 mL) at 0°C. The reaction mixture was warmed to 27°C and stirred for overnight. The mixture was concentrated under reduced pressure and was purified by C-18 gel column chromatography to give **Example 39h** (200 mg, yield: 10%) as a brown solid. LCMS [M+1]⁺ = 523.2.

### Step 5: Example 39i

To a solution of **Example 39h** (200 mg, 0.38 mmol) in MeOH (5 mL) was added NaOH (156 mg, 3.9 mmol) in H₂O (5 mL), which was stirred at 50°C for overnight. The mixture was concentrated to give the crude product **Example 39i** (700 mg crude, yield 100%) as a white solid.

### Step 6: Example 39j

To a solution of **Example 39i** (700 mg crude, 0.38 mmol) in con. HCl (5 mL) was stirred at r.t. for 2 h. The residue was purified by reverse phase column to afford the desired product **Example 39j** (105 mg, two steps' yield: 75%) as a brown solid.
LCMS [M+1]⁺ = 395.2.

### Step 7: Example 39

To a solution of **Example 39i** (100 mg, 0. 254 mmol), TEA (51 mg, 0.51 mmol) in DCM (10 mL) was added HATU (144 mg, 0.38 mmol). The reaction mixture was stirred at 25°C for 2 h. Then DCM (20 mL) was added to the reaction mixture, which was washed with brine (20 mL*2), dried over anhydrous Na₂SO₄ and concentrated. The residue was purified by Prep-HPLC to afford the desired product **Example 39** (4.1 mg, yield 4%) as a white solid. LCMS [M+1]⁺ = 377.1. ¹H NMR (400 MHz, DMSO-*d*₆) δ 9.07 (s, 1H), 8.50 (d, 1H), 8.35 (s, 1H), 8.24 (dd, 2H), 7.60 (d, 1H), 7.03 (d, 1H), 6.80 (d, 1H), 6.61 (s, 1H), 4.85 (d, 1H), 4.60 (d, 1H), 4.04 (d, 1H), 3.63 (d, 1H), 3.54 (t, 1H), 3.30 (s, 1H), 3.09 (d, 3H), 1.15 (d, 3H).

### Example 40:

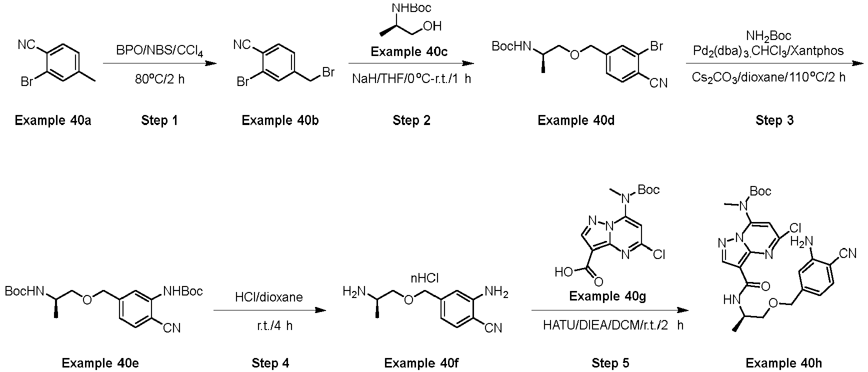

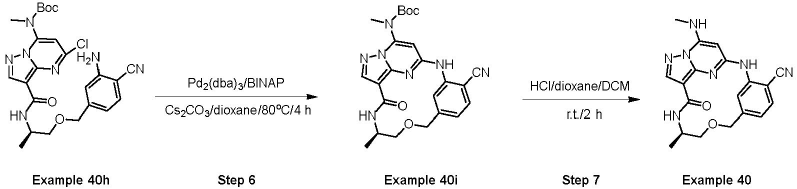

### Step 1: Example 40b

To a solution of **Example 40a** (10.0 g, 51.3 mmol, 1.0 eq) in CCl₄ (100 mL) was added NBS (10.04 g, 56.4 mmol, 1.1 eq) and BPO (1.24 g, 5.13 mmol, 0.1 eq) at room temperature. The mixture was stirred for 2 h at 80°C. After the reaction was completed, the mixture was cooled to room temperature. The suspension was diluted with EtOAc (150 mL), which was filtered through a pad of Celite and the filter cake was washed with EtOAc (150 mL). The filtrate was concentrated in vacuum, and the crude was purified by silica gel flash column chromatography to afford the product **Example 40b** (12.5 g, 88% yield) as a yellow solid. ¹H NMR (300 MHz, DMSO-*d*₆) δ 8.00 (d, 1H), 7.95 (d, 1H), 7.66 (dd, 1H), 4.75 (s, 2H).

### Step 2: Example 40d

A solution of **Example 40c** (3.53 g, 20.15 mmol, 1.1 eq) in THF (50 mL) was cooled to 0°C, and NaH (1.47 g, 60% in mineral oil, 36.64 mmol, 2.0 eq) was added in portions. The mixture was stirred for 30 min at 0°C, then **Example 40b** (5.0 g, 18.32 mmol, 1.0 eq) was added at 0°C, which was stirred for 1 h at room temperature. The mixture was quenched with NH₄Cl aqueous solution (100 mL) and extracted with EtOAc (100 mL*3). The combined organic layers were washed with brine, dried over Na₂SO₄ and concentrated vacuum. The crude product was purified by silica gel flash column chromatography to afford the product **Example 40d** (3.3 g, 50% yield) as an off-white solid. LCMS [M+1-100]⁺ = 269.1.

### Step 3: Example 40e

To a solution of **Example 40d** (2.0 g, 5.43 mmol, 1.0 eq) in dry dioxane (20 mL) were added Cs₂CO₃ (5.30 g, 16.3 mmol, 3.0 eq), NH₂-Boc (700 mg, 5.98 mmol, 1.1 eq), Xantphos (628 mg, 1.1 mmol, 0.2 eq) and Pd₂(dba)₃.CHCl₃ (560 mg, 0.543 mmol, 0.1 eq). The reaction mixture was stirred for 2 h at 110°C under N₂ protection. After the reaction was completed, the solvent was removed and the residue was purified by silica gel flash column chromatography to afford the product **Example 40e** (2.0 g, 91% yield) as a yellow solid. LCMS [M+23]⁺ = 428.3.

### Step 4: Example 40f

A solution of **Example 40e** (1.7 g, 4.2 mmol, 1.0 eq) in HCl/dioxane (4M, 20 mL) was stirred for 4 h at room temperature. After the reaction was completed, the mixture was concentrated in vacuum to afford the product **Example 40f** (860 mg, crude, 100% yield) as a yellow solid. LCMS [M+1]⁺ = 206.3.

### Step 5: Example 40h

To a solution of **Example 40g** (830 mg, 2.54 mmol, 0.8 eq) in DCM (8 mL) were added DIEA (1.65 g, 12.68 mmol, 4.0 eq) and HATU (964 mg, 2.54 mmol, 0.8 eq). The mixture was stirred for 20 min at room temperature. Then **Example 40f** (650 mg, 3.17 mmol, 1.0 eq) was added and the mixture was stirred for 2 h at room temperature. After the reaction was completed, it was concentrated in vacuum. The residue was purified by silica gel flash column chromatography to afford the product **Example 40h** (150 mg, 9% yield) as a yellow solid. LCMS [M+1]⁺ = 514.2.

### Step 6:Example 40i

To a solution of **Example 40h** (200 mg, 0.39 mmol, 1.0 eq) in dry dioxane (20 mL) were added Cs₂CO₃ (253.4 mg, 0.78 mmol, 2.0 eq), BINAP (48.5 mg, 0.078 mmol, 0.2 eq) and Pd₂(dba)₃ (40.4 mg, 0.039 mmol, 0.1 eq). The reaction mixture was stirred for 4 h at 80°C under N₂ protection. After the reaction was completed, the solvent was removed, and the residue was purified by Prep-TLC to afford the product **Example 40i** (110 mg, 59% yield) as an off-white solid. LCMS [M+1]⁺ = 478.3.

### Step 7: Example 40

To a solution of **Example 40i** (90 mg, 0.189 mmol, 1.0 eq) in DCM (2 mL) was added HCl/dioxane (1 mL, 4M in dioxane). The reaction was stirred at room temperature for 2 h. After the reaction was completed, the reaction was basified with NaHCO₃ (excess). The solid was filtered out, and the filtrate was concentrated. The crude product was purified by Prep-TLC to afford the product **Example 40** (52.0 mg, 73% yield) as an off-white solid. LCMS [M+1]⁺ = 378.1. ¹H NMR (300 MHz, DMSO-*d*₆) δ 9.78 (s, 1H), 8.60 (s, 1H), 8.18 (s, 1H), 8.11 (d, 1H), 8.06 (d, 1H), 7.76 (d, 1H), 7.12 (dd, 1H), 5.97 (s, 1H), 4.70 (d, 1H), 4.53 (d, 1H), 3.94-3.83 (m, 1H), 3.53 (dd, 1H), 3.28-3.20 (m, 1H), 2.96 (d, 3H), 1.15 (d, 3H).

### Example 41:

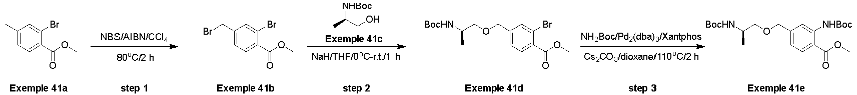

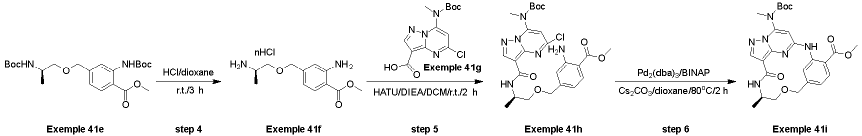

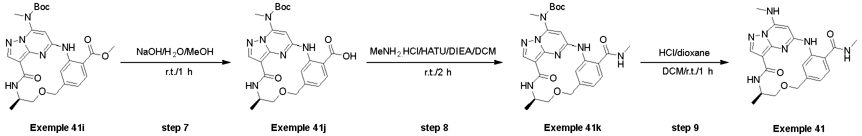

### Step 1: Example 41b

To a solution of **Example 41a** (10.0 g, 43.7 mmol, 1.0 eq) in CCl₄ (100 mL) were added NBS (11.66 g, 65.5 mmol, 1.5 eq) and AIBN (1.4 g, 8.7 mmol, 0.2 eq) at room temperature. The mixture was stirred for 2 h at 80°C. After the reaction was completed, the solid was filtered, and the filtrate was concentrated. The residue was purified by silica gel flash column chromatography to afford the product **Example 41b** (11.8 g, 88% yield) as a yellow solid.

### Step 2: Example 41d

To a solution of **Example 41c** (3.13 g, 17.86 mmol, 1.1 eq) in THF (50 mL) was added NaH (1.30 g, 32.47 mmol, 2.0 eq) in portions at 0°C. After stirring for 20 min, **Example 41b** (5.0 g, 16.23 mmol, 1.0 eq) was added at 0°C. The mixture was stirred for 1 h at room temperature under N₂. The mixture was quenched with NH₄Cl aqueous solution (100 mL), which was extracted with EtOAc (200 mL*3). The combined organic layers were washed brine, dried over Na₂SO₄ and concentrated. The crude product was purified by silica gel flash column chromatography to afford the product **Example 41d** (1.4 g, 22% yield) as an off-white solid. LCMS [M+1-100]⁺ = 302.1.

### Step 3: Example 41e

To a solution of **Example 41d** (1.4 g, 3.5 mmol, 1.0 eq) in dioxane (20 mL) were added Cs₂CO₃ (3.4 g, 10.5 mmol, 3.0 eq), NH₂-Boc (450 mg, 3.84 mmol, 1.1 eq), Xantphos (404 mg, 0.7 mmol, 0.2 eq) and Pd₂(dba)₃ (362 mg, 0.35 mmol, 0.1 eq). The reaction mixture was degassed with nitrogen for 3 times and stirred at 110°C for 2 h. The reaction was cooled to room temperature and concentrated in vacuum. The crude product was purified by silica gel flash column chromatography to afford the product **Example 41e** (1.42 g, 93% yield) as a yellow solid. LCMS [M+1+22]⁺ = 461.2

### Step 4: Example 41f

A solution of **Example 41e** (1.42 g, 3.24 mmol, 1.0 eq) in HCl/dioxane (4M, 20 mL) was stirred for 3 h at room temperature. After the reaction was completed, the mixture was concentrated in vacuum to afford the product **Example 41f** (800 mg, crude, 100% yield) as a yellow solid. LCMS [M+1]⁺ = 239.2.

### Step 5: Example 41h

To a solution of **Example 41g** (789 mg, 2.42 mmol, 0.8 eq) in DCM (20 mL) were added DIEA (1.57 g, 12.1 mmol, 4.0 eq) and HATU (1.72 g, 4.51 mmol, 1.5 eq). After stirring for 15 min, **Example 41f** (720 mg, 3.03 mmol, 1.0 eq) was added. The reaction mixture was stirred for 2 h at room temperature. After the reaction was completed, it was concentrated in vacuum. The residue was purified by silica gel flash column chromatography to afford the product **Example 41h** (520 mg, 39%yield) as a yellow solid. LCMS [M+1]⁺ = 547.3.

### Step 6:Example 41i

To a solution of **Example 41h** (440 mg, 0.81 mmol, 1.0 eq) in dioxane (20 mL) were added Cs₂CO₃ (523 mg, 1.61 mmol, 2.0 eq), BINAP (100 mg, 0.16 mmol, 0.2 eq) and Pd₂(dba)₃ (83.3 mg, 0.08 mmol, 0.1 eq). The reaction mixture was stirred for 2 h at 80°C under N₂ protection. The reaction mixture was cooled to room temperature, filtered and concentrated in vacuum. The crude product was purified by Prep-TLC to afford the product **Example 41i** (220 mg, 54% yield) as an off-white solid. LCMS [M+1]⁺ = 511.2.

### Step 7: Example 41j

To a solution of **Example 41i** (200 mg, 0.39 mmol, 1.0 eq) in MeOH (4 mL) was added 2 M NaOH aqueous solution (0.4 mL). The reaction mixture was stirred for 1 h at room temperature. After the reaction was completed, the mixture was acidified with HCl aqueous solution (1M), which was then extracted with EtOAc (20 mL*3). The combined organic layers were washed with brine and concentrated in vacuum to afford the product **Example 41j** (190 mg, 98%yield) as an off-white solid. LCMS [M+1]⁺ = 497.2.

### Step 8: Example 41j

To a solution of **Example 41j** (170 mg, 0.34 mmol, 1.0 eq) in DCM (8 mL) were added DIEA (178 mg, 1.37 mmol, 4.0 eq) and HATU (195 mg, 0.51 mmol, 1.5 eq). After stirring for 15 min, methylamine hydrochloride (46 mg, 0.69 mmol, 2.0 eq) was added to the reaction mixture, which was stirred for 2 h at room temperature. After the reaction was completed, it was concentrated in vacuum. The residue was purified by Prep-TLC to afford the product **Example 41k** (170 mg, 97% yield) as an off-white solid. LCMS [M+1]⁺ = 510.2.

### Step 9: Example 41

To a solution of **Example 41k** (100 mg, 0.196 mmol, 1.0 eq) in DCM (2 mL) was added HCl/dioxane (2 mL, 4M in dioxane) at room temperature. The reaction was stirred at room temperature for 1 h. After the reaction was completed, the reaction was basified with NaHCO₃ (excess). The solid was filtered out, and the filtrate was concentrated. The crude product was purified by Prep-TLC to afford the desired product **Example 41** (46.7 mg, 58% yield) as a white solid. LCMS [M+1]⁺ = 410.2 ¹H NMR (300 MHz, DMSO-*d*₆) δ 10.95 (s, 1H), 8.81 (d, 1H), 8.69 (d, 1H), 8.30 (d, 1H), 8.17 (s, 1H), 7.95 (d, 1H), 7.69 (d, 1H), 7.00-6.90 (m, 1H), 5.74 (s, 1H), 4.65 (d, 1H), 4.54 (d, 1H), 4.00-3.88 (m,1H), 3.61-3.54 (m, 1H), 3.44-3.37 (m, 1H), 2.97 (d, 3H), 2.83 (d, 3H), 1.18 (d, 3H).

### Example 42:

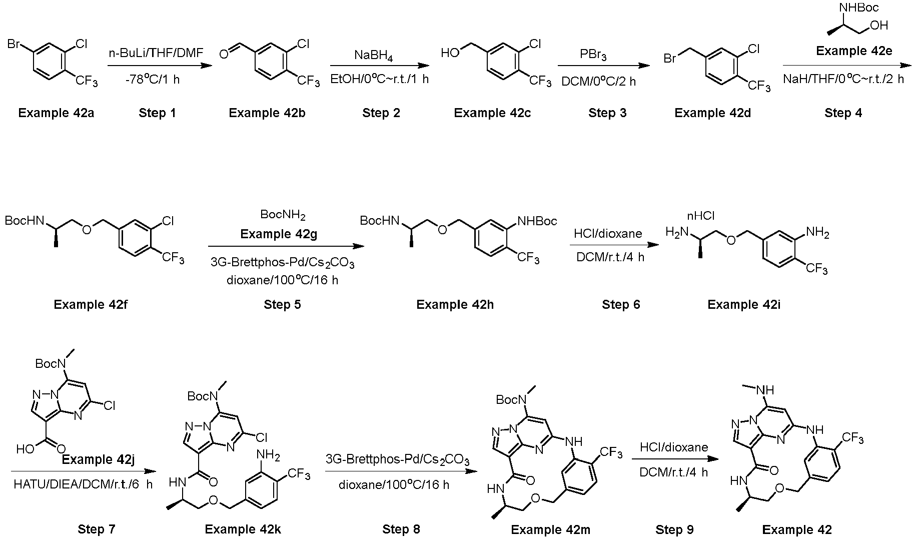

### Step 1: Example 42b

To a solution of **Example 42a** (10.0 g, 38.7 mmol, 1.0 eq) in dry THF (193 mL) was added n-BuLi (17 mL, 2.5 M in hexane, 42.6 mmol, 1.1 eq) dropwise at -78°C over a period of 15 min, followed by stirring for 20 min. DMF (28.3 g, 387 mmol, 10.0 eq) was added dropwise to the mixture at -78°C and the resulting mixture was stirred at -78°C for another 1 h under N₂. The reaction mixture was quenched with 1N NH₄Cl aqueous solution (100 mL), which was stirred for 30 min at 0°C. The reaction mixture was then extracted with EtOAc (100 mL*3). The combined organic layers were washed with brine, dried over Na₂SO₄, and concentrated. The residue was purified by silica column chromatography (Petroleum Ether) to afford the desired product **Example 42b** (6.1 g, 71% yield) as yellow oil.
¹H NMR (300 MHz, CDCl₃-*d*) δ 10.06 (s, 1H), 8.01 (s, 1H), 7.91-7.85 (m, 2H).

### Step 2: Example 42c

To a solution of **Example 42b** (4.9 g, 23.6 mmol, 1.0 eq) in ethanol (100 mL) was added NaBH₄ (985 mg, 25.9 mmol, 1.1 eq) in portions at 0°C. The reaction was stirred for 1 h at room temperature. The reaction mixture was quenched with 1N NH₄Cl aqueous solution (100 mL) and stirred for 30 min at 0°C. The reaction mixture was extracted with EtOAc (100 mL*3). The combined organic layers were washed with brine, dried over Na₂SO₄ and concentrated. The residue was purified by silica column chromatography (Petroleum Ether) to afford the desired product **Example 42c** (3.7 g, 75% yield) as a yellow oil.

### Step 3:Example 42d

To a solution of **Example 42c** (3.7 g, 17.6 mmol, 1.0 eq) in DCM (50 mL) was added PBr₃ (5.2 g, 19.4 mmol, 1.1 eq) at 0°C. The reaction was stirred for 2 h at 0°C. The reaction mixture was diluted with H₂O (40 mL), and extracted with EtOAc (40 mL*3). The combined organic layers were washed with NaHCO₃ aqueous solution, dried over Na₂SO₄ and concentrated. The residue was purified by silica column chromatography (Petroleum ether) to afford the desired product **Example 42d** (2.1 g, 44% yield) as yellow oil.

### Step 4: Example 42f

Toa solution of **Example 42e** (1.5 g, 8.49 mmol, 1.1 eq) in THF (50 mL) was added NaH (864 mg, 60% in mineral oil, 21.6 mmol, 2.8 eq) in portions at 0°C. After stirring for 15 min, **Example 42d** (2.1 g, 7.72 mmol, 1.0 eq) was added at 0°C. The mixture was stirred for 2 h at room temperature. The reaction was quenched with H₂O (50 mL) and extracted with EtOAc (50 mL*3). The combined organic layers were washed with brine, dried over Na₂SO₄ and concentrated. The residue was purified by silica gel chromatography to afford the desired product **Example 42f** (850 mg, 27% yield) as a white solid. LCMS [M+1-100]⁺ = 268.0.

### Step 5: Example 42h

To a solution of **Example 42f** (854 mg, 2.3 mmol, 1.0 eq) and **Example 42g** (523 mg, 3.0 mmol, 1.3 eq) in dioxane (20 mL) were added 3G-Brettphos-Pd (211 mg, 0.23 mmol, 0.1 eq) and Cs₂CO₃ (1.13 g, 3.54 mmol, 1.5 eq). The mixture reaction was degassed with nitrogen for 3 times and stirred at 100°C for 16 h. The reaction was cooled to room temperature and concentrated. The residue was purified by silica gel chromatography to afford the desired product **Example 42h** (720 mg, 70% yield) as a yellow solid. LCMS [M+1-100]⁺ = 349.2.

### Step 6: Example 42i

To a solution of **Example 42h** (720 mg, 1.6 mmol, 1.0 eq) in DCM (10 mL) was added HCl/dioxane (8 mL, 4M in dioxane, 32 mmol, 20.0 eq). The mixture was stirred at room temperature for 4 h. After the reaction was completed, the solvent was concentrated to give **Example 42i** (700 mg, crude) as a yellow solid. The crude was used next step directly without further purification. LCMS [M+1]⁺ = 249.2.

### Step 7: Example 42k

To a solution of **Example 42j** (791 mg, 2.42 mmol, 1.0 eq) in DCM (15 mL) were added DIEA (780 mg, 6.05 mmol, 2.5 eq) and HATU (1.01 g, 2.66 mmol, 1.1 eq). After stirring for 15 min, **Example 42i** (600 mg, 2.42 mmol, 1.0 eq) was added to the mixture. The reaction solution was stirred for 6 h at room temperature. After the reaction was completed, the solvent was removed and the crude was purified by silica gel chromatography to afford the desired product **Example 42k** (421 mg, 31% yield) as a yellow solid. LCMS [M+1]⁺ = 557.3.

### Step 8: Example 42m

To a solution of **Example 42k** (200 mg, 0.36 mmol, 1.0 eq) in dioxane (5 mL) were added Cs₂CO₃ (234 mg, 0.72 mmol, 2.0 eq), 3G-Brettphos-Pd (33.0 mg, 0.036 mmol, 0.1 eq). The reaction mixture was stirred for 16 h at 100°C under N₂. The reaction mixture was cooled to room temperature, filtered and concentrated in vacuum. The crude product was purified by prep-TLC to afford the desired product **Example 42m** (110 mg, 59% yield) as light yellow oil. LCMS [M+1]⁺ = 521.1.

### Step 9:Example 42

To a solution of **Example 42m** (100 mg, 3.1 mmol) in DCM (2 mL) was added HCl/dioxane (1 mL, 4M in dioxane) at room temperature. The reaction was stirred at room temperature for 4 h. After completed, the reaction was basified with NaHCO₃. The solid was filtered out, and the filtrate was concentrated. The crude product was purified by Prep-TLC (EtOAc) to afford the desired product **Example 42** (42.2 mg, 53% yield) as an off-white solid. LCMS [M+1]⁺ = 421.1. ¹H NMR (300 MHz, DMSO-*d*₆) δ 8.81 (s, 1H), 8.42 (s, 1H), 8.15 (s, 1H), 8.00-7.96 (m, 2H), 7.69 (d, 1H), 7.22 (d, 1H), 6.08 (s, 1H), 4.67 (d, 1H), 4.49 (d, 1H), 3.86-3.84 (m, 1H), 3.49-3.45 (m, 1H), 3.21-3.15 (m, 1H), 2.95 (d, 3H), 1.14 (d, 3H).

### Example 43:

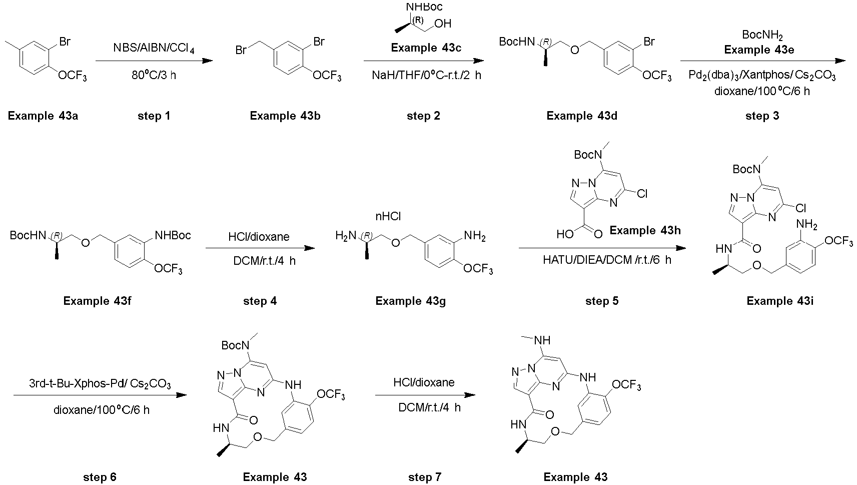

### Step 1: Example 43b

To a solution of **Example 43a** (2.0 g, 7.84 mmol, 1.0 eq) in CCl₄ (40 mL) were added NBS (1.54 g, 8.63 mmol, 1.10 eq), AIBN (129 mg, 0.78 mmol, 0.1 eq). The reaction was stirred for 3 h at 80°C under N₂. The mixture was cooled to room temperature. The suspension was diluted with EtOAc (50 mL), and then filtered through a pad of Celite. The filter cake was washed with EtOAc (50 mL). The filtrate was concentrated in vacuum, and the crude product was purified by silica column chromatography (Petroleum Ether) to afford the desired product **Example 43b** (2.2 g, 84% yield) as yellow oil. ¹H NMR (300 MHz, CDCl₃-*d*) δ 7.86 (d, 1H), 7.68 (s, 1H), 7.57 (d, 1H), 4.42 (s, 2H).

### Step 2: Example 43d

To a solution of **Example 43c** (1.3 g, 7.29 mmol, 1.1 eq) in THF (50 mL) was added NaH (742 mg, 60% in mineral oil, 18.6 mmol, 2.8 eq) in portions at 0°C. After stirring for 15 min, Example 43b (2.2 g, 6.62 mmol, 1.0 eq) was added to the mixture at 0°C, which was stirred for 2 h at room temperature under N₂. The reaction was quenched with H₂O (50 mL) and extracted with EtOAc (50 mL*3). The combined organic layers were washed with brine, dried over Na₂SO₄ and concentrated in vacuum. The crude product was purified by silica gel chromatography to afford the desired product **Example 43d** (1.3 g, 41% yield) as yellow oil. LCMS [M+1-100]⁺ = 328.0.

### Step 3: Example 43f

To a solution of **Example 43d** (800 mg, 1.87 mmol, 1.0 eq) and **Example 43e** (328 mg, 2.8 mmol, 1.5 eq) in dioxane (10 mL) were added Pd₂(dba)₃ (171 mg, 0.187 mmol, 0.1 eq), Xantphos (324 mg, 0.561 mmol, 0.3 eq) and Cs₂CO₃ (1.22 g, 3.74 mmol, 2.0 eq). The reaction mixture was degassed with nitrogen for 3 times and stirred at 100°C for 6 h. The reaction was cooled to room temperature and concentrated in vacuum. The crude product was purified by silica gel chromatography to afford the desired crude product **Example 43f** (612 mg, 70% yield) as a yellow solid. LCMS [M+1-100]⁺ = 365.2.

### Step 4: Example 43g

To a solution of **Example 43f** (612 mg, 1.31 mmol, 1.0 eq) in DCM (5 mL) was added HCl/dioxane (7 mL, 4M in dioxane, 26.2 mmol, 20.0 eq). The mixture was stirred at room temperature for 4 h. After the reaction was completed, the solvent was concentrated to give **Example 43g** (520 mg, crude) as a white solid. LCMS [M+1]⁺ = 265.2.

### Step 5: Example 43i

To a solution of **Example 43h** (572 mg, 1.75 mmol, 1.1 eq) in DCM (20 mL) were added DIEA (513 mg, 3.98 mmol, 2.5 eq) and HATU (725 mg, 1.91 mmol, 1.2 eq). After stirring for 15 min, **Example 43g** (420 mg, 1.59 mmol, 1.0 eq) was added to the mixture, which was stirred for 6 h at room temperature. After the reaction was completed, the solvent was removed and the crude product was purified by Prep-TLC to afford the desired product **Example 43i** (198 mg, 22% yield) as a yellow solid. LCMS [M+1]⁺ = 573.1.

### Step 6: Example 43j

To a solution of **Example 43i** (100 mg, 0.175 mmol, 1.0 eq) in dioxane (10 mL) were added Cs₂CO₃ (114 mg, 0.35 mmol, 2.0 eq) and 3rd-t-Bu-Xphos-Pd (27 mg, 0.035 mmol, 0.1 eq). The reaction mixture was stirred for 6 h at 100°C under N₂. The reaction mixture was cooled to room temperature, filtered and concentrated in vacuum. The crude product was purified by Prep-TLC to afford the desired product **Example 43j** (53 mg, 56% yield) as a light yellow solid. LCMS [M+1]⁺ = 537.4.

### Step 7:Example 43

To a solution of **Example 43j** (78 mg, 0.145 mmol, 1.0 eq) in DCM (1 mL) was added HCl/dioxane (0.7 mL, 4M in dioxane, 2.91 mmol, 20.0 eq) at room temperature. The reaction was stirred at room temperature for 4 h. After the reaction was completed, the reaction was basified with NaHCO₃ (excess). The solid was filtered out, and the filtrate was concentrated. The crude product was purified by prep-TLC (EtOAc) to afford the desired product **Example 43** (33.2 mg, 52% yield) as an off-white solid. LCMS [M+1]⁺ = 437.1. ¹H NMR (300 MHz, DMSO-*d*₆) δ 9.32 (s, 1H), 8.64 (d, 1H), 8.17 (s, 1H), 8.15 (d, 1H), 7.97 (d, 1H), 7.37 (dd, 1H), 7.03 (dd, 1H), 5.99 (s, 1H), 4.62 (d, 1H), 4.49 (d, 1H), 3.92-3.90 (m, 1H), 3.52 (dd, 1H), 3.35-3.30 (m, 1H), 2.95 (d, 1H), 1.16 (d, 1H).

### Example 44:

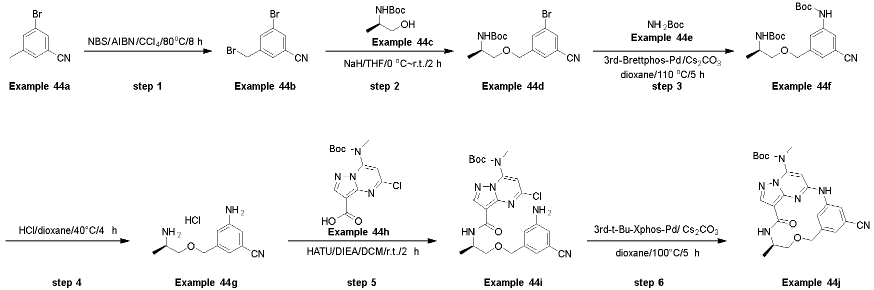

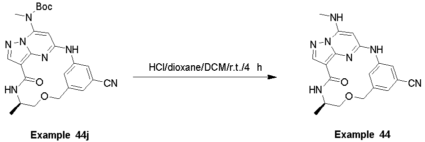

### Step 1: Example 44b

To a solution of **Example 44a** (11.0 g, 56.1 mmol, 1.0 eq) in CCl₄ (500 mL) were added NBS (15.0 g, 84.2 mmol, 1.5 eq) and AIBN (4.6 g, 28.1 mmol, 0.5 eq). The reaction mixture was stirred at 80°C for 8 h. After the insoluble solid was removed, the filtrate was concentrated, and the residue was purified by silica gel flash column chromatography to afford the product **Example 44b** (8.1 g, 53% yield) as a yellow solid. LCMS[M+1] ⁺ = 275.9.

### Step 2: Example 44d

To a solution of **Example 44c** (2.9 g, 16.4 mmol, 1.5 eq) in THF (40 mL) was added NaH (567 mg, 60% in mineral oil, 14.2 mmol, 1.3 eq) in portions at 0°C. After stirring for 10 min, a solution of **Example 44b** (3.0 g, 10.9 mmol, 1.0 eq) in THF (10 mL) was added dropwise. The reaction mixture was stirred for 2 h at 0°C~r.t. and then the solvent was concentrated in *vacuo.* The crude product was purified by silica gel flash column chromatography to afford the desired product **Example 44d** (1.9 g, 47% yield) as a yellow solid. LCMS [M+1]⁺ = 369.3.

### Step 3: Example 44f

To a solution of **Example 44d** (1.8 g, 4.9 mmol, 1.0 eq) in dioxane (50 mL) were added Cs₂CO₃ (3.2 g, 9.8 mmol, 2.0 eq) and 3rd-Brettphos-Pd (442 mg, 0.5 mmol, 0.1 eq). The reaction mixture was stirred at 110°C for 5 h under N₂. After cooled to room temperature, the solvent was removed, and the residue was purified by silica gel flash column chromatography to afford the product **Example 44f** (1.4 g, 71% yield) as yellow oil. LCMS [M+1]⁺ = 406.2.

### Step 4: Example 44g

A solution of **Example 44f** (700 mg, 1.7 mmol, 1.0 eq) in HCl/dioxane (15 mL, 4M in dioxane) was stirred for 4 h at 40°C. The reaction mixture was concentrated in vacuo to afford the desired product **Example 44g** (640 mg, crude) as a white solid. LCMS [M+1]⁺ = 206.2.

### Step 5: Example 44i

To a solution of **Example 44h** (465 mg, 1.4 mmol, 1.0 eq) in DCM (20 mL) were added DIEA (1.8 g, 14.2 mmol, 10.0 eq), HATU (649 mg, 1.7 mmol, 1.2 eq) and **Example 44g** (620 mg, 2.6 mmol, 1.8 eq). The reaction mixture was stirred for 2 h at r.t. The solvent was removed, and the residue was purified by silica gel flash column chromatography to afford the desired product **Example 44i** (185 mg, 25% yield) as yellow oil. LCMS [M+1]⁺ = 514.3.

### Step 6: Example 44j

To a solution of **Example 44i** (180 mg, 0.35 mmol, 1.0 eq) in dioxane (50 mL) were added Cs₂CO₃ (228 mg, 0.7 mmol, 2.0 eq) and 3rd-t-Bu-Xphos-Pd (93 mg, 0.11 mmol, 0.3 eq). The reaction mixture was stirred at 100°C for 5 h under N₂. After cooled to room temperature, the solvent was removed, and the residue was purified by silica gel flash column chromatography to afford the product **Example 44j** (95 mg, 57% yield) as a yellow solid. LCMS [M+1] ⁺ = 478.2.

### Step 7: Example 44

To a solution of **Example 44j** (90 mg, 0.19 mmol, 1.0 eq) in DCM (2 mL) was added HCl/dioxane (1 mL, 4M in dioxane). The reaction mixture was stirred at r.t. for 4 h and then concentrated in vacuum. The residue was dissolved in MeOH (5 mL), and basified with NaHCO₃. After concentration, the residue was purified by prep-TLC to afford the desired product Example 44 (41.5 mg, 58% yield) as an off-white solid. LCMS [M+1] ⁺ = 378.2. ¹H NMR (300 MHz, DMSO-*d*₆) δ 9.96 (s, 1H), 8.90 (s, 1H), 8.19-8.18 (m, 2H), 8.03 (d, 1H), 7.36 (s, 1H), 7.30 (s, 1H), 5.52 (s, 1H), 4.65 (d, 1H), 4.55 (d, 1H), 3.95-3.93 (m, 1H), 3.59 - 3.55 (m, 1H), 3.42- 3.37 (m, 1H), 2.95 (d, 3H), 1.18 (d, 3H).

### Example 45:

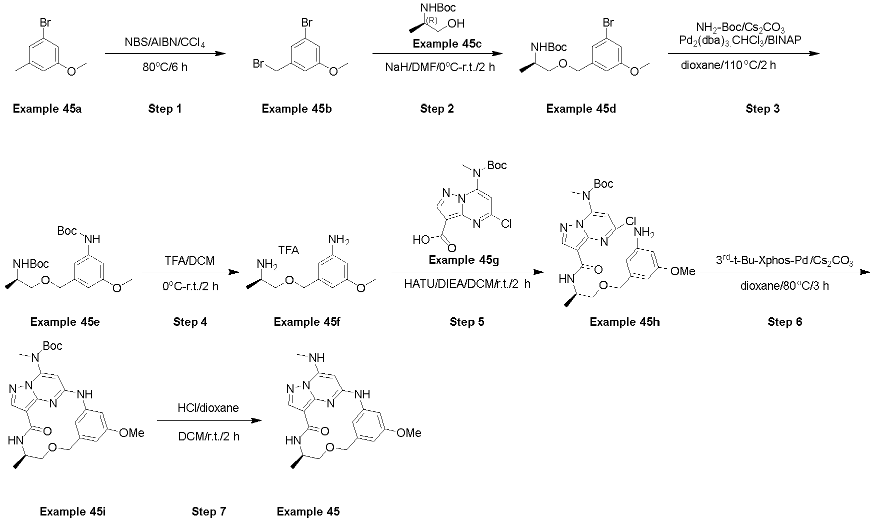

### Step1: Example 45b

To a solution of **Example 45a** (5.0 g, 24.9 mmol, 1.0 eq) in CCl₄ (50 mL) were added NBS (4.9 g, 27.4 mmol, 1.1 eq) and AIBN (410 mg, 2.5 mmol, 0.1 eq). The reaction mixture was stirred at 80°C for 6 h. After cooled to room temperature, the solvent was removed, and the residue was purified by silica gel flash column chromatography (Petroleum Ether) to afford the product **Example 45b** (4.5 g, 60% yield) as a white solid. LCMS [M+1]⁺ = 281.2.

### Step 2: Example 45d

To a solution of **Example 45c** (1.9 g, 10.7 mmol, 1.5 eq) in DMF (20 mL) was added NaH (340 mg, 60% in mineral oil, 8.5 mmol, 1.2 eq) in portions at 0°C. The mixture was stirred for 30 min at the same temperature, and then **Example 45b** (2.0 g,7.1 mmol, 1.0 eq) in DMF (20 mL) was added dropwise. The reaction mixture was stirred at r.t. for 2 h. The mixture was poured into saturated aqueous solution of NH₄Cl (50 mL), which was then extracted with EtOAc (70 mL*3). The combined organic layers were washed with brine, dried over Na₂SO₄, and concentrated. The crude product was purified by silica gel flash column chromatography to afford the product **Example 45d** (1.3 g, 50% yield) as a white solid. LCMS [M+1]⁺ = 374.3.

### Step 3: Example 45e

To a solution of **Example 45d** (1.3 g, 3.5 mmol, 1.0 eq) in dioxane (15 mL) were added Cs₂CO₃ (2.3 g, 7.0 mmol, 2.0 eq), NH₂-Boc (1.2 g, 10.5 mmol, 3.0 eq), BINAP (436.1 mg, 0.7 mmol, 0.2 eq) and Pd₂(dba)₃.CHCl₃ (362.3 mg, 0.35 mmol, 0.1 eq). The reaction mixture was stirred for 2 h at 110°C under N₂ protection. After cooled to room temperature, the solvent was removed. The crude product was purified by silica gel flash column chromatography to afford the product **Example 45e** (980 mg, 68% yield) as a yellow solid. LCMS [M+1]⁺ = 411.3.

### Step 4: Example 45f

To a solution of **Example 45e** (980 mg, 2.4 mmol, 1.0 eq) in DCM (5 mL) was added TFA (2.5 mL) dropwise at 0°C. The reaction mixture was stirred for 2 h at r.t. The solution was concentrated in vacuum to give the crude product **Example 45f** (1.6 g, crude, quant) as yellow oil, which was used to next step directly without purification. LCMS [M+1]⁺ = 211.3.

### Step 5: Example 45h

To a solution of **Example 45g** (392 mg, 1.2 mmol, 0.5 eq) in DCM (8 mL) were added HATU (1.0 g, 2.8 mmol, 1.2 eq) and DIEA (1.2 g, 9.2 mmol, 4.0 eq). The mixture was stirred for 20 min, then **Example 45f** (700 mg, 2.3 mmol, 1.0 eq) was added. The reaction mixture was stirred at r.t. for 2 h. The solution was concentrated in vacuum, and the crude product was purified by silica gel flash column chromatography to afford the product **Example 45h** (280 mg, 23% yield) as a yellow solid. LCMS [M+1]⁺ = 519.4.

### Step 6: Example45i

To a solution of **Example 45h** (260 mg, 0.5 mmol, 1.0 eq) in dioxane (3 mL) were added Cs₂CO₃ (326 mg,1.0 mmol, 2.0 eq) and 3^{rd}*t*-Bu-Xphos-Pd (44.1 mg, 0.05 mmol, 0.1 eq). The reaction mixture was stirred for 3 h at 80°C under N₂ protection. The solid was filtered out and filtrate was concentrated. The residue was purified by prep-TLC to afford the **Example45i** (120 mg, 50% yield) as a yellow solid. LCMS [M+1]⁺ = 483.3.

### Step 7: Example 45

To a solution of **Example 45i** (100 mg, 0.20 mmol, 1.0 eq) in DCM (3 mL) was added HCl/dioxane (3 mL, 4M in dioxane) dropwise at 0°C. The reaction mixture was stirred for 2 h at r.t. After completion, the reaction mixture was concentrated. The crude product was dissolved in MeOH (2 mL), and then NaHCO₃ (excess) was added to the mixture, which was stirred for 20 min at r.t. After DCM (20 mL) was added to the mixture, the solid was filtered out and the filtrate was concentrated. The residue was purified by Prep-TLC to afford the **Example 45** (42.8 mg, 54% yield) as an off-white solid. LCMS [M+1] ⁺ = 383.2. ¹H NMR (300 MHz, DMSO-*d*₆) δ 9.62 (s, 1H), 8.39 (d, 1H), 8.16-8.13 (m, 2H), 7.89 (d, 1H), 6.49 (s, 2H), 5.48(s, 1H), 4.60 (d, 1H), 4.49 (d, 1H), 3.92-3.89 (m, 1H), 3.76 (s, 3H), 3.54-3.50 (m,1H), 3.42-3.36 (m, 1H), 2.93 (d, 3H), 1.18 (d, 3H).

### Example 46:

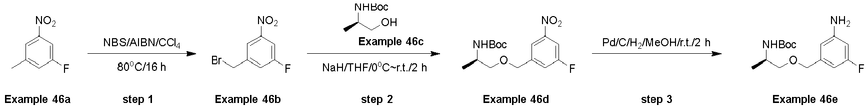

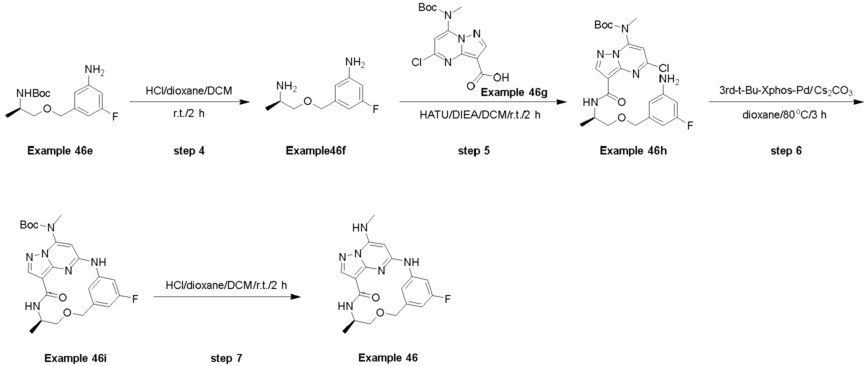

### Step 1: Example 46b

To a solution of **Example 46a** (5.0 g, 32.3 mmol, 1.0 eq) in CCl₄ (30 mL) was added BPO (2.3 g, 9.7l mmol, 0.3 eq) at 80°C. After stirring for 5 min, NBS (6.9 g, 38.76 mmol, 1.2 eq) was added, which was stirred for 16 h at 80°C. After the reaction was completed, EtOAc (150 mL) was added to the suspension, which was washed with saturated NaHCO₃ aqueous (100 mL*3). The organic layer was dried over Na₂SO₄ and concentrated to afford the crude product Example 46b (4.2 g, 56% yield) as yellow oil. LCMS [M+1]⁺ = 234.1

### Step 2: Example 46d

To a solution of **Example 46c** (2.7 g, 15.5 mmol, 1.2 eq) in THF (40 mL) was added NaH (770 mg, 60% in mineral oil, 19.4 mmol, 1.5 eq) in portions at 0°C. After stirring for 10 min, a solution of **Example 46b** (3.0 g, 12.9 mmol, 1.0 eq) in THF (5 mL) was added dropwise. The reaction mixture was stirred for 2 h at r.t. The reaction was quenched with saturated NH₄Cl aqueous (50 mL) at 0°C and extracted with EtOAc (100 mL*3). The combined organic layers were washed with brine (100 mL*2) , dried over Na₂SO₄ and concentrated in *vacuo.* The crude product was purified by silica gel flash column chromatography to afford the desired product **Example 46d** (3.1 g, 73% yield) as yellow oil. LCMS [M+1]⁺ = 329.3.

### Step 3: Example 46e

To a solution of **Example 46d** (2.5 g, 7.62 mmol, 3.0 eq) in MeOH (30 mL) was added 10% Pd/C (1.0 g) in portions under N₂ protection. The mixture was degassed with H₂ three times , which was stirred for 2 h at r.t. under H₂ balloon. The solid was filtered out, and the filtrate was concentrated in *vacuo* to give the desired product **Example 46e** (2.8 g, quant) as gray oil. LCMS [M+1]⁺ = 299.3.

### Step 4: Example 46f

To a solution of **Example 46e** (1.5 g, 5.03 mmol, 1.0 eq) in DCM (25 mL) was added HCl/dioxane (5 mL, 4 M in dioxane) at 0°C. The reaction mixture was stirred for 2 h at r.t. The reaction solution was concentrated in vacuo. The crude product was dissolved in MeOH, and Na₂CO₃ (excess) was added, which was stirred for 10 min at r.t. The solid was filtered out, and the filtrate was concentrated. The crude was purified by silica gel flash column chromatography to afford the desired product **Example 46f** (860 mg, 87% yield) as yellow oil. LCMS [M+1]⁺ = 199.2.

### Step 5: Example 46h

To a solution of **Example 46g** (200 mg, 1.01 mmol, 1.0 eq) and DIEA (521 mg, 4.04 mmol, 4.0 eq) in DCM (5 mL) were added HATU (460 mg, 1.21 mmol, 1.2 eq). After stirring for 10 min, **Example 46f** (329 mg, 1.01 mmol, 1.0 eq) was added, which was stirred for 2 h at r.t. The mixture was concentrated in vacuo. The residue was purified by silica gel flash column chromatography to afford the product **Example 46h** (160 mg, 31%yield) as a yellow solid. LCMS [M+1]⁺ = 507.3.

### Step 6: Example 46i

To a solution of **Example 46h** (160 mg, 0.32 mmol, 1.0 eq) in dioxane (8 mL) were added Cs₂CO₃ (308 mg, 0.96 mmol, 3.0 eq) and 3^{rd}-t-Bu-Xphos-Pd (84 mg, 0.096 mmol, 0.3 eq). The reaction mixture was stirred for 3 h at 80°C under N₂. The reaction solution was filtered and the filtrate was concentrated in *vacuo.* The crude product was purified by prep-TLC to afford the desired product **Example 46i** (45 mg, 30% yield) as a yellow solid. LCMS [M+1]⁺ = 471.3.

### Step 7: Example 46

To a solution of **Example 46i** (66 mg, 0.14 mmol, 1.0 eq) in DCM (4mL) was added HCl/dioxane (2 mL, 4 mol/L in dioxane) at 0°C, which was stirred for 2 h at r.t. The reaction solution was concentrated in *vacuo.* The crude product was dissolved in MeOH, and Na₂CO₃(excess) was added to the mixture, which was stirred for 10 min at r.t. The solid was filtered out, and the filtrate was concentrated. The crude was purified by prep-TLC to afford the desired product **Example 46** (20 mg, 36% yield) as a yellow solid. LCMS [M+1]⁺ = 371.3. ¹H NMR (300 MHz, DMSO-*d*₆) δ 9.79 (s, 1H), 8.46 (s, 1H), 8.31 (d, 1H), 8.16 (s, 1H), 7.93-8.01 (m, 1H), 6.66-6.75 (m, 2H), 5.49 (s, 1H), 4.60 (d, 1H), 4.50 (d, 1H), 3.87-3.90 (m, 1H), 3.55 (dd, 1H), 3.44-3.38 (m,1H), 2.93 (d, 3H), 1.18 (d, 3H).

### Example 47:

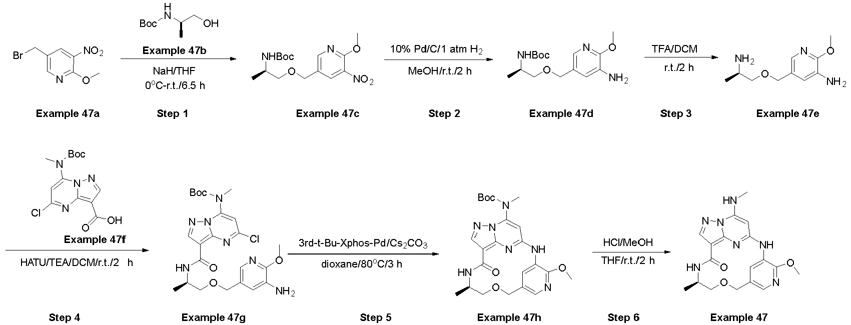

### Step 1: Example 47c

To a solution of **Example 47b** (525 mg, 3.0 mmol) in THF (15 mL) was added NaH (172 mg, 60% in mineral oil, 4.5 mmol) at 0°C. The reaction mixture was warmed to room temperature and stirred for 0.5 h. Then, **Example 47a** (741 mg, 3.0 mmol) was added. The resulting mixture was stirred at r.t. for 6 h. The mixture was quenched by aq.NH₄Cl, and then extracted by EtOAc, and dried over anhydrous Na₂SO₄. The solution was concentrated under reduced pressure and purified by silica gel column chromatography to give **Example 47c** (200 mg, yield: 20%) as a yellow solid. LCMS [M-174]⁺ = 167.0.

### Step 2: Example 47d

A mixture of **Example 47c** (200 mg, 0.58 mmol) and 10% Pd/C (30 mg) in MeOH (5 mL) was stirred at r.t. for 2 h under 1 atm H₂. The mixture was then filtered, and the filtrate was concentrated under reduced pressure to give crude **Example 47d** (200 mg crude, yield: ~100%) as a yellow solid, which was used in the next step directly. LCMS [M-174]⁺ = 137.1

### Step 3: Example 47e

To a solution of **Example 47d** (170 mg, 0.55 mmol) in DCM (5.0 mL) was added TFA (1.0 mL), which was stirred at r.t. for 2 h. The mixture was concentrated to give the crude product **Example 168g** (403.5 mg crude, yield: ~100%) as black oil.

### Step 4: Example 47g

To a solution of **Example 47e** (403 mg crude, 0.61 mmol), **Example 47f** (197 mg, 0.61 mmol), TEA (900 mg, 9.0 mmol) in DCM (10 mL) was added HATU (230 mg, 0.605 mmol). The reaction mixture was stirred at r.t. for 2 h. Then DCM (40 mL) was added to the reaction mixture, which was washed with brine (20 mL*2), dried over anhydrous Na₂SO₄ and concentrated. The residue was purified by silica gel column chromatography to afford the desired product **Example 47g** (200 mg, yield: 64%) as a brown solid. LCMS [M+1]⁺ = 520.2

### Step 5: Example 47h

To a mixture of **Example 47g** (200 mg, 0.39 mmol), Cs₂CO₃ (190 mg, 0.59 mmol) in dioxane (10.0 mL) was added 3rd-t-Bu-Xphos-Pd (35 mg, 0.039 mmol). The mixture was degassed with N₂ three times, and stirred for 3 h at 80°C. Then the reaction mixture diluted by EtOAc, washed by water, dried over anhydrous Na₂SO₄, and then concentrated under reduced pressure to afford crude **Example 47h** (240 mg crude, yield: ~100%) as a white solid, which was used in the next step without further purification. LCMS [M+1]⁺ = 484.2

### Step 6: Example 47

To a solution of **Example 47h** (240 mg crude, 0.49 mmol) in THF (1.4 mL) was added HCl/MeOH (2.0 mL, 6.0 moL/L), which was stirred at r.t. for 2 h. The mixture was concentrated, and the residue was purified by Prep-HPLC to afford the desired product **Example 47** (13.3 mg, yield: 7% over 2 steps) as a white solid. LCMS [M+1]⁺ = 384.2. ¹H NMR (400 MHz, DMSO-*d*₆) δ 9.13 (s, 1H), 8.57 (d, 1H), 8.11 (s, 1H), 8.05 (d, 1H), 7.90 (d, 1H), 7.71 (d, 1H), 5.90 (s, 1H), 4.54 (d, 1H), 4.43 (d, 1H), 3.95 (s, 3H), 3.86 (t, 1H), 3.46 (dd, 1H), 3.28 (dd, 1H), 2.89 (d, 3H), 1.12 (d, 3H).

### Example 48:

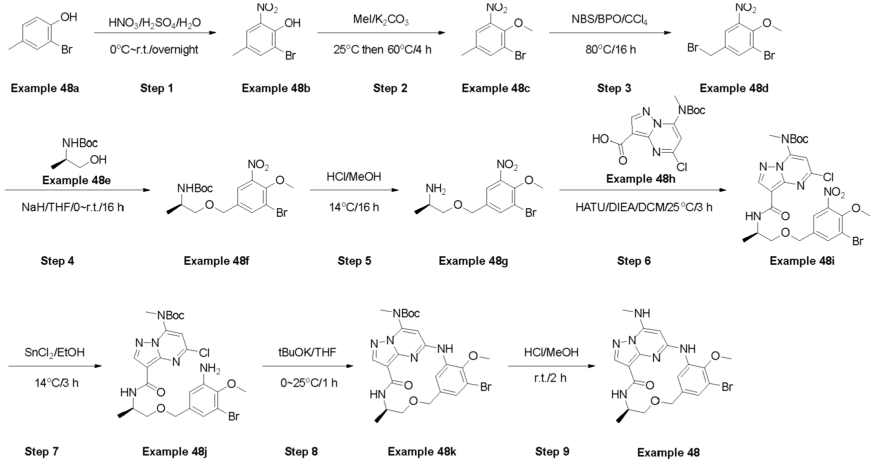

### Step 1: Example 48b

To a solution of H₂SO₄ (200 mL) in H₂O (620 mL) was added HNO₃ (56 g, 889 mmol) at 0°C. Then **Example 48a** (88 g, 471 mmol) was added and the resulting mixture was stirred at r.t. for overnight. After completion, the mixture was extracted by EtOAc, and dried over anhydrous Na₂SO₄. The solution was concentrated under reduced pressure, which was purified by silica gel column chromatography to give **Example 48b** (81 g, yield: 74%) as a yellow solid.

### Step 2: Example 48c

To a stirred solution of **Example 48b** (20 g, 86.6 mmol), and **K₂CO₃** (23.6 g,171mmol) in DMF (70 mL) was added CH₃I (17g, 111.8 mmol) at 25°C. Then the reaction mixture was stirred at 60°C for 4 h. The reaction mixture was extracted by EtOAc. The organic layer was washed by brine, and dried over anhydrous Na₂SO₄. The solution was concentrated under reduced pressure. The residue was purified by silica gel column chromatography to give **Example 48c** (22.5 g, yield: quant.) as a yellow solid.

### Step 3: Example 48d

To a solution of **Example 48c** (10.0 g, 40.64 mmol) in CCl₄ (10 mL) were added NBS (9.4 g, 52.84 mmol) and BPO (3.94 g, 16.26 mmol), which was stirred at 80°C for 16 h. The mixture was concentrated under reduced pressure, which was purified by silica gel column chromatography to give **Example 48d** (7.8 g, yield: 59%) as a yellow solid.

### Step 4: Example 48f

To a solution of NaH (1.0 g, 25.1 mmol) in THF (70 mL) were added **Example 48d** (6.8 g, 20.9 mmol) and **Example 48e** (4.4 g, 25.1 mmol) at 0°C. The mixture was stirred at r.t. for 16 h. The reaction mixture was quenched by H₂O, then concentrated under reduced pressure, which was purified by silica gel column chromatography to afford the desired product **Example 48f** (6.25 g, yield: 71%) as a yellow oil. LCMS [M-100+1] ⁺ = 319.0/321.0.

### Step 5: Example 48g

To a solution of HCl/MeOH (4M, 70 mL) was added **Example 48f** (6.25 g, 14.9 mmol). Then the reaction mixture was stirred at 14°C for 2 h. The mixture was concentrated to give crude **Example 48g** (5.17 g, crude) as a yellow solid, which was used in the next step without further purification. LCMS [M+1]⁺ = 319.0/321.0

### Step 6: Example 48i

To a stirred solution of **Example 48g** (5 g crude, 15.67 mmol) in DCM (50 mL) were added HATU (7.68 g, 23.5 mmol), DIEA (4.57 g, 47.0 mmol), and **Example 48h** (7.68 g, 23.5 mmol). The mixture was stirred at 25°C for 3 h. Then the reaction mixture was concentrated under reduced pressure, which was purified by silica gel column chromatography to afford the desired product **Example 48i** (8.4 g, yield: 85%) as a yellow solid. LCMS [M+1]⁺ = 627.1/629.1.

### Step 7: Example 48j

To a solution of **Example 48i** (1 g, 1.59 mmol) in EtOH (10 mL) was added SnCl₂ (0.91 g, 4.78 mmol), which was stirred at 14°C for 2 h. The mixture was concentrated and purified by silica gel column chromatography to afford the desired product **Example 48j** (1 g, crude) as a yellow solid. LCMS [M+1]⁺ = 597.1/599.1.

### Step 8: Example 48k

To a solution of **Example 232i** (700 mg, crude) in THF (30 mL) was added t-BuOK (394 mg, 3.51 mmol) at 0°C. Then the mixture solution was stirred at 25°C for 1 h. The mixture was concentrated, and the residue was purified by silica gel column chromatography to afford the desired product Example **48k** (450 mg, yield: 69%) as yellow oil. LCMS [M+1]⁺ = 561.1/563.1.

### Step 9: Example 48

To a solution of **Example 48k** (100 mg, 0.34 mmol) in MeOH (1 mL) was added HCl/MeOH (1.0 mL, 6.0 moL/L), which was stirred at r.t. for 2 h. The mixture was concentrated, and the residue was purified by Prep-HPLC to afford the desired product **Example 48** (36.5 mg, yield: 44%) as a white solid. LCMS [M+1]⁺ = 461.1. ¹H NMR (400 MHz, DMSO-*d*₆) δ 9.17 (s, 1H), 8.51 (s, 1H), 8.16 (s, 1H), 8.13 (s, 1H), 7.92 (d, 1H), 7.17 (d, 1H), 6.03 (s, 1H), 4.54 (d, 1H), 4.42 (d, 1H), 3.89-3.87 (m, 1H), 3.77 (s, 3H), 3.49 (d, 1H), 3.33 (d, 1H), 2.92 (d, 3H), 1.14 (d, 3H).

### Example 49:

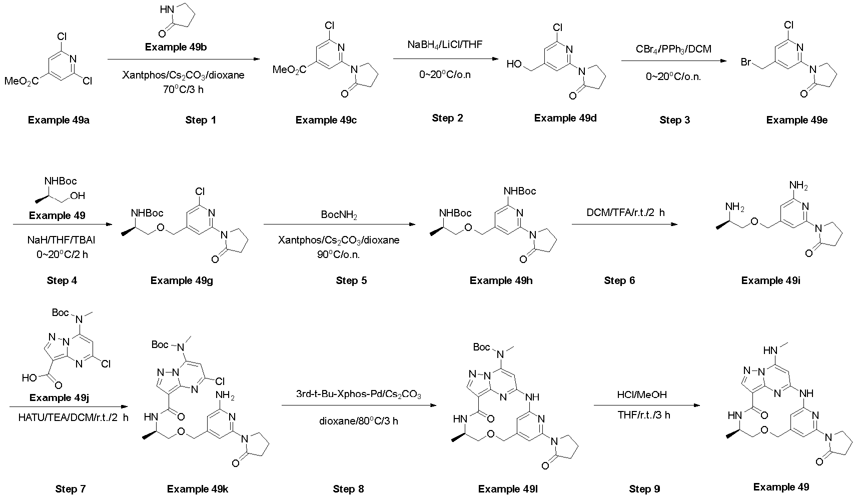

### Step 1: Example 49c

To a solution of **Example 49a** (2.04 g, 10.0 mmol), **Example 49b** (850 mg, 10.0 mmol), and Cs₂CO₃ (4.89 g, 15.0 mmol) in dioxane (30 mL) were added Pd₂(dba)₃ (458 mg, 0.5 mmol), and Xantphos (298 mg, 0.5 mmol). The mixture was degassed with N₂ three times, and stirred for 3 h at 70°C. Then the reaction mixture was diluted by EtOAc, washed by water, dried over anhydrous Na₂SO₄, and then concentrated under reduced pressure. The residue was purified by silica gel column chromatography to afford the desired product **Example 49c** (1.9 g, yield: 75%.) as a white solid. LCMS [M+1]⁺ = 255.0

### Step 2: Example 49d

To a solution of **Example 49c** (1.1 g, 4.33 mmol) in THF (30 mL) were added NaBH₄ (165 mg, 4.33 mmol) and LiCl (1.3 g, 34.64 mmol) at 0°C. The mixture was degassed with N₂ three times, and stirred for overnight at 20°C. The reaction mixture was then quenched by the addition of water (1.2 mL) at 0°C. The resulting solution was diluted with aqueous NaOH solution (15%, 3.6 mL), followed by EtOAc (1.2 mL) at room temperature. The solid was filtered off. The resulting filtrate was concentrated under reduced pressure, which was purified by silica gel column chromatography to afford the desired product **Example 49d** (400 mg, yield: 41%) as a yellow solid. LCMS [M+1] ⁺= 227.0

### Step 3: Example 49e

To a solution of **Example 49d** (400 mg, 1.77 mmol) in DCM (5 mL) was added PPh₃ (696 mg, 2.66 mmol). The mixture was cooled to 0°C, then a solution of CBr₄ (701 mg, 2.12 mmol) in DCM (5 mL) was added drop-wise. After addition, the reaction mixture was stirred at 20°C overnight. Then the solution was concentrated under reduced pressure, and purified by silica gel column chromatography to afford the desired product **Example 49e** (460 mg, yield: 90%) as a yellow solid. LCMS [M+1] ⁺ = 288.9.

### Step 4: Example 49g

To a solution of **Example 49e** (460 mg, 1.59 mmol), and **Example 49f** (332 mg, 1.89 mmol) in THF (10 mL) were added NaH (87 mg, 60% in mineral oil, 2.18 mmol), and TBAI (60 mg, 0.16 mmol) at 0°C. Then the reaction mixture was warmed to 20°C, and stirred for 2 h. The reaction was then quenched by the addition of aqueous NH₄Cl solution (10 mL), which was extracted with EtOAc for 3 times. The combined organic phase was dried over anhydrous Na₂SO₄, and then concentrated under reduced pressure. The residue was purified by silica gel column chromatography to afford the desired product **Example 49g** (520 mg, yield: 85%) as yellow oil. LCMS [M+1] ⁺ = 406.1.

### Step 5: Example 49h

To a solution of **Example 49g** (520 mg, 1.35 mmol), **NH₂Boc** (224 mg, 1.91 mmol), Cs₂CO₃ (625 mg, 1.92 mmol) in dioxane (10 mL) were added Pd₂(dba)₃ (114 mg, 0.12 mmol), and Xantphos (76 mg, 0.13 mmol). The mixture was degassed with N₂ three times, and stirred at 90°C overnight. Then the reaction mixture diluted with EtOAc, washed by water, dried over anhydrous Na₂SO₄, and then concentrated under reduced pressure. The residue was purified by silica gel column chromatography to afford the desired product **Example 49h** (650 mg crude, yield: quant.) as a white solid. LCMS [M+1]⁺ = 465.2.

### Step 6: Example 49i

To a solution of **Example 49h** (410 mg, 0.88 mmol) in DCM (8 mL) was added TFA (2 mL), which was stirred at r.t. for 2 h. The mixture was concentrated to give the crude product **Example 49i** (350 mg, crude, yield: quant.) as black oil. LCMS [M+1]⁺ = 265.1.

### Step 7: Example 49k

To a solution of **Example 49i** (350 mg, 0.76 mmol), **Example 49j** (248 mg, 0.76 mmol), and TEA (760 mg, 7.6 mmol) in DCM (15 mL) was added HATU (289 mg, 0.76 mmol). The reaction mixture was stirred at r.t. for 2 h. Then DCM (40 mL) was added to the reaction mixture, which was washed with brine (20 mL*2), dried over anhydrous Na₂SO₄ and concentrated. The residue was purified by silica gel column chromatography to afford the desired product **Example 49k** (304 mg, yield: 70%) as a brown solid. LCMS [M+1]⁺ = 573.2.

### Step 8: Example 49l

To a mixture of **Example 49k** (304 mg, 0.43 mmol), and Cs₂CO₃ (260 mg, 0.80 mmol) in dioxane (20 mL) was added 3rd-t-Bu-Xphos-Pd (46.3 mg, 0.053 mmol). The mixture was degassed with N₂ three times, and stirred at 80°C for 3 h. Then the reaction mixture was diluted with EtOAc, washed by water, dried over anhydrous Na₂SO₄, and then concentrated under reduced pressure to afford crude **Example 491** (200 mg, crude yield: 70%) as a brown solid, which was used in the next step without further purification. LCMS [M+1]⁺ = 537.2.

### Step 9: Example 49

To a solution of **Example 49l** (200 mg crude, 0.37 mmol) in THF (1.0 mL) was added HCl/MeOH (1.0 mL,6.0 moL/L), which was stirred at r.t. for 3 h. The mixture was concentrated, and the residue was purified by Prep-HPLC to afford the desired product **Example 49** (7.8 mg, yield: 5%) as a white solid. LCMS [M+1]⁺ = 437.2. ¹H NMR (400 MHz, DMSO-*d*₆) δ 9.95 (s, 1H), 8.32 (d, 1H), 8.26 (s, 1H), 8.16 (s, 1H), 8.00 (d, 1H), 7.77 (s, 1H), 5.81 (s, 1H), 4.58 (q, 2H), 4.00 (t, 2H), 3.90 (s, 1H), 3.56 (d, 1H), 3.43 (t, 1H), 2.90 (d, 3H), 2.56 (t, 2H), 2.09-1.99 (m, 2H), 1.18 (d, 3H).

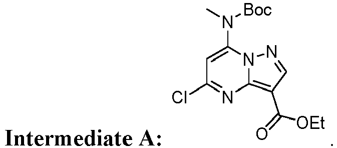

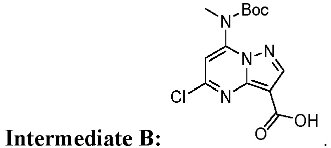

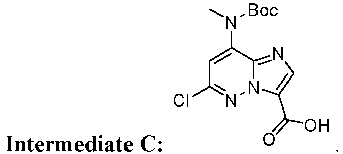

### Example 50:

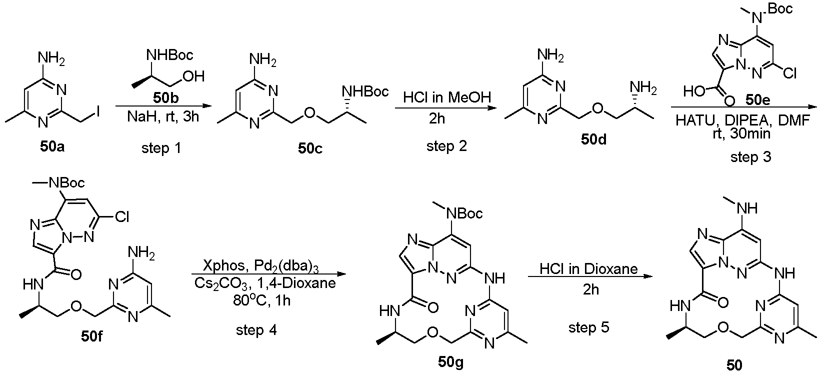

### Step 1: (R)-tert-butyl (1-((4-amino-6-methylpyrimidin-2-yl)methoxy)propan-2-yl) carbamate

To a solution of **50b** ( 90 mg, 0.5 mmol) in THF (4 mL) was added NaH (40 mg, 60%, 2 equiv, 1.0 mmol) at 0 °C. After 20 min, **50a** (80 mg, 0.32 mmol) (Studies on the Iodination of 4-Amino-2,6-dimethylpyrimidine-A Possibility of the Regiospecific Functionalization. Journal f. prakt. Chemie. Band 329, Heft 3, 1987, S. 400-408 ) was added, then the reaction mixture was warmed to rt. After being stirred at room temperature for 3 h, the reaction mixture was poured into water and then the product was extracted with EA (2×20 mL), dried over Na₂SO₄, and concentrated in vacuo. The residue was purified by flash chromatography to afford **1c** (56 mg) as a yellow solid. LC-MS (ESI): m/z =297.3 [M+H]⁺.

### Step 2: (R)-2-((2-aminopropoxy)methyl)-6-methylpyrimidin-4-amine

A solution of **50c** and hydrochloric acid (4M in MeOH ) (3 mL) was stirred at room temperature for 2h. Solvent was evaporated, and the crude product was partitioned between water and DCM. The aqueous layer was basified with NaHCO₃ and extracted with DCM. Combined organic layers were washed with brine, dried over sodium sulfate, filtered, and evaporated to give **50d** which was used in the next step without further purification.

### Step 3: (R)-tert-butyl (3-((1-((4-amino-6-methylpyrimidin-2-yl)methoxy)propan-2-yl)carbamoyl)-6-chloroimidazo[1,2-b]pyridazin-8-yl)(methyl)carbamate

To a solution of **50e** (66 mg, 0.2 mmol) in DMF (3 mL) were successively added HATU (76 mg, 0.2 mmol) and Et₃N (36 mg, 0.36 mmol). The reaction mixture was stirred at room temperature for 0.5 h, then the mixture was added **50d** (a solution in 1 mL DMF ) and stirred at room temperature for 0.5 h. The mixture was diluted with water (10 mL) and extracted with DCM (10 mL×3). The combined organic layers were then washed with water (10 mL×2) and brine (5 mL×1), dried over Na₂SO₄, filtered, and concentrated in vacuo. The residue was purified by flash chromatography to afford the title **50f** (81 mg, two steps 85%) as a yellow solid. LC-MS (ESI): m/z =505.3 [M+H]⁺.

### Step 4: tert-butyl ((7R,E)-3⁶,7-dimethyl-9-oxo-5-oxa-2,8-diaza-1(6,3)-imidazo[1,2-b]pyridazina-3(4,2)-pyrimidinacyclononaphane-1⁸-yl)(methyl)carbamate

To a solution **50f** (81 mg, 0.16 mmol) in 1,4-dioxane (4 mL) under argon was successively added Cesium Carbonate (0.13 g, 0.4 mmol), XPhos (24 mg, 0.05 mmol) and Pd₂(dba)₃ (23 mg, 0.025 mmol). The reaction mixture was heated at 80°C for 1h. After cooling to room temperature, the mixture was diluted with water and EtOAc. The organic layer was separated and the aqueous layer extracted with EtOAc. The combined organic layers were then washed with water and brine, dried over Na₂SO₄, filtered, and concentrated in vacuo. The residue was purified by flash chromatography to afford 50g (40 mg, 53%) as a yellow solid. LC-MS (ESI): m/z =469.3 [M+H]⁺.

### Step 5: (7R,E)-3⁶,7-dimethyl-1⁸-(methylamino)-5-oxa-2,8-diaza-1(6,3)-imidazo[1,2-b]pyridazina-3(4,2)-pyrimidinacyclononaphan-9-one

A solution of **50g** (40 mg, 0.085 mmol) and hydrochloric acid (2M in 1,4-dioxane ) (3 mL)) was stirred at room temperature for 2h. Solvent was evaporated, and the crude product was partitioned between water and DCM. The aqueous layer was basified with NaHCO₃ and extracted with DCM. Combined organic layers were washed with brine, dried over Na₂SO₄, filtered, and concentrated in vacuo. The residue was purified by flash chromatography to afford **example 50** (12 mg, 38%) as a yellow solid. LC-MS (ESI): m/z =369.2 [M+H]⁺. ¹H NMR (400 MHz, DMSO-d6) δ 10.11 (s, 1H), 9.98 (d, 1H), 7.88 (s, 1H), 7.65 (d, 1H), 6.63 (s, 1H), 5.85 (s, 1H), 4.56 (d, 2H), 3.98-3.84 (m, 1H), 3.68-3.63 (m, 1H), 3.53 - 3.43 (m, 1H), 2.91 (d, 3H), 2.35 (s, 3H), 1.23 (s, 3H).

### Example 51:

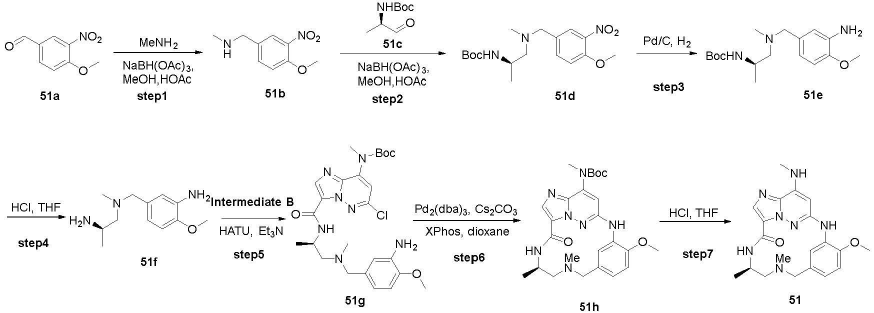

### Step 1: 1-(4-methoxy-3-nitrophenyl)-N-methylmethanamine (51b)

4-methoxy-3-nitrobenzaldehyde (5 g, 27.6 mmol), CH₃NH₂ (2.0 g, 64.5 mmol) and AcOH (3 mL) were added to MeOH (50 mL). The reaction mixture was stirred at 0°C for 1h. NaBH(OAc)₃(2.0 g, 64.5 mmol) was added and then stirred at rt for overnight. The reaction mixture was then poured into water (200 mL), and was extracted with ethyl acetate. The organic layer was washed with HCl (1M), the aqueous layer was basified with NaHCO₃ and extracted with ethyl acetate, the organic layer was dried over magnesium sulfate, filtered, and concentrated in vacuo to give title compound **51b.**(2 g, 44%). LC-MS (ESI): m/z =197.3 [M+H]⁺

### Step 2: tert-butyl(R)-(1-((4-methoxy-3-nitrobenzyl)(methyl)amino)propan-2-yl)carbamate (51d)

51b (1.6 g,8.16 mmol), tert-butyl(*R*)-(1-oxopropan-2-yl)carbamate (1.73 g,10.0 mmol) and AcOH (1 mL) was dissolved in methanol (10 mL), the mixture was stirred for 1h. NaBH(OAc)₃ (2.0 g, 64.5 mmol) was added and then stirred at rt for overnight. The reaction mixture was then poured into water (100 mL), The aqueous layer was basified with K₂CO₃ and extracted with ethyl acetate. The organic layer was dried over magnesium sulfate, filtered, and concentrated in vacuo to give title compound **51d** (1.05 g, 36.5%). LC-MS (ESI): m/z =354.3 [M+H]⁺

### Step 3: tert-butyl(R)-(1-((3-amino-4-methoxybenzyl)(methyl)amino)propan-2-yl)carbamate (51e)

**51d** (1 g, 0.40 mmol), Pd/C (37 mg) was added to MeOH (20 mL) and the mixture was stirred under H₂ ball for overnight, the suspension was diluted with dichloromethane and filtered through Celite. The solvent was removed to give a brown residue which was purified by column chromatography on silica gel to give the title compound **51e** (500 mg, 77.4%). LC-MS (ESI): m/z =324.3 [M+H]⁺

### Step 4: (R)-N¹-(3-amino-4-methoxybenzyl)-N¹-methylpropane-1,2-diamine (51f)

To a solution of compound **51e** (500 mg,1.55 mmol) in THF (10 mL) was added HCl/dioxane (5 mL), The mixture was stirred at rt for overnight and then was concentrated under reduced pressure, the residue was directly used for the next step without purification. LC-MS (ESI): m/z =224.2 [M+H]⁺

### Step 5: tert-butyl(R)-(3-((1-((3-amino-4-methoxybenzyl)(methyl)amino)propan-2-yl)carbamoyl)-6-chloroimidazo[1,2-b]pyridazin-8-yl)(methyl)carbamate (51g)

To a solution of compound 2e (345 mg, 1.55 mmol) and 8-((tert-butoxycarbonyl) (methyl) amino)-6-chloroimidazo[1,2-b]pyridazine-3-carboxylic acid **(Intermediate B,** 600 mg, 1.84 mmol) in DMF (10 mL) was added TEA (3 mL) and HATU (1.52 g, 4 mmol) at room temperature, The reaction mixture was stirred for overnight and then poured into crashed ice, and was extracted with ethyl acetate. The organic layer was dried over magnesium sulfate, filtered, and concentrated in vacuum. The residue was purified column chromatography on silica gel to give the title compound **51g** (550 mg, 67%). LC-MS (ESI): m/z =533.3 [M+H]⁺

### Step 6: tert-butyl ((7R,E)-3⁶-methoxy-5,7-dimethyl-9-oxo-2,5,8-triaza-1(6,3) -imidazo [1,2-b] pyridazina-3(1,3)-benzenacyclononaphane-18-yl)(methyl)carbamate (51h)

To a solution of **51g** (300 mg, 0.56 mmol) in 1,4-dioxane (10 mL) was added Pd₂(dba)₃ (50 mg, 0.054 mmol), Cs₂CO₃ (400 mg, 1.22 mmol) and Xphos (30 mg, 0.05 mmol). The reaction mixture was heated to 95 °C and then stirred for 3.5 h under N_{2.} After cooled to rt, the mixture was filtered. The filtrate was then suspended in 50 mL of water, extracted with ethyl acetate, the organic layer was dried over magnesium sulfate, filtered, and concentrated in vacuum. The residue was purified by column chromatography on silica gel to give the title compound **51h** (30 mg, 10.8%). LC-MS (ESI): m/z =496.3 [M+H]⁺

### Step 7: (7R,E)-3⁶-methoxy-5,7-dimethyl-1⁸-(methylamino)-2,5,8-triaza-1(6,3)-imidazo[1,2-b]pyridazina-3(1,3)-benzenacyclononaphan-9-one

To a solution of **51h** (30 mg, 0.06 mmol) in MeOH (2 mL) was added HCl /dioxane (5 mL), The mixture was stirred at rt for overnight and then was concentrated under reduced pressure, the residue was poured into crashed ice, and then K₂CO₃ was added until PH>10, extracted with ethyl acetate, the organic layer was dried over magnesium sulfate, filtered, and concentrated in vacuum. The residue was purified by column chromatography on silica gel to give **example 51** (5 mg, 21.0%). ¹H NMR (400 MHz, DMSO-d6) *δ* 8.73 (d, 1H), 8.36 (s, 1H), 8.18 (d, 1H), 7.78 (s, 1H), 7.37(d, 1H), 6.95 (d, 1H), 6.80 (d, 1H), 6.17 (s, 1H), 4.04-4.00 (m, 1H), 3.86 (s, 3H), 3.74 (d, 1H), 3.12 (d, 1H), 2.87 (d, 3H), 2.46-2.34 (m, 1H), 2.36 (s, 1H), 1.96-1.92 (m, 1H), 1.02 (d, 3H). LC-MS (ESI): m/z =396.3 [M+H]⁺.

### Example 52:

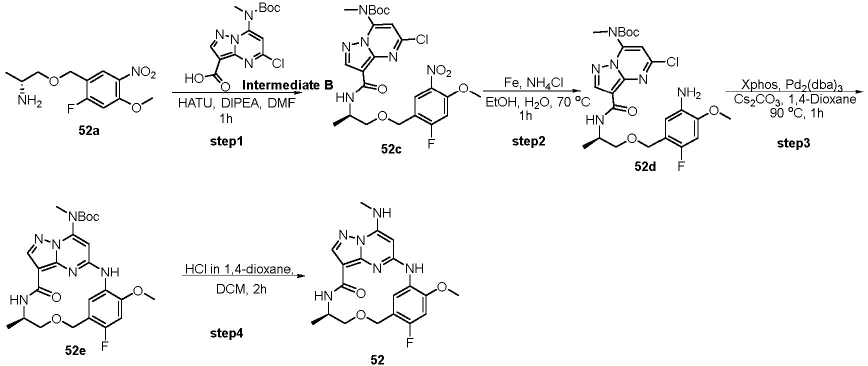

### Step1: (R)-tert-butyl (5-chloro-3-((1-((2-fluoro-4-methoxy-5-nitrobenzyl)oxy)propan-2-yl)carbamoyl)pyrazolo[1,5-a]pyrimidin-7-yl)(methyl)carbamate (52c)

To a solution of **Intermediate B** (0.24 g, 0.74 mmol) in DMF (10 mL) were successively added HATU (0.31 g, 0.81 mmol) and Et₃N (0.15 g, 1.5 mmol). The reaction mixture was stirred at room temperature for 0.5 h, then the mixture was added **52a** (0.19 g, 0.74 mmol) (a solution in 3 mL DMF ) and stirred at room temperature for 0.5 h. The mixture was diluted with water and extracted with DCM. The combined organic layers were then washed with brine, dried over Na₂SO₄, filtered, and concentrated in vacuo. The residue was purified by flash chromatography to afford **52c** (0.33 g, 79%) as a yellow solid. LC-MS (ESI): m/z =567.2 [M+H]⁺.

### Step2: (R)-tert-butyl (3-((1-((5-amino-2-fluoro-4-methoxybenzyl)oxy)propan-2-yl)carbamoyl)-5-chloropyrazolo[1,5-a]pyrimidin-7-yl)(methyl)carbamate (52d)

To a solution **52c** (0.33 g, 0.58 mmol) in EtOH (20 mL) and water (5 mL) was successively added Iron powder (0.35 g, 6 mmol), NH₄Cl (18 mg, 0.3 mmol) The reaction mixture was heated at 70 °C for 1h. After cooling to room temperature, Then the reaction mixture was filtered and the solvent was removed. the mixture was diluted with water and EtOAc. The organic layer was separated and the aqueous layer extracted with EtOAc. The combined organic layers were then washed with water and brine, dried over Na₂SO₄, filtered, and concentrated in vacuo. The residue was purified by flash chromatography to afford **52d** (0.19 g, 61%) as a yellow solid. LC-MS (ESI): m/z =537.3 [M+H]⁺.

### Step3: tert-butyl ((R,1³E,1⁴E)-3⁴-fluoro-3⁶-methoxy-7-methyl-9-oxo-5-oxa-2,8-diaza-1(5,3)-pyrazolo[1,5-a]pyrimidina-3(1,3)-benzenacyclononaphane-17-yl)(methyl)carbamate (52e)

To a solution **52d** (0.19 g, 0.35 mmol) in 1,4-dioxane (15 mL) under argon was successively added Cesium Carbonate (0.23 g, 7 mmol), XPhos (78 mg, 0.1 mmol) and Pd₂(dba)₃ (46 mg, 0.05 mmol). The reaction mixture was heated at 90 °C for 1h. After cooling to room temperature, the mixture was diluted with water and EtOAc. The organic layer was separated and the aqueous layer extracted with EtOAc. The combined organic layers were then washed with water and brine, dried over Na₂SO₄, filtered, and concentrated in vacuo. The residue was purified by flash chromatography to afford **3e** (0.13 g, 74%) as a yellow solid. LC-MS (ESI): m/z =501.3 [M+H]⁺.

### Step 4: (R,1³E,1⁴E)-3⁴-fluoro-3⁶-methoxy-7-methyl-1⁷-(methylamino)-5-oxa-2,8-diaza-1(5,3)-pyrazolo[1,5-a]pyrimidina-3(1,3)-benzenacyclononaphan-9-one

A solution of **52e** (0.13 g, 0.26 mmol) (4 mL) in DCM and hydrochloric acid (2M in 1,4-dioxane) (6 mL) was stirred at room temperature for 2h. Solvent was evaporated, and the crude product was partitioned between water and DCM. The aqueous layer was basified with NaHCO₃ and extracted with DCM. Combined organic layers were washed with brine, dried over sodium sulfate, filtered, and evaporated in vacuo. The residue was purified by flash chromatography to afford **example 52** (31 mg, 30%) as a white solid. LC-MS (ESI): m/z =401.3. [M+H]⁺. ¹H NMR (400 MHz, DMSO) δ 8.88 (s, 1H), 8.36 (d, 1H), 8.18 (d, 1H), 8.10 (s, 1H), 7.80 (d, 1H), 6.96 (d, 1H), 5.90 (s, 1H), 4.56 (d, 2H), 3.89 (s, 3H), 3.54 - 3.47 (m, 1H), 3.35 - 3.29 (m, 1H),2.91 (d, 3H), 1.30 - 1.19 (m, 1H), 1.15 (d, 3H).

### Example 53:

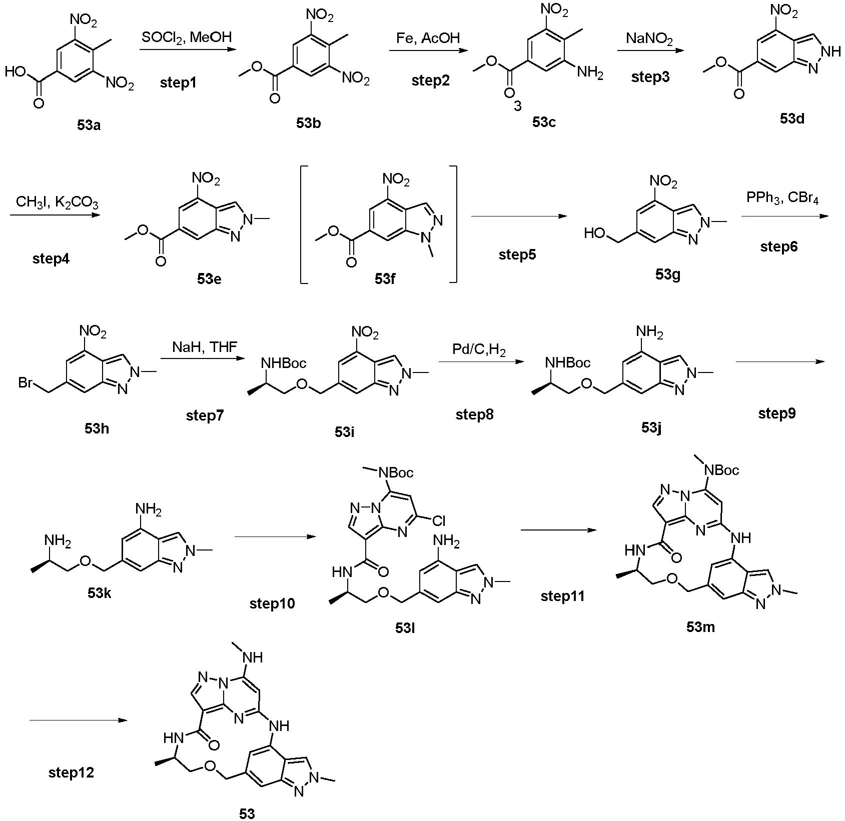

### Step 1: methyl 4-methyl-3,5-dinitrobenzoate (53b)

4-methyl-3,5-dinitrobenzoic acid (5g, 22.1 mmol) was added to MeOH (50 mL). SOCl₂ (6.6 g, 3.0 mmol) was added dropwise at 0-20 °C. Then the reaction mixture was heated to 60 °C and stirred for 2h. After cooled to rt, the reaction mixture was concentrated in vacuum, the residue was washed with MTBE and dried to give **53b** (5g, 94.2%). LC-MS (ESI): m/z =241.3 [M+H] ⁺

### Step 2: methyl 3-amino-4-methyl-5-nitrobenzoate (53c)

**53b** (5 g, 20.8 mmol) was added to AcOH (50 mL). Fe (1.68 g, 3.0 mmol) was added by batches and then the mixture was stirred for 1h. The reaction mixture was then poured into water (200 mL) and extracted with ethyl acetate, the organic layer was dried over magnesium sulfate, filtered, and concentrated in vacuum to give the title **53c** (4 g, 91.6%). LC-MS (ESI): m/z =211.3 [M+H] ⁺

### Step 3: methyl 4-nitro-2H-indazole-6-carboxylate (53d)

**53c** (4 g, 19 mmol) was added to AcOH (50 mL). NaNO₂ (1.72 g in 10 mL H₂O, 2.5 mmol) was added dropwise and then the mixture was heated to 40 °C for 1h. The reaction mixture was then poured into water (200 mL) and extracted with ethyl acetate, the organic layer was dried over magnesium sulfate, filtered, and concentrated in vacuum to give the title 4d (3 g, 71.5%). LC-MS (ESI): m/z =222.3 [M+H] ⁺

### Step 4: methyl 2-methyl-4-nitro-2H-indazole-6-carboxylate (53e)

53d (3 g, 13 mmol) and K₂CO₃ (2.8 g, 20 mmol) was added to DMF (50 mL). CH₃I (3.1 g, 22 mmol) was added dropwise and then the mixture was stirred at rt for 1h. The reaction mixture was then poured into water (200 mL) and extracted with ethyl acetate, the organic layer was dried over magnesium sulfate, filtered, and concentrated in vacuum. The residue was purified by column chromatography on silica gel (PE: EA = 2:1) to give **53e** (0.7g, 23%) and **4f** (0.7g, 23%). LC-MS (ESI): m/z =236.3 [M+H] ⁺

### Step 5: (2-methyl-4-nitro-2H-indazol-6-yl)methanol (53g)

**53e** (0.7 g, 3.0 mmol) was added to THF (20 mL) under N₂. After cooled to -60 °C, DIBAL-H (1mol/L in toluene, 6 mL, 6 mmol) was added dropwise and then stirred for 2h. The reaction mixture was then poured into water (100 mL), and was extracted with ethyl acetate. the organic layer was dried over magnesium sulfate, filtered, and concentrated in vacuum. The residue was purified by column chromatography on silica gel (PE: EA = 1:1) to give the **53g** (0.4 g, 64.5%. LC-MS (ESI): m/z =208.2 [M+H] ⁺

### Step 6: 6-(bromomethyl)-2-methyl-4-nitro-2H-indazole (4h)

**53g** (400 mg, 1.92 mmol) was dissolved in DCM (10 mL), CBr₄ (760 mg, 2.3 mmol) and PPh₃ (600 mg, 2.3 mmol) were added and then stirred at rt for 2h. The reaction mixture was then poured into water (100 mL) and extracted with ethyl acetate, the organic layer was dried over magnesium sulfate, filtered, and concentrated in vacuum. The residue was purified by column chromatography on silica gel (n-hexane: ethyl acetate = 5:1) to give the title **53h** (300 mg, 58.5%). LC-MS (ESI): m/z =271.2 [M+H] ⁺

### Step 7: tert-butyl (R)-(1-((2-methyl-4-nitro-2H-indazol-6-yl)methoxy) Propan-2-yl) carbamate (53i)

Tert-butyl (*R*)-(1-hydroxypropan-2-yl)carbamate (200 mg, 1.15 mmol) was added to THF (20 mL) under N₂, NaH (50 mg, 1.3 mmol) was added at 0°C, the suspension was stirred at rt for 0.5h, **53h** (300 mg, 1.10 mmol) was added and then the mixture was stirred at rt for 4h, The reaction mixture was then poured into water (100 mL) and extracted with ethyl acetate, The organic layer was dried over magnesium sulfate, filtered, and concentrated in vacuum. The residue was purified by column chromatography on silica gel (n-hexane: ethyl acetate = 2:1) to give **53i** (300 mg, 74.9%). LC-MS (ESI): m/z =365.2 [M+H] ⁺

### Step8: tert-butyl(R)-(1-((4-amino-2-methyl-2H-indazol-6-yl)methoxy)propan-2-yl)carbamate (53j)

**53i** (60 mg, 0.16 mmol) and Pd/C (10 mg) was added to MeOH (10 mL) and the mixture was stirred under H₂ ball for overnight, the suspension was diluted with dichloromethane and filtered through Celite. The solvent was removed to give a brown residue which was purified by column chromatography on silica gel (n-hexane: ethyl acetate = 1: 1) to give **53j** (50 mg, 93.56%). LC-MS (ESI): m/z =335.2 [M+H] ⁺

### Step 9: (R)-6-((2-aminopropoxy)methyl)-2-methyl-2H-indazol-4-amine (53k)

To a solution of **53j** (50 mg, 0.15 mmol) in THF(2 mL) was added HCl/dioxane (6 mol/L, 1 mL), The mixture was stirred at rt for overnight and then was concentrated under reduced pressure, the residue was directly used for the next step without purification. LC-MS (ESI): m/z =235.2 [M+H] ⁺

### Step10: tert-butyl(R)-(3-((1-((4-amino-2-methyl-2H-indazol-6-yl)methoxy)propan-2-yl) carbamoyl)-5-chloropyrazolo[1,5-a]pyrimidin-7-yl)(methyl)carbamate (53l)

To a solution of **53k** (30 mg, 0.13 mmol) and 7-((tert-butoxycarbonyl)(methyl)amino)-5-chloropyrazolo [1,5-a] pyrimidine- 3-carboxylic acid (42 mg, 0.13 mmol) in DMF (2 mL) was added TEA (0.1 mL) and HATU (50 mg,0.13 mmol) at room temperature, the reaction mixture was stirred for overnight and then poured into crashed ice, and was extracted with ethyl acetate. The organic layer was dried over magnesium sulfate, filtered, and concentrated in vacuum. The residue was purified column *chromatography* on silica gel (DCM: MeOH = 20:1) to give **53l** (20 mg, 28.3%). LC-MS (ESI): m/z =544.3 [M+H] ⁺

### Step11: tert-butyl((R,1³E,1⁴E,3⁴E)-3²,7-dimethyl-9-oxo-3²H-5-oxa-2,8-diaza-1(5,3)-pyrazolo[1,5-a] pyrimidina-3(4,6)-indazolacyclononaphane-17-yl)(methyl)carbamate (53m)

To a solution of **53l** (20 mg, 0.036 mmol) in 1,4-dioxane (2 mL) was added Pd₂(dba)₃ (10 mg, 0.01 mmol), Cs₂CO₃ (20 mg, 0.06 mmol) and X-Phos (6 mg, 0.01 mmol). The reaction mixture was heated to 95 °C and then stirred for 3.5 h under N_{2.} After cooled to rt, the mixture was filtered, the filtrate was then suspended in 20 mL of water, extracted with ethyl acetate, the organic layer was dried over magnesium sulfate, filtered, and concentrated in vacuum. The residue was purified by column chromatography on silica gel (DCM: MeOH = 10: 1) to give the **53m** (15 mg, 82.2%). LC-MS (ESI): m/z =507.3 [M+H] ⁺

### Step 12: (R,1³E,1⁴E,3⁴E)-3²,7-dimethyl-1⁷-(methylamino)-3²H-5-oxa-2,8-diaza-1(5,3)-pyrazolo[1,5-a]pyrimidina-3(4,6)-indazolacyclononaphan-9-one

To a solution of **53m** (15 mg, 0.03 mmol) in MeOH (1 mL) was added HCl /dioxane (1 mL), The mixture was stirred at rt for overnight and then was concentrated under reduced pressure, the residue was poured into crashed ice, and then K₂CO₃ was added until PH>10, extracted with ethyl acetate, the organic layer was dried over magnesium sulfate, filtered, and concentrated in vacuum. The residue was purified by column chromatography on silica gel (DCM: MeOH = 10:1) to give the product (5 mg, 41%). LC-MS (ESI): m/z =407.3 [M+H] ⁺

### Example 54:

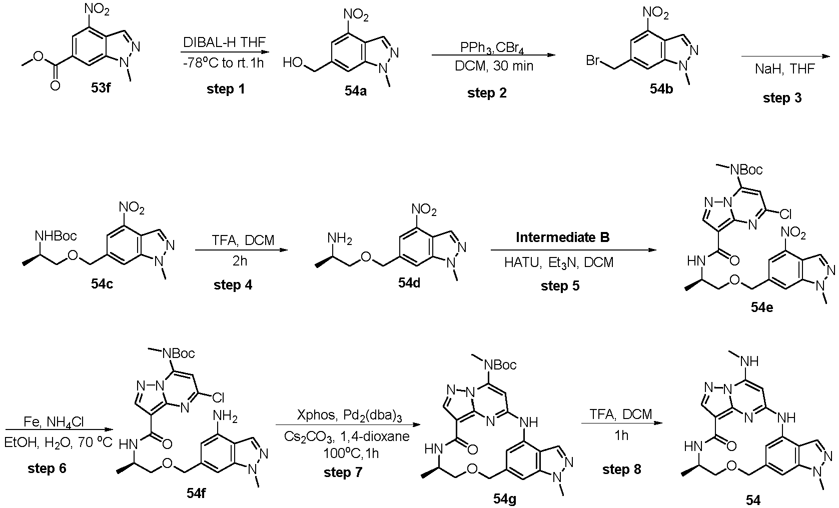

### Step 1: (1-methyl-4-nitro-1H-indazol-6-yl)methanol (54a)

A solution of methyl **53f** (1.5 g, 6.4 mmol) in anhydrous tetrahydrofuran (40 mL) was cooled to -78°C, under a nitrogen atmosphere. 1 M Diisobutylaluminum hydride in tetrahydrofuran (13 mL) was then added dropwise and the reaction stirred at -78°C for 30 min, then warmed to room temperature and stirred for 1h After this time, the reaction was carefully treated with 10% NH₄Cl (20 mL) maintaining the temperature below 25 °C. After the addition was complete, the layers were separated and the aqueous phase was extracted with methylene chloride and the combined organic layers were dried over sodium sulfate. The drying agent was removed by filtration and the filtrate was concentrated under reduced pressure to afford **54a** as a yellow solid: which was used in the next step without further purification.

### Step 2: 6-(bromomethyl)-1-methyl-4-nitro-1H-indazole (54b)

To a solution of **54a** (1.32 g, 6.4mmol) and tetrabromomethane (3.18 g, 9.6mmol) in DCM (20 ml) was added triphenylphosphine (2.52 g, 9.6 mmol) at 0°C and the mixture was stirred at the same temperature for 1 hour. The mixture was evaporated under reduced pressure. The residue was purified by silica gel column chromatography to give **54b** (1.2g, 69%). ¹H NMR (400 MHz, CDCl₃) δ 8.58 (s, 1H), 8.18 (s, 1H), 7.77 (s, 1H), 4.68 (s, 2H), 4.17 (s, 3H).

### Step 3: (R)-tert-butyl (1-((1-methyl-4-nitro-1H-indazol-6-yl)methoxy)propan-2-yl)carbamate (54c)

To a solution of **54b** (0.53 g, 3 mmol) in THF (20 mL) was added NaH (0.26 g, 60%, 2.2 equiv, 6.6 mmol) at 0 °C. After 20 min, **50b** (0.68 g, 2.5 mmol) was added, then the reaction mixture was warmed to rt. After being stirred at room temperature for 2 h, the reaction mixture was poured into water and then the product was extracted with EA (2×50 mL), dried over Na₂SO₄, and concentrated in vacuo. The residue was purified by flash chromatography to afford **54c** (0.63 g, 69%) as a yellow solid

### Step 4: (R)-1-((1-methyl-4-nitro-1H-indazol-6-yl)methoxy)propan-2-amine (54d)

A solution of **54c** (0.63 g, 1.7 mmol) in DCM (20mL) was added TFA (6 mL)) and stirred at room temperature for 2h. Solvent was evaporated, and the crude product was partitioned between water (30 mL) and DCM (50 mL). The aqueous layer was basified with NaHCO₃ and extracted with DCM (40 mL). Combined organic layers were washed with brine, dried over sodium sulfate, filtered, and evaporated to give **54d** which was used in the next step without further purification.

### Step 5: (R)-tert-butyl(5-chloro-3-((1-((1-methyl-4-nitro-1H-indazol-6-yl)methoxy)propan-2-yl)carbamoyl)pyrazolo[1,5-a]pyrimidin-7-yl)(methyl)carbamate (54e)

To a solution of **54d** (0.45 g, 1.7 mmol) and **Intermediate B** (0.53 g, 1.7 mmol) in DCM (20 mL) were successively added Et₃N (0.3 g, 3 mmol) and HATU (0.76 g, 2 mmol) The reaction mixture was stirred at room temperature for 0.5 h. Then the mixture was diluted with water (30 mL) and extracted with DCM (40 mL×2). The combined organic layers were then washed with brine (50 mL), dried over Na₂SO₄, filtered, and concentrated in vacuo. The residue was purified by flash chromatography to afford the title **54e** (0.82 g, 84%) as a yellow solid. LC-MS (ESI): m/z =573.3 [M+H]⁺.

### Step 6: (R)-tert-butyl (3-((1-((4-amino-1-methyl-1H-indazol-6-yl)methoxy)propan-2-yl)carbamoyl)-5-chloropyrazolo[1,5-a]pyrimidin-7-yl)(methyl)carbamate (54f)

To a solution of **54e** (0.82 g, 1.43 mmol) in EtOH (20 mL) and water (5 mL) was successively added Fe (0.67 g, 12 mmol), NH₄Cl (54 mg, 1 mmol), The reaction mixture was heated at 80 °C for 40min. After cooling to room temperature, Then the reaction mixture was filtered and the solvent was removed. the mixture was diluted with water (40 mL) and EtOAc (40 mL). The organic layer was separated and the aqueous layer extracted with EtOAc (40 mL). The combined organic layers were then washed with water and brine, dried over Na₂SO₄, filtered, and concentrated in vacuo. The residue was purified by flash chromatography to afford **54f** (0.53 g, 68%) as a yellow solid. LC-MS (ESI): m/z =543.3 [M+H]⁺.

### Step7: tert-butyl ((R,1³E,1⁴E)-3¹,7-dimethyl-9-oxo-3¹H-5-oxa-2,8-diaza-1(5,3)-pyrazolo[1,5-a]pyrimidina-3(4,6)-indazolacyclononaphane-1⁷-yl)(methyl)carbamate (54g)

To a solution **5f** (0.53 g, 0.98 mmol) in 1,4-dioxane (20 mL) under argon was successively added Cesium Carbonate (0.65 g, 2 mmol), XPhos (98 mg, 0.2 mmol) and Pd₂(dba)₃ (46 mg, 0.5 mmol). The reaction mixture was heated at 80 °C for 1h. After cooling to room temperature, the mixture was diluted with water (30 mL) and EtOAc (30 mL). The organic layer was separated and the aqueous layer extracted with EtOAc (40 mL×2). The combined organic layers were then washed with brine (40 mL), dried over Na₂SO₄, filtered, and concentrated in vacuo. The residue was purified by flash chromatography to afford **54g** (0.31 g, 53%) as a yellow solid. LC-MS (ESI): m/z =469.3 [M+H]⁺.

### Step 8: (R,1³E,1⁴E)-3¹,7-dimethyl-1⁷-(methylamino)-3¹H-5-oxa-2,8-diaza-1(5,3)-pyrazolo[1,5-a]pyrimidina-3(4,6)-indazolacyclononaphan-9-one

A solution of **54g** (0.31 g, 0.61 mmol) in DCM (10 mL) was added TFA (4 mL) was stirred at room temperature for 1h. Solvent was evaporated, and the crude product was partitioned between water (40 mL) and DCM (40 mL). The aqueous layer was basified with NaHCO₃ and extracted with DCM (40 mL×2). Combined organic layers were washed with brine, dried over sodium sulfate, filtered, and evaporated, purified by flash chromatography to afford **example 54** (0.13 g, 52%) as a white solid. LC-MS (ESI): m/z =407.3 [M+H]⁺. ¹H NMR (400 MHz, DMSO-d6) δ 9.80 (s, 1H), 8.40 (d, 1H), 8.27 (s, 1H), 8.24 (s, 1H), 8.16 (s, 1H), 7.97 - 7.92 m, 1H), 7.16 (s, 1H), 5.75 (s, 1H), 4.75 (d, 1H), 4.62 (d, 1H)), 4.01 (s, 3H), 3.58 - 3.53 (m, 1H), 3.44 - 3.37 (m, 1H), 2.97 (d, 3H), 1.24 (s, 1H), 1.14 (d, 3H).

### Example 55:

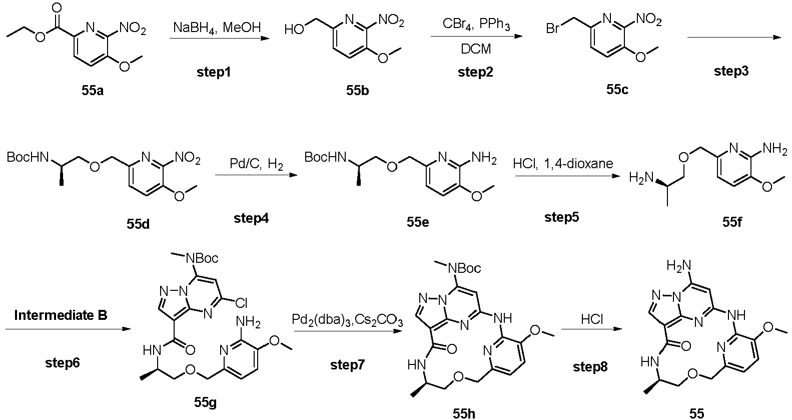

### Step 1: (5-methoxy-6-nitropyridin-2-yl) methanol (55b)

Ethyl 5-methoxy-6-nitropicolinate (1 g, 4.42 mmol) was added to MeOH (20 mL). NaBH₄ (2.0 g, 64.5 mmol) was added and then stirred at rt for 2h. The reaction mixture was then poured into water (100 mL), and was extracted with ethyl acetate. the organic layer was dried over magnesium sulfate, filtered, and concentrated in vacuo to give 55b (600mg, 74%). LC-MS (ESI): m/z =195.3 [M+H] ⁺

### Step 2: 6-(bromomethyl)-3-methoxy-2-nitropyridine (55c)

**55b** (600 mg, 3.26 mmol) was dissolved in DCM (10 mL), CBr₄(1.3 g,3.9 mmol) and PPh₃ (1.0 g,3.9 mmol) were added and then stirred at rt for 2h. The reaction mixture was then poured into water (100 mL) and extracted with ethyl acetate, the organic layer was dried over magnesium sulfate, filtered, and concentrated in vacuum. The residue was purified by column chromatography on silica gel (PE: EA = 5:1) to give 55c (350 mg, 43.7%). LC-MS (ESI): m/z =248.2 [M+H] ⁺

### Step 3: tert-butyl (R)-(1-((5-methoxy-6-nitropyridin-2-yl)methoxy)propan-2-yl)carbamate (55d)

**50b** (200 mg, 1.15 mmol) was added to THF (20 mL) under N₂ ball, NaH (100 mg, 2.5 mmol) was added at 0 °C, the suspension was stirred at rt for 0.5h, **55c** (250mg,1.00mmol) was added and then the mixture was stirred at rt for 4h, The reaction mixture was then poured into water (100 mL) and extracted with ethyl acetate, The organic layer was dried over magnesium sulfate, filtered, and concentrated in vacuum. The residue was purified by column chromatography on silica gel (PE: EA = 3:1) to give **55d** (300 mg, 64.6%). LC-MS (ESI): m/z =342.2 [M+H] ⁺

### Step 4: tert-butyl (R)-(1-((6-amino-5-methoxypyridin-2-yl)methoxy) propan-2-yl) carbamate (55e)

**55d** (250 mg, 0.73 mmol) and Pd/C (30 mg) was added to MeOH (10 mL), the mixture was stirred under H₂ ball for overnight, the suspension was diluted with dichloromethane and filtered through Celite. The solvent was removed to give a brown residue which was purified by column chromatography on silica gel (n-hexane: ethyl acetate = 3:1) to give **55e** (200 mg, 88.3%). LC-MS (ESI): m/z =312.3 [M+H] ⁺

### Step 5: (R)-5-((2-aminopropoxy) methyl)-2-methoxyaniline (55f)

To a solution of **55e** (200 mg, 1.55 mmol) in THF (10 mL) was added HCl /dioxane (4M, 5 mL), The mixture was stirred at rt for overnight and then was concentrated under reduced pressure, the residue was directly used for the next step without purification. LC-MS (ESI): m/z =212.2 [M+H] ⁺

### Step 6: tert-butyl (R)-(3-((1-((6-amino-5-methoxypyridin-2-yl)methoxy)propan-2-yl)carbamoyl)-5-chloropyrazolo[1,5-a]pyrimidin-7-yl)(methyl)carbamate (55g)

To a solution of **55f** (100 mg, 0.47 mmol) and **Intermediate B** (186 mg, 0.57 mmol) in DMF (3 mL) was added TEA (0.5 mL) and HATU (216 mg,0.57 mmol) at room temperature. The reaction mixture was stirred for overnight and then poured into crashed ice, and was extracted with ethyl acetate. The organic layer was dried over magnesium sulfate, filtered, and concentrated in vacuum. The residue was purified column chroma*tograp*hy on silica gel (DCM: MeOH = 20:1) to **55g** (100 mg, 41%). LC-MS (ESI): m/z =520.3 [M+H] ⁺.

### Step7: tert-butyl ((R,1³E,1⁴E)-3³-methoxy-7-methyl-9-oxo-5-oxa-2,8-diaza-1(5,3)-pyrazolo[1,5-a]pyrimidina-3(2,6)-pyridinacyclononaphane-17-yl)(methyl)carbamate (55h)

To a solution of **55g** (100 mg, 0.19 mmol) in 1,4-dioxane (30 mL) was added Pd₂(dba)₃ (30 mg, 0.03 mmol), Cs₂CO₃(200 mg, 0.61mmol) and X-Phos (20 mg, 0.03 mmol). The reaction mixture was heated to 95 °C and then stirred for 3.5 h under N_{2.} After cooled to rt, the mixture was filtered, the filtrate was then suspended in 20 mL of water, extracted with ethyl acetate, the organic layer was dried over magnesium sulfate, filtered, and concentrated in vacuum. The residue was purified by column chromatography on silica gel (DCM: MeOH = 10: 1) to give the **55h** (80 mg, 84.2%). LC-MS (ESI): m/z =484.3 [M+H] ⁺

### Step 8: (R,1³E,1⁴E)-33-methoxy-7-methyl-1⁷-(methylamino)-5-oxa-2,8-diaza-1(5,3)-pyrazolo[1,5-a]pyrimidina-3(2,6)-pyridinacyclononaphan-9-one

To a solution of **55h** (50 mg, 0.10 mmol) in MeOH (2 mL) was added HCl /dioxane (2M, 5 mL), The mixture was stirred at rt for overnight and then was concentrated under reduced pressure, the residue was poured into crashed ice, and then K₂CO₃ was added until PH>10, extracted with ethyl acetate, the organic layer was dried over magnesium sulfate, filtered, and concentrated in vacuum. The residue was purified by column chromatography on silica gel (DCM: MeOH = 10: 1) to give the **Example 55** (20 mg, 52.2%). ¹H NMR (400 MHz, CDCl₃) *δ* 8.73 (d, 1H), 8.36 (s, 1H), 8.18 (d, 1H), 7.78 (s, 1H), 7.37(d, 1H), 6.95 (d, 1H), 6.80 (d, 1H), 6.17 (s, 1H), 4.04 - 4.00 (m, 1H), 3.86 (s, 3H), 3.74 (d, 1H), 3.12 (d, 1H), 2.87 (d, 3H), 2.46 - 2.34 (m, 1H), 2.36 (s, 1H), 1.96 - 1.92 (m, 1H),1.02 (d, 3H). LC-MS (ESI): m/z =384.3 [M+H] ⁺.

### Example 56:

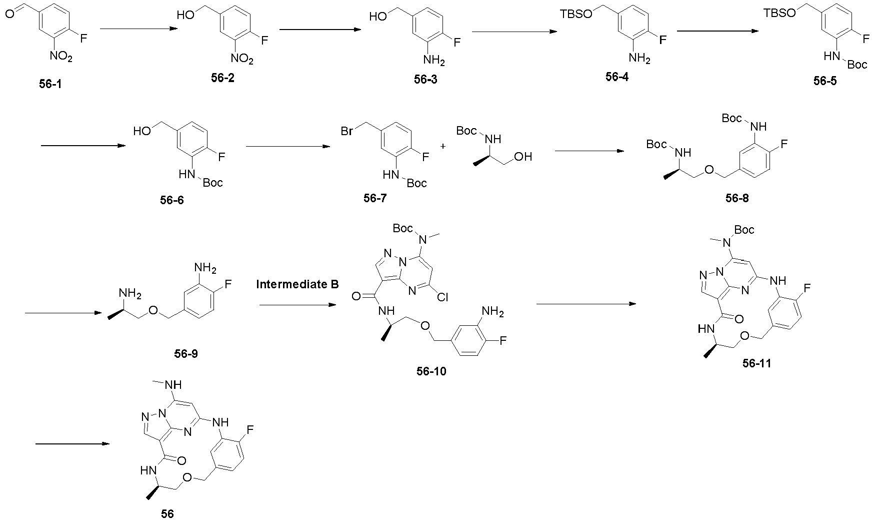

### Step1 : (4-fluoro-3-nitro-phenyl)methanol (56-2)

Sodium borohydride (1.9 g, 35.5 mmol) was added portionwise to a stirring solution of 4-fluoro-3-nitro-benzaldehyde **(56-1)** (3.0 g, 17.75 mmol) in methanol (100 ml) at 0°C. After 30 min of stirring at r.t., the methanol was removed in vacuo. The residue was treated with cold water and extracted with dichloromethane. The combined organic layer was washed with brine, dried (Na₂SO₄) and then evaporated in vacuo to give the title compound **(56-2)** as a crude solid (2.5 g, 82%). LC-MS (ESI): m/z =172.1 [M+H]+

### Step 2 : (3-amino-4-fluoro-phenyl)methanol (56-3)

(4-fluoro-3-nitro-phenyl)methanol **(56-2)** (1.0 g, 5.84 mmol) was dissolved in ethanol (9 mL) and H₂O (3 mL), Fe powder (3.3 g, 58.4 mmol) and NH₄Cl(4.06 g, 58.4 mmol) were added to solution, then the reaction mixture heated to 85 °C for 3 h, After cooling to room temperature, reaction filtered, filtrate was removed in vacuo. The residue was purified by flash chromatography to afford the title compound **(56-3)**(0.7 g, 80%) as a white solid. LC-MS (ESI): m/z =142.2 [M+H]+

### Step 3 : 5-[[tert-butyl(dimethyl)silyl]oxymethyl]-2-fluoro-aniline (56-4)

To a solution of (3-amino-4-fluoro-phenyl)methanol **(56-3)** (1.5 g, 10.6 mmol) in DCM was added TBSCl (2.4 g, 15.9 mmol) and imidazole (1.22 g, 18.0 mmol) at 0 °C, the mixture was stirred at r.t. overnight, the mixture treated with cold water and extracted with ethyl acetate. The combined organic layer was washed with brine, dried (Na₂SO₄) and then the residue was purified by flash chromatography to afford the title compound **(56-4)**(2.24 g, 83%). LC-MS (ESI): m/z =256.2 [M+H]+

### Step 4: tert-butyl N-[5-[[tert-butyl(dimethyl)silyl]oxymethyl]-2-fluoro-phenyl]carbamate (56-5)

To a solution of **56-4** in DCM (2.24 g, 8.78 mmol) was added Boc₂O (3.8 g, 17.56 mmol), triethylamine (2.66 g, 26.35 mmol) and DMAP (110 mg, 0.9 mmol) at 0 °C, the mixture was stirred at r.t. for 2 h, the mixture treated with cold water and extracted with ethyl acetate. The combined organic layer was washed with brine, dried (Na₂SO₄) and then the residue was purified by flash chromatography to afford the title compound **(56-5)**(2.1 g, 67%). LC-MS (ESI): m/z =356.1 [M+H]+

### Step 5: tert-butyl N-[2-fluoro-5-(hydroxymethyl)phenyl]carbamate (56-6)

To a solution of **56-5** (2.0 g, 5.63 mmol) in THF was added TBAF (2.9 g, 11.27 mmol) at 0 °C, the mixture was stirred at r.t. for 3 h, the mixture treated with cold water and extracted with ethyl acetate. The combined organic layer was washed with brine, dried (Na₂SO₄) and then the residue was purified by flash chromatography to afford the title compound **(56-6)**(1.06 g, 78%) as a brown solid.
LC-MS (ESI): m/z =242.2 [M+H]+

### Step 6: tert-butyl N-[5-(bromomethyl)-2-fluoro-phenyl]carbamate (56-7)

Carbon tetrabromide (2.2 g, 6.64 mmol) in anhydrous diethyl ether (5 mL) was added dropwise to a stirred solution of tert-butyl N-[2-fluoro-5-(hydroxymethyl)phenyl]carbamate **(56-6)** (0.8 g, 3.32 mmol) and Triphenylphosphine (1.74 g, 6.64 mmol) in anhydrous diethyl ether (15 mL). The mixture was stirred overnight before it was concentrated. chromatography with ethyl acetate in hexane (0-10%) gave the title compound **(56-7)** as pale yellow solid (0.73 g, 72%). LC-MS (ESI): m/z =304.2 [M+H]⁺

### Step 7: tert-butyl N-[(1R)-2-[[3-(tert-butoxycarbonylamino)-4-fluoro-phenyl]methoxy]-1-methylethyl]carbamate (56-8)

Potassium tert-butoxide (220 mg, 2.0 mmol) was added to a stirred solution of tert-butyl N-[(1R)-2-hydroxy-1-methyl-ethyl] carbamate (350 mg, 2.0 mmol) and tert-butyl N-[5-(bromomethyl)-2-fluoro-phenyl]carbamate **(56-7)** (400 mg, 1.3 mmol) in THF (15 mL) at 0°C, the mixture was stirred at 75°C for 5 min under microwave. The mixture was treated with cold water and extracted with ethyl acetate. The combined organic layer was washed with brine, dried (Na₂SO₄) and then the residue was purified by flash chromatography to afford the title compound **(56-8)**(40 mg, 7.6%) as a brown solid. LC-MS (ESI): m/z =399.3 [M+1]⁺

### Step 8: 5-[[(2R)-2-aminopropoxy]methyl]-2-fluoro-aniline (56-9)

Trifluoroacetic acid (1 mL) was added to a solution of tert-butyl N-[(1R)-2-[[3-(tert-butoxycarbonylamino)-4-fluoro-phenyl]methoxy]-1-methyl-ethyl]carbamate **(56-8)** (40 mg, 0.1mmol) in DCM (3 mL), The mixture was stirred 2 h, The mixture solution was evaporated to dryness, then the title compound **(56-9)** (18 mg. 90%) was obtained as brown liquid, which was used in the next step without further purification. LC-MS (ESI): m/z =199.3 [M+H]⁺

### Step 9: tert-butyl N-[3-[[(1R)-2-[(3-amino-4-fluoro-phenyl)methoxy]-1-methyl-ethyl]carbamoyl]-5-chloro-pyrazolo[1,5-a]pyrimidin-7-yl]-N-methyl-carbamate (56-10)

5-[[(2R)-2-aminopropoxy]methyl]-2-fluoro-aniline **(56-9)** (20 mg, 0.1 mmol) was dissolved in DMF (5 mL), TCFH (42 mg, 0.15 mmol), 1-methylimidazole ( 41 mg, 0.5 mmol) and **intermediate B** ( WO2019023468 ) (33 mg, 0.1 mmol) were added to the solution in room temperature. After 1 h pf stirring at r.t., the solution mixture was diluted with EA (30 mL), washed with water (2× 30 mL) and brine (30 mL), dried with Na₂SO₄ and concentrated. The crude product was purified by flash chromatography (PE/EA = 3:1) to afford the title compound **(56-10)** (30 mg, 59%) as a white solid. LC-MS (ESI): m/z =508.2 [M+H]⁺

### Step 10: tert-butyl ((R,1³E,1⁴E)-3⁶-fluoro-7-methyl-9-oxo-5-oxa-2,8-diaza-1(5,3)-pyrazolo[1,5-a]pyrimidina-3(1,3)-benzenacyclononaphane-1⁷-yl)(methyl)carbamate (56-11)

To a solution of tert-butyl N-[3-[[(1R)-2-[(3-amino-4-fluoro-phenyl)methoxy]-1-methylethyl]carbamoyl]-5-chloro-pyrazolo[1,5-a]pyrimidin-7-yl]-N-methyl-carbamate **(56-10)** (30 mg, 0.06 mmol) in 1,4-dioxane ( 3 mL) were added Cs₂CO₃ (40 mg, 0.12 mmol) and 3rd-*t*-Bu-Xphos-Pd (5 mg). The reaction mixture was stirred at 85°C for 2 h under N₂. After cooled to room temperature, the solvent was removed, and the residue was purified by silica gel flash column chromatography to afford the product **(56-11)** (9 mg, 30%) as a white solid. LC-MS (ESI): m/z =471.3 [M+H]⁺

### Step 11: (R,1³E,1⁴E)-3⁶-fluoro-7-methyl-1⁷-(methylamino)-5-oxa-2,8-diaza-1(5,3)-pyrazolo[1,5-a]pyrimidina-3(1,3)-benzenacyclononaphan-9-one

A solution of tert-butyl ((R,1³E,1⁴E)-3⁶-fluoro-7-methyl-9-oxo-5-oxa-2,8-diaza-1(5,3)-pyrazolo[1,5-a]pyrimidina-3(1,3)-benzenacyclononaphane-1⁷-yl)(methyl)carbamate **(56-11)** (9 mg, 0.02 mmol) and trifluoroacetic acid (0.5 mL) in DCM (2 mL) was stirred at room temperature for 3 h. Solvent was evaporated, and the crude product was partitioned between water and DCM. The aqueous layer was basified with NaHCO₃ and extracted with DCM. Combined organic layers were washed with brine, dried over sodium sulfate, filtered, and evaporated, the residue was purified by silica gel flash column chromatography to afford the product **example 56** (3 mg, 40%) as a white solid. LC-MS (ESI): m/z =371.2[M+H]⁺. ¹H NMR (400 MHz, CDCl₃) δ 8.68 (d, 1H), 8.55 (s, 1H), 8.43-8.39 (m, 1H), 8.11 (s, 1H), 7.08 (dd, 1H), 6.88-6.81 (m, 1H), 6.52-6.46 (m, 1H), 5.42 (s, 1H), 4.58 (dd, 2H), 4.21 - 4.16 (m, 1H), 3.63 - 3.57 (m, 1H), 3.51 - 3.47 (m, 1H), 3.11 (d, 3H), 1.34 (d, 3H).

### Example 57

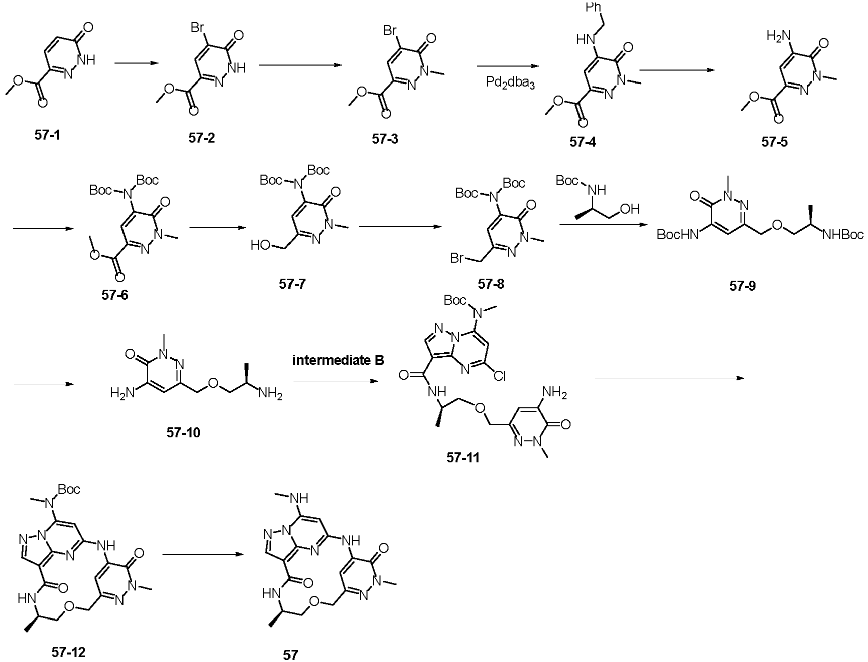

### Step 1: methyl 5-bromo-6-oxo-1H-pyridazine-3-carboxylate (57-2)

To a solution of methyl 6-oxo-1H-pyridazine-3-carboxylate **(57-1)** (15 g, 97.3 mmol) in AcOH (200 mL) was added AcOK (34 g, 346 mmol) at -10 °C, the mixture was stirred for 20 min, Bromine (34.2 g, 214 mmol) was added dropwise over 20 min, After an additional hour of stirring at 80 °C, The residue was treated with cold water and extracted with dichloromethane. The combined organic layer was washed with brine, dried (Na₂SO₄) and then evaporated in vacuo to give the title compound **(57-2)** as a crude solid (10.5 g, 46.3%).LC-MS (ESI): m/z =233.1 [M+H]⁺.

### Step 2: methyl 5-bromo-1-methyl-6-oxo-pyridazine-3-carboxylate (57-3)

To a solution of methyl 5-bromo-6-oxo-1H-pyridazine-3-carboxylate **(57-2)** (6 g, 26 mmol) in DMF (30 mL) was added Cs₂CO₃ (17 g, 51 mmol) and Iodomethane (4.4 g, 31 mmol) at 0°C., the mixture was stirred at r.t. for 4h, The residue was treated with cold water and extracted with EtOAc. The combined organic layer was washed with brine, dried (Na₂SO₄) and then evaporated in vacuo to give the title compound **(57-3)** as a crude solid (4.5 g, 71%). LC-MS (ESI): m/z =247.0 [M+H]⁺.

### Step 3: methyl 5-(benzylamino)-1-methyl-6-oxo-pyridazine-3-carboxylate (57-4)

To a solution of methyl 5-bromo-1-methyl-6-oxo-pyridazine-3-carboxylate **(57-3)** (2.5 g, 10.1 mmol) and phenylmethanamine (1.08 g, 10.1 mmol) in 1,4-dioxane (30 mL) was added Pd₂(dba)₃ (2.78 g, 3.04 mmol), Cs₂CO₃ (6.6 g, 20.2 mmol) and Xantphos (3.51 g, 6.07 mmol) under N₂ atmosphere, the mixture was stirred at 100 °C for 4h, The residue was treated with cold water and extracted with EtOAc. The combined organic layer was washed with brine, dried (Na₂SO₄) and then evaporated in vacuo to give the title compound **(57-4)** (1.6 g, 58%). LC-MS (ESI): m/z =274.0 [M+H]⁺.

### Step 4: methyl 5-amino-1-methyl-6-oxo-pyridazine-3-carboxylate (57-5)

To a solution of methyl 5-(benzylamino)-1-methyl-6-oxo-pyridazine-3-carboxylate **(57-4)** (2.2 g, 8.0 mmol) in methanol (30 mL) was added10% Pd/C (2.0 g) at r.t. the mixture was exchanged hydrogen three times and stirred at 50 °C for 6h under hydrogen atmosphere, filtered and washed with EA, then evaporated in vacuo to give the title compound **(57-5)** as a crude solid (1.3 g, 88%). LC-MS (ESI): m/z =184.2 [M+H]⁺.

### Step 5 : methyl 5-[bis(tert-butoxycarbonyl)amino]-1-methyl-6-oxo-pyridazine-3-carboxylate (57-6)

To a solution of methyl 5-amino-1-methyl-6-oxo-pyridazine-3-carboxylate **(57-5)** in DCM (1.3 g, 7.1 mmol) was added Boc₂O (3.9 g, 18 mmol) and DMAP (0.87 g, 7.1 mmol) at 0 °C, the mixture was stirred at 60 °C for 4 h, the mixture treated with cold water and extracted with ethyl acetate. The combined organic layer was washed with brine, dried (Na₂SO₄) and then the residue was purified by flash chromatography to afford the title compound **(57-6)**(2.3 g, 85%). LC-MS (ESI): m/z =384.1 [M+H]+

### Step 6: tert-butyl N-tert-butoxycarbonyl-N-[6-(hydroxymethyl)-2-methyl-3-oxo-pyridazin-4-yl]carbamate (57-7)

Sodium borohydride (0.41 g, 11.0 mmol) was added portionwise to a stirring solution of methyl 5-[bis(tert-butoxycarbonyl)amino]-1-methyl-6-oxo-pyridazine-3-carboxylate **(57-6)**(2.1 g, 5.48 mmol) in methanol (20 mL) at 0°C. After 2 hours, the methanol was removed in vacuo. The mixture was treated with cold water and extracted with dichloromethane. The combined organic layer was washed with brine, dried (Na₂SO₄) and then evaporated in vacuo, chromatography with ethyl acetate in hexane (0-10%) gave the title compound **(57-7)** as pale yellow solid (1.6 g, 82%)
LC-MS (ESI): m/z =356.3 [M+H]+

### Step 7: tert-butyl N-[6-(bromomethyl)-2-methyl-3-oxo-pyridazin-4-yl]-N-tert-butoxycarbonyl-carbamate (57-8)

Carbon tetrabromide (3.0 g, 9.0 mmol) in anhydrous DCM (30 mL) was added dropwise to a stirred solution of tert-butyl N-tert-butoxycarbonyl-N-[6-(hydroxymethyl)-2-methyl-3-oxo-pyridazin-4-yl]carbamate **(57-7)** (1.6 g, 4.5 mmol) and Triphenylphosphine (2.36 g,9.0 mmol) in DCM (15 mL). The mixture was stirred overnight before it was concentrated. chromatography with ethyl acetate in hexane (0-10%) gave the title compound **(57-8)** as pale yellow solid (1.2 g, 64%)
LC-MS (ESI): m/z =418.2 [M+H]⁺

### Step 8: tert-butyl N-[(1R)-2-[[5-(tert-butoxycarbonylamino)-1-methyl-6-oxo-pyridazin-3-yl]methoxy]-1-methyl-ethyl]carbamate (57-9)

Sodium hydride (95 mg, 3.94 mmol) was added portionwise to a stirred solution of tert-butyl N-[(1R)-2-hydroxy-1-methyl-ethyl]carbamate (0.69 g, 3.94 mmol) in THF (15 mL) at 0°C, the mixture was stirred at 0°C for 20 min. then tert-butyl N-tert-butoxycarbonyl-N-[6-(hydroxymethyl)-2-methyl-3-oxo-pyridazin-4-yl]carbamate **(57-8)** (1.1 g, 2.63 mmol) was added to the mixture at 0°C,after 4h of stirring at r.t., The mixture was treated with cold water and extracted with ethyl acetate. The combined organic layer was washed with brine, dried (Na₂SO₄) and then the residue was purified by flash chromatography to afford the title compound **(57-9)**(0.9 g, 80%) as a brown solid. LC-MS (ESI): m/z =413.1[M+1]⁺

### Step 9: 4-amino-6-[[(2R)-2-aminopropoxy]methyl]-2-methyl-pyridazin-3-one (57-10)

Trifluoroacetic acid (2 mL) was added to a solution of tert-butyl N-[(1R)-2-[[5-(tert-butoxycarbonylamino)-1-methyl-6-oxo-pyridazin-3-yl]methoxy]-1-methyl-ethyl]carbamate **(57-9)**(0.6 g, 1.5 mmol) in DCM (5 mL), The mixture was stirred 2 h, The mixture solution was evaporated to dryness, then the title compound **(57-10)** (0.28 g. 91%) was obtained as brown liquid, which was used in the next step without further purification. LC-MS (ESI): m/z =213.2 [M+H]⁺

### Step 10: tert-butyl N-[3-[[(1R)-2-[(5-amino-1-methyl-6-oxo-pyridazin-3-yl)methoxy]-1-methylethyl]carbamoyl]-5-chloro-pyrazolo[1,5-a]pyrimidin-7-yl]-N-methyl-carbamate (57-11)

4-amino-6-[[(2R)-2-aminopropoxy]methyl]-2-methyl-pyridazin-3-one **(57-10)** (200 mg, 0.94 mmol) and **Intermediate B** (308 mg, 0.94 mmol) was dissolved in DMF (5 mL), N,N,N',N'-Tetramethylchloroformamidinium hexafluorophosphate (397 mg, 1.41 mmol) and 1-methylimidazole ( 387 mg, 4.71 mmol) were added to the solution in room temperature. After 1 h of stirring at r.t., the solution mixture was diluted with EA (30 mL), washed with water (2× 30 mL) and brine (30 mL), dried with Na₂SO₄ and concentrated. The crude product was purified by flash chromatography (PE/EA = 3:1) to afford the title compound **(57-11)** (210 mg, 43%) as a white solid.
LC-MS (ESI): m/z =521.1 [M+H]⁺

### Step 11: tert-butyl ((R,1³E,1⁴E,3⁴E)-3¹,7-dimethyl-3⁶,9-dioxo-3¹,3⁶-dihydro-5-oxa-2,8-diaza-1(5,3)-pyrazolo[1,5-a]pyrimidina-3(5,3)-pyridazinacyclononaphane-1⁷-yl)(methyl)carbamate (57-12)

To a solution of tert-butyl N-[3-[[(1R)-2-[(5-amino-1-methyl-6-oxo-pyridazin-3-yl)methoxy]-1-methyl-ethyl]carbamoyl]-5-chloro-pyrazolo[1,5-a]pyrimidin-7-yl]-N-methyl-carbamate **(57-11)** (200 mg, 0.38 mmol) in 1,4-dioxane (20 mL) were added Cs₂CO₃ (250 mg, 0.77 mmol) and 3rd-*t*-Bu-Xphos-Pd (20 mg). The reaction mixture was stirred at 85°C for 2 h under N₂. After cooled to room temperature, the solvent was removed, and the residue was purified by silica gel flash column chromatography to afford the product **(57-12)**(130 mg, 70%) as a white solid.
LC-MS (ESI): m/z =485.0 [M+H]⁺

### Step 12: (R,1³E,1⁴E,3⁴E)-3¹,7-dimethyl-1⁷-(methylamino)-3¹,3⁶-dihydro-5-oxa-2,8-diaza-1(5,3)-pyrazolo[1,5-a]pyrimidina-3(5,3)-pyridazinacyclononaphane-36,9-dione

A solution of tert-butyl ((R,1³E,1⁴E,3⁴E)-3¹,7-dimethyl-3⁶,9-dioxo-3¹,3⁶-dihydro-5-oxa-2,8-diaza-1(5,3)-pyrazolo[1,5-a]pyrimidina-3(5,3)-pyridazinacyclononaphane-1⁷-yl)(methyl)carbamate **(57-12)** (120 mg, 0.25 mmol) and trifluoroacetic acid (1 mL) in DCM (4 mL) was stirred at room temperature for 3 h. Solvent was evaporated, and the crude product was partitioned between water and DCM. The aqueous layer was basified with NaHCO₃ and extracted with DCM. Combined organic layers were washed with brine, dried over sodium sulfate, filtered, and evaporated, the residue was purified by silica gel flash column chromatography to afford the product (50 mg, 53%) as a white solid. LC-MS (ESI): m/z =385.2 [M+H]⁺. ¹H NMR (400 MHz, DMSO-*d*₆) δ 9.75 (s, 1H), 8.48 (s, 1H), 8.23 (s, 1H), 8.17 - 8.07 (m, 2H), 6.30 (s, 1H), 4.45 (dd, 2H), 4.05-3.96 (m, 1H), 3.71 (s, 3H), 3.68-3.62 (m, 1H), 3.58-3.53 (m, 1H), 2.92 (d, 3H), 1.18 (d, 3H).

### Example 58:

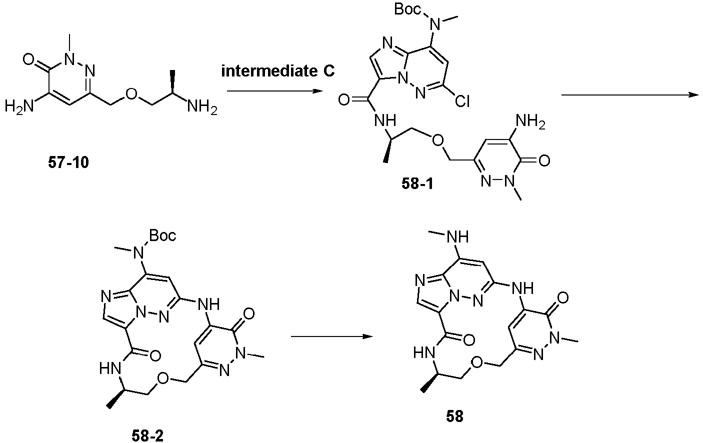

### Step 1: tert-butyl N-[3-[[(1R)-2-[(5-amino-1-methyl-6-oxo-pyridazin-3-yl)methoxy]-1-methylethyl]carbamoyl]-6-chloro-imidazo[1,2-b]pyridazin-8-yl]-N-methyl-carbamate (58-1)

4-amino-6-[[(2R)-2-aminopropoxy]methyl]-2-methyl-pyridazin-3-one (57-10)(220 mg, 1.04 mmol) and Intermediate C (340 mg, 1.04 mmol) was dissolved in DMF (5 mL), DIPEA (0.67 g, 5.18 mmol) and HATU ( 0.6 g, 1.55 mmol) were added to the solution in room temperature. After 6 h, the solution mixture was diluted with EA (30 mL), washed with water (2× 30 mL) and brine (30 mL), dried with Na₂SO₄ and concentrated. The crude product was purified by flash chromatography (PE/EA = 3:1) to afford the title compound (58-1) (0.31 g, 57.4%) as a white solid. LC-MS (ESI): m/z =521.2 [M+H]⁺

### Step 2: tert-butyl ((1⁵E,3⁴E,7R)-3¹,7-dimethyl-3⁶,9-dioxo-3¹,3⁶-dihydro-5-oxa-2,8-diaza-1(6,3)-imidazo[1,2-b]pyridazina-3(5,3)-pyridazinacyclononaphane-1⁸-yl)(methyl)carbamate (58-2)

To a solution of tert-butyl N-[3-[[(1R)-2-[(5-amino-1-methyl-6-oxo-pyridazin-3-yl)methoxy]-1-methyl-ethyl]carbamoyl]-6-chloro-imidazo[1,2-b]pyridazin-8-yl]-N-methyl-carbamate (58-1) (220 mg, 0.42 mmol) in 1,4-dioxane (20 mL) were added Cs₂CO₃ (275 mg, 0.84 mmol) and 3rd-*t*-Bu-Xphos-Pd (30 mg). The reaction mixture was stirred at 80 °C for 2 h under N₂ atmosphere. After cooled to room temperature, the solvent was removed, and the residue was purified by silica gel flash column chromatography to afford the product (58-2)(160 mg, 78.2%) as a white solid. LC-MS (ESI): m/z =485.2 [M+H]⁺

### Step 3: (15E,34E,7R)-31,7-dimethyl-18-(methylamino)-31,36-dihydro-5-oxa-2,8-diaza-1(6,3)-imidazo[1,2-b]pyridazina-3(5,3)-pyridazinacyclononaphane-36,9-dione

A solution of tert-butyl ((1⁵E,3⁴E,7R)-3¹,7-dimethyl-3⁶,9-dioxo-3¹,3⁶-dihydro-5-oxa-2,8-diaza-1(6,3)-imidazo[1,2-b]pyridazina-3(5,3)-pyridazinacyclononaphane-1⁸-yl)(methyl)carbamate **(58-2)** (180 mg, 0.372 mmol) and p-TsOH (192 mg, 1.11 mmol) in DCM (10 mL) was stirred at 40 °C for 1 h. Solvent was evaporated, and the crude product was partitioned between water and DCM. The aqueous layer was basified with NaHCO₃ and extracted with DCM. Combined organic layers were washed with brine, dried over sodium sulfate, filtered, and evaporated, the residue was purified by silica gel flash column chromatography to afford the product **example 58** (110 mg, 77%) as a white solid. LC-MS (ESI): m/z =385.2 [M+H]⁺. ¹H NMR (400 MHz, CDCl₃) δ 9.50 (s, 1H), 8.58 (d, 1H), 8.14 (s, 1H), 7.89 (s, 1H), 7.72-7.64 (m, 1H), 6.49 (s, 1H), 4.58 (d, 1H), 4.26 (d, 1H), 4.15 - 4.03 (m, 1H), 3.71 (s, 3H), 3.64 - 3.58 (m, 1H), 3.49 - 3.41 (m, 1H), 2.89 (d, 3H), 1.13 (d, 3H).

### Example 59:

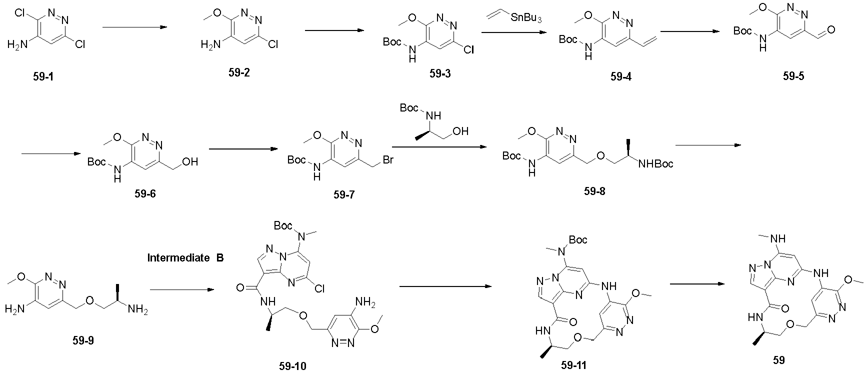

### Step 1: 6-chloro-3-methoxy-pyridazin-4-amine (59-2)

To a solution of 59-1 (5.0 g, 30.5 mmol) in DMSO (20 mL) were added LiOH(1.46 g, 61 mmol) and methanol (30 mL) at r.t., The reaction mixture was stirred at 80 °C for 12 h. After cooled to room temperature, the mixture was treated with cold water and extracted with ethyl acetate. The combined organic layer was washed with brine, dried (Na₂SO₄) and then the residue was purified by flash chromatography to afford the title compound (59-2)(4.1 g, 85%) as a brown solid. LC-MS (ESI): m/z =160.1 [M+H]⁺

### Step 2: tert-butyl N-(6-chloro-3-methoxy-pyridazin-4-yl)carbamate (59-3)

To a solution of 6-chloro-3-methoxy-pyridazin-4-amine **(59-2)** (4.0 g, 25.16 mmol) in DCM was added Boc₂O (11.0 g, 50.31 mmol), triethylamine (7.6 g, 75.5 mmol) and DMAP (307 mg, 2.52 mmol) at 0 °C, the mixture was stirred at r.t. for 3 h, the mixture treated with cold water and extracted with DCM. The combined organic layer was washed with brine, dried (Na₂SO₄) and then the residue was purified by flash chromatography to afford the title compound (59-3)(3.71 g, 57%) as a brown solid. LC-MS (ESI): m/z =260.2 [M+H]+

### Step 3: tert-butyl N-(3-methoxy-6-vinyl-pyridazin-4-yl)carbamate (59-4)

To a solution of tert-butyl N-(6-chloro-3-methoxy-pyridazin-4-yl)carbamate **(59-3)** (3.5 g, 13.51 mmol) and tributyl(vinyl)Tin (8.57 g, 27.03 mmol) in DMF (30 mL) was added CuCl (4.01 g, 40.53 mmol), Pd(PPh₃)₄ (1.56 g, 1.35 mmol), at r.t. under N₂ atmosphere, the mixture was stirred at 80 °C for 4h, then treated with cold water and extracted with EtOAc. The combined organic layer was washed with brine, dried (Na₂SO₄) and then the residue was purified by flash chromatography to afford the title compound (59-4)(2.58 g, 76%). LC-MS (ESI): m/z =252.1 [M+H]⁺

### Step 4: tert-butyl N-(6-formyl-3-methoxy-pyridazin-4-yl)carbamate (59-5)

To a solution of tert-butyl N-(3-methoxy-6-vinyl-pyridazin-4-yl)carbamate **(59-4)** (2.5 g, 10 mmol) in DCM (30 mL) was added RuCl₃ (225 mg, 1 mmol) and 4-methylmorpholine N-oxide (3.51 g, 30 mmol) at 0 °C, warmed to r.t. and stirred for 1h, then treated with cold water and extracted with EtOAc. The combined organic layer was washed with brine, dried (Na₂SO₄) and then the residue was purified by flash chromatography to afford the title compound (59-5)(1.87 g, 74%). LC-MS (ESI): m/z =254.1 [M+H]⁺

### Step 5: tert-butyl N-[6-(hydroxymethyl)-3-methoxy-pyridazin-4-yl]carbamate (59-6)

Sodium borohydride (537 mg, 14.2 mmol) was added portionwise to a stirring solution of tert-butyl N-(6-formyl-3-methoxy-pyridazin-4-yl)carbamate (59-5) (1.8 g,7.1 mmol) in THF (10 mL) at 0°C. After 2 hours of stirring at r.t., the THF was removed in vacuo. The residue was treated with cold water and extracted with dichloromethane. The combined organic layer was washed with brine, dried (Na₂SO₄) and then evaporated in vacuo to give the title compound (59-6) as a crude solid (1.7 g, 94%). LC-MS (ESI): m/z =256.1 [M+H]⁺

### Step 6 : tert-butyl N-[6-(bromomethyl)-3-methoxy-pyridazin-4-yl]carbamate (59-7)

Carbon tetrabromide (4.42 g,13.34 mmol) in anhydrous diethyl ether (5 mL) was added dropwise to a stirred solution of tert-butyl N-[6-(hydroxymethyl)-3-methoxy-pyridazin-4-yl]carbamate **(59-6)** (1.7 g,6.67 mmol) and Triphenylphosphine (3.50 g,13.34 mmol) in anhydrous diethyl ether (15 mL). The mixture was stirred overnight before it was concentrated. chromatography with ethyl acetate in hexane (0-10%) gave the title compound **(59-7)** as pale yellow solid (1.08 g, 51%). LC-MS (ESI): m/z =318.1 [M+H]⁺

### Step 7 : tert-butyl N-[(1R)-2-[[5-(tert-butoxycarbonylamino)-6-methoxy-pyridazin-3-yl]methoxy]-1-methyl-ethyl]carbamate (59-8)

Sodium hydride (273 mg, 6.82 mmol, 60%) was added portionwise to a stirred solution of tert-butyl N-(2-hydroxy-1-methyl-ethyl)carbamate (0.9 g, 5.11 mmol) in THF (15 mL) at 0°C, the mixture was stirred at 0°C for 10 min. then tert-butyl N-[6-(bromomethyl)-3-methoxy-pyridazin-4-yl]carbamate **(59-7)**(1.08 g, 3.41 mmol) was added to the mixture at 0°C,after 30 min, The mixture was treated with cold water and extracted with ethyl acetate. The combined organic layer was washed with brine, dried (Na₂SO₄) and then the residue was purified by flash chromatography to afford the title compound (59-8)(930 mg, 66%) as a brown solid. LC-MS (ESI): m/z =413.2 [M+1]⁺

### Step 8: 6-[[(2R)-2-aminopropoxy]methyl]-3-methoxy-pyridazin-4-amine (59-9)

Trifluoroacetic acid (2 mL) was added to a solution of tert-butyl N-[(1R)-2-[[5-(tert-butoxycarbonylamino)-6-methoxy-pyridazin-3-yl]methoxy]-1-methyl-ethyl]carbamate (59-8) (200 mg, 0.48 mmol) in DCM (8 mL), The mixture was stirred 2 h, The mixture solution was evaporated to dryness, then the title compound (59-9) (95 mg. 92%) was obtained as brown liquid, which was used in the next step without further purification. LC-MS (ESI): m/z =213.2 [M+H]⁺

### Step 9: tert-butyl N-[3-[[(1R)-2-[(5-amino-6-methoxy-pyridazin-3-yl)methoxy]-1-methylethyl]carbamoyl]-5-chloro-pyrazolo[1,5-a]pyrimidin-7-yl]-N-methyl-carbamate (59-10)

6-[[(2R)-2-aminopropoxy]methyl]-3-methoxy-pyridazin-4-amine (59-9) (95 mg, 0.45 mmol) and **Intermediate B** (145 mg, 0.45 mmol) was dissolved in DMF (5 mL), HATU (256 mg, 0.67 mmol) and DIPEA ( 116 mg, 0.90 mmol) were added to the solution in room temperature. After 3 h, the solution mixture was diluted with EA (30 mL), washed with water (2× 30 mL) and brine (30 mL), dried with Na₂SO₄ and concentrated. The crude product was purified by flash chromatography (PE/EA = 3:1) to afford the title compound (59-10) (108 mg, 46%) as a white solid. LC-MS (ESI): m/z =521.0 [M+H]+.

### Step 10: tert-butyl ((R,1³E,1⁴E)-3⁶-methoxy-7-methyl-9-oxo-5-oxa-2,8-diaza-1(5,3)-pyrazolo[1,5-a]pyrimidina-3(5,3)-pyridazinacyclononaphane-1⁷-yl)(methyl)carbamate (59-11)

To a solution of tert-butyl N-[3-[[(1R)-2-[(5-amino-6-methoxy-pyridazin-3-yl)methoxy]-1-methyl-ethyl]carbamoyl]-5-chloro-pyrazolo[1,5-a]pyrimidin-7-yl]-N-methyl-carbamate (59-10) (108 mg, 0.21 mmol) in 1,4-dioxane (10 mL) were added Cs₂CO₃ (136 mg, 0.42 mmol) and 3rd-*t*-Bu-Xphos-Pd (40 mg). The reaction mixture was stirred at 80 °C for 2 h under N₂. After cooled to room temperature, the solvent was removed, and the residue was purified by silica gel flash column chromatography to afford the product (59-11)(22 mg, 21.7%) as a white solid. LC-MS (ESI): m/z =485.1 [M+H]⁺

### Step 11: (R,1³E,1⁴E)-3⁶-methoxy-7-methyl-1⁷-(methylamino)-5-oxa-2,8-diaza-1(5,3)-pyrazolo[1,5-a]pyrimidina-3(5,3)-pyridazinacyclononaphan-9-one

A solution of tert-butyl ((R,1³E,1⁴E)-3⁶-methoxy-7-methyl-9-oxo-5-oxa-2,8-diaza-1(5,3)-pyrazolo[1,5-a]pyrimidina-3(5,3)-pyridazinacyclononaphane-1⁷-yl)(methyl)carbamate **(59-11)** (14 mg, 0.044 mmol) and trifluoroacetic acid (0.25 mL) in DCM (2 mL) was stirred at room temperature for 3 h. Solvent was evaporated, and the crude product was partitioned between water and DCM. The aqueous layer was basified with NaHCO₃ and extracted with DCM. Combined organic layers were washed with brine, dried over sodium sulfate, filtered, and evaporated, the residue was purified by silica gel flash column chromatography to afford the product **example 59** (5 mg, 50%) as a white solid. LC-MS (ESI): m/z =385.1 [M+H]⁺. ¹H NMR (400 MHz, CDCl₃) δ 8.85 (s, 1H), 8.38 (s, 1H), 8.09 (s, 1H), 7.22 (s, 1H), 6.39 (s, 1H), 5.51 (s, 1H), 4.80 (dd, 2H), 4.24 (s, 3H), 4.22-4.18 (m, 1H), 3.73-3.68 (m, 1H), 3.62 - 3.55 (m, 1H), 3.13 (d, 3H), 1.35 (d, 3H).

### Example 60:

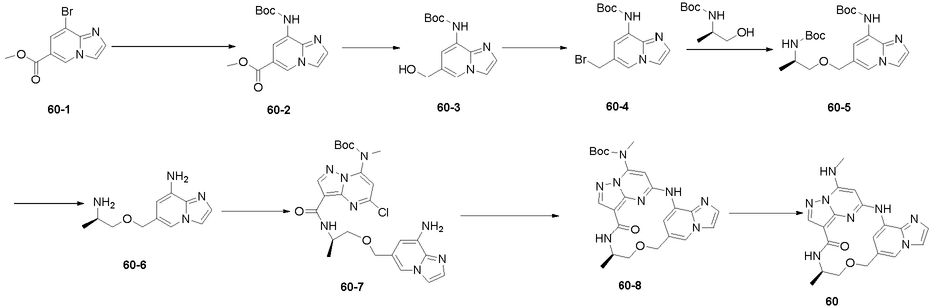

### Step1 : methyl 8-(tert-butoxycarbonylamino)imidazo[1,2-a]pyridine-6-carboxylate (60-2)

To a solution of (60-1) (2.0 g, 7.84 mmol) and tert-butyl carbamate (1.41 g, 11.76 mmol) in 1,4-dioxane (100 mL) were added t-BuONa(1.2 g, 11.76 mmol), Pd₂(dba)₃ (1.44 g, 1.57 mmol) and Dpephos (1.7 g, 3.14 mmol) under N₂ atmosphere. The reaction mixture was stirred at 80 °C for 2 h under N₂. After cooled to room temperature, the solvent was removed, and the residue was purified by silica gel flash column chromatography to afford the product **(60-2)(450** mg, 20%) as a white solid. LC-MS (ESI): m/z =292.3 [M+H]⁺

### Step2: tert-butyl N-[6-(hydroxymethyl)imidazo[1,2-a]pyridin-8-yl]carbamate (60-3)

Sodium borohydride (176 mg, 4.64 mmol) was added portion-wise to a stirring solution of methyl 8-(tert-butoxycarbonylamino)imidazo[1,2-a]pyridine-6-carboxylate **(60-2)(0.54** g, 1.86 mmol) in methanol (10 mL) at r.t. then the mixture warmed to 50 °C and stirred for 1 hours, the methanol was removed in vacuo. The residue was treated with cold water and extracted with dichloromethane. The combined organic layer was washed with brine, dried (Na₂SO₄) and then evaporated in vacuo to give the title compound **(60-3)** as a crude solid (0.27 g, 55%). LC-MS (ESI): m/z =264.3 [M+H]⁺

### Step3: tert-butyl N-[6-(bromomethyl)imidazo[1,2-a]pyridin-8-yl]carbamate (60-4)

Carbon tetrabromide (0.68 g, 2.06 mmol) in anhydrous diethyl ether (5 mL) was added dropwise to a stirred solution of tert-butyl N-[6-(hydroxymethyl)imidazo[1,2-a]pyridin-8-yl]carbamate (60-3) (0.27 g, 1.03 mmol) and triphephosphine (0.54 g, 2.06 mmol) in anhydrous diethyl ether (15 mL). The mixture was stirred overnight before it was concentrated. chromatography with ethyl acetate in hexane (0-10%) gave the title compound **(60-4)** as pale yellow solid (0.18 g, 54%). LC-MS (ESI): m/z =327.2 [M+H]⁺

### Step4: tert-butyl N-[(1R)-2-[[8-(tert-butoxycarbonylamino)imidazo[1,2-a]pyridin-6-yl]methoxy]-1-methyl-ethyl]carbamate (60-5)

Sodium hydride (44 mg, 1.1 mmol) was added portion-wise to a stirred solution of tert-butyl N-(2-hydroxy-1-methyl-ethyl)carbamate (0.19 g, 1.1 mmol) in THF (15 mL) at 0°C, the mixture was stirred at 0°C for 10 min. then tert-butyl N-[6-(bromomethyl)imidazo[1,2-a]pyridin-8-yl]carbamate **(60-4**)(0.18 g, 0.55 mmol) was added to the mixture at 0°C, warmed to r.t. and stirred for 1h, quenched by cold water, and extracted with ethyl acetate. The combined organic layer was washed with brine, dried (Na₂SO₄) and then the residue was purified by flash chromatography to afford the title compound **(60-5**)(0.2 g, 86%) as a brown solid. LC-MS (ESI): m/z =421.5 [M+H]⁺

### Step5: 6-[[(2R)-2-aminopropoxy]methyl]imidazo[1,2-a]pyridin-8-amine (60-6)

Trifluoroacetic acid (1 mL) was added to a solution of tert-butyl N-[(1R)-2-[[8-(tert-butoxycarbonylamino)imidazo[1,2-a]pyridin-6-yl]methoxy]-1-methyl-ethyl]carbamate (**60-5**)(0.2 g, 0.48 mmol) in DCM (5 mL), The mixture was stirred at r.t. for 2 h, The mixture solution was evaporated to dryness, then the title compound **(60-6)** (0.11 g. 100%) was obtained as brown liquid, which was used in the next step without further purification. LC-MS (ESI): m/z =221.3 [M+H]⁺

### step6: tert-butyl N-[3-[[(1R)-2-[(8-aminoimidazo[1,2-a]pyridin-6-yl)methoxy]-1-methylethyl]carbamoyl]-5-chloro-pyrazolo[1,5-a]pyrimidin-7-yl]-N-methyl-carbamate (60-7)

tert-butyl N-[3-[[(1R)-2-[(8-aminoimidazo[1,2-a]pyridin-6-yl)methoxy]-1-methylethyl]carbamoyl]-5-chloro-pyrazolo[1,5-a]pyrimidin-7-yl]-N-methyl-carbamate (**60-6**)(0.11 g, 0.5 mmol) and **Intermediate B** (0.32 g, 0.5 mmol) was dissolved in DMF (5 mL), N,N,N',N'-Tetramethylchloroformamidinium hexafluorophosphate (0.21 g, 0.75 mmol) and 1-methylimidazole ( 0.08 g, 1.0 mmol) were added to the solution in room temperature. After stirring for 3h, the solution mixture was diluted with EA (30 mL), washed with water (2× 10 mL) and brine (10 mL), dried with Na₂SO₄ and concentrated. The crude product was purified by flash chromatography (PE/EA = 3:1) to afford the title compound **(60-7)** (0.12 g, 45%) as a white solid. LC-MS (ESI): m/z =529.0 [M+H]⁺

### Step 7: tert-butyl methyl((R,1³E,1⁴E,3⁷E)-7-methyl-9-oxo-5-oxa-2,8-diaza-1(5,3)-pyrazolo[1,5-a]pyrimidina-3(8,6)-imidazo[1,2-a]pyridinacyclononaphane-1⁷-yl)carbamate (60-8)

To a solution of **(60-7)** (120 mg, 0.23 mmol) in 1,4-dioxane (20 mL) were added Cs₂CO₃ (150 mg, 0.46 mmol) and 3rd-*t*-Bu-Xphos-Pd (120 mg). The reaction mixture was stirred at 80°C for 2 h under N₂. After cooled to room temperature, the solvent was removed, and the residue was purified by silica gel flash column chromatography to afford the product (60-8)(80 mg, 72%) as a white solid. LC-MS (ESI): m/z =493.5 [M+H]⁺

### Step 8: (R,1³E,1⁴E,3⁷E)-7-methyl-1⁷-(methylamino)-5-oxa-2,8-diaza-1(5,3)-pyrazolo[1,5-a]pyrimidina-3(8,6)-imidazo[1,2-a]pyridinacyclononaphan-9-one

A solution of tert-butyl methyl((R,1³E,1⁴E,3⁷E)-7-methyl-9-oxo-5-oxa-2,8-diaza-1(5,3)-pyrazolo[1,5-a]pyrimidina-3(8,6)-imidazo[1,2-a]pyridinacyclononaphane-1⁷-yl)carbamate (60-8) (80mg, 0.16 mmol) and trifluoroacetic acid (0.5 mL) in DCM (4 mL) was stirred at room temperature for 2 h. Solvent was evaporated, and the crude product was partitioned between water and DCM. The aqueous layer was basified with NaHCO₃ and extracted with DCM. Combined organic layers were washed with brine, dried over sodium sulfate, filtered, and evaporated, the residue was purified by silica gel flash column chromatography to afford the product **example 60** (20 mg, 29.5%) as a white solid. ¹H NMR (400 MHz, CD₃OD) δ 8.50 (d, 1H), 8.29 (d, 1H), 8.14 (s, 1H), 7.99 (s, 1H), 7.77 (d, 1H), 7.50 (d, 1H), 4.52 (q, 2H), 4.13 - 4.03 (m, 1H), 3.59 (dd, 1H), 3.49 (dd, 1H), 2.99 (s, 3H), 1.22 (d, 3H). LC-MS (ESI): m/z =393.5 [M+H]⁺

### Example 62:

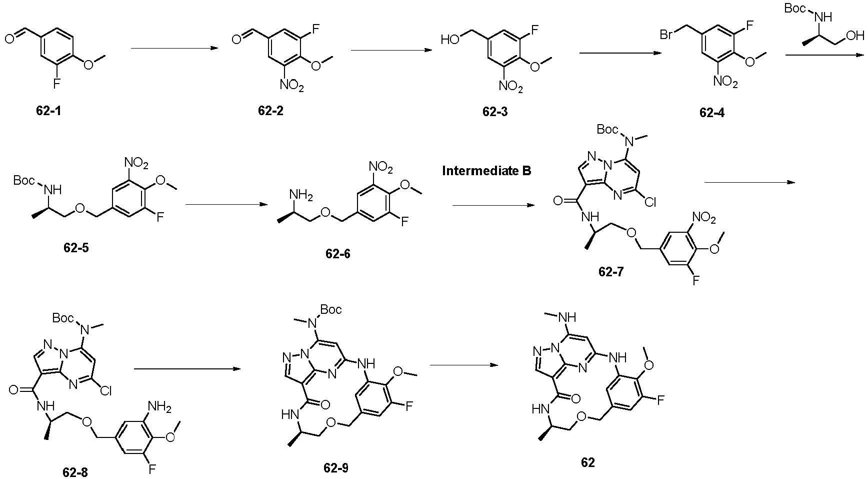

### Step 1: 3-fluoro-4-methoxy-5-nitro-benzaldehyde (62-2)

3-fluoro-4-methoxy-benzaldehyde **(62-1)** (3.6 g, 23.37 mmol ) was dissolved in concentrated sulfuric acid (30 mL) and cooled to -10°C. Concentrated nitric acid (2.5 mL) in concentrated sulfur acid (4 mL) was added dropwise over 20 min. After an additional hour of stirring at below -10°C, the mixture was poured into crushed ice. the precipitate was collected by filtration and partitioned between dichloromethane (40 mL) and saturated sodium hydrogen carbonate (30mL). The organic layer was dried (Na₂SO₄) and evaporated in vacuo to give the title compound **(62-2)(1.6** g, 34.23%) as an oil. LC-MS (ESI): m/z =200.1 [M+H]⁺.

### Step 2 : (3-fluoro-4-methoxy-5-nitro-phenyl)methanol (62-3)

To a stirring solution of 3-fluoro-4-methoxy-5-nitro-benzaldehyde **(62-2)** (1.6 g,8.0 mmol) in methanol (20 mL) was added sodium borohydride (0.38 g,10.04 mmol) portionwise at 0°C. After 2 hours, the methanol was removed in vacuo. The residue was treated with cold water and extracted with dichloromethane. The combined organic layer was washed with brine, dried (Na₂SO₄) and then evaporated in vacuo to give the title compound **(62-3)** as a crude solid(1.4 g,87.06%). LC-MS (ESI): m/z =202.1 [M+H]⁺

### Step 3 : 5-(bromomethyl)-1-fluoro-2-methoxy-3-nitro-benzene (62-4).

To a solution of (3-fluoro-4-methoxy-5-nitro-phenyl)methanol **(62-3)** (1.4 g, 6.96mmol) and triphephosphine (2.61g, 9.95 mmol) in anhydrous diethyl ether (30 mL) was added carbon tetrabromide (3.3 g, 9.95 mmol) in anhydrous diethyl ether (5 mL) dropwise. The mixture was stirred overnight before it was concentrated down to a sticky oil. Silica gel chromatography gave the title compound **(62-4)** as a pale yellow solid (1.3g, 70.97%). LC-MS (ESI): m/z =264.1 [M+H]⁺

### Step 4: tert-butylN-[(1R)-2-[(3-fluoro-4-methoxy-5-nitro-phenyl)methoxy]-1-methylethyl]carbamate (62-5)

To a stirred solution of tert-butyl N-(2-hydroxy-1-methyl-ethyl)carbamate (0.63 g, 3.61 mmol) in THF (15 mL) was added sodium hydride (144 mg, 3.61 mmol) portionwise at 0°C, the mixture was stirred at 0°C for 10 min. then 5-(bromomethyl)-1-fluoro-2-methoxy-3-nitro-benzene (62-4)(0.95 g, 3.61 mmol) was added to the mixture at 0°C, after 30 min, the mixture was quenched with cold water and extracted with ethyl acetate. The combined organic layer was washed with brine, dried (Na₂SO₄) and then the residue was purified by flash chromatography to afford the title compound (**62-5**)(0.63 g, 48.83%) as a brown solid. LC-MS (ESI): m/z =359.1 [M+H]⁺

### Step 5: (2R)-1-[(3-fluoro-4-methoxy-5-nitro-phenyl)methoxy]propan-2-amine (62-6)

Trifluoroacetic acid (1.5 mL) was added to a solution of tert-butylN-[(1R)-2-[(3-fluoro-4-methoxy-5-nitro-phenyl)methoxy]-1-methyl-ethyl]carbamate (62-5) (0.63g, 1.76 mmol) in DCM(5 mL), The mixture was stirred 2 h, The mixture solution was evaporated to dryness, then the title compound **(62-6)** (0.6 g. 91.46%) was obtained as brown liquid, which was used in the next step without further purification. LC-MS (ESI): m/z =259.2 [M+H]⁺

### Step 6: tert-butylN-[5-chloro-3-[[(1R)-2-[(3-fluoro-4-methoxy-5-nitro-phenyl)methoxy]-1-methylethyl]carbamoyl]pyrazolo[1,5-a]pyrimidin-7-yl]-N-methyl-carbamate (62-7)

(2R)-1-[(3-fluoro-4-methoxy-5-nitro-phenyl)methoxy]propan-2-amine (62-6)(0.6 g, 1.6 mmol) was dissolved in DMF (10 mL),HATU (0.91g, 2.41mmol), DIPEA(0.41g, 3.2 mmol) and intermediate **B** (0.52 g, 1.6 mmol) were added to the solution in room temperature. After 18h, the solution mixture was diluted with EA (50 mL), washed with water (2×50 mL) and brine (50 mL), dried with Na₂SO₄ and concentrated. The crude product was purified by flash chromatography to afford the title compound **(62-7)** (545 mg, 59.89%) as a white solid. LC-MS (ESI): m/z =567.2 [M+H]⁺

### Step 7: tert-butylN-[3-[[(1R)-2-[(3-amino-5-fluoro-4-methoxy-phenyl)methoxy]-1-methylethyl]carbamoyl]-5-chloro-pyrazolo[1,5-a]pyrimidin-7-yl]-N-methyl-carbamate(62-8)

tert-butylN-[5-chloro-3-[[(1R)-2-[(3-fluoro-4-methoxy-5-nitro-phenyl)methoxy]-1-methylethyl]carbamoyl]pyrazolo[1,5-a]pyrimidin-7-yl]-N-methyl-carbamate **(62-7)** (545 mg, 0.96 mmol) was dissolved in ethanol (45 mL) and H₂O (15 mL), iron powder (540 mg,9.62 mmol) and NH₄Cl (310 mg,5.77 mmol) were added to solution, then the reaction mixture heated to 85 °C for 3 h, After cooling to room temperature, reaction filtered, filtrate was removed in vacuo, The residue was purified by flash chromatography (PE/EA = 2:1) to afford the title compound **(62-8)(450** mg, 87.2%) as a white solid. LC-MS (ESI): m/z =537.1 [M+H]⁺

### Step 8: tert-butyl((7R,E)-3⁵-fluoro-3⁶-methoxy-7-methyl-9-oxo-5-oxa-2,8-diaza-1(6,3)-imidazo[1,2-b]pyridazina-3(1,3)-benzenacyclononaphane-1⁸-yl)(methyl)carbamate (62-9)

To a solution of **(62-8)** (450 mg, 0.84 mmol) in 1,4-dioxane (100 mL) were added Cs₂CO₃ (820 mg, 2.51 mmol) and 3rd-*t*-Bu-Xphos-Pd (250 mg). The reaction mixture was stirred at 80°C for 2 h under N₂. After cooled to room temperature, the solvent was removed, and the residue was purified by silica gel flash column chromatography to afford the product (62-9)(220 mg, 52.50%) as a white solid. LC-MS (ESI): m/z =501.3 [M+H]⁺

### Step 9: (7R,E)-3⁵-fluoro-3⁶-methoxy-7-methyl-1⁸-(methylamino)-5-oxa-2,8-diaza-1(6, 3)-imidazo[1, 2-b]pyridazina-3(1, 3)-benzenacyclononaphan-9-one

A solution of **(62-9)** (220 mg, 0.44 mmol) and trifluoroacetic acid (1 mL) in DCM (5 mL) was stirred at room temperature for 2 h. Solvent was evaporated, and the crude product was partitioned between water and DCM. The aqueous layer was basified with NaHCO₃ and extracted with DCM. Combined organic layers were washed with brine, dried over sodium sulfate, filtered, and evaporated, the residue was purified by silica gel flash column chromatography to afford the product (71 mg, 40.34%) as a white solid. LC-MS (ESI): m/z =401.3 [M+H]⁺. ¹H NMR (400 MHz, CDCl₃) δ 8.56 (s, 1H), 8.40 - 8.14 (m, 2H), 7.14 (s, 1H), 6.62 (d, 1H), 6.22 (d, 1H), 5.38 (s, 1H), 4.53 (dd, 2H), 4.18 (dd, 1H), 4.03 (d, 3H), 3.57 (m, 2H), 3.10 (d, 3H), 1.34 (d, 3H).

### Example A. TYK2 JH2 Domain Binding Assay

Binding constants for the compounds described herein against the JH2 domain were determined by the following protocol for a KINOMEscan^{®} assay (DiscoveRx). A fusion protein of a partial length construct of human TYK2 (JH2domain-pseudokinase) (amino acids G556 to D888 based on reference sequence NP_003322.3) and the DNA binding domain of NFkB is expressed in transiently transfected HEK293 cells. From these HEK 293 cells, extracts are prepared in M-PER extraction buffer (Pierce) in the presence of Protease Inhibitor Cocktail Complete (Roche) and Phosphatase Inhibitor Cocktail Set II (Merck) per manufacturers' instructions. The TYK2 (JH2domain-pseudokinase) fusion protein is labeled with a chimeric double-stranded DNA tag containing the NFkB binding site fused to an amplicon for qPCR readout, which is added directly to the expression extract (the final concentration of DNA-tag in the binding reaction is 0.1 nM).

Streptavidin-coated magnetic beads (Dynal M280) are treated with a biotinylated small molecule ligand for 30 minutes at room temperature to generate affinity resins the binding assays. The liganded beads are blocked with excess biotin and washed with blocking buffer (SeaBlock (Pierce), 1% BSA, 0.05% Tween 20, 1 mM DTT) to remove unbound ligand and to reduce nonspecific binding.

The binding reaction is assembled by combining 16 µl of DNA-tagged kinase extract, 3.8 µl liganded affinity beads, and 0.18 µl test compound (PBS/0.05% Tween 20/10 mM DTT/0.1% BSA/2 µg/ml sonicated salmon sperm DNA)]. Extracts are used directly in binding assays without any enzyme purification steps at a ≥10,000-fold overall stock dilution (final DNA-tagged enzyme concentration <0.1 nM). Extracts are loaded with DNA-tag and diluted into the binding reaction in a two-step process. First extracts are diluted 1:100 in 1× binding buffer (PBS/0.05% Tween 20/10 mM DTT/0.1% BSA/2 µg/ml sonicated salmon sperm DNA) containing 10 nM DNA-tag. This dilution is allowed to equilibrate at room temperature for 15 minutes and then subsequently diluted 1:100 in 1× binding buffer. Test compounds were prepared as 111× stocks in 100% DMSO. Kds were determined using an 11-point 3-fold compound dilution series with three DMSO control points. All compounds for Kd measurements are distributed by acoustic transfer (non-contact dispensing) in 100% DMSO. The compounds are then diluted directly into the assays such that the final concentration of DMSO was 0.9%. All reactions are performed in polypropylene 384-well plates. Each was a final volume of 0.02 mL. Assays are incubated with shaking for 1 hour at room temperature. Then the beads are pelleted and washed with wash buffer (1×PBS, 0.05% Tween 20) to remove displaced kinase and test compound. The washed based are resuspended in elution buffer (1×PBS, 0.05% Tween 20, 0.5 µM non-biotinylated affinity ligand) and incubated at room temperature with shaking for 30 minutes. The kinase concentration in the eluates was measured by qPCR. qPCR reactions are assembled by adding 2.5 µL of kinase eluate to 7.5 µL of qPCR master mix containing 0.15 µM amplicon primers and 0.15 µM amplicon probe. The qPCR protocol consisted of a 10 minute hot start at 95° C., followed by 35 cycles of 95° C. for 15 seconds, 60° C. for 1 minute.

Test compounds are prepared as 111× stocks in 100% DMSO. Kds were determined using an 11-point 3-fold compound dilution series with three DMSO control points. All compounds for Kd measurements are distributed by acoustic transfer (non-contact dispensing) in 100% DMSO. The compounds are then diluted directly into the assays such that the final concentration of DMSO was 0.9%. The Kds are determined using a compound top concentration of 30,000 nM. Kd measurements are performed in duplicate.

Binding constants (Kds) were calculated with a standard dose-response curve using the Hill equation: $Response = Background + \frac{\left(Signal-Background\right)}{\left(1+\left(\frac{{Kd}^{Hill Slope}}{{Dose}^{Hill Slope}}\right)\right.}$

The Hill Slope was set to -1. Curves were fitted using a non-linear least square fit with the Levenberg-Marquardt algorithm (Levenberg, K., A method for the solution of certain non-linear problems in least squares, Q. Appl. Math. 2, 164-168 (1944)).

The results are shown in table 1.

**TABLE 1**

| **Ex.** | **TYK2 (JH2 domain) binding Kd (nM)** |
|---|---|
| **1** | 0.24 |
| **2** | 0.14 |
| **3** | 0.96 |
| **4** | 0.38 |
| **6** | 0.071 |
| **7** | 0.0071 |
| **10** | 0.0023 |

### Example B: IL-12 induced pSTAT4 in human PBMC

Fresh Human PBMCs were resuspended in RPMI 1640 medium with 10% FBS. Cells were seeded in a round bottom 96-well plate at the concentration of 200,000 cells/well. A 10-point dilution series of test compound (top dose 10uM, 1:5 dilution) was added to the well using the liquid dispenser (Tecan D300e) and incubated for 1 hour at 37C. Then human IL-12 recombinant protein (R&D Systems) was added to the well at the final concentration of 10 ng/ml and incubated for 15 minutes at 37C. Cell lysates were prepared and analyzed by Phospho STAT4 (Tyr693) Kit (Meso Scale Discovery) following manufacturer's protocol.

For calculation of the inhibition rate, the relative pSTAT4 signal of each well = pSTAT4 signal of each well - the average pSTAT4 signal of baseline. 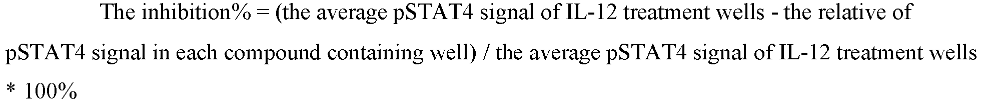 The inhibition% = (the average pSTAT4 signal of IL-12 treatment wells - the relative of pSTAT4 signal in each compound containing well) / the average pSTAT4 signal of IL-12 treatment wells * 100%

The curve was plotted as the inhibition% (y-axis) vs. compounds concentration (x-axis) and was fitted with log(inhibitor) vs. normalized response -- Variable slope by GraphPad Prism7.0.

The results are shown in table 2.

**TABLE 2**

| **Ex.** | **IL-12-induced PBMC assay, JAK2/TYK2 activity (IC50, nM)** |
|---|---|
| **1** | 280.9 |
| **2** | 12.23 |
| **3** | 1147 |
| **4** | 16.09 |

### Example C: INFα induced pSTAT3 or pSTATS in human PBMC

Fresh Human PBMCs were resuspended in RPMI 1640 medium with 10% FBS. Cells were seeded in a round bottom 96-well plate at the concentration of 200,000 cells/well. A 10-point dilution series of test compound (top dose 10uM, 1:5 dilution) was added to the well using the liquid dispenser (Tecan D300e) and incubated for 1 hour at 37C. Then human INFα recombinant protein (R&D Systems) was added to the well at the final concentration of 5000 units/ml and incubated for 15 minutes at 37C. Cell lysates were prepared and analyzed by Phospho STAT3 (Tyr705) cellular kit (Cisbio) or Phospho STAT5 (Tyr693) Kit (Meso Scale Discovery) following manufacturer's protocol.

For calculation of the inhibition rate, the relative pSTAT signal of each well = pSTAT signal of each well - the average pSTAT signal of baseline.

The inhibition% = (the average pSTAT signal of INFα treatment wells - the relative of pSTAT signal in each compound containing well) / the average pSTAT signal of INFα treatment wells * 100%

The curve was plotted as the inhibition% (y-axis) vs. compounds concentration (x-axis) and was fitted with log(inhibitor) vs. normalized response -- Variable slope by GraphPad Prism7.0. Control is BMS-986165:

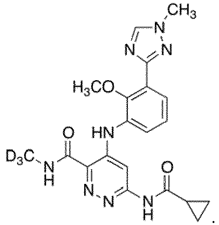

The results are shown in table 3.

**TABLE 3**

| **Ex.** | **p-STAT3 IC₅₀ (nM)** | **p-STAT5 IC₅₀ (nM)** | **Relative IC₅₀ to control** |
|---|---|---|---|
| **1** | 87.55 | | 18.6 |
| **3** | 1455 | | 207.3 |
| **5** | | 150.9 | 243.3 |
| **6** | | 26.2 | 42.2 |
| **7** | | 2.3 | 2.4 |
| **8** | | 13.9 | 15.5 |
| **9** | | 923.1 | 1024.8 |
| **10** | | 1.2 | 1.2 |
| **11** | | 436.3 | 418.3 |
| **12** | | 2.2 | 2.1 |
| **13** | | 459.8 | 482.2 |
| **14** | | 3.0 | 2.5 |
| **15** | | 5.1 | 6.8 |
| **16** | | 3.6 | 5.7 |
| **17** | | 1.0 | 1.0 |
| **18** | | 1.3 | 1.3 |
| **19** | | 8.7 | 8.0 |
| **20** | | 15.3 | 18.2 |
| **21** | | 101.7 | 59.7 |
| **22** | | 10.7 | 17.8 |
| **24** | | 0.5 | 0.8 |
| **25** | | 2.1 | 1.3 |
| **26** | | >200 | |
| **27** | | 7.2 | 4.4 |
| **28** | | 8.3 | 7.5 |
| **29** | | 3.1 | 1.9 |
| **30** | | >200 | |
| **31** | | >200 | |
| **32** | | 0.7 | 0.7 |
| **33** | | 42.8 | 41.5 |
| **34** | | 2.5 | 1.7 |
| **35** | | 1.7 | 1.8 |
| **36** | | 0.7 | 0.6 |
| **37** | | 90.1 | 68.6 |
| **38** | | >200 | |
| **40** | | 2.4 | 1.8 |
| **41** | | 3.6 | 3.5 |
| **42** | | 13.6 | 10.3 |
| **43** | | 3.8 | 3.7 |
| **44** | | 2.0 | 1.8 |
| **45** | | 1.1 | 1.0 |
| **46** | | 2.8 | 2.3 |
| **47** | | 2.4 | 2.5 |
| **48** | | 16.7 | 12.7 |
| **49** | | 1.7 | 1.32 |
| **50** | | >1000 | |
| **51** | | 209.6 | 250.2 |
| **52** | | 5.9 | 3.6 |
| **53** | | 9.4 | 8.1 |
| **54** | | 0.3 | 0.3 |
| **55** | | >200 | |
| **56** | | 28.8 | 19.1 |
| **57** | | 21.2 | 13.1 |
| **58** | | 7.7 | 13.4 |
| **59** | | 39.1 | 41.6 |
| **60** | | 3.8 | 4.0 |
| **62** | | 3.8 | 2.4 |

### Example D: JAK1 JH2 and JAK2 JH1 Domain Binding Assay

Similar to the method for TYK2 JH2 binding described above, JAK1 JH2 and JAK2 JH1 domain binding assay was performed using DiscoverX's KINOMEscan^{™}, but with change of kinase domain. These assays were performed to compare the binding selectivity of test compounds to JAK1 JH2 and JAK2 JH1 domain. The results are shown in table 4.

**TABLE 4**

| **Ex.** | **JAK1 (JH2 domain) binding Kd (nM)** | **JAK2 (JH1 domain) binding Kd (nM)** |
|---|---|---|
| **control** | 0.11 | 2100 |
| **10** | 430 | 7300 |

### Example E: GM-CSF-induced pSTAT5 and IL-2-induced pSTAT5 in human PBMC in human PBMC

Similar to the method for INFα induced pSTAT5 in human PBMC described above, these assays were performed to check if test compounds have cross-activity to JAK1. JAK2 and JAK3 pathways in human PBMC. The procedure is as described with change of stimuli to 10 ng/ml of GM-CSF or 20 ng/ml of IL-2. The data are shown in Table 5.

**TABLE 5**

| **Ex.** | **GM-CSF-induced pSTAT5 IC₅₀ (nM)** | **IL-2-induced pSTAT5 IC₅₀ (nM)** |
|---|---|---|
| **control** | 1313 | 442 |
| **10** | | >10000 |
| **19** | | 38472 |
| **24** | | 9389 |

### Example F: Pharmacokinetic Studies

The pharmacokinetics of test compounds were evaluated in male Sprague Dawley rats when administered via oral gavage and IV injection. The test compound was suspended in 0.5% methylcellulose for oral gavage and dissolved in 5% DMSO/5% Solutol/90% saline for IV injection. The animals were fasted overnight before administration. Plasma samples were collected predose and at 0.5, 1, 3, 6, 9, 12, and 24 hours postdose. The samples were analyzed by LC/MS/MS and the concentration of test compound at each timepoint was determined by linear regression. Pharmacokinetic parameters were calculated from the plasma concentrations using Pheonix WinNonlin. The PK results were summarized in the table 6.

**TABLE 6: Pharmacokinetic parameters of test compounds in Sprague Dawley rats.**

| **Ex.** | **Route of administrat ion & dose** | **C₀ or Cₘₐₓ (ng/mL )** | **AUC (h·ng·m L⁻¹)** | **T_{1/2} (h)** | **Tₘₐₓ (h)** | **F%** | **CL(mL· kg-1·min-1)** | **Vdss(L/k g)** |
|---|---|---|---|---|---|---|---|---|
| **contr ol** | Oral (5 mg/kg) | 792±26 0 | 3197±60 0 | 2.93±1.2 | 1.0±0.87 | 32.6±5 .4 | | |
| | I.V. (1 mg/kg) | 1743±2 76 | 1966±16 6 | 2.42±1.7 | | | 8.41±0.7 | 0.933±0. 12 |
| 7 | Oral (5 mg/kg) | 18.2±3. 1 | 84.9±36 | 3.97±1.1 | 0.667±0. 29 | 5.34±2 .3 | | |
| | I.V. (1 mg/kg) | 1229±1 53 | 318±30 | 0.300±0.0 16 | | | 52.4±5.3 | 0.956±0. 10 |
| **15** | Oral (5 mg/kg) | 22.2±5. 0 | 147±40 | 4.11±1.90 | 2.67±1.2 | 6.23±1 .7 | | |
| | I.V. (1 mg/kg) | 539±54 | 472±93 | 1.02±0.05 9 | | | 36.1±8.0 | 2.17±0.0 8 |

### Example G: Pharmaceutical Compositions

### Example G1: Parenteral Composition

To prepare a parenteral pharmaceutical composition suitable for administration by injection, 100 mg of a water-soluble salt of a compound described herein is dissolved in DMSO and then mixed with 10 mL of 0.9% sterile saline. The mixture is incorporated into a dosage unit form suitable for administration by injection.

### Example G2: Oral Composition

To prepare a pharmaceutical composition for oral delivery, 100 mg of a compound described herein is mixed with 750 mg of starch. The mixture is incorporated into an oral dosage unit for, such as a hard gelatin capsule, which is suitable for oral administration.

### Example G3: Sublingual (Hard Lozenge) Composition

To prepare a pharmaceutical composition for buccal delivery, such as a hard lozenge, mix 100 mg of a compound described herein, with 420 mg of powdered sugar mixed, with 1.6 mL of light corn syrup, 2.4 mL distilled water, and 0.42 mL mint extract. The mixture is gently blended and poured into a mold to form a lozenge suitable for buccal administration.

The examples and embodiments described herein are for illustrative purposes only and in some embodiments, various modifications or changes are to be included within the purview of disclosure and scope of the appended claims.

## Claims

1. A compound of Formula (II), or a pharmaceutically acceptable salt, solvate, or stereoisomer thereof: 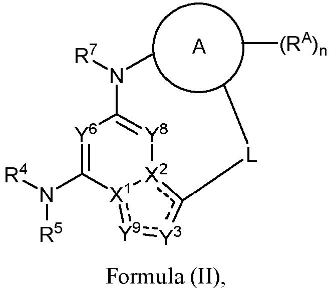 wherein:
L is 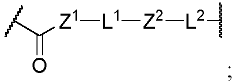 wherein Z¹ and Z² are independently -O-, -S-, or -NR^{Z}; each R^{Z} is independently hydrogen or C₁-C₆alkyl; and L¹ and L² are independently C₁-C₆ alkylene optionally substituted with one or more R^{L};
each R^{L} is independently deuterium, halogen, -CN, -OR^{b}, -SR^{b}, -S(=O)R^{a}, -S(=O)₂R^{a}, -NO₂, -NR^{c}R^{d}, - NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, - OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{L} on the same carbon are taken together to form an oxo, a cycloalkyl, or heterocycloalkyl; or two R^{L} on different carbons are taken together to form a cycloalkyl or heterocycloalkyl;
Ring A is aryl, heteroaryl, heterocycloalkyl, or cycloalkyl;
each R^{A} is independently -OR^{b}, halogen, -CN, deuterium, -SR^{b}, -S(=O)R^{a}, -S(=O)₂R^{a}, -NO₂, -NR^{c}R^{d}, - NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, - OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more R^{A1}; or two R^{A} on the same carbon are taken together to form an oxo;
each R^{A1} is independently deuterium, halogen, -CN, -OR^{b}, -SR^{b}, -S(=O)R^{a}, -S(=O)₂R^{a}, -NO₂, -NR^{c}R^{d}, - NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, - OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; or two R^{A1} on the same carbon are taken together to form an oxo
n is 0-4;
-̅ -̅ -̅ is a single bond or a double bond;
X¹ and X² are -N- or -C=; provided that one of X¹ or X² is -N- and the other is -C=;
Y⁸ is CR⁸ or N;
Y⁶ is CR⁶ or N;
Y³ is CR³ or N;
Y⁹ is CR⁹ or N;
R³, R⁶, R⁸, and R⁹ are independently hydrogen, deuterium, halogen, -CN, -OR^{b}, -SR^{b}, -S(=O)R^{a}, - S(=O)₂R^{a}, -NO₂, -NR^{c}R^{d}, -NHS(=O)₂R^{a}, -S(=O)₂NR^{c}R^{d}, -C(=O)R^{a}, -OC(=O)R^{a}, -C(=O)OR^{b}, - OC(=O)OR^{b}, -C(=O)NR^{c}R^{d}, -OC(=O)NR^{c}R^{d}, -NR^{b}C(=O)NR^{c}R^{d}, -NR^{b}C(=O)R^{a}, -NR^{b}C(=O)OR^{b}, C₁-C₆alkyl, C₁-C₆haloalkyl, C₁-C₆deuteroalkyl, C₁-C₆hydroxyalkyl, C₁-C₆aminoalkyl, C₂-C₆alkenyl, or C₂-C₆alkynyl;
R⁴ is hydrogen, C₁-C₆alkyl, C₁-C₆heteroalkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl are optionally substituted with one or more R^{4a};
each R^{4a} is independently deuterium, halogen, -CN, -OR^{b}, -NR^{c}R^{d}, -C(=O)R^{a}, -C(=O)OR^{b}, -C(=O)NR^{c}R^{d}, C₁-C₆alkyl, C₁-C₆haloalkyl, C₁-C₆deuteroalkyl, C₁-C₆hydroxyalkyl, C₁-C₆aminoalkyl, cycloalkyl, heterocycloalkyl, aryl, or heteroaryl; or two R^{4a} on the same carbon are taken together to form an oxo;
R⁵ is hydrogen, C₁-C₆alkyl, C₁-C₆haloalkyl, or C₁-C₆deuteroalkyl;
R⁷ is hydrogen, C₁-C₆alkyl, C₁-C₆haloalkyl, or C₁-C₆deuteroalkyl;
each R^{a} is independently C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OMe, - NH₂, -C(=O)Me, -C(=O)OH, -C(=O)OMe, C₁-C₆alkyl, or C₁-C₆haloalkyl;
each R^{b} is independently hydrogen, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OMe, - NH₂, -C(=O)Me, -C(=O)OH, -C(=O)OMe, C₁-C₆alkyl, or C₁-C₆haloalkyl; and
each R^{c} and R^{d} is independently hydrogen, C₁-C₆alkyl, C₁-C₆haloalkyl, C₁-C₆deuteroalkyl, C₁-C₆hydroxyalkyl, C₁-C₆aminoalkyl, C₂-C₆alkenyl, C₂-C₆alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OMe, -NH₂, -C(=O)Me, -C(=O)OH, -C(=O)OMe, C₁-C₆alkyl, or C₁-C₆haloalkyl;
or R^{c} and R^{d} are taken together with the nitrogen atom to which they are attached to form a heterocycloalkyl optionally substituted with one or more oxo, deuterium, halogen, -CN, -OH, -OMe, -NH₂, -C(=O)Me, -C(=O)OH, -C(=O)OMe, C₁-C₆alkyl, or C₁-C₆haloalkyl.

2. The compound of claim 1, or a pharmaceutically acceptable salt, stereoisomer, or solvate thereof, wherein the compound of Formula (II) is of Formula (IIa): 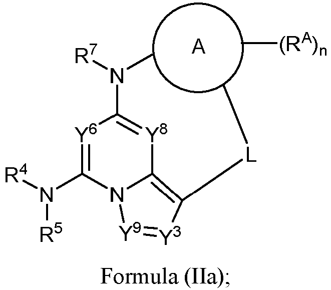 or Formula (IIb):

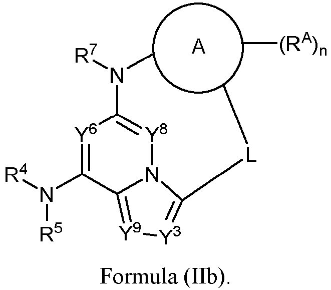

3. The compound of any of claims 1-2, or a pharmaceutically acceptable salt, stereoisomer, or solvate thereof, wherein:
Y⁹ is N.

4. The compound of any of claims 1-3, or a pharmaceutically acceptable salt, stereoisomer, or solvate thereof, wherein:
Y⁶ is CR⁶; optionally wherein R⁶ is hydrogen.

5. The compound of any of claims 1-4, or a pharmaceutically acceptable salt, stereoisomer, or solvate thereof, wherein:
(a) Y³ is CR³; optionally wherein R³ is hydrogen; and/or
(b) Y⁸ is N or CR⁸; optionally wherein R⁸ is hydrogen.

6. The compound of any of claims 1-5, or a pharmaceutically acceptable salt, stereoisomer, or solvate thereof, wherein:
(a) R⁴ is hydrogen, C₁-C₆alkyl, C₁-C₆haloalkyl, or C₁-C₆deuteroalkyl;
optionally wherein R⁴ is C₁-C₆alkyl or C₁-C₆deuteroalkyl; and/or
(b) R⁵ is hydrogen; and/or
(c) R⁷ is hydrogen or C₁-C₆alkyl.

7. The compound of any one of claims 1-6, or a pharmaceutically acceptable salt, stereoisomer, or solvate thereof wherein:
Ring A is aryl, heteroaryl, or heterocycloalkyl;
optionally wherein Ring A is:
(a) aryl; or
(b) heteroaryl.

8. The compound of any one of claims 1-7, or a pharmaceutically acceptable salt, stereoisomer, or solvate thereof wherein:
each R^{A} is independently -OR^{b}, halogen, -CN, deuterium, -NR^{c}R^{d}, -C(=O)R^{a}, C₁-C₆alkyl, C₁-C₆haloalkyl, or C₁-C₆deuteroalkyl.

9. The compound of any one of claims 1-8, or a pharmaceutically acceptable salt, stereoisomer, or solvate thereof wherein:
each R^{L} is independently deuterium, halogen, -CN, -OR^{b}, -NR^{c}R^{d}, C₁-C₆alkyl, C₁-C₆haloalkyl, or C₁-C₆deuteroalkyl; or two R^{L} on the same carbon are taken together to form an oxo.

10. The compound of any one of claims 1-8, or a pharmaceutically acceptable salt, stereoisomer, or solvate thereof wherein:
each R^{L} is independently deuterium, halogen, C₁-C₆alkyl, C₁-C₆haloalkyl, or C₁-C₆deuteroalkyl; or two R^{L} on the same carbon are taken together to form an oxo or a cycloalkyl; or two R^{L} on different carbons are taken together to form a cycloalkyl;
optionally wherein:
each R^{L} is independently deuterium, halogen, C₁-C₆alkyl, C₁-C₆haloalkyl, or C₁-C₆deuteroalkyl; or two R^{L} on the same carbon are taken together to form an oxo.

11. The compound of any one of claims 1-10, or a pharmaceutically acceptable salt, stereoisomer, or solvate thereof wherein:
L is 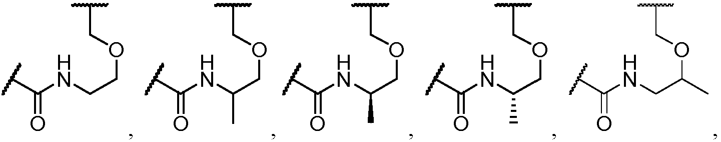 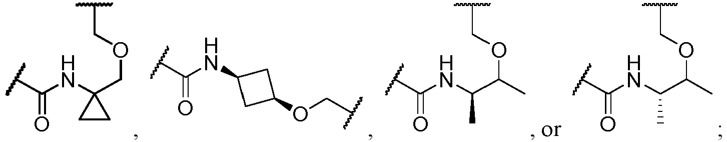
optionally wherein:
L is

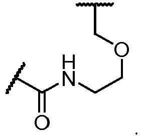

12. The compound of claim 1, or a pharmaceutically acceptable salt, stereoisomer, or solvate thereof wherein the compound is:

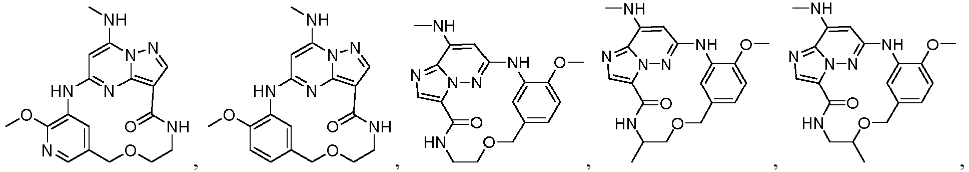

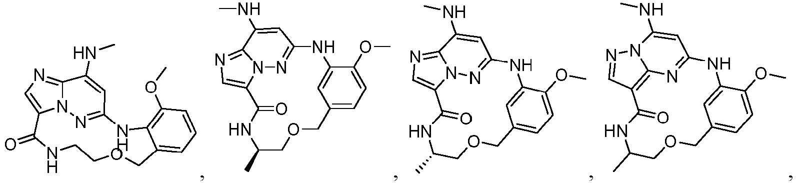

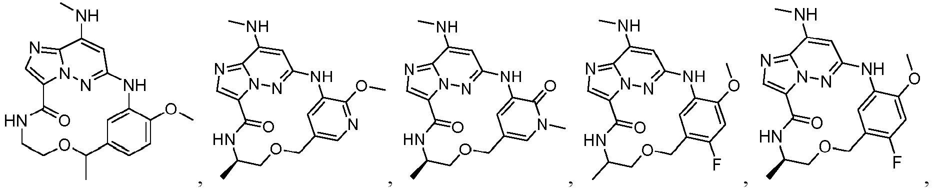

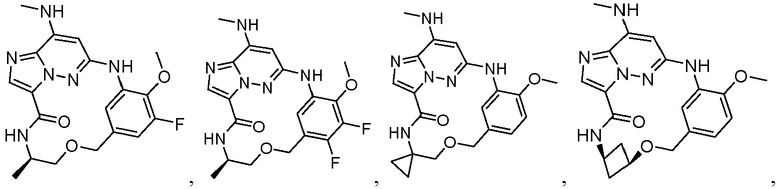

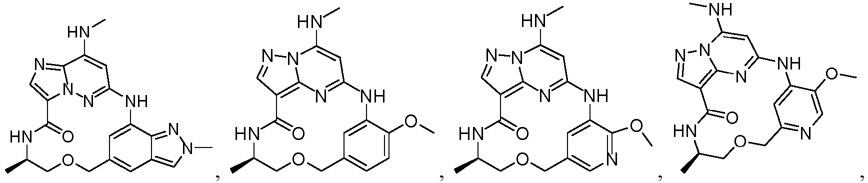

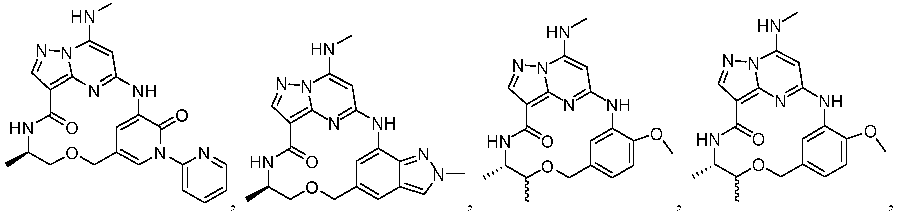

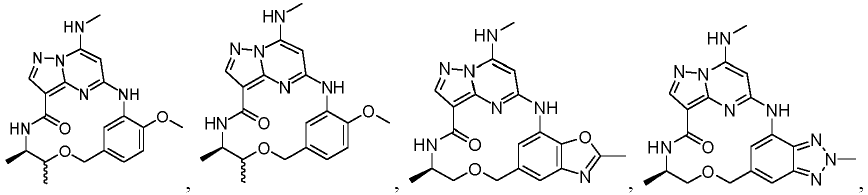

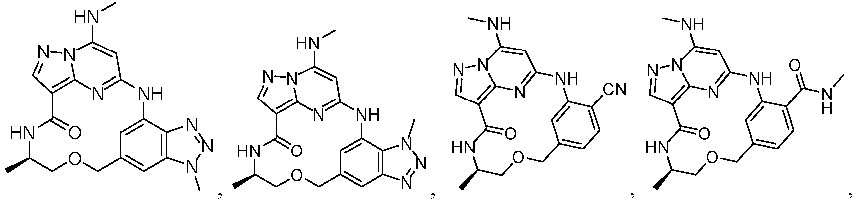

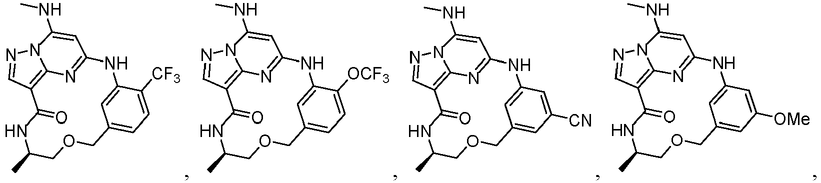

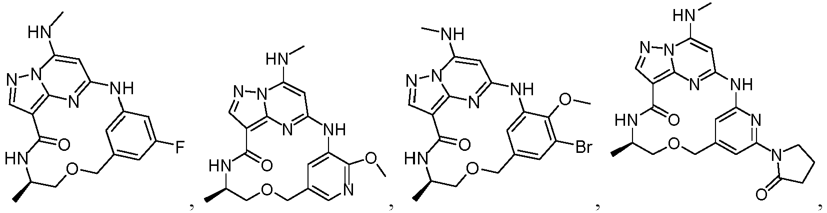

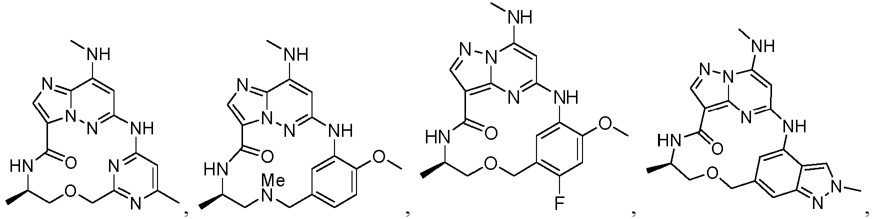

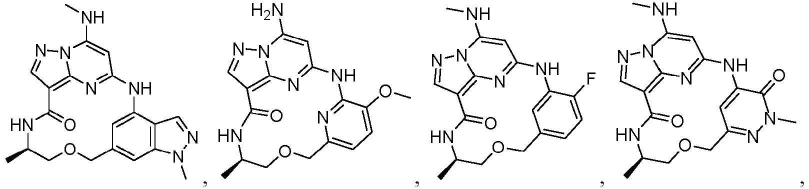

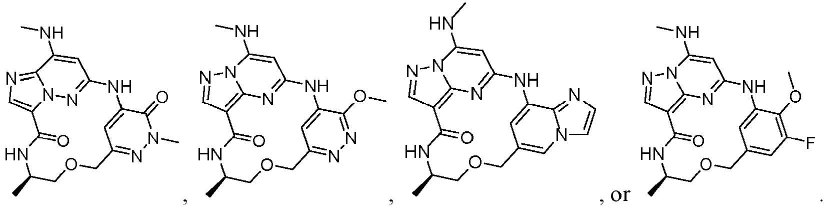

13. A compound, or a pharmaceutically acceptable salt, stereoisomer, or solvate thereof wherein the compound is: 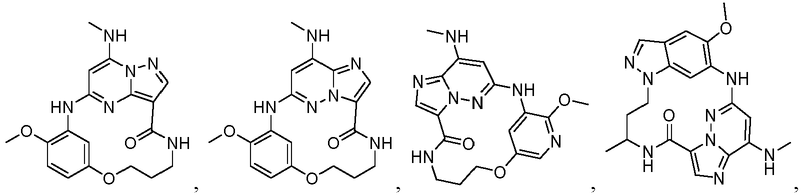 or

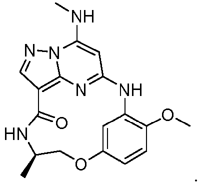

14. A pharmaceutical composition comprising a therapeutically effective amount of the compound of any one of claims 1-13, or a pharmaceutically acceptable salt, stereoisomer, or solvate thereof, and a pharmaceutically acceptable excipient.

15. A compound of any one of claims 1-13, or a pharmaceutically acceptable salt, stereoisomer, or solvate thereof, for use in a method of:
(a) inhibiting a TYK2 enzyme in a patient or biological sample comprising contacting said patient or biological sample with said compound, pharmaceutically acceptable salt, stereoisomer, or solvate; or
(b) treating a TYK2-mediated disorder comprising administering to a patient in need thereof said compound, pharmaceutically acceptable salt, stereoisomer, or solvate;
optionally wherein the TYK2-mediated disorder is:
(i) an autoimmune disorder, an inflammatory disorder, a proliferative disorder, an endocrine disorder, a neurological disorder, or a disorder associated with transplantation; or
(ii) associated with type I interferon, IL-10, IL-12, or IL-23 signaling.

## Patentansprüche

1. Verbindung der Formel (II) oder ein pharmazeutisch unbedenkliches Salz, Solvat oder Stereoisomer davon: 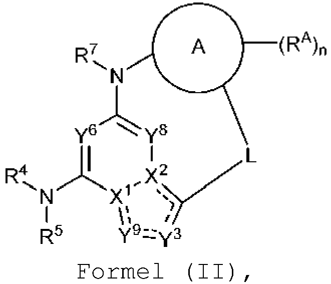 wobei:
L für 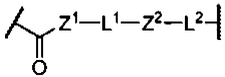 steht; wobei Z¹ und Z² unabhängig für -O-, -S- oder -NR^{Z} stehen; R^{Z} jeweils unabhängig für Wasserstoff oder C₁-C₆-Alkyl steht und L¹ und L² unabhängig für C₁-C₆-Alkylen, das gegebenenfalls durch ein oder mehrere R^{L} substituiert ist, stehen;
R^{L} jeweils unabhängig für Deuterium, Halogen, -CN, -OR^{b}, -SR^{b}, -S(=O)R^{a}, -S(=O)₂R^{a}, -NO₂, -NR^{c}R^{d}, -NHS(=O)₂R^{a}, - S(=O)₂NR^{c}R^{d}, -C(=O)R^{a}, -OC(=O)R^{a}, -C(=O)OR^{b}, -OC(=O)OR^{b}, - C(=O)NR^{c}R^{d}, -OC(=O)NR^{c}R^{d}, -NR^{b}C(=O)NR^{c}R^{d}, -NR^{b}C(=O)R^{a}, - NR^{b}C(=O)OR^{b}, C₁-C₆-Alkyl, C₁-C₆-Halogenalkyl, C₁-C₆-Deuteroalkyl, C₁-C₆-Hydroxyalkyl, C₁-C₆-Aminoalkyl, C₂-C₆-Alkenyl, C₂-C₆-Alkinyl, Cycloalkyl, Heterocycloalkyl, Aryl oder Heteroaryl steht oder zwei R^{L} an demselben Kohlenstoff zusammen ein Oxo, ein Cycloalkyl oder Heterocycloalkyl bilden oder zwei R^{L} an verschiedenen Kohlenstoffatomen zusammen ein Cycloalkyl oder Heterocycloalkyl bilden;
Ring A für Aryl, Heteroaryl, Heterocycloalkyl oder Cycloalkyl steht;
R^{A} jeweils unabhängig für -OR^{b}, Halogen, -CN, Deuterium, -SR^{b}, -S(=O)R^{a}, -S(=O)₂R^{a}, -NO₂, -NR^{c}R^{d}, -NHS(=O)₂R^{a}, - S(=O)₂NR^{c}R^{d}, -C(=O)R^{a}, -OC(=O)R^{a}, -C(=O)OR^{b}, -OC(=O)OR^{b}, - C(=O)NR^{c}R^{d}, -OC(=O)NR^{c}R^{d}, -NR^{b}C(=O)NR^{c}R^{d}, -NR^{b}C(=O)R^{a}, - NR^{b}C(=O)OR^{b}, C₁-C₆-Alkyl, C₁-C₆-Halogenalkyl, C₁-C₆-Deuteroalkyl, C₁-C₆-Hydroxyalkyl, C₁-C₆-Aminoalkyl, C₂-C₆-Alkenyl, C₂-C₆-Alkinyl, Cycloalkyl, Heterocycloalkyl, Aryl oder Heteroaryl steht; wobei jedes Alkyl, Alkenyl, Alkinyl, Cycloalkyl, Heterocycloalkyl, Aryl und Heteroaryl unabhängig gegebenenfalls durch ein oder mehrere R^{A1} substituiert ist; oder zwei R^{A} an demselben Kohlenstoff zusammen ein Oxo bilden;
R^{A1} jeweils unabhängig für Deuterium, Halogen, -CN, -OR^{b}, -SR^{b}, -S(=O)R^{a}, -S(=O)₂R^{a}, -NO₂, -NR^{c}R^{d}, -NHS(=O)₂R^{a}, - S(=O)₂NR^{c}R^{d}, -C(=O)R^{a}, -OC(=O)R^{a}, -C(=O)OR^{b}, -OC(=O)OR^{b}, - C(=O)NR^{c}R^{d}, -OC(=O)NR^{c}R^{d}, -NR^{b}C(=O)NR^{c}R^{d}, -NR^{b}C(=O)R^{a}, - NR^{b}C(=O)OR^{b}, C₁-C₆-Alkyl, C₁-C₆-Halogenalkyl, C₁-C₆-Deuteroalkyl, C₁-C₆-Hydroxyalkyl, C₁-C₆-Aminoalkyl, C₂-C₆-Alkenyl, C₂-C₆-Alkinyl, Cycloalkyl, Heterocycloalkyl, Aryl oder Heteroaryl steht oder zwei R^{A1} an demselben Kohlenstoff zusammen ein Oxo bilden;
n für 0-4 steht;
-̅ -̅ -̅ für eine Einfachbindung oder eine Doppelbindung steht;
X¹ und X² für -N- oder -C= stehen; vorausgesetzt, dass eines von X¹ oder X² für -N- steht und das andere für -C= steht;
Y⁸ für CR⁸ oder N steht;
Y⁶ für CR⁶ oder N steht;
Y³ für CR³ oder N steht;
Y⁹ für CR⁹ oder N steht;
R³, R⁶, R⁸ und R⁹ unabhängig voneinander für Wasserstoff, Deuterium, Halogen, -CN, -OR^{b}, -SR^{b}, -S(=O)R^{a}, -S(=O)₂R^{a}, -NO₂, -NR^{c}R^{d}, -NHS(=O)₂R^{a}, -S(=O)₂NR^{c}R^{d}, -C(=O)R^{a}, - OC(=O)R^{a}, -C(=O)OR^{b}, -OC(=O)OR^{b}, -C(=O)NR^{c}R^{d}, -OC(=O)NR^{c}R^{d}, -NR^{b}C(=O)NR^{c}R^{d}, -NR^{b}C(=O)R^{a}, -NR^{b}C(=O)OR^{b}, C₁-C₆-Alkyl, C₁-C₆-Halogenalkyl, C₁-C₆-Deuteroalkyl, C₁-C₆-Hydroxyalkyl, C₁-C₆-Aminoalkyl, C₂-C₆-Alkenyl oder C₂-C₆-Alkinyl stehen; R⁴ für Wasserstoff, C₁-C₆-Alkyl, C₁-C₆-Heteroalkyl, C₁-C₆-Halogenalkyl, C₁-C₆-Deuteroalkyl, C₁-C₆-Hydroxyalkyl, C₁-C₆-Aminoalkyl, C₂-C₆-Alkenyl, C₂-C₆-Alkinyl, Cycloalkyl, Heterocycloalkyl, Aryl oder Heteroaryl steht; wobei jedes Alkyl, Alkenyl, Alkinyl, Cycloalkyl, Heterocycloalkyl, Aryl und Heteroaryl gegebenenfalls durch ein oder mehrere R^{4a} substituiert ist;
R^{4a} jeweils unabhängig für Deuterium, Halogen, -CN, -OR^{b}, -NR^{c}R^{d}, -C(=O)R^{a}, -C(=O)OR^{b}, -C(=O)NR^{c}R^{d}, C₁-C₆-Alkyl, C₁-C₆-Halogenalkyl, C₁-C₆-Deuteroalkyl, C₁-C₆-Hydroxyalkyl, C₁-C₆-Aminoalkyl, Cycloalkyl, Heterocycloalkyl, Aryl oder Heteroaryl steht oder zwei R^{4a} an demselben Kohlenstoff zusammen ein Oxo bilden;
R⁵ für Wasserstoff, C₁-C₆-Alkyl, C₁-C₆-Halogenalkyl oder C₁-C₆-Deuteroalkyl steht;
R⁷ für Wasserstoff, C₁-C₆-Alkyl, C₁-C₆-Halogenalkyl oder C₁-C₆-Deuteroalkyl steht;
R^{a} jeweils unabhängig für C₁-C₆-Alkyl, C₁-C₆-Halogenalkyl, C₁-C₆-Deuteroalkyl, C₁-C₆-Hydroxyalkyl, C₁-C₆-Aminoalkyl, C₂-C₆-Alkenyl, C₂-C₆-Alkinyl, Cycloalkyl, Heterocycloalkyl, Aryl oder Heteroaryl steht; wobei jedes Alkyl, Alkenyl, Alkinyl, Cycloalkyl, Heterocycloalkyl, Aryl und Heteroaryl unabhängig gegebenenfalls durch ein oder mehrere Oxo, Deuterium, Halogen, -CN, -OH, -OMe, - NH₂, -C(=O)Me, -C(=O)OH, -C(=O)OMe, C₁-C₆-Alkyl oder C₁-C₆-Halogenalkyl substituiert ist;
R^{b} jeweils unabhängig für Wasserstoff, C₁-C₆-Alkyl, C₁-C₆-Halogenalkyl, C₁-C₆-Deuteroalkyl, C₁-C₆-Hydroxyalkyl, C₁-C₆-Aminoalkyl, C₂-C₆-Alkenyl, C₂-C₆-Alkinyl, Cycloalkyl, Heterocycloalkyl, Aryl oder Heteroaryl steht; wobei jedes Alkyl, Alkenyl, Alkinyl, Cycloalkyl, Heterocycloalkyl, Aryl und Heteroaryl unabhängig gegebenenfalls durch ein oder mehrere Oxo, Deuterium, Halogen, -CN, -OH, -OMe, - NH₂, -C(=O)Me, -C(=O)OH, -C(=O)OMe, C₁-C₆-Alkyl oder C₁-C₆-Halogenalkyl substituiert ist; und
R^{c} und R^{d} jeweils unabhängig für Wasserstoff, C₁-C₆-Alkyl, C₁-C₆-Halogenalkyl, C₁-C₆-Deuteroalkyl, C₁-C₆-Hydroxyalkyl, C₁-C₆-Aminoalkyl, C₂-C₆-Alkenyl, C₂-C₆-Alkinyl, Cycloalkyl, Heterocycloalkyl, Aryl oder Heteroaryl stehen; wobei jedes Alkyl, Alkenyl, Alkinyl, Cycloalkyl, Heterocycloalkyl, Aryl und Heteroaryl unabhängig gegebenenfalls durch ein oder mehrere Oxo, Deuterium, Halogen, -CN, -OH, -OMe, -NH₂, -C(=O)Me, - C(=O)OH, -C(=O)OMe, C₁-C₆-Alkyl oder C₁-C₆-Halogenalkyl substituiert ist;
oder R^{c} und R^{d} zusammen mit dem Stickstoffatom, an das sie gebunden sind, ein Heterocycloalkyl bilden, das gegebenenfalls durch ein oder mehrere Oxo, Deuterium, Halogen, -CN, -OH, -OMe, -NH₂, -C(=O)Me, -C(=O)OH, - C(=O)OMe, C₁-C₆-Alkyl oder C₁-C₆-Halogenalkyl substituiert ist.

2. Verbindung nach Anspruch 1 oder ein pharmazeutisch unbedenkliches Salz, Stereoisomer oder Solvat davon, wobei die Verbindung der Formel (II) die Formel (IIa): 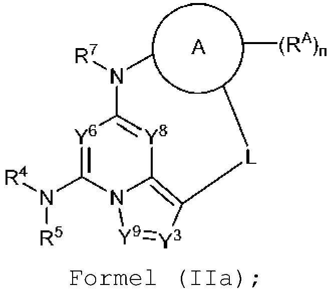 oder Formel (IIb): 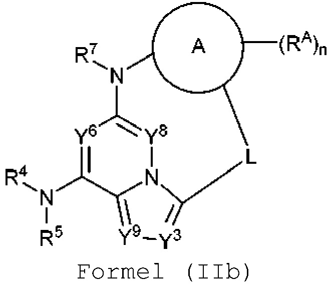 aufweist.

3. Verbindung nach einem der Ansprüche 1-2 oder ein pharmazeutisch unbedenkliches Salz, Stereoisomer oder Solvat davon, wobei:
Y⁹ für N steht.

4. Verbindung nach einem der Ansprüche 1-3 oder ein pharmazeutisch unbedenkliches Salz, Stereoisomer oder Solvat davon, wobei:
Y⁶ für CR⁶ steht; gegebenenfalls wobei R⁶ für Wasserstoff steht.

5. Verbindung nach einem der Ansprüche 1-4 oder ein pharmazeutisch unbedenkliches Salz, Stereoisomer oder Solvat davon, wobei:
(a) Y³ für CR³ Steht; gegebenenfalls wobei R³ für Wasserstoff steht; und/oder
(b) Y⁸ für N oder CR⁸ steht; gegebenenfalls wobei R⁸ für Wasserstoff steht.

6. Verbindung nach einem der Ansprüche 1-5 oder ein pharmazeutisch unbedenkliches Salz, Stereoisomer oder Solvat davon, wobei:
(a) R⁴ für Wasserstoff, C₁-C₆-Alkyl, C₁-C₆-Halogenalkyl oder C₁-C₆-Deuteroalkyl steht;
gegebenenfalls wobei R⁴ für C₁-C₆-Alkyl oder C₁-C₆-Deuteroalkyl steht; und/oder
(b) R⁵ für Wasserstoff steht; und/oder
(c) R⁷ für Wasserstoff oder C₁-C₆-Alkyl steht.

7. Verbindung nach einem der Ansprüche 1-6 oder ein pharmazeutisch unbedenkliches Salz, Stereoisomer oder Solvat davon, wobei:
Ring A für Aryl, Heteroaryl oder Heterocycloalkyl steht; gegebenenfalls wobei Ring A für Folgendes steht:
(a) Aryl; oder
(b) Heteroaryl.

8. Verbindung nach einem der Ansprüche 1-7 oder ein pharmazeutisch unbedenkliches Salz, Stereoisomer oder Solvat davon, wobei:
R^{A} jeweils unabhängig für -OR^{b}, Halogen, -CN, Deuterium, -NR^{c}R^{d}, -C(=O)R^{a}, C₁-C₆-Alkyl, C₁-C₆-Halogenalkyl oder C₁-C₆-Deuteroalkyl steht.

9. Verbindung nach einem der Ansprüche 1-8 oder ein pharmazeutisch unbedenkliches Salz, Stereoisomer oder Solvat davon, wobei:
R^{L} jeweils unabhängig für Deuterium, Halogen, -CN, -OR^{b}, -NR^{c}R^{d}, C₁-C₆-Alkyl, C₁-C₆-Halogenalkyl oder C₁-C₆-Deuteroalkyl steht oder zwei R^{L} an demselben Kohlenstoff zusammen ein Oxo bilden.

10. Verbindung nach einem der Ansprüche 1-8 oder ein pharmazeutisch unbedenkliches Salz, Stereoisomer oder Solvat davon, wobei:
R^{L} jeweils unabhängig für Deuterium, Halogen, C₁-C₆-Alkyl, C₁-C₆-Halogenalkyl oder C₁-C₆-Deuteroalkyl steht oder zwei R^{L} an demselben Kohlenstoff zusammen ein Oxo oder ein Cycloalkyl bilden oder zwei R^{L} an verschiedenen Kohlenstoffatomen zusammen ein Cycloalkyl bilden; gegebenenfalls wobei:
R^{L} jeweils unabhängig für Deuterium, Halogen, C₁-C₆-Alkyl, C₁-C₆-Halogenalkyl oder C₁-C₆-Deuteroalkyl steht oder zwei R^{L} an demselben Kohlenstoff zusammen ein Oxo bilden.

11. Verbindung nach einem der Ansprüche 1-10 oder ein pharmazeutisch unbedenkliches Salz, Stereoisomer oder Solvat davon, wobei:
L für 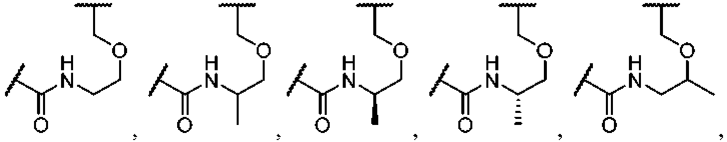 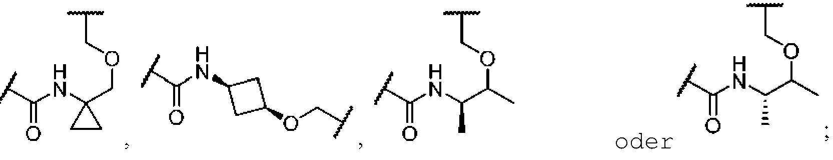 steht
gegebenenfalls wobei:
L für 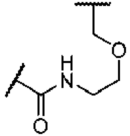 steht.

12. Verbindung nach Anspruch 1 oder ein pharmazeutisch unbedenkliches Salz, Stereoisomer oder Solvat davon, wobei es sich bei der Verbindung um Folgendes handelt:

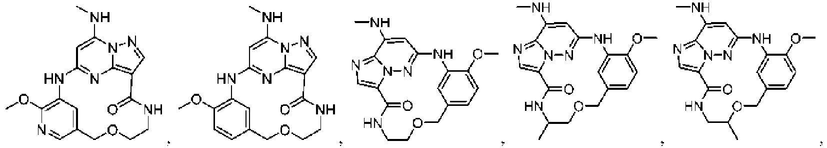

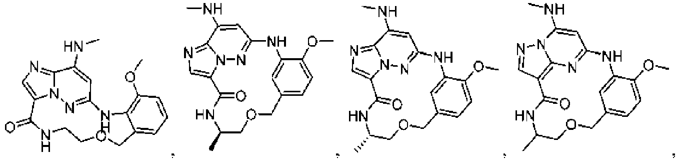

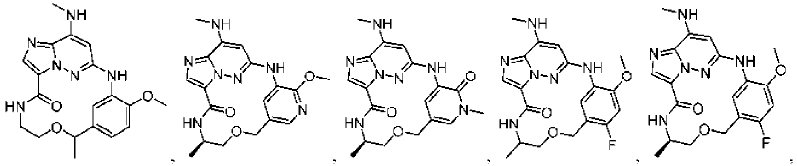

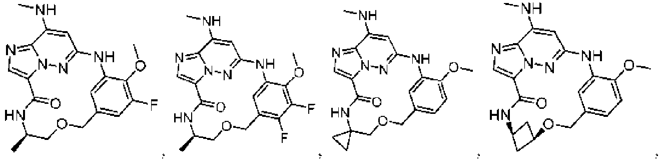

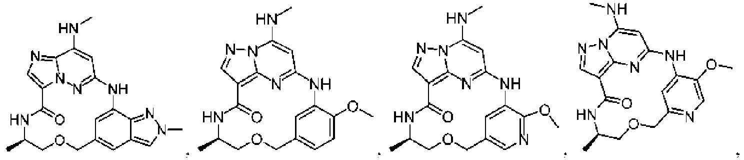

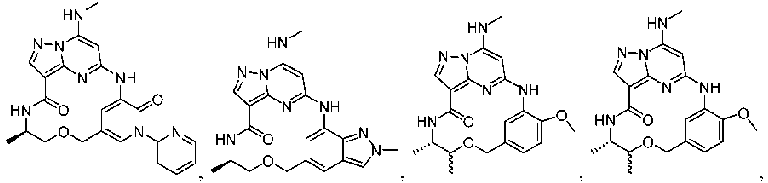

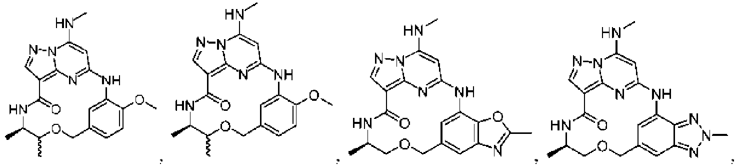

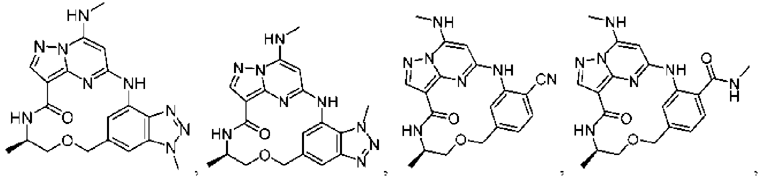

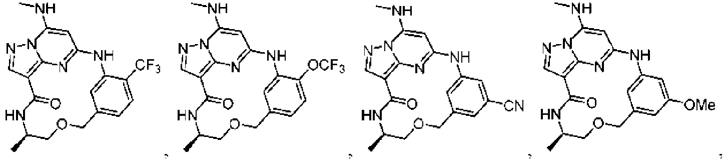

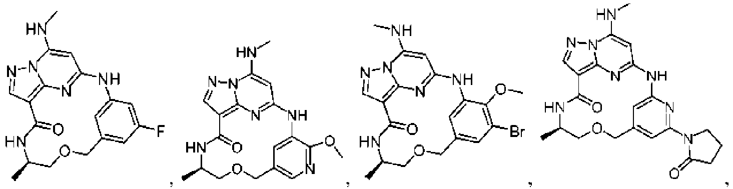

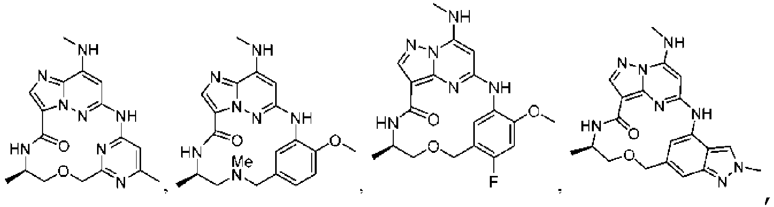

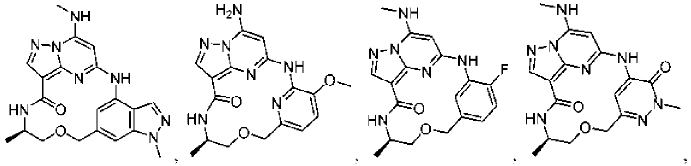

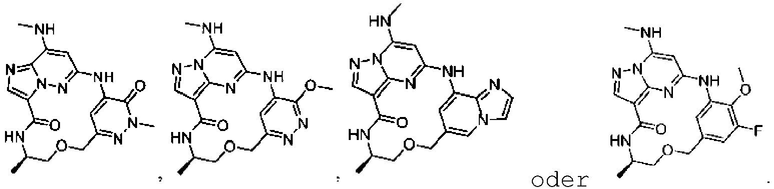

13. Verbindung oder ein pharmazeutisch unbedenkliches Salz, Stereoisomer oder Solvat davon, wobei es sich bei der Verbindung um Folgendes handelt: 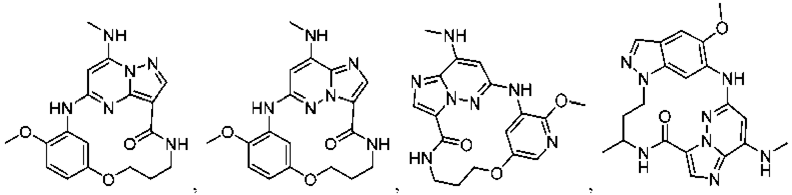 oder

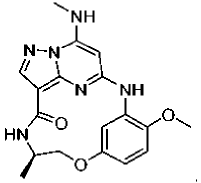

14. Pharmazeutische Zusammensetzung, umfassend eine therapeutisch wirksame Menge der Verbindung nach einem der Ansprüche 1-13 oder eines pharmazeutisch unbedenklichen Salzes, Stereoisomers oder Solvats davon und einen pharmazeutisch unbedenklichen Hilfsstoff.

15. Verbindung nach einem der Ansprüche 1-13 oder ein pharmazeutisch unbedenkliches Salz, Stereoisomer oder Solvat davon zur Verwendung bei einem Verfahren zum:
(a) Inhibieren eines TYK2-Enzyms in einem Patienten oder einer biologischen Probe, umfassend das Inkontaktbringen des Patienten oder der biologischen Probe mit der Verbindung oder dem pharmazeutisch unbedenklichen Salz, Stereoisomer oder Solvat; oder
(b) Behandeln einer durch TYK2 vermittelten Störung, umfassend das Verabreichen der Verbindung oder des pharmazeutisch unbedenklichen Salzes, Stereoisomers oder Solvats an einen Patienten, bei dem diesbezüglicher Bedarf besteht;
gegebenenfalls wobei die durch TYK2 vermittelte Störung:
(i) eine Autoimmunstörung, eine entzündliche Störung, eine Proliferationsstörung, eine endokrine Störung, eine neurologische Störung oder eine mit einer Transplantation assoziierte Störung ist; oder
(ii) mit Typ-I-Interferon-, IL-10-, IL-12- oder IL-23-Signalgebung assoziiert ist.

## Revendications

1. Composé de formule (II), ou sel, solvate ou stéréoisomère pharmaceutiquement acceptable correspondant : 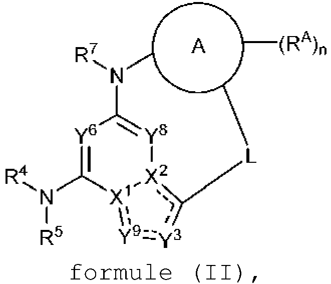
L étant 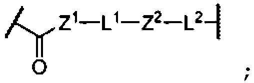 Z¹ et Z² étant indépendamment - O-, -S-, ou -NR^{Z} ; chaque R^{Z} étant indépendamment hydrogène ou C₁-C₆alkyle ; et L¹ et L² étant indépendamment C₁-C₆ alkylène éventuellement substitué par un ou plusieurs R^{L} ;
chaque R^{L} étant indépendamment deutérium, halogène, -CN, -OR^{b}, -SR^{b}, -S(=O)R^{a}, -S(=O)₂R^{a}, -NO₂, -NR^{c}R^{d}, -NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, -OC(=O)NR^{c}R^{d}, -NR^{b}C(=O)NR^{c}R^{d}, -NR^{b}C(=O)R^{a}, - NR^{b}C(=O)OR^{b}, C₁-C₆alkyle, C₁-C₆halogénoalkyle, C₁-C₆deutéroalkyle, C₁-C₆hydroxyalkyle, C₁-C₆aminoalkyle, C₂-C₆alcényle, C₂-C₆alcynyle, cycloalkyle, hétérocycloalkyle, aryle, ou hétéroaryle ; ou deux R^{L} sur le même carbone étant pris ensemble pour former un oxo, un cycloalkyle, ou hétérocycloalkyle ; ou deux R^{L} sur des carbones différents étant pris ensemble pour former un cycloalkyle ou hétérocycloalkyle ;
le cycle A étant aryle, hétéroaryle, hétérocycloalkyle, ou cycloalkyle ;
chaque R^{A} étant indépendamment -OR^{b}, halogène, -CN, deutérium, -SR^{b}, -S(=O)R^{a}, -S(=O)₂R^{a}, -NO₂, -NR^{c}R^{d}, - NHS(=O)₂R^{a}, -S(=O)₂NR^{c}R^{d}, -C(=O)R^{a}, -OC(=O)R^{a}, -C(=O)OR^{b}, - OC(=O)OR^{b}, -C(=O)NR^{c}R^{d}, -OC(=O)NR^{c}R^{d}, -NR^{b}C(=O)NR^{c}R^{d}, - NR^{b}C(=O)R^{a}, -NR^{b}C(=O)OR^{b}, C₁-C₆alkyle, C₁-C₆halogénoalkyle, C₁-C₆deutéroalkyle, C₁-C₆hydroxyalkyle, C₁-C₆aminoalkyle, C₂-C₆alcényle, C₂-C₆alcynyle, cycloalkyle, hétérocycloalkyle, aryle, ou hétéroaryle ; chaque alkyle, alcényle, alcynyle, cycloalkyle, hétérocycloalkyle, aryle, et hétéroaryle étant indépendamment éventuellement substitué par un ou plusieurs R^{A1} ; ou deux R^{A} sur le même carbone étant pris ensemble pour former un oxo ;
chaque R^{A1} étant indépendamment deutérium, halogène, -CN, -OR^{b}, -SR^{b}, -S(=O)R^{a}, -S(=O)₂R^{a}, -NO₂, -NR^{c}R^{d}, -NHS(=O)₂R^{a}, -S(=O)₂NR^{c}R^{d}, -C(=O)R^{a}, -OC(=O)R^{a}, -C(=O)OR^{b}, -OC(=O)OR^{b}, -C(=O)NR^{c}R^{d}, -OC(=O)NR^{c}R^{d}, -NR^{b}C(=O)NR^{c}R^{d}, -NR^{b}C(=O)R^{a}, - NR^{b}C(=O)OR^{b}, C₁-C₆alkyle, C₁-C₆halogénoalkyle, C₁-C₆deutéroalkyle, C₁-C₆hydroxyalkyle, C₁-C₆aminoalkyle, C₂-C₆alcényle, C₂-C₆alcynyle, cycloalkyle, hétérocycloalkyle, aryle, ou hétéroaryle ; ou deux R^{A1} sur le même carbone étant pris ensemble pour former un oxo
n étant 0-4 ;
-̅ -̅ -̅ étant une simple liaison ou une double liaison ;
X¹ et X² étant -N- ou -C= ; étant entendu que l'un parmi
X¹ ou X² est -N- et l'autre est -C= ;
Y⁸ étant CR⁸ ou N ;
Y⁶ étant CR⁶ ou N ;
Y³ étant CR³ ou N ;
Y⁹ étant CR⁹ ou N ;
R³, R⁶, R⁸, et R⁹ étant indépendamment hydrogène, deutérium, halogène, -CN, -OR^{b}, -SR^{b}, -S(=O)R^{a}, -S(=O)₂R^{a}, -NO₂, -NR^{c}R^{d}, -NHS(=O)₂R^{a}, -S(=O)₂NR^{c}R^{d}, -C(=O)R^{a}, - OC(=O)R^{a}, -C(=O)OR^{b}, -OC(=O)OR^{b}, -C(=O)NR^{c}R^{d}, -OC(=O)NR^{c}R^{d}, -NR^{b}C(=O)NR^{c}R^{d}, -NR^{b}C(=O)R^{a}, -NR^{b}C(=O)OR^{b}, C₁-C₆alkyle, C₁-C₆halogénoalkyle, C₁-C₆deutéroalkyle, C₁-C₆hydroxyalkyle, C₁-C₆aminoalkyle, C₂-C₆alcényle, ou C₂-C₆alcynyle ;
R⁴ étant hydrogène, C₁-C₆alkyle, C₁-C₆hétéroalkyle, C₁-C₆halogénoalkyle, C₁-C₆deutéroalkyle, C₁-C₆hydroxyalkyle, C₁-C₆aminoalkyle, C₂-C₆alcényle, C₂-C₆alcynyle, cycloalkyle, hétérocycloalkyle, aryle, ou hétéroaryle ; chaque alkyle, alcényle, alcynyle, cycloalkyle, hétérocycloalkyle, aryle, et hétéroaryle étant éventuellement substitué par un ou plusieurs R^{4a} ; chaque R^{4a} étant indépendamment deutérium, halogène, -CN, -OR^{b}, -NR^{c}R^{d}, -C(=O)R^{a}, -C(=O)OR^{b}, -C(=O)NR^{c}R^{d}, C₁-C₆alkyle, C₁-C₆halogénoalkyle, C₁-C₆deutéroalkyle, C₁-C₆hydroxyalkyle, C₁-C₆aminoalkyle, cycloalkyle, hétérocycloalkyle, aryle, ou hétéroaryle ; ou deux R^{4a} sur le même carbone étant pris ensemble pour former un oxo ;
R⁵ étant hydrogène, C₁-C₆alkyle, C₁-C₆halogénoalkyle, ou C₁-C₆deutéroalkyle ;
R⁷ étant hydrogène, C₁-C₆alkyle, C₁-C₆halogénoalkyle, ou C₁-C₆deutéroalkyle ; chaque R^{a} étant indépendamment C₁-C₆alkyle, C₁-C₆halogénoalkyle, C₁-C₆deutéroalkyle, C₁-C₆hydroxyalkyle, C₁-C₆aminoalkyle, C₂-C₆alcényle, C₂-C₆alcynyle, cycloalkyle, hétérocycloalkyle, aryle, ou hétéroaryle ; chaque alkyle, alcényle, alcynyle, cycloalkyle, hétérocycloalkyle, aryle, et hétéroaryle étant indépendamment éventuellement substitué par un ou plusieurs oxo, deutérium, halogène, -CN, -OH, -OMe, -NH₂, -C(=O)Me, -C(=O)OH, -C(=O)OMe, C₁-C₆alkyle, ou C₁-C₆halogénoalkyle ;
chaque R^{b} étant indépendamment hydrogène, C₁-C₆alkyle, C₁-C₆halogénoalkyle, C₁-C₆deutéroalkyle, C₁-C₆hydroxyalkyle, C₁-C₆aminoalkyle, C₂-C₆alcényle, C₂-C₆alcynyle, cycloalkyle, hétérocycloalkyle, aryle, ou hétéroaryle ; chaque alkyle, alcényle, alcynyle, cycloalkyle, hétérocycloalkyle, aryle, et hétéroaryle étant indépendamment éventuellement substitué par un ou plusieurs oxo, deutérium, halogène, -CN, -OH, -OMe, -NH₂, -C(=O)Me, -C (=O) OH, -C(=O)OMe, C₁-C₆alkyle, ou C₁-C₆halogénoalkyle ; et
chaque R^{c} et R^{d} étant indépendamment hydrogène, C₁-C₆alkyle, C₁-C₆halogénoalkyle, C₁-C₆deutéroalkyle, C₁-C₆hydroxyalkyle, C₁-C₆aminoalkyle, C₂-C₆alcényle, C₂-C₆alcynyle, cycloalkyle, hétérocycloalkyle, aryle, ou hétéroaryle ; chaque alkyle, alcényle, alcynyle, cycloalkyle, hétérocycloalkyle, aryle, et hétéroaryle étant indépendamment éventuellement substitué par un ou plusieurs oxo, deutérium, halogène, -CN, -OH, -OMe, -NH₂, -C(=O)Me, -C(=O)OH, -C(=O)OMe, C₁-C₆alkyle, ou C₁-C₆halogénoalkyle ;
ou R^{c} et R^{d} étant pris ensemble avec l'atome d'azote auquel ils sont fixés pour former un hétérocycloalkyle éventuellement substitué par un ou plusieurs oxo, deutérium, halogène, -CN, -OH, -OMe, -NH₂, -C(=O)Me, - C(=O)OH, -C(=O)OMe, C₁-C₆alkyle, ou C₁-C₆halogénoalkyle.

2. Composé selon la revendication 1, ou sel, stéréoisomère ou solvate pharmaceutiquement acceptable correspondant, le composé de formule (II) étant de formule (IIa) : 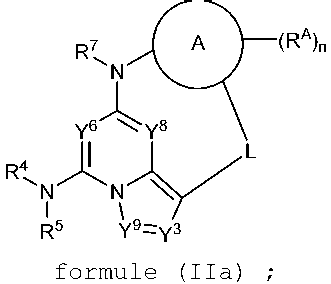 ou formule (IIb):

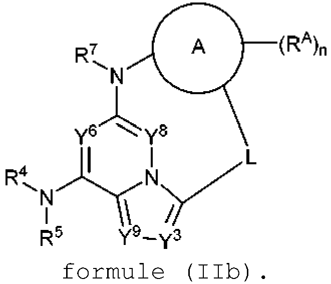

3. Composé selon l'une quelconque des revendications 1-2, ou sel, stéréoisomère ou solvate pharmaceutiquement acceptable correspondant,
Y⁹ étant N.

4. Composé selon l'une quelconque des revendications 1-3, ou sel, stéréoisomère ou solvate pharmaceutiquement acceptable correspondant,
Y⁶ étant CR⁶ ; éventuellement, R⁶ étant hydrogène.

5. Composé selon l'une quelconque des revendications 1-4, ou sel, stéréoisomère ou solvate pharmaceutiquement acceptable correspondant,
(a) Y³ étant CR³ ; éventuellement, R³ étant hydrogène ; et/ou
(b) Y⁸ étant N ou CR⁸ ; éventuellement, R⁸ étant hydrogène.

6. Composé selon l'une quelconque des revendications 1-5, ou sel, stéréoisomère ou solvate pharmaceutiquement acceptable correspondant,
(a) R⁴ étant hydrogène, C₁-C₆alkyle, C₁-C₆halogénoalkyle, ou C₁-C₆deutéroalkyle ;
éventuellement, R⁴ étant C₁-C₆alkyle ou C₁-C₆deutéroalkyle ; et/ou
(b) R⁵ étant hydrogène ; et/ou
(c) R⁷ étant hydrogène ou C₁-C₆alkyle.

7. Composé selon l'une quelconque des revendications 1-6, ou sel, stéréoisomère ou solvate pharmaceutiquement acceptable correspondant,
le cycle A étant aryle, hétéroaryle, ou hétérocycloalkyle ;
éventuellement, le cycle A étant :
(a) aryle ; ou
(b) hétéroaryle.

8. Composé selon l'une quelconque des revendications 1-7, ou sel, stéréoisomère ou solvate pharmaceutiquement acceptable correspondant,
chaque R^{A} étant indépendamment -OR^{b}, halogène, -CN, deutérium, -NR^{c}R^{d}, -C(=O)R^{a}, C₁-C₆alkyle, C₁-C₆halogénoalkyle, ou C₁-C₆deutéroalkyle.

9. Composé selon l'une quelconque des revendications 1-8, ou sel, stéréoisomère ou solvate pharmaceutiquement acceptable correspondant,
chaque R^{L} étant indépendamment deutérium, halogène, -CN, -OR^{b}, -NR^{c}R^{d}, C₁-C₆alkyle, C₁-C₆halogénoalkyle, ou C₁-C₆deutéroalkyle ; ou deux R^{L} sur le même carbone étant pris ensemble pour former un oxo.

10. Composé selon l'une quelconque des revendications 1-8, ou sel, stéréoisomère ou solvate pharmaceutiquement acceptable correspondant,
chaque R^{L} étant indépendamment deutérium, halogène, C₁-C₆alkyle, C₁-C₆halogénoalkyle, ou C₁-C₆deutéroalkyle ; ou deux R^{L} sur le même carbone étant pris ensemble pour former un oxo ou un cycloalkyle ; ou deux R^{L} sur des carbones différents étant pris ensemble pour former un cycloalkyle ;
éventuellement,
chaque R^{L} étant indépendamment deutérium, halogène, C₁-C₆alkyle, C₁-C₆halogénoalkyle, ou C₁-C₆deutéroalkyle ; ou deux R^{L} sur le même carbone étant pris ensemble pour former un oxo.

11. Composé selon l'une quelconque des revendications 1-10, ou sel, stéréoisomère ou solvate pharmaceutiquement acceptable correspondant,
L étant 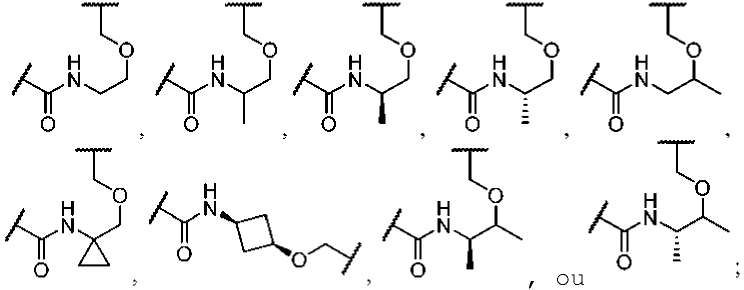
éventuellement,
L étant

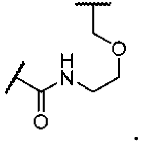

12. Composé de la revendication 1, ou sel, stéréoisomère, ou solvate pharmaceutiquement acceptable de celui-ci, le composé étant :

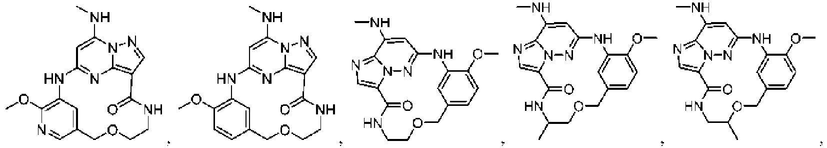

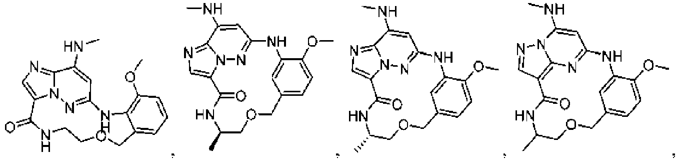

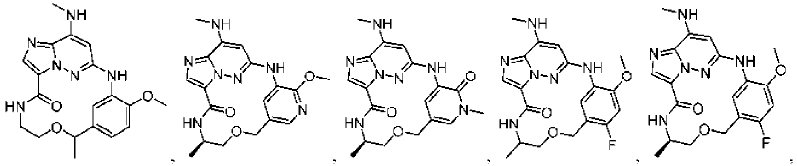

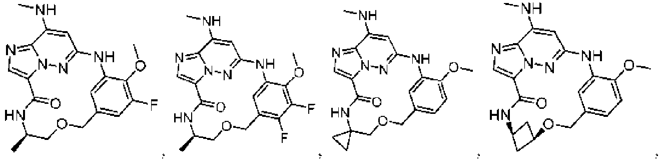

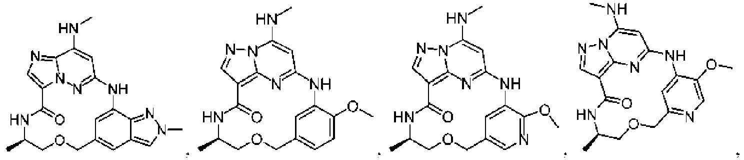

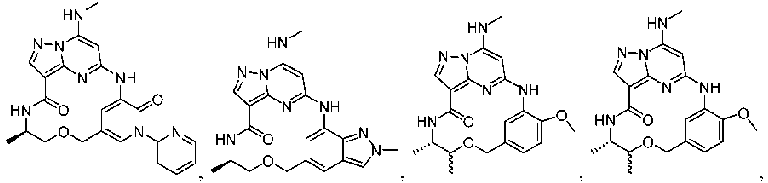

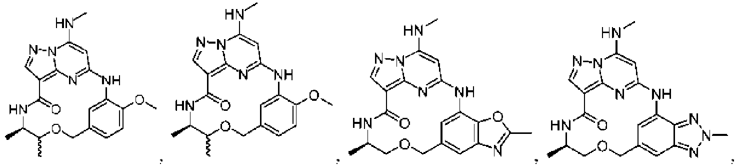

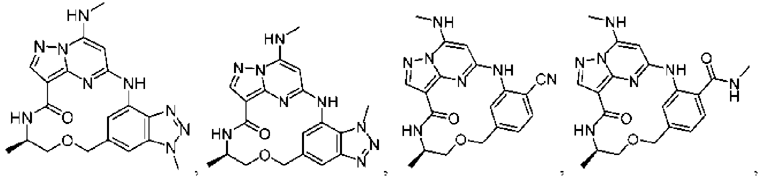

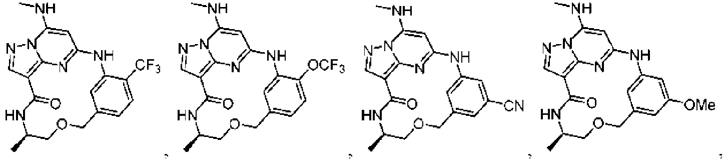

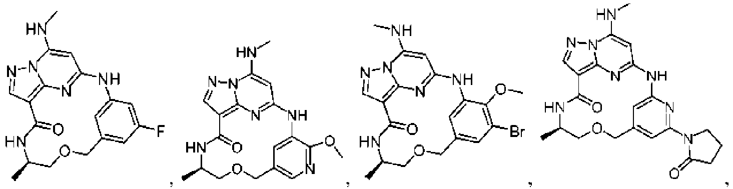

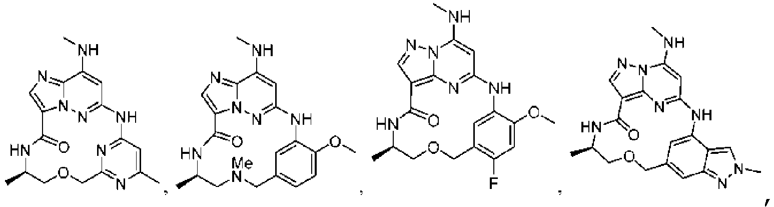

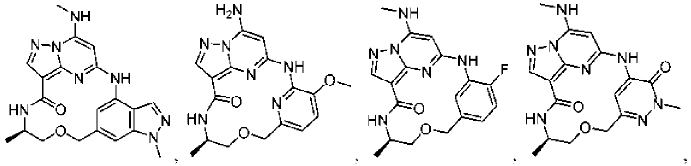

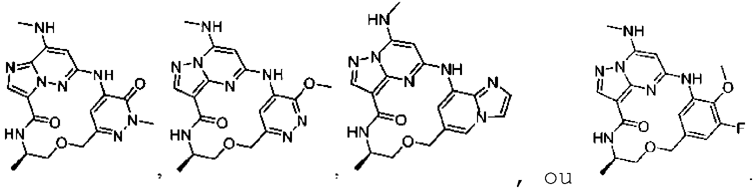

13. Composé, ou sel, stéréoisomère, ou solvate pharmaceutiquement acceptable de celui-ci, le composé étant : 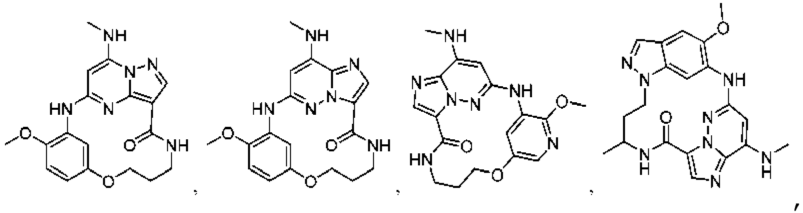 ou

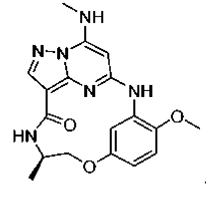

14. Composition pharmaceutique comprenant une quantité thérapeutiquement efficace du composé selon l'une quelconque des revendications 1 à 13, ou d'un sel, stéréoisomère ou solvate pharmaceutiquement acceptable de celui-ci, et un excipient pharmaceutiquement acceptable.

15. Composé selon l'une quelconque des revendications 1 à 13, ou sel, stéréoisomère ou solvate pharmaceutiquement acceptable de celui-ci, pour une utilisation dans un procédé de :
(a) inhibition d'une enzyme TYK2 chez un patient ou dans un échantillon biologique comprenant la mise en contact dudit patient ou échantillon biologique avec ledit composé, sel, stéréoisomère ou solvate pharmaceutiquement acceptable ; ou
(b) traitement d'un trouble médié par TYK2 comprenant l'administration à un patient en ayant besoin dudit composé, sel, stéréoisomère ou solvate pharmaceutiquement acceptable ;
éventuellement, dans lequel le trouble associé à TYK2 est
(i) un trouble auto-immun, un trouble inflammatoire, un trouble prolifératif, un trouble endocrinien, un trouble neurologique, ou un trouble associé à une transplantation ; ou
(ii) associé à la signalisation de l'interféron de type I, IL-10, IL-12 ou IL-23.
